# EUROPEAN PATENT SPECIFICATION

(11) **EP 3 898 646 B1**
(45) Date of publication and mention of the grant of the patent: **18.02.2026**
(21) Application number: 19850836.8
(22) Date of filing: 20.12.2019
(51) Int. Cl.: C07J 43/00, A61K 31/58, A61P 25/00, A61P 25/24, C07J 3/00, C07J 41/00, C07J 7/00, C07J 21/00, C07J 1/00, C07J 13/00, C07J 51/00

(54) **3.ALPHA.-HYDROXY-17.BETA.-AMIDE NEUROACTIVE STEROIDS AND COMPOSITIONS THEREOF**
NEUROAKTIVE 3.ALPHA.-HYDROXY-17.BETA.-AMID-STEROIDE UND ZUSAMMENSETZUNGEN DARAUS
STÉROÏDES NEUROACTIFS DE 3.ALPHA.-HYDROXY-17.BÊTA.-AMIDE ET COMPOSITIONS ASSOCIÉES

(30) Priority: 21.12.2018 US 201862784229 P; 21.12.2018 US 201862784222 P
(43) Date of publication of application: 27.10.2021
(73) Proprietor: Sage Therapeutics, LLC, Rockville, MD 20850 (US)
(72) Inventor: BLANCO-PILLADO, Maria, Jesus, Arlington, MA 02474 (US); SALITURO, Francesco, G., Marlborough, MA 01752 (US)
(74) Representative: Coles, Andrea Birgit
(86) International application number: PCT/US2019/067953
(87) International publication number: WO 2020/132504

(56) References cited:
- WO-A1-2018/013613

## Description

### Cross-Reference To Related Applications

This application claims priority to U.S.S.N. 62/784,222 filed December 21, 2018, and U.S.S.N. 62/784,229 filed December 21, 2018.

### Background of the Invention

Brain excitability is defined as the level of arousal of an animal, a continuum that ranges from coma to convulsions, and is regulated by various neurotransmitters. In general, neurotransmitters are responsible for regulating the conductance of ions across neuronal membranes. At rest, the neuronal membrane possesses a potential (or membrane voltage) of approximately -70 mV, the cell interior being negative with respect to the cell exterior. The potential (voltage) is the result of ion (K⁺, Na⁺, Cl⁻, organic anions) balance across the neuronal semipermeable membrane. Neurotransmitters are stored in presynaptic vesicles and are released under the influence of neuronal action potentials. When released into the synaptic cleft, an excitatory chemical transmitter such as acetylcholine will cause membrane depolarization (change of potential from -70 mV to -50 mV). This effect is mediated by postsynaptic nicotinic receptors which are stimulated by acetylcholine to increase membrane permeability to Na⁺ ions. The reduced membrane potential stimulates neuronal excitability in the form of a postsynaptic action potential.

In the case of the GABA receptor complex (GRC), the effect on brain excitability is mediated by γ-aminobutyric acid (GABA), a neurotransmitter. GABA has a profound influence on overall brain excitability because up to 40% of the neurons in the brain utilize GABA as a neurotransmitter. GABA regulates the excitability of individual neurons by regulating the conductance of chloride ions across the neuronal membrane. GABA interacts with its recognition site on the GRC to facilitate the flow of chloride ions down an electrochemical gradient of the GRC into the cell. An intracellular increase in the levels of this anion causes hyperpolarization of the transmembrane potential, rendering the neuron less susceptible to excitatory inputs, *i.e.,* reduced neuron excitability. In other words, the higher the chloride ion concentration in the neuron, the lower the brain excitability and level of arousal.

It is well-documented that the GRC is responsible for the mediation of anxiety, seizure activity, and sedation. Thus, GABA and drugs that act like GABA or facilitate the effects of GABA (e.g., the therapeutically useful barbiturates and benzodiazepines (BZs), such as Valium^{®}) produce their therapeutically useful effects by interacting with specific regulatory sites on the GRC. Accumulated evidence has now indicated that in addition to the benzodiazepine and barbiturate binding site, the GRC contains a distinct site for neuroactive steroids. See, e.g., Lan, N. C. et al., Neurochem. Res. (1991) 16:347-356.

Neuroactive steroids can occur endogenously. The most potent endogenous neuroactive steroids are 3α-hydroxy-5-reduced pregnan-20-one and 3α-21-dihydroxy-5-reduced pregnan-20-one, metabolites of hormonal steroids progesterone and deoxycorticosterone, respectively. The ability of these steroid metabolites to alter brain excitability was recognized in 1986 (Majewska, M. D. et al., Science 232:1004-1007 (1986); Harrison, N. L. et al., J Pharmacol. Exp. Ther. 241:346-353 (1987)). Furthermore, WO-A-2018/013613 discloses N-substituted, 3.alpha.-hydroxy-androstan-17.beta.-amide compounds active as GABA receptor modulators and useful in the treatment of CNS-related disorders.

New and improved compounds are needed that act as modulating agents for brain excitability, as well as agents for the prevention and treatment of CNS-related diseases. The compounds, compositions, and methods described herein are directed toward this end.

### Summary of the Invention

Provided herein are compounds designed, for example, to act as GABA modulators. In some embodiments, such compounds are envisioned to be useful as therapeutic agents for treating a CNS-_{related} disorder. The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

In some embodiments, provided herein is a compound of Formula (I): 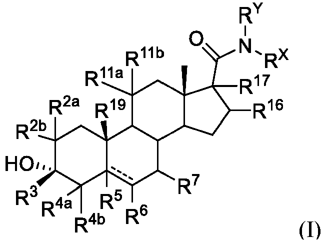 or a pharmaceutically acceptable salt thereof.

In some embodiments, provided herein is a compound of Formula (I-X): 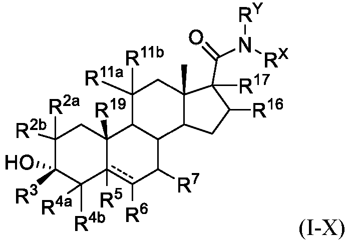 or a pharmaceutically acceptable salt thereof.

In some embodiments, provided herein is a compound of Formula (I): 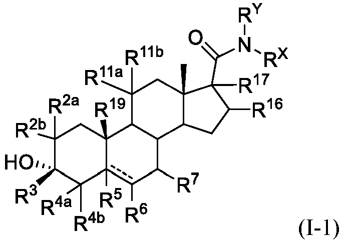 or a pharmaceutically acceptable salt thereof.

In some embodiments, the compound of Formula (I) is a compound of Formula (I-a): 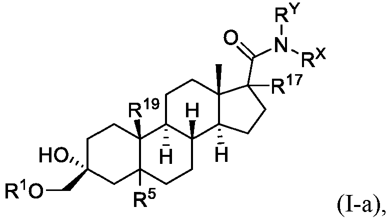 or a pharmaceutically acceptable salt thereof,

In some embodiments, the compound of Formula (I) is a compound of Formula (I-b1) or Formula (I-b2): 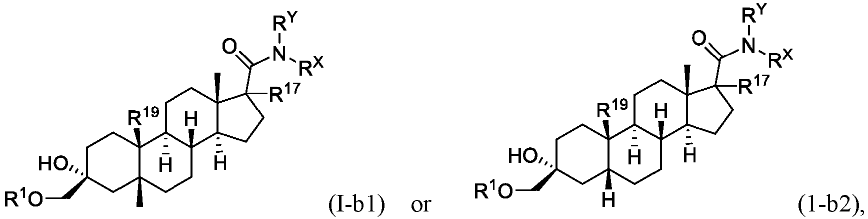 or a pharmaceutically acceptable salt thereof.

In some embodiments, the compound of Formula (I) is a compound of Formula (I-c1) or Formula (I-c2): 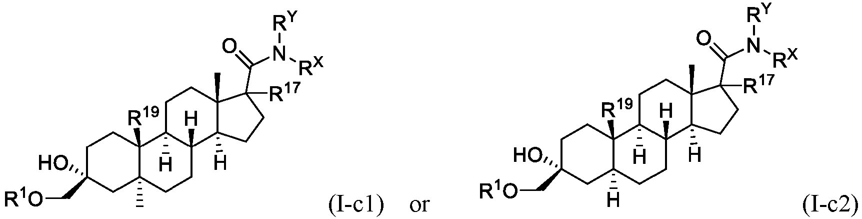 or a pharmaceutically acceptable salt thereof.

In some embodiments, the compound of Formula (I) is a compound of Formula (I-d1) or Formula (I-d2): 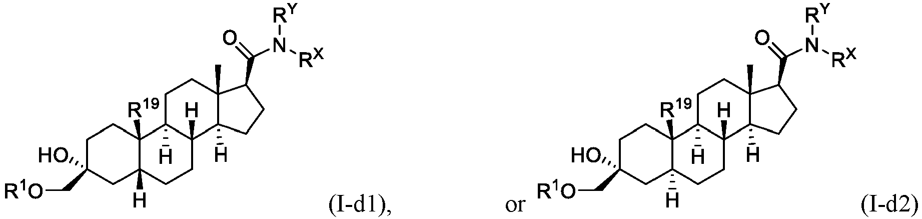 or a pharmaceutically acceptable salt thereof.

In some embodiments, the compound of Formula (I) is a compound of Formula (I-d1): 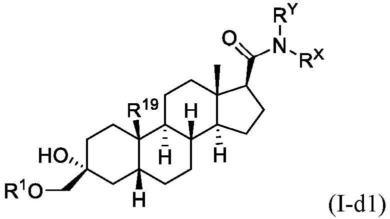 or a pharmaceutically acceptable salt thereof.

In some embodiments, the compound of Formula (I) is a compound of Formula (I-d2): 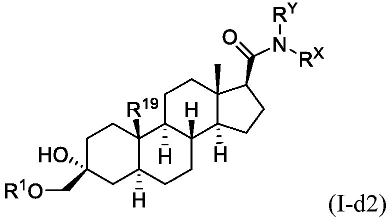 or a pharmaceutically acceptable salt thereof.

In some embodiments, the compound of Formula (I), is a compound of Formula (I-e): 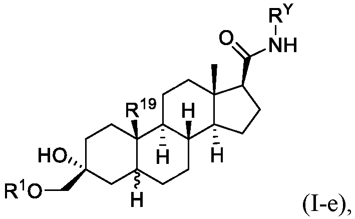 or a pharmaceutically acceptable salt thereof, wherein R^{Y} is heteroaryl, and R¹ is -CH₂CH₃ or -CH₃.

In some embodiments, the compound of Formula (I) is a compound of Formula (If): 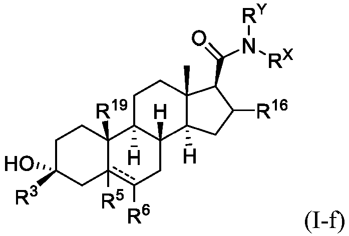 or a pharmaceutically acceptable salt thereof.

In some embodiments, the compound of Formula (I) is a compound of Formula (I-g) 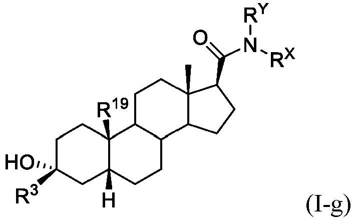 or a pharmaceutically acceptable salt thereof.

In some embodiments, a pharmaceutical composition comprises a compound described herein or pharmaceutically acceptable salt thereof, and a pharmaceutically acceptable excipient.

In some embodiments, a method of treating a CNS-related disorder in a subject in need thereof, comprises administering to the subject an effective amount of a compound described herein or a pharmaceutically acceptable salt thereof. In some embodiments, the CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In some embodiments, the CNS-related disorder is depression. In some embodiments, the CNS-related disorder is postpartum depression. In some embodiments, the CNS-related disorder is major depressive disorder. In some embodiments, the major depressive disorder is moderate major depressive disorder. In some embodiments, the major depressive disorder is severe major depressive disorder.

In some embodiments, the compound is selected from the group consisting of the compounds identified in **Table 1** herein.

In one aspect, provided herein is a pharmaceutically acceptable salt of a compound described herein (*e*.*g.,* a compound of Formula (I)).

In one aspect, provided herein is a pharmaceutical composition comprising a compound described herein (*e.g.,* a compound of Formula (I)) or a pharmaceutically acceptable salt thereof, and a pharmaceutically acceptable excipient. In certain embodiments, the compound of the present invention is provided in an effective amount in the pharmaceutical composition. In certain embodiments, the compound of the present invention is provided in a therapeutically effective amount.

Compounds of the present invention as described herein, act, in certain embodiments, as GABA modulators, *e.g*., effecting the GABA_{A} receptor in either a positive or negative manner. As modulators of the excitability of the central nervous system (CNS), as mediated by their ability to modulate GABA_{A} receptor, such compounds are expected to have CNS-activity.

Thus, in another aspect, provided are methods of treating a CNS-related disorder in a subject in need thereof, comprising administering to the subject an effective amount of a compound of the present invention. In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder. In certain embodiments, the compound is administered orally, subcutaneously, intravenously, or intramuscularly. In certain embodiments, the compound is administered orally. In certain embodiments, the compound is administered chronically. In certain embodiments, the compound is administered continuously, e.g., by continuous intravenous infusion.

In a preferred embodiment, compounds described herein selectively modulate certain subunit compositions of the GABA_{A} receptor, e.g., α4β3δ subunit composition.

### Detailed Description of Certain Embodiments of the Invention

As generally described herein, the present invention provides neuroactive steroids designed, for example, to act as GABA_{A} receptor modulators. In a preferred embodiment, compounds described herein selectively modulate certain subunit compositions of the GABA_{A} receptor, e.g., α4β3δ subunit composition. As used herein, "selectively modulates" means a higher or greater modulation of certain subunit compositions when compared to other subunit compositions of the GABA_{A} receptor. In certain embodiments, such compounds are envisioned to be useful as therapeutic agents for treating a CNS-related disorder (e.g., a disorder as described herein, for example depression, such as post-partum depression or major depressive disorder).

### Definitions

### Chemical definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

Isomers, e.g., stereoisomers, can be isolated from mixtures by methods known to those skilled in the art, including chiral high pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, *Stereochemistry of Carbon Compounds* (McGraw-Hill, NY, 1962); and Wilen, Tables of Resolving Agents and Optical Resolutions p. 268 (E.L. Eliel, Ed., Univ. of Notre Dame Press, Notre Dame, IN 1972).

As used herein a pure enantiomeric compound is substantially free from other enantiomers or stereoisomers of the compound (*i.e.,* in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of the enantiomer. In certain embodiments, the weights are based upon total weight of all enantiomers or stereoisomers of the compound.

In the compositions provided herein, an enantiomerically pure compound can be present with other active or inactive ingredients. For example, a pharmaceutical composition comprising enantiomerically pure R-position/center/ carbon compound can comprise, for example, about 90% excipient and about 10% enantiomerically pure R- compound. In certain embodiments, the enantiomerically pure R-compound in such compositions can, for example, comprise, at least about 95% by weight R-compound and at most about 5% by weight S-compound, by total weight of the compound. For example, a pharmaceutical composition comprising enantiomerically pure S-compound can comprise, for example, about 90% excipient and about 10% enantiomerically pure S-compound. In certain embodiments, the enantiomerically pure S-compound in such compositions can, for example, comprise, at least about 95% by weight S-compound and at most about 5% by weight R-compound, by total weight of the compound. In certain embodiments, the active ingredient can be formulated with little or no excipient or carrier.

The term "diastereomierically pure" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 98.5% by weight, more than 99% by weight, more than 99.2% by weight, more than 99.5% by weight, more than 99.6% by weight, more than 99.7% by weight, more than 99.8% by weight or more than 99.9% by weight, of a single diastereomer. Methods for determining diastereomeric and enantiomeric purity are well-known in the art. Diastereomeric purity can be determined by any analytical method capable of quantitatively distinguishing between a compound and its diastereomers, such as high performance liquid chromatography (HPLC).

"Stereoisomers": It is also to be understood that compounds that have the same molecular formula but differ in the nature or sequence of bonding of their atoms or the arrangement of their atoms in space are termed "isomers." Isomers that differ in the arrangement of their atoms in space are termed "stereoisomers." Stereoisomers that are not mirror images of one another are termed "diastereomers" and those that are non-superimposable mirror images of each other are termed "enantiomers." When a compound has an asymmetric center, for example, it is bonded to four different groups, a pair of enantiomers is possible. An enantiomer can be characterized by the absolute configuration of its asymmetric center and is described by the R- and S-sequencing rules of Cahn and Prelog, or by the manner in which the molecule rotates the plane of polarized light and designated as dextrorotatory or levorotatory (*i.e.,* as (+) or (-)-isomers respectively). A chiral compound can exist as either individual enantiomer or as a mixture thereof. A mixture containing equal proportions of the enantiomers is called a "racemic mixture".

The articles "a" and "an" may be used herein to refer to one or to more than one (*i.e.* at least one) of the grammatical objects of the article. By way of example "an analogue" means one analogue or more than one analogue.

When a range of values is listed, it is intended to encompass each value and sub-range within the range. For example "C₁₋₆ alkyl" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆ alkyl.

The following terms are intended to have the meanings presented therewith below and are useful in understanding the description and intended scope of the present invention.

"Alkyl" refers to a radical of a straight-chain or branched saturated hydrocarbon group having from 1 to 20 carbon atoms ("C₁₋₂₀ alkyl"). In some embodiments, an alkyl group has 1 to 12 carbon atoms ("C₁₋₁₂ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl", also referred to herein as "lower alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently optionally substituted, *i.e.,* unsubstituted (an "unsubstituted alkyl") or substituted (a "substituted alkyl") with one or more substituents; e.g., for instance from 1 to 5 substituents, 1 to 3 substituents, or 1 substituent. In certain embodiments, the alkyl group is unsubstituted C₁₋₁₀ alkyl (*e.g.,* -CH₃). In certain embodiments, the alkyl group is substituted C₁₋₁₀ alkyl. Common alkyl abbreviations include Me (-CH₃), Et (-CH₂CH₃), iPr (-CH(CH₃)₂), nPr (-CH₂CH₂CH₃), n-Bu (-CH₂CH₂CH₂CH₃), or i-Bu (-CH₂CH(CH₃)₂).

"Alkylene" refers to an alkyl group wherein two hydrogens are removed to provide a divalent radical, and which may be substituted or unsubstituted. Unsubstituted alkylene groups include, but are not limited to, methylene (-CH₂-), ethylene (-CH₂CH₂-), propylene (-CH₂CH₂CH₂-), butylene (-CH₂CH₂CH₂CH₂-), pentylene (-CH₂CH₂CH₂CH₂CH₂-), hexylene (-CH₂CH₂CH₂CH₂CH₂CH₂-), and the like. Exemplary substituted alkylene groups, e.g., substituted with one or more alkyl (methyl) groups, include but are not limited to, substituted methylene (-CH(CH₃)-, (-C(CH₃)₂-), substituted ethylene (-CH(CH₃)CH₂-,-CH₂CH(CH₃)-, -C(CH₃)₂CH₂-,-CH₂C(CH₃)₂-), substituted propylene (-CH(CH₃)CH₂CH₂-, - CH₂CH(CH₃)CH₂-, -CH₂CH₂CH(CH₃)-, -C(CH₃)₂CH₂CH₂-, -CH₂C(CH₃)₂CH₂-, - CH₂CH₂C(CH₃)₂-), and the like. When a range or number of carbons is provided for a particular alkylene group, it is understood that the range or number refers to the range or number of carbons in the linear carbon divalent chain. Alkylene groups may be substituted or unsubstituted with one or more substituents as described herein.

"Alkenyl" refers to a radical of a straight-chain or branched hydrocarbon group having from 2 to 20 carbon atoms, one or more carbon-carbon double bonds *(e.g.,* 1, 2, 3, or 4 carbon-carbon double bonds), and optionally one or more carbon-carbon triple bonds *(e.g.,* 1, 2, 3, or 4 carbon-carbon triple bonds) ("C₂₋₂₀ alkenyl"). In certain embodiments, alkenyl does not contain any triple bonds. In some embodiments, an alkenyl group has 2 to 10 carbon atoms ("C₂₋₁₀ alkenyl"). In some embodiments, an alkenyl group has 2 to 9 carbon atoms ("C₂₋₉ alkenyl"). In some embodiments, an alkenyl group has 2 to 8 carbon atoms ("C₂₋₈ alkenyl"). In some embodiments, an alkenyl group has 2 to 7 carbon atoms ("C₂₋₇ alkenyl"). In some embodiments, an alkenyl group has 2 to 6 carbon atoms ("C₂₋₆ alkenyl"). In some embodiments, an alkenyl group has 2 to 5 carbon atoms ("C₂₋₅ alkenyl"). In some embodiments, an alkenyl group has 2 to 4 carbon atoms ("C₂₋₄ alkenyl"). In some embodiments, an alkenyl group has 2 to 3 carbon atoms ("C₂₋₃ alkenyl"). In some embodiments, an alkenyl group has 2 carbon atoms ("C₂ alkenyl"). The one or more carbon-carbon double bonds can be internal (such as in 2-butenyl) or terminal (such as in 1-butenyl). Examples of C₂₋₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂₋₆ alkenyl groups include the aforementioned C₂₋₄ alkenyl groups as well as pentenyl (C₅), pentadienyl (C₅), hexenyl (C₆), and the like. Additional examples of alkenyl include heptenyl (C₇), octenyl (C₈), octatrienyl (C₈), and the like. Unless otherwise specified, each instance of an alkenyl group is independently optionally substituted, *i.e.,* unsubstituted (an "unsubstituted alkenyl") or substituted (a "substituted alkenyl") with one or more substituents *e.g.*, for instance from 1 to 5 substituents, 1 to 3 substituents, or 1 substituent. In certain embodiments, the alkenyl group is unsubstituted C₂₋₁₀ alkenyl. In certain embodiments, the alkenyl group is substituted C₂₋₁₀ alkenyl.

"Alkynyl" refers to a radical of a straight-chain or branched hydrocarbon group having from 2 to 20 carbon atoms, one or more carbon-carbon triple bonds (*e.g.,* 1, 2, 3, or 4 carbon-carbon triple bonds), and optionally one or more carbon-carbon double bonds (*e.g.,* 1, 2, 3, or 4 carbon-carbon double bonds) ("C₂₋₂₀ alkynyl"). In certain embodiments, alkynyl does not contain any double bonds. In some embodiments, an alkynyl group has 2 to 10 carbon atoms ("C₂₋₁₀ alkynyl"). In some embodiments, an alkynyl group has 2 to 9 carbon atoms ("C₂₋₉ alkynyl"). In some embodiments, an alkynyl group has 2 to 8 carbon atoms ("C₂₋₈ alkynyl"). In some embodiments, an alkynyl group has 2 to 7 carbon atoms ("C₂₋₇ alkynyl"). In some embodiments, an alkynyl group has 2 to 6 carbon atoms ("C₂₋₆ alkynyl"). In some embodiments, an alkynyl group has 2 to 5 carbon atoms ("C₂₋₅ alkynyl"). In some embodiments, an alkynyl group has 2 to 4 carbon atoms ("C₂₋₄ alkynyl"). In some embodiments, an alkynyl group has 2 to 3 carbon atoms ("C₂₋₃ alkynyl"). In some embodiments, an alkynyl group has 2 carbon atoms ("C₂ alkynyl"). The one or more carbon-carbon triple bonds can be internal (such as in 2-butynyl) or terminal (such as in 1-butynyl). Examples of C₂₋₄ alkynyl groups include, without limitation, ethynyl (C₂), 1-propynyl (C₃), 2-propynyl (C₃), 1-butynyl (C₄), 2-butynyl (C₄), and the like. Examples of C₂₋₆ alkenyl groups include the aforementioned C₂₋₄ alkynyl groups as well as pentynyl (C₅), hexynyl (C₆), and the like. Additional examples of alkynyl include heptynyl (C₇), octynyl (C₈), and the like. Unless otherwise specified, each instance of an alkynyl group is independently optionally substituted, *i.e.,* unsubstituted (an "unsubstituted alkynyl") or substituted (a "substituted alkynyl") with one or more substituents; e.g., for instance from 1 to 5 substituents, 1 to 3 substituents, or 1 substituent. In certain embodiments, the alkynyl group is unsubstituted C₂₋₁₀ alkynyl. In certain embodiments, the alkynyl group is substituted C₂₋₁₀ alkynyl.

The term "heteroalkyl," as used herein, refers to an alkyl group, as defined herein, which further comprises 1 or more (*e.g.,* 1, 2, 3, or 4) heteroatoms (*e.g.,* oxygen, sulfur, nitrogen, boron, silicon, phosphorus) within the parent chain, wherein the one or more heteroatoms is inserted between adjacent carbon atoms within the parent carbon chain and/or one or more heteroatoms is inserted between a carbon atom and the parent molecule, *i.e.,* between the point of attachment. In certain embodiments, a heteroalkyl group refers to a saturated group having from 1 to 10 carbon atoms and 1, 2, 3, or 4 heteroatoms ("heteroC₁₋₁₀ alkyl"). In some embodiments, a heteroalkyl group is a saturated group having 1 to 9 carbon atoms and 1, 2, 3, or 4 heteroatoms ("heteroC₁₋₉ alkyl"). In some embodiments, a heteroalkyl group is a saturated group having 1 to 8 carbon atoms and 1, 2, 3, or 4 heteroatoms ("heteroC₁₋₈ alkyl"). In some embodiments, a heteroalkyl group is a saturated group having 1 to 7 carbon atoms and 1, 2, 3, or 4 heteroatoms ("heteroC₁₋₇ alkyl"). In some embodiments, a heteroalkyl group is a group having 1 to 6 carbon atoms and 1, 2, or 3 heteroatoms ("heteroC₁₋₆ alkyl"). In some embodiments, a heteroalkyl group is a saturated group having 1 to 5 carbon atoms and 1 or 2 heteroatoms ("heteroC₁₋₅ alkyl"). In some embodiments, a heteroalkyl group is a saturated group having 1 to 4 carbon atoms and 1or 2 heteroatoms ("heteroC₁₋₄ alkyl"). In some embodiments, a heteroalkyl group is a saturated group having 1 to 3 carbon atoms and 1 heteroatom ("heteroC₁₋₃ alkyl"). In some embodiments, a heteroalkyl group is a saturated group having 1 to 2 carbon atoms and 1 heteroatom ("heteroC₁₋₂ alkyl"). In some embodiments, a heteroalkyl group is a saturated group having 1 carbon atom and 1 heteroatom ("heteroC₁ alkyl"). In some embodiments, a heteroalkyl group is a saturated group having 2 to 6 carbon atoms and 1 or 2 heteroatoms ("heteroC₂₋₆ alkyl"). Unless otherwise specified, each instance of a heteroalkyl group is independently unsubstituted (an "unsubstituted heteroalkyl") or substituted (a "substituted heteroalkyl") with one or more substituents. In certain embodiments, the heteroalkyl group is an unsubstituted heteroC₁₋₁₀ alkyl. In certain embodiments, the heteroalkyl group is a substituted heteroC₁₋₁₀ alkyl.

"Aryl" refers to a radical of a monocyclic or polycyclic (*e.g*., bicyclic or tricyclic) 4n+2 aromatic ring system (*e.g*., having 6, 10, or 14 π electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; *e.g.,* phenyl). In some embodiments, an aryl group has ten ring carbon atoms ("C₁₀ aryl"; *e.g.*, naphthyl such as 1-naphthyl and 2-naphthyl). In some embodiments, an aryl group has fourteen ring carbon atoms ("C₁₄ aryl"; *e.g*., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Typical aryl groups include, but are not limited to, groups derived from aceanthrylene, acenaphthylene, acephenanthrylene, anthracene, azulene, benzene, chrysene, coronene, fluoranthene, fluorene, hexacene, hexaphene, hexalene, as-indacene, s-indacene, indane, indene, naphthalene, octacene, octaphene, octalene, ovalene, penta-2,4-diene, pentacene, pentalene, pentaphene, perylene, phenalene, phenanthrene, picene, pleiadene, pyrene, pyranthrene, rubicene, triphenylene, and trinaphthalene. Particularly aryl groups include phenyl, naphthyl, indenyl, and tetrahydronaphthyl. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

In certain embodiments, an aryl group substituted with one or more of groups selected from halo, C₁-C₈ alkyl, C₁-C₈ haloalkyl, cyano, hydroxy, C₁-C₈ alkoxy, and amino.

Examples of representative substituted aryls include the following 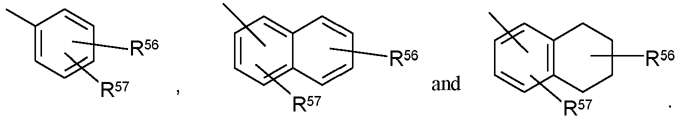 wherein one of R⁵⁶ and R⁵⁷ may be hydrogen and at least one of R⁵⁶ and R⁵⁷ is each independently selected from C₁-C₈ alkyl, C₁-C₈ haloalkyl, 4-10 membered heterocyclyl, alkanoyl, C₁-C₈ alkoxy, heteroaryloxy, alkylamino, arylamino, heteroarylamino, NR⁵⁸COR⁵⁹, NR⁵⁸SOR⁵⁹ NR⁵⁸SO₂R⁵⁹, COOalkyl, COOaryl, CONR⁵⁸R⁵⁹ CONR⁵⁸OR⁵⁹, NR⁵⁸R⁵⁹, SO₂NR⁵⁸R⁵⁹, S-alkyl, SOalkyl, SO₂alkyl, Saryl, SOaryl, SO₂aryl; or R⁵⁶ and R⁵⁷ may be joined to form a cyclic ring (saturated or unsaturated) from 5 to 8 atoms, optionally containing one or more heteroatoms selected from the group N, O, or S. R⁶⁰ and R⁶¹ are independently hydrogen, C₁-C₈ alkyl, C₁-C₄ haloalkyl, C₃-C₁₀ cycloalkyl, 4-10 membered heterocyclyl, C₆-C₁₀ aryl, substituted C₆-C₁₀ aryl, 5-10 membered heteroaryl, or substituted 5-10 membered heteroaryl .

"Fused aryl" refers to an aryl having two of its ring carbon in common with a second aryl or heteroaryl ring or with a carbocyclyl or heterocyclyl ring.

"Heteroaryl" refers to a radical of a 5-10 membered monocyclic or bicyclic 4n+2 aromatic ring system (*e.g*., having 6 or 10 π electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e.g*., indolyl, quinolinyl, carbazolyl, and the like) the point of attachment can be on either ring, *i.e.,* either the ring bearing a heteroatom (*e.g*., 2-indolyl) or the ring that does not contain a heteroatom (*e.g.,* 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

Examples of representative heteroaryls include the following: 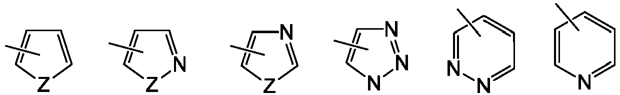 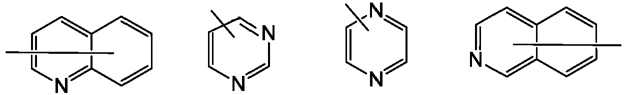 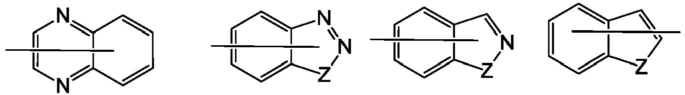 wherein each Z is selected from carbonyl, N, NR⁶⁵, O, and S; and R⁶⁵ is independently hydrogen, C₁-C₈ alkyl, C₃-C₁₀ cycloalkyl, 4-10 membered heterocyclyl, C₆-C₁₀ aryl, and 5-10 membered heteroaryl.

"Carbocyclyl" or "carbocyclic" refers to a radical of a non-aromatic cyclic hydrocarbon group having from 3 to 10 ring carbon atoms ("C₃₋₁₀ carbocyclyl") and zero heteroatoms in the non-aromatic ring system. In some embodiments, a carbocyclyl group has 3 to 8 ring carbon atoms ("C₃₋₈ carbocyclyl"). In some embodiments, a carbocyclyl group has 3 to 6 ring carbon atoms ("C₃₋₆ carbocyclyl"). In some embodiments, a carbocyclyl group has 3 to 6 ring carbon atoms ("C₃₋₆ carbocyclyl"). In some embodiments, a carbocyclyl group has 5 to 10 ring carbon atoms ("C₅₋₁₀ carbocyclyl"). Exemplary C₃₋₆ carbocyclyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃₋₈ carbocyclyl groups include, without limitation, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptanyl (C₇), bicyclo[2.2.2]octanyl (C₈), and the like. Exemplary C₃₋₁₀ carbocyclyl groups include, without limitation, the aforementioned C₃₋₈ carbocyclyl groups as well as cyclononyl (C₉), cyclononenyl (C₉), cyclodecyl (C₁₀), cyclodecenyl (C₁₀), octahydro-1H-indenyl (C₉), decahydronaphthalenyl (C₁₀), spiro[4.5]decanyl (C₁₀), and the like. As the foregoing examples illustrate, in certain embodiments, the carbocyclyl group is either monocyclic ("monocyclic carbocyclyl") or contain a fused, bridged or spiro ring system such as a bicyclic system ("bicyclic carbocyclyl") and can be saturated or can be partially unsaturated. "Carbocyclyl" also includes ring systems wherein the carbocyclyl ring, as defined above, is fused with one or more aryl or heteroaryl groups wherein the point of attachment is on the carbocyclyl ring, and in such instances, the number of carbons continue to designate the number of carbons in the carbocyclic ring system. Unless otherwise specified, each instance of a carbocyclyl group is independently optionally substituted, *i.e.,* unsubstituted (an "unsubstituted carbocyclyl") or substituted (a "substituted carbocyclyl") with one or more substituents. In certain embodiments, the carbocyclyl group is unsubstituted C₃₋₁₀ carbocyclyl. In certain embodiments, the carbocyclyl group is a substituted C₃₋₁₀ carbocyclyl.

In some embodiments, "carbocyclyl" is a monocyclic, saturated carbocyclyl group having from 3 to 10 ring carbon atoms ("C₃₋₁₀ cycloalkyl"). In some embodiments, a cycloalkyl group has 3 to 8 ring carbon atoms ("C₃₋₈ cycloalkyl"). In some embodiments, a cycloalkyl group has 3 to 6 ring carbon atoms ("C₃₋₆ cycloalkyl"). In some embodiments, a cycloalkyl group has 5 to 6 ring carbon atoms ("C₅₋₆ cycloalkyl"). In some embodiments, a cycloalkyl group has 5 to 10 ring carbon atoms ("C₅₋₁₀ cycloalkyl"). Examples of C₅₋₆ cycloalkyl groups include cyclopentyl (C₅) and cyclohexyl (C₅). Examples of C₃₋₆ cycloalkyl groups include the aforementioned C₅₋₆ cycloalkyl groups as well as cyclopropyl (C₃) and cyclobutyl (C₄). Examples of C₃₋₈ cycloalkyl groups include the aforementioned C₃₋₆ cycloalkyl groups as well as cycloheptyl (C₇) and cyclooctyl (C₈). Unless otherwise specified, each instance of a cycloalkyl group is independently unsubstituted (an "unsubstituted cycloalkyl") or substituted (a "substituted cycloalkyl") with one or more substituents. In certain embodiments, the cycloalkyl group is unsubstituted C₃₋₁₀ cycloalkyl. In certain embodiments, the cycloalkyl group is substituted C₃₋₁₀ cycloalkyl.

"Heterocyclyl" or "heterocyclic" refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more carbocyclyl groups wherein the point of attachment is either on the carbocyclyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. Unless otherwise specified, each instance of heterocyclyl is independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Nitrogen-containing heterocyclyl" group means a 4- to 7- membered non-aromatic cyclic group containing at least one nitrogen atom, for example, but without limitation, morpholine, piperidine (*e.g*. 2-piperidinyl, 3-piperidinyl and 4-piperidinyl), pyrrolidine (*e.g*. 2-pyrrolidinyl and 3-pyrrolidinyl), azetidine, pyrrolidone, imidazoline, imidazolidinone, 2-pyrazoline, pyrazolidine, piperazine, and N-alkyl piperazines such as N-methyl piperazine. Particular examples include azetidine, piperidone and piperazone.

"Hetero" when used to describe a compound or a group present on a compound means that one or more carbon atoms in the compound or group have been replaced by a nitrogen, oxygen, or sulfur heteroatom. Hetero may be applied to any of the hydrocarbyl groups described above such as alkyl, *e.g.,* heteroalkyl, cycloalkyl, *e.g.,* heterocyclyl, aryl, *e.g,.* heteroaryl, cycloalkenyl, *e.g,.* cycloheteroalkenyl, and the like having from 1 to 5, and particularly from 1 to 3 heteroatoms.

"Acyl" refers to a radical -C(O)R²⁰, where R²⁰ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, as defined herein. "Alkanoyl" is an acyl group wherein R²⁰ is a group other than hydrogen. Representative acyl groups include, but are not limited to, formyl (-CHO), acetyl (-C(=O)CH₃), cyclohexylcarbonyl, cyclohexylmethylcarbonyl, benzoyl (-C(=O)Ph), benzylcarbonyl (-C(=O)CH₂Ph), -C(O)-C₁-C₈ alkyl, -C(O)-(CH₂)ₜ(C₆-C₁₀ aryl), -C(O)-(CH₂)ₜ(5-10 membered heteroaryl), -C(O)-(CH₂)ₜ(C₃-C₁₀ cycloalkyl), and -C(O)-(CH₂)ₜ(4-10 membered heterocyclyl), wherein t is an integer from 0 to 4. In certain embodiments, R²¹ is C₁-C₈ alkyl, substituted with halo or hydroxy; or C₃-C₁₀ cycloalkyl, 4-10 membered heterocyclyl, C₆-C₁₀ aryl, arylalkyl, 5-10 membered heteroaryl or heteroarylalkyl, each of which is substituted with unsubstituted C₁₋C₄ alkyl, halo, unsubstituted C₁-C₄ alkoxy, unsubstituted C₁-C₄ haloalkyl, unsubstituted C₁₋C₄ hydroxyalkyl, or unsubstituted C₁-C₄ haloalkoxy or hydroxy.

"Alkoxy" refers to the group -OR²⁹ where R²⁹ is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl. Particular alkoxy groups are methoxy, ethoxy, n-propoxy, isopropoxy, n-butoxy, tert-butoxy, sec-butoxy, n-pentoxy, n-hexoxy, and 1,2-dimethylbutoxy. Particular alkoxy groups are lower alkoxy, *i.e.* with between 1 and 6 carbon atoms. Further particular alkoxy groups have between 1 and 4 carbon atoms.

In certain embodiments, R²⁹ is a group that has 1 or more substituents, for instance from 1 to 5 substituents, and particularly from 1 to 3 substituents, in particular 1 substituent, selected from the group consisting of amino, substituted amino, C₆-C₁₀ aryl, aryloxy, carboxyl, cyano, C₃-C₁₀ cycloalkyl, 4-10 membered heterocyclyl, halogen, 5-10 membered heteroaryl, hydroxyl, nitro, thioalkoxy, thioaryloxy, thiol, alkyl-S(O)-, aryl-S(O)-, alkylS(O)₂- and aryl-S(O)₂-. Exemplary 'substituted alkoxy' groups include, but are not limited to, -O-(CH₂)ₜ(C₆-C₁₀ aryl), -O-(CH₂)ₜ(5-10 membered heteroaryl), -O-(CH₂)ₜ(C₃-C₁₀ cycloalkyl), and -O-(CH₂)ₜ(4-10 membered heterocyclyl), wherein t is an integer from 0 to 4 and any aryl, heteroaryl, cycloalkyl or heterocyclyl groups present, may themselves be substituted by unsubstituted C₁-C₄ alkyl, halo, unsubstituted C₁-C₄ alkoxy, unsubstituted C₁₋C₄ haloalkyl, unsubstituted C₁-C₄ hydroxyalkyl, or unsubstituted C₁-C₄ haloalkoxy or hydroxy. Particular exemplary 'substituted alkoxy' groups are -OCF₃, -OCH₂CF₃, -OCH₂Ph, -OCH₂-cyclopropyl, -OCH₂CH₂OH, and -OCH₂CH₂NMe₂.

"Amino" refers to the radical -NH₂.

"Oxo group" refers to -C(=O)-.

"Substituted amino" refers to an amino group of the formula -N(R³⁸)₂ wherein R³⁸ is hydrogen, substituted or unsubstituted alkyl, substituted or unsubstitued alkenyl, substituted or unsubstitued alkynyl, substituted or unsubstitued carbocyclyl, substituted or unsubstituted heterocyclyl, substituted or unsubstituted aryl, substituted or unsubstitued heteroaryl, or an amino protecting group, wherein at least one of R³⁸ is not a hydrogen. In certain embodiments, each R³⁸ is independently selected from hydrogen, C₁-C₈ alkyl, C₃-C₈ alkenyl, C₃-C₈ alkynyl, C₆-C₁₀ aryl, 5-10 membered heteroaryl, 4-10 membered heterocyclyl, or C₃-C₁₀ cycloalkyl; or C₁-C₈ alkyl, substituted with halo or hydroxy; C₃-C₈ alkenyl, substituted with halo or hydroxy; C₃-C₈ alkynyl, substituted with halo or hydroxy, or - (CH₂)ₜ(C₆-C₁₀ aryl), -(CH₂)ₜ(5-10 membered heteroaryl), -(CH₂)ₜ(C₃-C₁₀ cycloalkyl), or - (CH₂)ₜ(4-10 membered heterocyclyl), wherein t is an integer between 0 and 8, each of which is substituted by unsubstituted C₁-C₄ alkyl, halo, unsubstituted C₁-C₄ alkoxy, unsubstituted C₁-C₄ haloalkyl, unsubstituted C₁-C₄ hydroxyalkyl, or unsubstituted C₁-C₄ haloalkoxy or hydroxy; or both R³⁸ groups are joined to form an alkylene group.

Exemplary "substituted amino" groups include, but are not limited to, -NR³⁹-C₁-C₈ alkyl, -NR³⁹-(CH₂)ₜ(C₆-C₁₀ aryl), -NR³⁹-(CH₂)ₜ(5-10 membered heteroaryl), -NR³⁹-(CH₂)ₜ(C₃-C₁₀ cycloalkyl), and -NR³⁹-(CH₂)ₜ(4-10 membered heterocyclyl), wherein t is an integer from 0 to 4, for instance 1 or 2, each R³⁹ independently represents H or C₁-C₈ alkyl; and any alkyl groups present, may themselves be substituted by halo, substituted or unsubstituted amino, or hydroxy; and any aryl, heteroaryl, cycloalkyl, or heterocyclyl groups present, may themselves be substituted by unsubstituted C₁-C₄ alkyl, halo, unsubstituted C₁₋C₄ alkoxy, unsubstituted C₁-C₄ haloalkyl, unsubstituted C₁-C₄ hydroxyalkyl, or unsubstituted C₁-C₄ haloalkoxy or hydroxy. For the avoidance of doubt the term 'substituted amino' includes the groups alkylamino, substituted alkylamino, alkylarylamino, substituted alkylarylamino, arylamino, substituted arylamino, dialkylamino, and substituted dialkylamino as defined below. Substituted amino encompasses both monosubstituted amino and disubstituted amino groups.

"Carboxy" refers to the radical -C(O)OH.

"Cyano" refers to the radical -CN.

"Halo" or "halogen" refers to fluoro (F), chloro (Cl), bromo (Br), and iodo (I). In certain embodiments, the halo group is either fluoro or chloro.

"Haloalkyl" refers to an alkyl radical in which the alkyl group is substituted with one or more halogens. Typical haloalkyl groups include, but are not limited to, trifluoromethyl, difluoromethyl, fluoromethyl, chloromethyl, dichloromethyl, dibromoethyl, tribromomethyl, tetrafluoroethyl, and the like.

"Hydroxy" refers to the radical -OH.

"Nitro" refers to the radical -NO₂.

"Thioketo" refers to the group =S.

Alkyl, alkenyl, alkynyl, carbocyclyl, heterocyclyl, aryl, and heteroaryl groups, as defined herein, are optionally substituted (*e.g*., "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g.,* a carbon or nitrogen atom) is replaced with a permissible substituent, *e.g*., a substituent which upon substitution results in a stable compound, *e.g.,* a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, any of the substituents described herein that results in the formation of a stable compound. The present invention contemplates any and all such combinations in order to arrive at a stable compound. For purposes of this invention, heteroatoms such as nitrogen may have hydrogen substituents and/or any suitable substituent as described herein which satisfy the valencies of the heteroatoms and results in the formation of a stable moiety.

Exemplary carbon atom substituents include, but are not limited to, halogen, -CN, -NO₂, -N₃, -SO₂H, -SO₃H, -OH, -OR^{aa}, -ON(R^{bb})₂, -N(R^{bb})₂, -N(R^{bb})₃⁺X⁻, -N(OR^{cc})R^{bb}, -SH, -SR^{aa}, -SSR^{cc}, -C(=O)R^{aa}, -CO₂H, -CHO, -C(OR^{cc})₂, -CO₂R^{aa}, -OC(=O)R^{aa}, - OCO₂R^{aa}, -C(=O)N(R^{bb})₂, -OC(=O)N(R^{bb})₂, -NR^{bb}C(=O)R^{aa}, -NR^{bb}CO₂R^{aa}, - NR^{bb}C(=O)N(R^{bb})₂, -C(=NR^{bb})R^{aa}, -C(=NR^{bb})OR^{aa}, -OC(=NR^{bb})R^{aa}, -OC(=NR^{bb})OR^{aa}, - C(=NR^{bb})N(R^{bb})₂, -OC(=NR^{bb})N(R^{bb})₂, -NR^{bb}C(=NR^{bb})N(R^{bb})₂, -C(=O)NR^{bb}SO₂R^{aa}, - NR^{bb}SO₂R^{aa}, -SO₂N(R^{bb})₂, -SO₂R^{aa}, -SO₂OR^{aa}, -OSO₂R^{aa}, -S(=O)R^{aa}, -OS(=O)R^{aa}, - Si(R^{aa})₃, -OSi(R^{aa})₃ -C(=S)N(R^{bb})₂, -C(=O)SR^{aa}, -C(=S)SR^{aa}, -SC(=S)SR^{aa}, -SC(=O)SR^{aa}, -OC(=O)SR^{aa}, -SC(=O)OR^{aa}, -SC(=O)R^{aa}, -P(=O)₂R^{aa}, -OP(=O)₂R^{aa}, -P(=O)(R^{aa})₂, - OP(=O)(R^{aa})₂, -OP(=O)(OR^{cc})₂, -P(=O)₂N(R^{bb})₂, -OP(=O)₂N(R^{bb})₂, -P(=O)(NR^{bb})₂, - OP(=O)(NR^{bb})₂, -NR^{bb}P(=O)(OR^{cc})₂, -NR^{bb}P(=O)(NR^{bb})₂, -P(R^{cc})₂, -P(R^{cc})₃, -OP(R^{cc})₂, - OP(R^{cc})₃, -B(R^{aa})₂, -B(OR^{cc})₂, -BR^{aa}(OR^{cc}), C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, wherein each alkyl, alkenyl, alkynyl, carbocyclyl, heterocyclyl, aryl, and heteroaryl is independently substituted with 0, 1, 2, 3, 4, or 5 R^{dd} groups; or two geminal hydrogens on a carbon atom are replaced with the group =O, =S, =NN(R^{bb})₂, =NNR^{bb}C(=O)R^{aa}, =NNR^{bb}C(=O)OR^{aa}, =NNR^{bb}S(=O)₂R^{aa}, =NR^{bb}, or =NOR^{cc};
each instance of R^{aa} is, independently, selected from C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{aa} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{bb} is, independently, selected from hydrogen, -OH, -OR^{aa}, - N(R^{cc})₂, -CN, -C(=O)R^{aa}, -C(=O)N(R^{cc})₂, -CO₂R^{aa}, -SO₂R^{aa}, -C(=NR^{cc})OR^{aa}, - C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, - C(=S)SR^{cc}, -P(=O)₂R^{aa}, -P(=O)(R^{aa})₂, -P(=O)₂N(R^{cc})2, -P(=O)(NR^{cc})2, C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{bb} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{cc} is, independently, selected from hydrogen, C₁₋₁₀ alkyl, C₁₋₁₀ haloalkyl, C₂₋₁₀ alkenyl, C₂₋₁₀ alkynyl, C₃₋₁₀ carbocyclyl, 3-14 membered heterocyclyl, C₆₋₁₄ aryl, and 5-14 membered heteroaryl, or two R^{cc} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{dd} groups;
each instance of R^{dd} is, independently, selected from halogen, -CN, -NO₂, -N₃, - SO₂H, -SO₃H, -OH, -OR^{ee}, -ON(R^{ff})₂, -N(R^{ff})₂, -N(R^{ff})₃⁺X⁻, -N(OR^{ee})R^{ff}, -SH, -SR^{ee}, - SSR^{ee}, -C(=O)R^{ee}, -CO₂H, -CO₂R^{ee}, -OC(=O)R^{ee}, -OCO₂R^{ee}, -C(=O)N(R^{ff})₂, - OC(=O)N(R^{ff})₂, -NR^{ff}C(=O)R^{ee}, -NR^{ff}CO₂R^{ee}, -NR^{ff}C(=O)N(R^{ff})₂, -C(=NR^{ff})OR^{ee}, - OC(=NR^{ff})R^{ee}, -OC(=NR^{ff})OR^{ee}, -C(=NR^{ff})N(R^{ff})₂, -OC(=NR^{ff})N(R^{ff})₂, - NR^{ff}C(=NR^{ff})N(R^{ff})₂,-NR^{ff}SO₂R^{ee}, -SO₂N(R^{ff})₂, -SO₂R^{ee}, -SO₂OR^{ee}, -OSO₂R^{ee}, -S(=O)R^{ee}, -Si(R^{ee})₃, -OSi(R^{ee})₃, -C(=S)N(R^{ff})₂, -C(=O)SR^{ee}, -C(=S)SR^{ee}, -SC(=S)SR^{ee}, -P(=O)₂R^{ee}, - P(=O)(R^{ee})₂, -OP(=O)(R^{ee})₂, -OP(=O)(OR^{ee})₂, C₁₋₆ alkyl, C₁₋₆ haloalkyl, C₂₋₆ alkenyl, C₂₋₆ alkynyl, C₃₋₁₀ carbocyclyl, 3-10 membered heterocyclyl, C₆₋₁₀ aryl, 5-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups, or two geminal R^{dd} substituents can be joined to form =O or =S;
each instance of R^{ee} is, independently, selected from C₁₋₆ alkyl, C₁₋₆ haloalkyl, C₂₋₆ alkenyl, C₂₋₆ alkynyl, C₃₋₁₀ carbocyclyl, C₆₋₁₀ aryl, 3-10 membered heterocyclyl, and 3-10 membered heteroaryl, wherein each alkyl, alkenyl, alkynyl, carbocyclyl, heterocyclyl, aryl, and heteroaryl is independently substituted with 0, 1, 2, 3, 4, or 5 R^{gg} groups;
each instance of R^{ff} is, independently, selected from hydrogen, C₁₋₆ alkyl, C₁₋₆ haloalkyl, C₂₋₆ alkenyl, C₂₋₆ alkynyl, C₃₋₁₀ carbocyclyl, 3-10 membered heterocyclyl, C₆₋₁₀ aryl and 5-10 membered heteroaryl, or two R^{ff} groups are joined to form a 3-14 membered heterocyclyl or 5-14 membered heteroaryl ring, wherein each alkyl, alkenyl, alkynyl, carbocyclyl, heterocyclyl, aryl, and heteroaryl is independently substituted with 0, 1, 2, 3, 4, or 5 R^{gg} groups; and
each instance of R^{gg} is, independently, halogen, -CN, -NO₂, -N₃, -SO₂H, -SO₃H, -OH, -OC₁₋₆ alkyl, -ON(C₁₋₆ alkyl)₂, -N(C₁₋₆ alkyl)₂, -N(C₁₋₆ alkyl)₃⁺X⁻, -NH(C₁₋₆ alkyl)₂⁺X⁻, -NH₂(C₁₋₆ alkyl)⁺X⁻, -NH₃⁺X⁻, -N(OC₁₋₆ alkyl)(C₁₋₆ alkyl), -N(OH)(C₁₋₆ alkyl), -NH(OH), -SH, -SC₁₋₆ alkyl, -SS(C₁₋₆ alkyl), -C(=O)(C₁₋₆ alkyl), -CO₂H, -CO₂(C₁₋₆ alkyl), -OC(=O)(C₁₋₆ alkyl), -OCO₂(C₁₋₆ alkyl), -C(=O)NH₂, -C(=O)N(C₁₋₆ alkyl)₂, - OC(=O)NH(C₁₋₆ alkyl), -NHC(=O)( C₁₋₆ alkyl), -N(C₁₋₆ alkyl)C(=O)( C₁₋₆ alkyl), - NHCO₂(C₁₋₆ alkyl), -NHC(=O)N(C₁₋₆ alkyl)₂, -NHC(=O)NH(C₁₋₆ alkyl), -NHC(=O)NH₂, -C(=NH)O(C₁₋₆ alkyl),-OC(=NH)(C₁₋₆ alkyl), -OC(=NH)OC₁₋₆ alkyl, -C(=NH)N(C₁₋₆ alkyl)₂, -C(=NH)NH(C₁₋₆ alkyl), -C(=NH)NH₂, -OC(=NH)N(C₁₋₆ alkyl)₂, - OC(NH)NH(C₁₋₆ alkyl), -OC(NH)NH₂, -NHC(NH)N(C₁₋₆ alkyl)₂, -NHC(=NH)NH₂, - NHSO₂(C₁₋₆ alkyl), -SO₂N(C₁₋₆ alkyl)₂, -SO₂NH(C₁₋₆ alkyl), -SO₂NH₂,-SO₂C₁₋₆ alkyl, - SO₂OC₁₋₆ alkyl, -OSO₂C₁₋₆ alkyl, -SOC₁₋₆ alkyl, -Si(C₁₋₆ alkyl)₃, -OSi(C₁₋₆ alkyl)₃ - C(=S)N(C₁₋₆ alkyl)₂, C(=S)NH(C₁₋₆ alkyl), C(=S)NH₂, -C(=O)S(C₁₋₆ alkyl), -C(=S)SC₁₋₆ alkyl, -SC(=S)SC₁₋₆ alkyl, -P(=O)₂(C₁₋₆ alkyl), -P(=O)(C₁₋₆ alkyl)₂, -OP(=O)(C₁₋₆ alkyl)₂, - OP(=O)(OC₁₋₆ alkyl)₂, C₁₋₆ alkyl, C₁₋₆ haloalkyl, C₂₋₆ alkenyl, C₂₋₆ alkynyl, C₃₋₁₀ carbocyclyl, C₆₋₁₀ aryl, 3-10 membered heterocyclyl, 5-10 membered heteroaryl; or two geminal R^{gg} substituents can be joined to form =O or =S; wherein X⁻ is a counterion.

A "counterion" or "anionic counterion" is a negatively charged group associated with a cationic quaternary amino group in order to maintain electronic neutrality. Exemplary counterions include halide ions *(e.g.,* F⁻, Cl⁻, Br⁻, I⁻), NO₃⁻, ClO₄⁻, OH⁻, H₂PO₄⁻, HSO₄⁻, sulfonate ions (*e.g*., methansulfonate, trifluoromethanesulfonate, p-toluenesulfonate, benzenesulfonate, 10-camphor sulfonate, naphthalene-2-sulfonate, naphthalene-1-sulfonic acid-5-sulfonate, ethan-1-sulfonic acid-2-sulfonate, and the like), and carboxylate ions (*e.g.,* acetate, ethanoate, propanoate, benzoate, glycerate, lactate, tartrate, glycolate, and the like).

These and other exemplary substituents are described in more detail in the **Detailed Description,** and **Claims.** The invention is not intended to be limited in any manner by the above exemplary listing of substituents.

### Other definitions

As used herein, the term "modulation" refers to the inhibition or potentiation of GABA_{A} receptor function. A "modulator" (*e.g.,* a modulator compound) may be, for example, an agonist, partial agonist, antagonist, or partial antagonist of the GABA_{A} receptor.

"Pharmaceutically acceptable" means approved or approvable by a regulatory agency of the Federal or a state government or the corresponding agency in countries other than the United States, or that is listed in the U.S. Pharmacopoeia or other generally recognized pharmacopoeia for use in animals, and more particularly, in humans.

"Pharmaceutically acceptable salt" refers to a salt of a compound of the invention that is pharmaceutically acceptable and that possesses the desired pharmacological activity of the parent compound. In particular, such salts are non-toxic may be inorganic or organic acid addition salts and base addition salts. Specifically, such salts include: (1) acid addition salts, formed with inorganic acids such as hydrochloric acid, hydrobromic acid, sulfuric acid, nitric acid, phosphoric acid, and the like; or formed with organic acids such as acetic acid, propionic acid, hexanoic acid, cyclopentanepropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl) benzoic acid, cinnamic acid, mandelic acid, methanesulfonic acid, ethanesulfonic acid, 1,2-ethane-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2.2.2]-oct-2-ene-1-carboxylic acid, glucoheptonic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; or (2) salts formed when an acidic proton present in the parent compound either is replaced by a metal ion, *e.g.,* an alkali metal ion, an alkaline earth ion, or an aluminum ion; or coordinates with an organic base such as ethanolamine, diethanolamine, triethanolamine, N-methylglucamine and the like. Salts further include, by way of example only, sodium, potassium, calcium, magnesium, ammonium, tetraalkylammonium, and the like; and when the compound contains a basic functionality, salts of non-toxic organic or inorganic acids, such as hydrochloride, hydrobromide, tartrate, mesylate, acetate, maleate, oxalate and the like. The term "pharmaceutically acceptable cation" refers to an acceptable cationic counterion of an acidic functional group. Such cations are exemplified by sodium, potassium, calcium, magnesium, ammonium, tetraalkylammonium cations, and the like. See, *e.g.,* Berge, et al., J. Pharm. Sci. (1977) 66(1): 1-79.

"Tautomers" refer to compounds that are interchangeable forms of a particular compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Another example of tautomerism is the aci- and nitro- forms of phenylnitromethane, that are likewise formed by treatment with acid or base. Tautomeric forms may be relevant to the attainment of the optimal chemical reactivity and biological activity of a compound of interest.

A "subject" to which administration is contemplated includes, but is not limited to, humans (*i.e.,* a male or female of any age group, *e.g.,* a pediatric subject (*e.g,* infant, child, adolescent) or adult subject (*e.g*., young adult, middle-aged adult or senior adult)) and/or a non-human animal, *e.g.,* a mammal such as primates (*e.g.,* cynomolgus monkeys, rhesus monkeys), cattle, pigs, horses, sheep, goats, rodents, cats, and/or dogs. In certain embodiments, the subject is a human. In certain embodiments, the subject is a non-human animal.

In certain embodiments, the substituent present on an oxygen atom is an oxygen protecting group (also referred to as a hydroxyl protecting group). Oxygen protecting groups include, but are not limited to, -R^{aa}, -N(R^{bb})₂, -C(=O)SR^{aa}, -C(=O)R^{aa}, -CO₂R^{aa}, - C(=O)N(R^{bb})_{2,} -C(=NR^{bb})R^{aa}, -C(=NR^{bb})OR^{aa}, -C(=NR^{bb})N(R^{bb})₂, -S(=O)R^{aa}, -SO₂R^{aa}, - Si(R^{aa})₃, -P(R^{cc})₂, -P(R^{cc})₃, -P(=O)₂R^{aa}, -P(=O)(R^{aa})₂, -P(=O)(OR^{cc})₂, -P(=O)₂N(R^{bb})₂, and - P(=O)(NR^{bb})₂, wherein R^{aa}, R^{bb}, and R^{cc} are as defined herein. Oxygen protecting groups are well known in the art and include those described in detail in Protecting Groups in Organic Synthesis, T. W. Greene and P. G. M. Wuts, 3rd edition, John Wiley & Sons, 1999.

Exemplary oxygen protecting groups include, but are not limited to, methyl, methoxylmethyl (MOM), 2-methoxyethoxymethyl (MEM), benzyl (Bn), triisopropylsilyl (TIPS), t-butyldimethylsilyl (TBDMS), t-butylmethoxyphenylsilyl (TBMPS), methanesulfonate (mesylate), and tosylate (Ts).

In certain embodiments, the substituent present on an sulfur atom is an sulfur protecting group (also referred to as a thiol protecting group). Sulfur protecting groups include, but are not limited to, -R^{aa}, -N(R^{bb})₂, -C(=O)SR^{aa}, -C(=O)R^{aa}, -CO₂R^{aa}, - C(=O)N(R^{bb})₂, -C(=NR^{bb})R^{aa}, -C(=NR^{bb})OR^{aa}, -C(=NR^{bb})N(R^{bb})₂, -S(=O)R^{aa}, -SO₂R^{aa}, - Si(R^{aa})₃, -P(R^{cc})₂, -P(R^{cc})₃, -P(=O)₂R^{aa}, -P(=O)(R^{aa})₂, -P(=O)(OR^{cc})₂, -P(=O)₂N(R^{bb})₂, and - P(=O)(NR^{bb})₂, wherein R^{aa}, R^{bb}, and R^{cc} are as defined herein. Sulfur protecting groups are well known in the art and include those described in detail in Protecting Groups in Organic Synthesis, T. W. Greene and P. G. M. Wuts, 3rd edition, John Wiley & Sons, 1999.

In certain embodiments, the substituent present on a nitrogen atom is an amino protecting group (also referred to herein as a nitrogen protecting group). Amino protecting groups include, but are not limited to, -OH, -OR^{aa}, -C(=O)R^{aa}, -C(=O)OR^{aa}, - C(=O)N(R^{cc})₂, -S(=O)₂R^{aa}, -C(=NR^{cc})R^{aa}, -C(=NR^{cc})OR^{aa}, -C(=NR^{cc})N(R^{cc})₂, -SO₂N(R^{cc})₂, -SO₂R^{cc}, -SO₂OR^{cc}, -SOR^{aa}, -C(=S)N(R^{cc})₂, -C(=O)SR^{cc}, -C(=S)SR^{cc}, C₁₋₁₀ alkyl, C₂₋₁₀ alkenyl, C₂₋₁₀ alkynyl, C₃₋₁₀ carbocyclyl, 3-14-membered heterocyclyl, C₆₋₁₄ aryl, and 5-14-membered heteroaryl groups, wherein each alkyl, alkenyl, alkynyl, carbocyclyl, heterocyclyl, aryl, and heteroaryl is independently substituted with 0, 1, 2, 3, 4, or 5 R^{dd} groups, and wherein R^{aa}, R^{bb}, R^{cc} and R^{dd} are as defined herein. Amino protecting groups are well known in the art and include those described in detail in *Protecting Groups in Organic Synthesis, T.* W. Greene and P. G. M. Wuts, 3^{rd} edition, John Wiley & Sons, 1999.

Exemplary amino protecting groups include, but are not limited to amide groups (*e.g.,* -C(=O)R^{aa}), which include, but are not limited to, formamide and acetamide; carbamate groups (*e.g.,* -C(=O)OR^{aa}), which include, but are not limited to, 9-fluorenylmethyl carbamate (Fmoc), t-butyl carbamate (BOC), and benzyl carbamate (Cbz); sulfonamide groups (*e.g.,* -S(=O)₂R^{aa}), which include, but are not limited to, *p-*toluenesulfonamide (Ts), methanesulfonamide (Ms), and N-[2-(trimethylsilyl)ethoxy]methylamine (SEM).
*Disease, disorder, and condition are used interchangeably herein.*

As used herein, and unless otherwise specified, the terms "treat," "treating" and "treatment" contemplate an action that occurs while a subject is suffering from the specified disease, disorder or condition, which reduces the severity of the disease, disorder or condition, or retards or slows the progression of the disease, disorder or condition ("therapeutic treatment"), and also contemplates an action that occurs before a subject begins to suffer from the specified disease, disorder or condition.

In general, the "effective amount" of a compound refers to an amount sufficient to elicit the desired biological response, *e.g.,* to treat a CNS-related disorder, is sufficient to induce anesthesia or sedation. As will be appreciated by those of ordinary skill in this art, the effective amount of a compound of the invention may vary depending on such factors as the desired biological endpoint, the pharmacokinetics of the compound, the disease being treated, the mode of administration, and the age, weight, health, and condition of the subject.

As used herein, and unless otherwise specified, a "therapeutically effective amount" of a compound is an amount sufficient to provide a therapeutic benefit in the treatment of a disease, disorder or condition, or to delay or minimize one or more symptoms associated with the disease, disorder or condition. A therapeutically effective amount of a compound means an amount of therapeutic agent, alone or in combination with other therapies, which provides a therapeutic benefit in the treatment of the disease, disorder or condition. The term "therapeutically effective amount" can encompass an amount that improves overall therapy, reduces or avoids symptoms or causes of disease or condition, or enhances the therapeutic efficacy of another therapeutic agent.

In an alternate embodiment, the present invention contemplates administration of the compounds of the present invention or a pharmaceutically acceptable salt or a pharmaceutically acceptable composition thereof, as a prophylactic before a subject begins to suffer from the specified disease, disorder or condition. As used herein, and unless otherwise specified, a "prophylactically effective amount" of a compound is an amount sufficient to prevent a disease, disorder or condition, or one or more symptoms associated with the disease, disorder or condition, or prevent its recurrence. A prophylactically effective amount of a compound means an amount of a therapeutic agent, alone or in combination with other agents, which provides a prophylactic benefit in the prevention of the disease, disorder or condition. The term "prophylactically effective amount" can encompass an amount that improves overall prophylaxis or enhances the prophylactic efficacy of another prophylactic agent.

As used herein, an "episodic dosing regimen" is a dosing regimen wherein a compound of Formula (I) or a composition comprising a compound of Formula (I) is administered to a subject for a finite period of time in response to the diagnosis of a disorder or symptom thereof, *e*.g, a diagnosis or symptom of depression. an episode of major depressive disorder, bipolar depression, anxiety, or postpartum depression. In some embodiments, the major depressive disorder is moderate major depressive disorder. In some embodiments, the major depressive disorder is severe major depressive disorder In some embodiments, the compound is formulated as individual dosage units, each unit comprising a compound of Formula (I) and one or more suitable pharmaceutical excipients. In some embodiments, the episodic dosing regimen has a duration of a plurality of weeks, *e.g*. about 8 weeks. In contrast with chronic administration as defined herein, episodic dosing of a compound occurs over a finite period of time, e.g., from about 2 weeks to about 8 weeks, in response to a diagnosis of a disorder, *e.g*., depression, or a symptom thereof. In some embodiments, episodic dosing occurs once per day across a plurality of weeks, *e.g*., from about 2 weeks to about 6 weeks. In one embodiment, the episodic dosing has a duration of two weeks. In some embodiments, more than one episodic dosing regimen is administered to the subject, *e.g*., two or more episodic regimens throughout the subject's life.

### Compounds

It should be appreciated that formulas described herein may reference particular carbon atoms, such as C17, C3, C19, etc. These references are based on the position of carbon atoms according to steroid nomenclature known and used in the industry, as shown below: 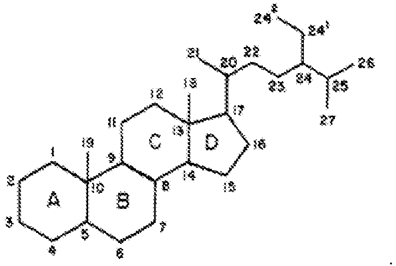 For example, C17 refers to the carbon at position 17 and C3 refers to the carbon at position 3.

In some embodiments, provided herein is a compound of Formula (I) 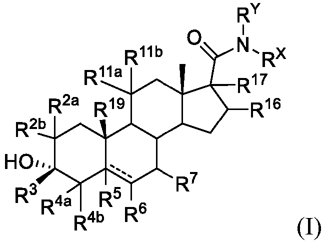 Or a pharmaceutically acceptable salt thereof;
wherein:
- - - - - - represents a single or a double bond as valency permits;
each of R^{2a}, R^{2b}, R^{4a}, R^{4b}, R⁶, R⁷, R^{11a}, and R^{11b}, is independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, an oxygen protecting group when attached to an oxygen atom, a nitrogen protecting group when attached to a nitrogen atom, or two R^{D1} groups are joined to form an substituted or unsubstituted heterocyclic ring; or any one of R^{2a} and R^{2b}, R^{4a} and R^{4b}, R^{7a} and R^{7b}, R^{11a} and R^{11b} are joined to form an oxo (=O) group;
each of R¹⁶ and R¹⁷ is each independently hydrogen, halogen, cyano, hydroxyl, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}),-CN(R^{A1})₂, -C(O)R^{A1}, -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, - SC(=O)N(R^{A1})₂,-NHC(=O)R^{A1}, -NHC(=O)OR^{A1}, -NHC(=O)SR^{A1}, -NHC(=O)N(R^{A1})₂, - OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or - S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, -SO₂R^{A2}, -C(O)R^{A2}, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R³ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R⁵ is hydrogen or methyl when both of the ------ are single bonds; and when one of the - - - - - - is a double bond, R⁵ is absent;
R¹⁹ is hydrogen or substituted or unsubstituted alkyl;
R^{X} is independently hydrogen, or substituted or unsubstituted alkyl
R^{Y} is independently substituted or unsubstituted heteroaryl.

In some embodiments, provided herein is a compound of Formula (I-X) 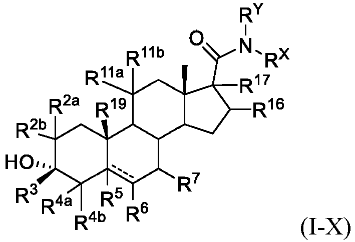 wherein:
- - - - - - represents a single or a double bond as valency permits;
each of R^{2a}, R^{2b}, R^{4a}, R^{4b}, R⁶, R⁷, R^{11a}, and R^{11b}, is independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, an oxygen protecting group when attached to an oxygen atom, a nitrogen protecting group when attached to a nitrogen atom, or two R^{D1} groups are joined to form an substituted or unsubstituted heterocyclic ring; or any one of R^{2a} and R^{2b}, R^{4a} and R^{4b}, R^{7a} and R^{7b}, R^{11a} and R^{11b} are joined to form an oxo (=O) group;
each of R¹⁶ and R¹⁷ is each independently hydrogen, halogen, cyano, hydroxyl, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}),-CN(R^{A1})₂, -C(O)R^{A1}, -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, - SC(=O)N(R^{A1})₂,-NHC(=O)R^{A1}, -NHC(=O)OR^{A1}, -NHC(=O)SR^{A1}, -NHC(=O)N(R^{A1})₂, - OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or - S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, -SO₂R^{A2}, -C(O)R^{A2}, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R³ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R⁵ is hydrogen or methyl when both of the - - - - - - are single bonds; and when one of the - - - - - - is a double bond, R⁵ is absent;
R¹⁹ is hydrogen or methyl;
R^{X} is independently hydrogen, or substituted or unsubstituted alkyl
R^{Y} is independently substituted or unsubstituted heteroaryl.
or a pharmaceutically acceptable salt thereof.

In some embodiments, provided herein is a compound of Formula (I-1) 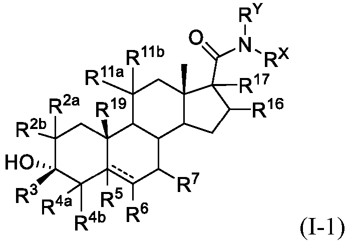 wherein:
- - - - - - represents a single or a double bond as valency permits;
each of R^{2a}, R^{2b}, R^{4a}, R^{4b}, R⁶, R⁷, R^{11a}, and R^{11b}, is independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, an oxygen protecting group when attached to an oxygen atom, a nitrogen protecting group when attached to a nitrogen atom, or two R^{D1} groups are joined to form an substituted or unsubstituted heterocyclic ring; or any one of R^{2a} and R^{2b}, R^{4a} and R^{4b}, R^{7a} and R^{7b}, R^{11a} and R^{11b} are joined to form an oxo (=O) group;
each of R¹⁶ and R¹⁷ is each independently hydrogen, halogen, cyano, hydroxyl, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}),-CN(R^{A1})₂, -C(O)R^{A1}, -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, - SC(=O)N(R^{A1})₂,-NHC(=O)R^{A1}, -NHC(=O)OR^{A1}, -NHC(=O)SR^{A1}, -NHC(=O)N(R^{A1})₂, - OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or - S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, -SO₂R^{A2}, -C(O)R^{A2}, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R³ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R⁵ is hydrogen when both of the - - - - - - are single bonds; and when one of the - - - - - - is a double bond, R⁵ is absent;
R¹⁹ is hydrogen or methyl;
R^{X} is independently hydrogen, or substituted or unsubstituted alkyl
R^{Y} is independently substituted or unsubstituted heteroaryl.
or a pharmaceutically acceptable salt thereof.

### Groups R^{2a} and R^{2b}

In some aspects, R^{2a} and R^{2b} is each independently hydrogen, halogen, cyano, hydroxyl, substituted or unsubstituted alkyl, substituted or unsubstituted alkenyl,-ORD1,-OC(=O)RD1, -NH2, or -N(RD1)2, wherein each instance of RD1 is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments each of R^{2a} and R^{2b} is independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH.

In some embodiments R^{2a} and R^{2b} is each independently hydrogen, -CH₃, - CH₂CH₃, -OH, -OCH₃, or -CH(CH₃)₂.

In some embodiments R^{2a} and R^{2b} is each independently hydrogen.

In some embodiments, R^{2a} and R^{2b} are both hydrogen.

### Groups R^{4a} and R^{4b}

In some embodiments, R^{4a} and R^{4b} is each independently is each independently hydrogen, halogen, cyano, hydroxyl, substituted or unsubstituted alkyl, substituted or unsubstituted alkenyl,-OR^{D1},-OC(=O)R^{D1}, -NH₂, or -N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, R^{4a} and R^{4b} is independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH.

In some embodiments, R^{4a} and R^{4b} is independently hydrogen, -CH₃, -CH₂CH₃, - OH, -OCH₃, or -CH(CH₃)₂.

In some embodiments, R^{4a} and R^{4b} is each independently hydrogen.

In some embodiments, R^{4a} and R^{4b} are both hydrogen.

### Groups R^{11a} and R^{11b}

In some embodiments, R^{11a} and R^{11b} is each independently hydrogen, halogen, cyano, substituted or unsubstituted alkyl, substituted or unsubstituted alkenyl,-OR^{D1},-OC(=O)R^{D1}, -NH₂, or -N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, R^{11a} and R^{11b} is independently hydrogen, substituted or unsubstituted C₁-C₆ alkyl, substituted or unsubstituted C₁-C₆ haloalkyl, substituted or unsubstituted C₁-C₆ alkoxy, or substituted or unsubstituted C₁-C₆ alkoxyhalo.

In some embodiments, R^{11a} and R^{11b} is independently hydrogen, -CH₃, -CH₂CH₃, -OCH₃, or -CH(CH₃)₂.

In some embodiments, R^{11a} and R^{11b} is each independently hydrogen.

In some embodiments, R^{11a} and R^{11b} are both hydrogen.

### Groups R⁷

In some embodiments, R⁷ is hydrogen, halogen, cyano, hydroxyl, substituted or unsubstituted alkyl, substituted or unsubstituted alkenyl,-OR^{D1},-OC(=O)R^{D1}, -NH₂, or - N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, R⁷ is independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH.

In some embodiments, R⁷ is independently hydrogen, -CH₃, -CH₂CH₃, -OH, - OCH₃, or -CH(CH₃)₂.

In some embodiments, R⁷ is hydrogen.

### Group R⁶

In some embodiments, R⁶ is independently hydrogen, halogen, cyano, hydroxyl, substituted or unsubstituted alkyl, substituted or unsubstituted alkenyl,-OR^{D1},-OC(=O)R^{D1}, - NH₂, or -N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, R⁶ is independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH.

In some embodiments, R⁶ is independently hydrogen, -CH₃, -CH₂CH₃, -OH, - OCH₃, or -CH(CH₃)₂.

In some embodiments, R⁶ is hydrogen.

### Group R¹⁶

R¹⁶ is independently hydrogen, halogen, cyano, hydroxyl, substituted or unsubstituted alkyl, substituted or unsubstituted alkenyl,-OR^{D1},-OC(=O)R^{D1}, -NH₂, or - N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, R¹⁶ is independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH.

In some embodiments, R¹⁶ is independently hydrogen, -CH₃, -CH₂CH₃, -OH, - OCH₃, or -CH(CH₃)₂.

In some embodiments, R¹⁶ is hydrogen.

### Group R¹⁷

In some embodiments, R¹⁷ is independently hydrogen, halogen, cyano, hydroxyl, substituted or unsubstituted alkyl, substituted or unsubstituted alkenyl,-ORD1,-OC(=O)R^{D1}, -NH₂, or -N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl.

In some embodiments, each of R¹⁷ is independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH.

In some embodiments, R¹⁷ is independently hydrogen, -CH₃, -CH₂CH₃, -OH, - OCH₃, or -CH(CH₃)₂.

In some embodiments, R¹⁷ is hydrogen.

### Group R¹⁹

In some embodiments, R¹⁹ is independently hydrogen, or substituted or unsubstituted C₁-C₆ alkyl.

In some embodiments, R¹⁹ is independently hydrogen, or substituted or unsubstituted C₁-C₄ alkyl.

In some embodiments, R¹⁹ is independently hydrogen, -CH₃, -CH₂CH₃, or - CH₂OCH(CH₃)₂.

In some embodiments, R¹⁹ is hydrogen.

In some embodiments, R¹⁹ is -CH₂OCH(CH₃)₂.

In some embodiments R¹⁹ is -CH₃.

In some embodiments R¹⁹ is -CH₂CH₃.

### Group R³

In some embodiments, R³ is substituted or unsubstituted alkyl, substituted or unsubstituted alkenyl, or substituted or unsubstituted alkynyl, substituted or unsubstituted carbocyclyl, or substituted or unsubstituted heterocyclyl.

In some embodiments, R³ is substituted or unsubstituted alkyl.

In some embodiments, R³ is substituted or unsubstituted C₁-C₆ alkyl.

In some embodiments, R³ is -CH₂CH₃ or -CH₃, -CH₂OCH₂CH₃, -CH₂OCH₃

In some embodiments, the compound of Formula (I) is a compound of Formula (I-a): 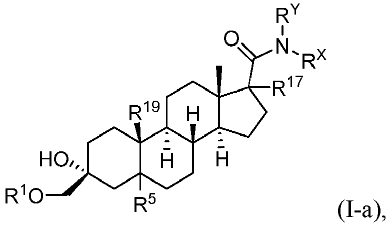
or a pharmaceutically acceptable salt thereof,
wherein R¹ is substituted or unsubstituted alkyl.

### Group R¹

In some embodiments R¹ is C₁-C₆alkyl

In some embodiments, R¹ is -CH₂CH₃ or -CH₃.

In some embodiments, R¹ is -CH₂CH₃.

In some embodiments, R¹ is -CH₃.

### Group R⁵

In some embodiments, the compound of Formula (I) is a compound of Formula (I-b1) or Formula (I-b2): 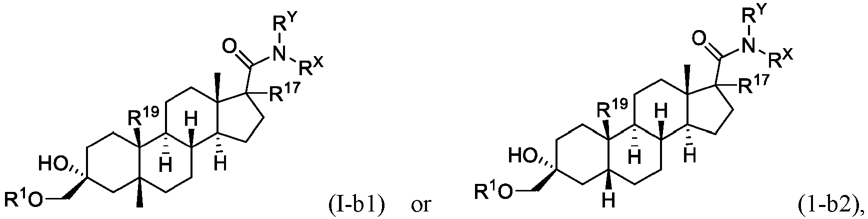 or a pharmaceutically acceptable salt thereof.

In some embodiments, the compound of Formula (I) is a compound of Formula (I-c1) or Formula (I-c2): 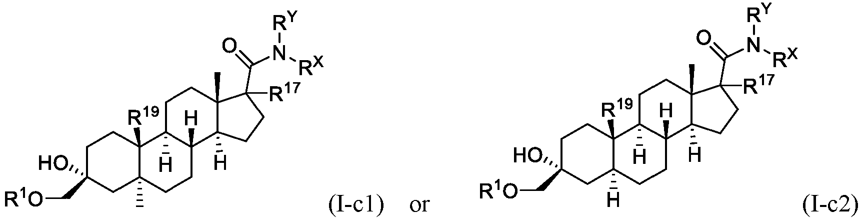 or a pharmaceutically acceptable salt thereof.

In some embodiments, the compound of Formula (I) is a compound of Formula (I-d1) or Formula (I-d2): 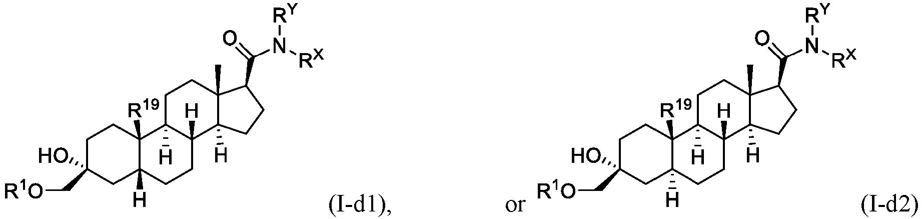 or a pharmaceutically acceptable salt thereof.

In some embodiments, the compound of Formula (I) is a compound of Formula (I-d1): 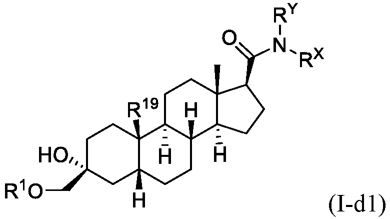 or a pharmaceutically acceptable salt thereof.

In some embodiments, the compound of Formula (I) is a compound of Formula (I-d2): 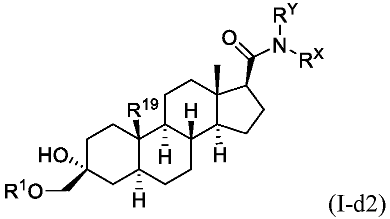 or a pharmaceutically acceptable salt thereof.

### Group R^{X}

In some embodiments, R^{X} is independently hydrogen, or substituted or unsubstituted C₁₋₆ alkyl.

In some embodiments, R^{X} is independently hydrogen, or substituted or unsubstituted C1-4 alkyl.

In some embodiments, R^{X} is independently hydrogen, -CH₃, or -CH₂CH₃.

In some embodiments, R^{X} is hydrogen.

### Group R^{Y}

In some embodiments, the compound of Formula (I), is a compound of Formula (I-e): 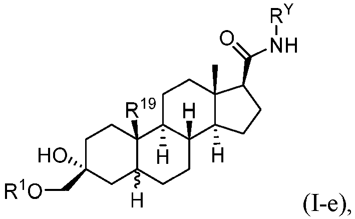 or a pharmaceutically acceptable salt thereof, wherein R^{Y} is heteroaryl, and R¹ is -CH₂CH₃ or -CH₃.

In some embodiments, R^{Y} is selected from: 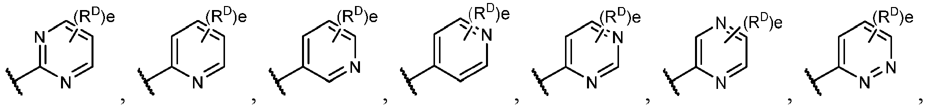 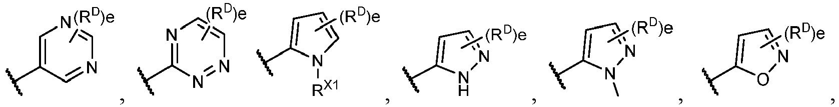 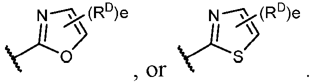
wherein each instance of R^{D} is, independently hydrogen, halogen, -NO₂, -CN, -OR^{GA}, -N(R^{GA})₂, -C(=O)R^{GA}, -C(=O)OR^{GA}, -C(=O)N(R^{GA})₂, -OC(=O)R^{GA}, -OC(=O)OR^{GA}, - N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, - OS(=O)₂R^{GA}, -S(=O)₂N(R^{GA})₂, or -N(R^{GA})S(=O)₂R^{GA}, substituted or unsubstituted C₁₋₆ alkyl, substituted or unsubstituted C₂₋₆ alkenyl, substituted or unsubstituted C₂₋₆ alkynyl, substituted or unsubstituted C₃₋₄ carbocyclyl, substituted or unsubstituted 3- to 6-membered heterocyclyl, C₅₋₁₀ substituted or unsubstituted aryl, substituted or unsubstituted 5- to 10- membered heteroaryl, or optionally two R^{GA} are taken with the intervening atoms to form a substituted or unsubstituted 3- to 4-membered carbocyclic or heterocyclic ring;
wherein each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocyclyl, substituted or unsubstituted 3- to 6- membered heterocyclyl, substituted or unsubstituted aryl, substituted or unsubstituted heteroaryl, an oxygen protecting group when attached to oxygen, a nitrogen protecting group when attached to nitrogen, or two R^{GA} groups are taken with the intervening atoms to form a substituted or unsubstituted carbocyclic or heterocyclic ring;
wherein each instance R^{X1} is hydrogen or substituted or unsubstituted alkyl, substituted or unsubstituted cycloalkyl, substituted or unsubstituted aryl, or substituted or unsubstituted carbocyclyl and
e is 0, 1, 2, 3, 4, or 5.

In some embodiments, R^{Y} is selected from: 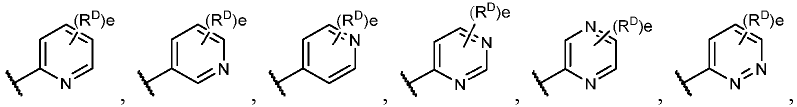 or

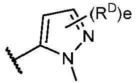

In some embodiments, R^{D} is, independently hydrogen, halogen, substituted or unsubstituted C₁₋₆ alkyl, -CN, substituted or unsubstituted 5-6 membered heteroaryl, substituted or unsubstituted 5-membered heterocyclyl,
e is 0, 1, 2, 3, or 4.

In some embodiments R^{Y} is selected from: 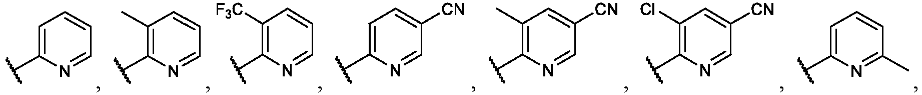 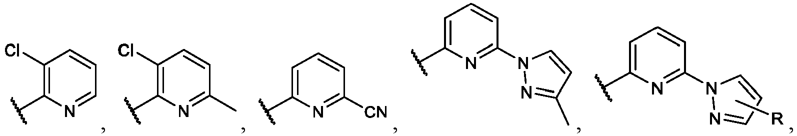 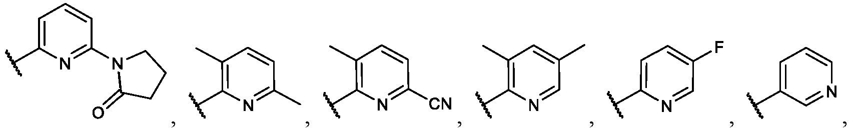 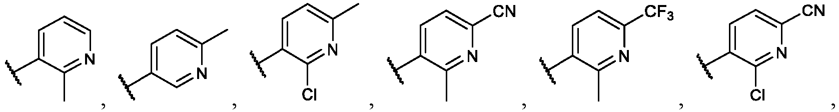 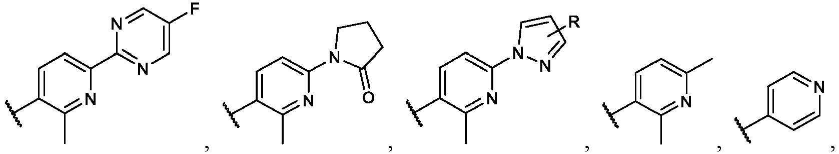 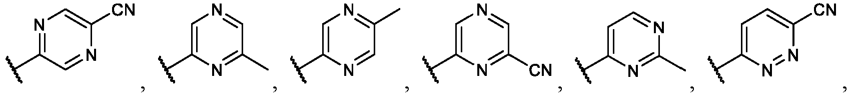 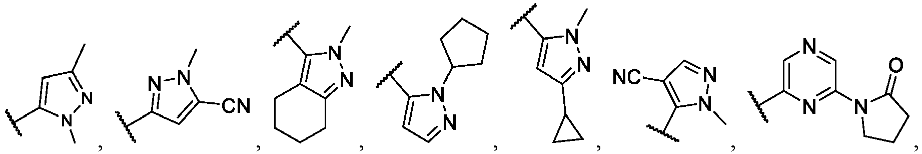 or 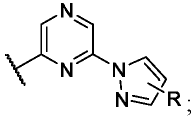 wherein R is -CH₃, -CH₂CH₃,-*i*-Pr, cyclopropyl or -CN.

In some embodiments, R^{Y} is selected from:

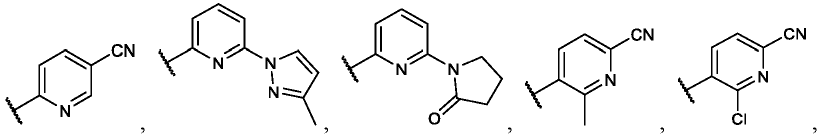

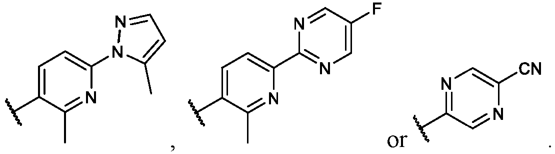

In some embodiments, R^{Y} is:

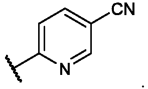

In some embodiments, R^{Y} is:

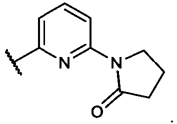

In some embodiments, the compound of Formula (I) is a compound of Formula (If): 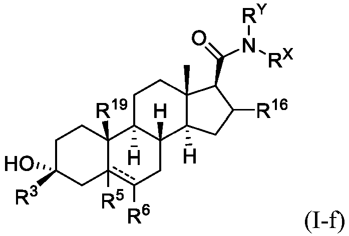 or a pharmaceutically acceptable salt thereof.

In some embodiments, the compound of Formula (I) is a compound of Formula (I-g) 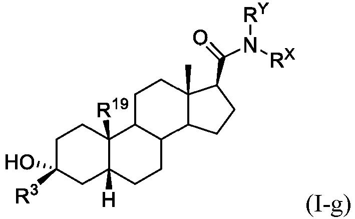 or a pharmaceutically acceptable salt thereof.

In some embodiments, the compound of Formula (I) is the compound is of Formula (Ia), Formula (I-b1), Formula (I-b2), Formula (I-c1), Formula (I-c2), Formula (I-d1), Formula (I-d2). Formula (I-e), Formula (I-f), or Formula (I-g).

In some embodiments, a pharmaceutical composition comprises a compound described herein or pharmaceutically acceptable salt thereof, and a pharmaceutically acceptable excipient.

In some embodiments, a method of treating a CNS-related disorder in a subject in need thereof, comprises administering to the subject an effective amount of a compound described herein or a pharmaceutically acceptable salt thereof. In some embodiments, the CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In some embodiments, the CNS-related disorder is depression. In some embodiments, the CNS-related disorder is postpartum depression. In some embodiments, the CNS-related disorder is major depressive disorder. In some embodiments, the major depressive disorder is moderate major depressive disorder. In some embodiments, the major depressive disorder is severe major depressive disorder.

In some embodiments, the compound is selected from the group consisting of the compounds identified in **Table 1** below:

**Table 1.**

| **Example** | **STRUCTURE** |
|---|---|
| 1 | 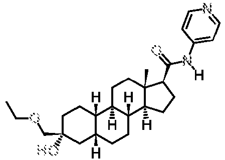 |
| 2 | 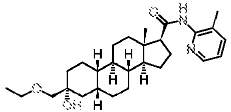 |
| 3 | 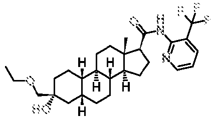 |
| 4 | 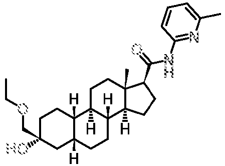 |
| 5 | 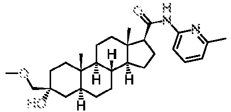 |
| 6 | 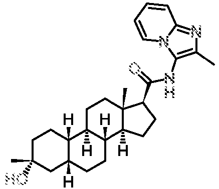 |
| 7 | 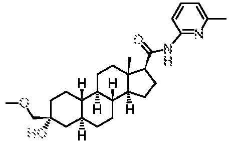 |
| 8 | 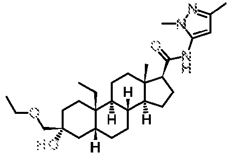 |
| 9 | 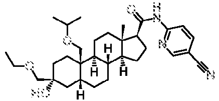 |
| 10 | 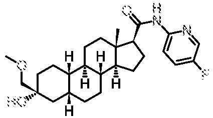 |
| 11 | 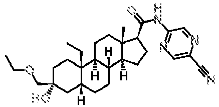 |
| 12 | 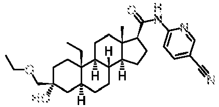 |
| 13 | 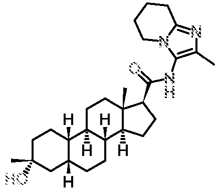 |
| 14 | 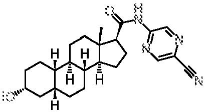 |
| 15 | 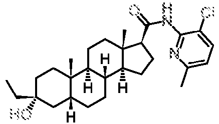 |
| 16 | 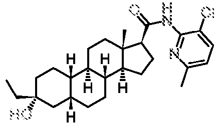 |
| 20 | 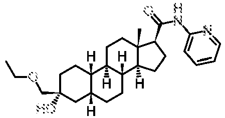 |
| 21 | 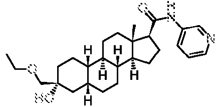 |
| 22 | 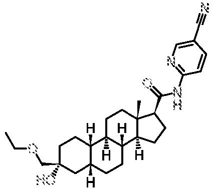 |
| 23 | 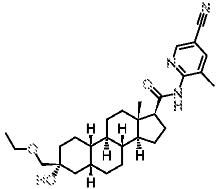 |
| 24 | 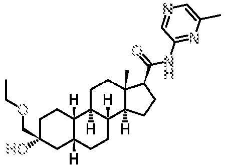 |
| 25 | 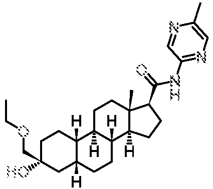 |
| 26 | 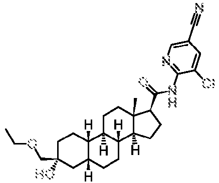 |
| 27 | 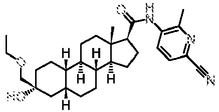 |
| 28 | 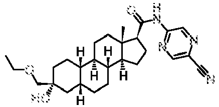 |
| 29 | 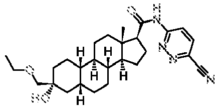 |
| 30 | 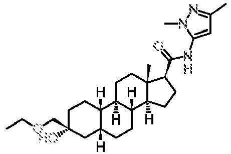 |
| 31 | 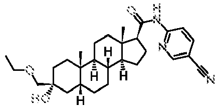 |
| 32 | 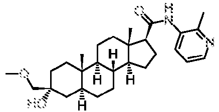 |
| 33 | 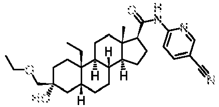 |
| 35 | 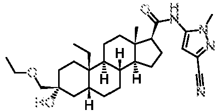 |
| 36 | 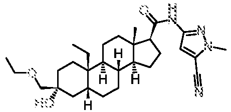 |
| 37 | 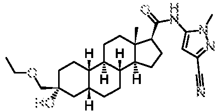 |
| 38 | 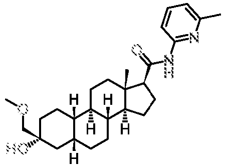 |
| 39 | 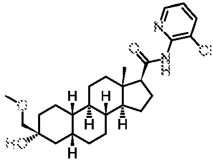 |
| 40 | 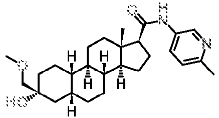 |
| 41 | 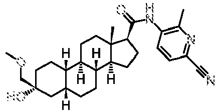 |
| 42 | 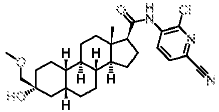 |
| 43 | 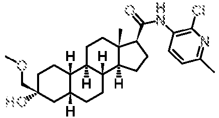 |
| 44 | 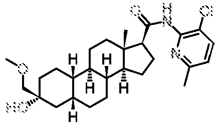 |
| 45 | 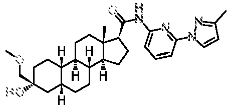 |
| 46 | 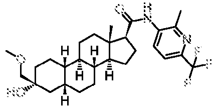 |
| 47 | 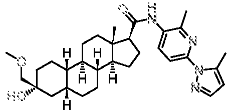 |
| 48 | 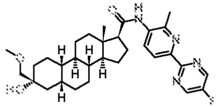 |
| 49 | 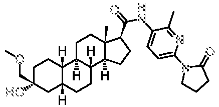 |
| 50 | 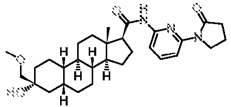 |
| 51 | 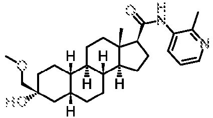 |
| 52 | 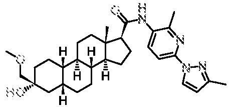 |
| 53 | 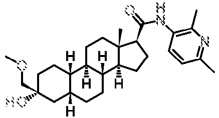 |
| 54 | 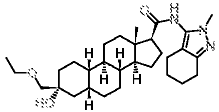 |
| 55 | 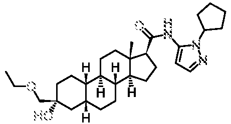 |
| 56 | 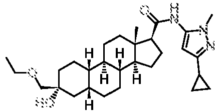 |
| 57 | 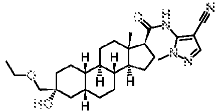 |
| 58 | 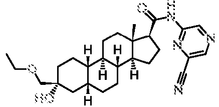 |
| 59 | 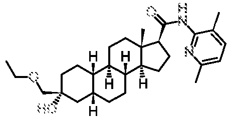 |
| 60 | 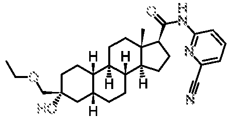 |
| 61 | 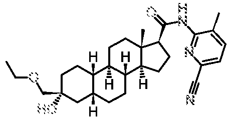 |
| 62 | 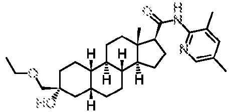 |
| 63 | 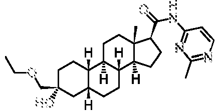 |
| 64 | 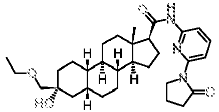 |
| 65 | 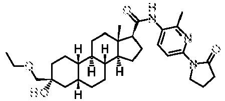 |
| 66 | 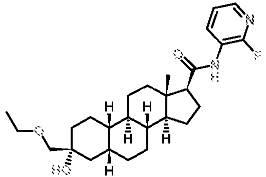 |
| 67 | 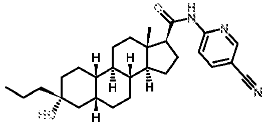 |
| 68 | 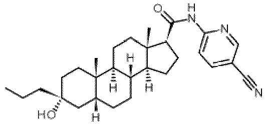 |

In one aspect, provided herein is a pharmaceutically acceptable salt of a compound described herein (*e.g.,* a compound of Formula (I)).

In one aspect, provided herein is a pharmaceutical composition comprising a compound described herein (*e.g.,* a compound of Formula (I)) or a pharmaceutically acceptable salt thereof, and a pharmaceutically acceptable excipient. In certain embodiments, the compound of the present invention is provided in an effective amount in the pharmaceutical composition. In certain embodiments, the compound of the present invention is provided in a therapeutically effective amount.

Compounds of the present invention as described herein, act, in certain embodiments, as GABA modulators, e.g., effecting the GABA_{A} receptor in either a positive or negative manner. As modulators of the excitability of the central nervous system (CNS), as mediated by their ability to modulate GABA_{A} receptor, such compounds are expected to have CNS-activity.

Thus, in another aspect, provided are methods of treating a CNS-related disorder in a subject in need thereof, comprising administering to the subject an effective amount of a compound of the present invention. In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder. In certain embodiments, the compound is administered orally, subcutaneously, intravenously, or intramuscularly. In certain embodiments, the compound is administered orally. In certain embodiments, the compound is administered chronically. In certain embodiments, the compound is administered continuously, *e.g.*, by continuous intravenous infusion.

Exemplary compounds of the invention may be synthesized from the following known starting materials using methods known to one skilled in the art or certain references, In one aspect, provided herein is a pharmaceutically acceptable salt of a compound described herein (*e.g.,* a compound of Formula (I)).

### Alternative Embodiments

In an alternative embodiment, compounds described herein may also comprise one or more isotopic substitutions. For example, hydrogen may be ²H (D or deuterium) or ³H (T or tritium); carbon may be, for example, ¹³C or ¹⁴C; oxygen may be, for example, ¹⁸O; nitrogen may be, for example, ¹⁵N, and the like. In other embodiments, a particular isotope (e.g., ³H, ¹³C, ¹⁴C, ¹⁸O, or ¹⁵N) can represent at least 1%, at least 5%, at least 10%, at least 15%, at least 20%, at least 25%, at least 30%, at least 35%, at least 40%, at least 45%, at least 50%, at least 60%, at least 65%, at least 70%, at least 75%, at least 80%, at least 85%, at least 90%, at least 95%, at least 99%, or at least 99.9% of the total isotopic abundance of an element that occupies a specific site of the compound.

### Pharmaceutical Compositions

In one aspect, provided herein is a pharmaceutical composition comprising a compound described herein (*e.g.,* a compound of Formula (I)) or a pharmaceutically acceptable salt thereof, and a pharmaceutically acceptable excipient. In certain embodiments, the compound of the present invention is provided in an effective amount in the pharmaceutical composition. In certain embodiments, the compound of the present invention is provided in a therapeutically effective amount.

In certain embodiments, the pharmaceutical composition comprises an effective amount of the active ingredient. In certain embodiments, the pharmaceutical composition comprises a therapeutically effective amount of the active ingredient.

The pharmaceutical compositions provided herein can be administered by a variety of routes including, but not limited to, oral (enteral) administration, parenteral (by injection) administration, rectal administration, transdermal administration, intradermal administration, intrathecal administration, subcutaneous (SC) administration, intravenous (IV) administration, intramuscular (IM) administration, and intranasal administration.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms, and the like.

When used to prevent the onset of a CNS-disorder, the compounds provided herein will be administered to a subject at risk for developing the condition, typically on the advice and under the supervision of a physician, at the dosage levels described above. Subjects at risk for developing a particular condition generally include those that have a family history of the condition, or those who have been identified by genetic testing or screening to be particularly susceptible to developing the condition.

The pharmaceutical compositions provided herein can also be administered chronically ("chronic administration"). Chronic administration refers to administration of a compound or pharmaceutical composition thereof over an extended period of time, e.g., for example, over 3 months, 6 months, 1 year, 2 years, 3 years, 5 years, etc, or may be continued indefinitely, for example, for the rest of the subject's life. In certain embodiments, the chronic administration is intended to provide a constant level of the compound in the blood, e.g., within the therapeutic window over the extended period of time.

The pharmaceutical compositions of the present invention may be further delivered using a variety of dosing methods. For example, in certain embodiments, the pharmaceutical composition may be given as a bolus, e.g., in order to raise the concentration of the compound in the blood to an effective level. The placement of the bolus dose depends on the systemic levels of the active ingredient desired throughout the body, e.g., an intramuscular or subcutaneous bolus dose allows a slow release of the active ingredient, while a bolus delivered directly to the veins (e.g., through an IV drip) allows a much faster delivery which quickly raises the concentration of the active ingredient in the blood to an effective level. In other embodiments, the pharmaceutical composition may be administered as a continuous infusion, e.g., by IV drip, to provide maintenance of a steady-state concentration of the active ingredient in the subject's body. Furthermore, in still yet other embodiments, the pharmaceutical composition may be administered as first as a bolus dose, followed by continuous infusion.

The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions or pills, tablets, capsules or the like in the case of solid compositions. In such compositions, the compound is usually a minor component (from about 0.1 to about 50% by weight or preferably from about 1 to about 40% by weight) with the remainder being various vehicles or excipients and processing aids helpful for forming the desired dosing form.

With oral dosing, one to five and especially two to four and typically three oral doses per day are representative regimens. Using these dosing patterns, each dose provides from about 0.01 to about 20 mg/kg of the compound provided herein, with preferred doses each providing from about 0.1 to about 10 mg/kg, and especially about 1 to about 5 mg/kg.

Transdermal doses are generally selected to provide similar or lower blood levels than are achieved using injection doses, generally in an amount ranging from about 0.01 to about 20% by weight, preferably from about 0.1 to about 20% by weight, preferably from about 0.1 to about 10% by weight, and more preferably from about 0.5 to about 15% by weight.

Injection dose levels range from about 0.1 mg/kg/hour to at least 20 mg/kg/hour, all for from about 1 to about 120 hours and especially 24 to 96 hours. A preloading bolus of from about 0.1 mg/kg to about 10 mg/kg or more may also be administered to achieve adequate steady state levels. The maximum total dose is not expected to exceed about 5 g/day for a 40 to 80 kg human patient.

Liquid forms suitable for oral administration may include a suitable aqueous or nonaqueous vehicle with buffers, suspending and dispensing agents, colorants, flavors and the like. Solid forms may include, for example, any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable excipients known in the art. As before, the active compound in such compositions is typically a minor component, often being from about 0.05 to 10% by weight with the remainder being the injectable excipient and the like.

Transdermal compositions are typically formulated as a topical ointment or cream containing the active ingredient(s). When formulated as an ointment, the active ingredients will typically be combined with either a paraffinic or a water-miscible ointment base. Alternatively, the active ingredients may be formulated in a cream with, for example an oil-in-water cream base. Such transdermal formulations are well-known in the art and generally include additional ingredients to enhance the dermal penetration of stability of the active ingredients or Formulation. All such known transdermal formulations and ingredients are included within the scope provided herein.

The compounds provided herein can also be administered by a transdermal device. Accordingly, transdermal administration can be accomplished using a patch either of the reservoir or porous membrane type, or of a solid matrix variety.

The above-described components for orally administrable, injectable or topically administrable compositions are merely representative. Other materials as well as processing techniques and the like are set forth in Part 8 of Remington's Pharmaceutical Sciences, 17th edition, 1985, Mack Publishing Company, Easton, Pennsylvania.

The compounds of the present invention can also be administered in sustained release forms or from sustained release drug delivery systems. A description of representative sustained release materials can be found in Remington's Pharmaceutical Sciences.

The present invention also relates to the pharmaceutically acceptable acid addition salt of a compound of the present invention. The acid which may be used to prepare the pharmaceutically acceptable salt is that which forms a non-toxic acid addition salt, i.e., a salt containing pharmacologically acceptable anions such as the hydrochloride, hydroiodide, hydrobromide, nitrate, sulfate, bisulfate, phosphate, acetate, lactate, citrate, tartrate, succinate, maleate, fumarate, benzoate, para-toluenesulfonate, and the like.

In another aspect, the invention provides a pharmaceutical composition comprising a compound of the present invention and a pharmaceutically acceptable excipient, e.g., a composition suitable for injection, such as for intravenous (IV) administration.

Pharmaceutically acceptable excipients include any and all diluents or other liquid vehicles, dispersion or suspension aids, surface active agents, isotonic agents, preservatives, lubricants and the like, as suited to the particular dosage form desired, e.g., injection. General considerations in the formulation and/or manufacture of pharmaceutical compositions agents can be found, for example, in Remington's Pharmaceutical Sciences, Sixteenth Edition, E. W. Martin (Mack Publishing Co., Easton, Pa., 1980), and Remington: The Science and Practice of Pharmacy, 21st Edition (Lippincott Williams & Wilkins, 2005).

For example, injectable preparations, such as sterile injectable aqueous suspensions, can be formulated according to the known art using suitable dispersing or wetting agents and suspending agents. Exemplary excipients that can be employed include, but are not limited to, water, sterile saline or phosphate-buffered saline, or Ringer's solution.

In certain embodiments, the pharmaceutical composition further comprises a cyclodextrin derivative. The most common cyclodextrins are α-, β- and γ- cyclodextrins consisting of 6, 7 and 8 α-1 ,4-linked glucose units, respectively, optionally comprising one or more substituents on the linked sugar moieties, which include, but are not limited to, substituted or unsubstituted methylated, hydroxyalkylated, acylated, and sulfoalkylether substitution. In certain embodiments, the cyclodextrin is a sulfoalkyl ether β-cyclodextrin, e.g., for example, sulfobutyl ether β-cyclodextrin, also known as CAPTISOL^{®}. See, e.g., U.S. 5,376,645. In certain embodiments, the composition comprises hexapropyl-β-cyclodextrin. In a more particular embodiment, the composition comprises hexapropyl-β-cyclodextrin (10-50% in water).

The injectable composition can be sterilized, for example, by filtration through a bacterial-retaining filter, or by incorporating sterilizing agents in the form of sterile solid compositions which can be dissolved or dispersed in sterile water or other sterile injectable medium prior to use.

Generally, the compounds provided herein are administered in an effective amount. The amount of the compound actually administered will typically be determined by a physician, in the light of the relevant circumstances, including the condition to be treated, the chosen route of administration, the actual compound administered, the age, weight, response of the individual patient, the severity of the patient's symptoms, and the like.

The compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampules or syringes of the liquid compositions. In such compositions, the compound is usually a minor component (from about 0.1% to about 50% by weight or preferably from about 1% to about 40% by weight) with the remainder being various vehicles or carriers and processing aids helpful for forming the desired dosing form.

The compounds provided herein can be administered as the sole active agent, or they can be administered in combination with other active agents. In one aspect, the present invention provides a combination of a compound of the present invention and another pharmacologically active agent. Administration in combination can proceed by any technique apparent to those of skill in the art including, for example, separate, sequential, concurrent, and alternating administration.

Although the descriptions of pharmaceutical compositions provided herein are principally directed to pharmaceutical compositions which are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation. General considerations in the formulation and/or manufacture of pharmaceutical compositions can be found, for example, in Remington: The Science and Practice of Pharmacy 21st ed., Lippincott Williams & Wilkins, 2005.

In one aspect, provided is a kit comprising a composition (e.g., a solid composition) comprising a compound of Formula (I).

### Medicinal indications

The references to methods of treatment in the summary and detailed description of the invention in this description are to be interpreted as references to the compounds, pharmaceutical compositions and medicaments of the present invention for use in a method for treatment of the human (or animal) body by therapy (or for diagnosis).

In an aspect, compounds described herein, e.g., compounds of Formula (I), are envisioned to be useful as therapeutic agents for treating a CNS-related disorder (e.g., sleep disorder, a mood disorder such as depression, a schizophrenia spectrum disorder, a convulsive disorder, epileptogenesis, a disorder of memory and/or cognition, a movement disorder, a personality disorder, autism spectrum disorder, pain, traumatic brain injury, a vascular disease, a substance abuse disorder and/or withdrawal syndrome, or tinnitus) in a subject in need (e.g., a subject with Rett syndrome, Fragile X syndrome, or Angelman syndrome). Exemplary CNS conditions related to GABA-modulation include, but are not limited to, sleep disorders [e.g., insomnia], mood disorders [e.g., depression (e.g., major depressive disorder (MDD)), dysthymic disorder (e.g., mild depression), bipolar disorder (e.g., I and/or II), anxiety disorders (e.g., generalized anxiety disorder (GAD), social anxiety disorder), stress, post-traumatic stress disorder (PTSD), compulsive disorders (e.g., obsessive compulsive disorder (OCD))], schizophrenia spectrum disorders [e.g., schizophrenia, schizoaffective disorder], convulsive disorders [e.g., epilepsy (e.g., status epilepticus (SE)), seizures], disorders of memory and/or cognition [e.g., attention disorders (e.g., attention deficit hyperactivity disorder (ADHD)), dementia (e.g., Alzheimer's type dementia, Lewis body type dementia, vascular type dementia], movement disorders [e.g., Huntington's disease, Parkinson's disease], personality disorders [e.g., anti-social personality disorder, obsessive compulsive personality disorder], autism spectrum disorders (ASD) [e.g., autism, monogenetic causes of autism such as synaptophathy's, e.g., Rett syndrome, Fragile X syndrome, Angelman syndrome], pain [e.g., neuropathic pain, injury related pain syndromes, acute pain, chronic pain], traumatic brain injury (TBI), vascular diseases [e.g., stroke, ischemia, vascular malformations], substance abuse disorders and/or withdrawal syndromes [e.g., addition to opiates, cocaine, and/or alcohol], and tinnitus.

In certain embodiments, CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus. In certain embodiments, the CNS-related disorder is depression. In certain embodiments, the CNS-related disorder is postpartum depression. In certain embodiments, the CNS-related disorder is major depressive disorder. In certain embodiments, the major depressive disorder is moderate major depressive disorder. In certain embodiments, the major depressive disorder is severe major depressive disorder.

In an aspect, provided is a method of alleviating or preventing seizure activity in a subject, comprising administering to the subject in need of such treatment an effective amount of a compound of the present invention. In some embodiments, the method alleviates or prevents epileptogenesis.

In yet another aspect, provided is a combination of a compound of the present invention and another pharmacologically active agent. The compounds provided herein can be administered as the sole active agent or they can be administered in combination with other agents. Administration in combination can proceed by any technique apparent to those of skill in the art including, for example, separate, sequential, concurrent and alternating administration.

In another aspect, provided is a method of treating or preventing brain excitability in a subject susceptible to or afflicted with a condition associated with brain excitability, comprising administering to the subject an effective amount of a compound of the present invention to the subject.

In yet another aspect, provided is a method of treating or preventing stress or anxiety in a subject, comprising administering to the subject in need of such treatment an effective amount of a compound of the present invention, or a composition thereof.

In yet another aspect, provided is a method of alleviating or preventing insomnia in a subject, comprising administering to the subject in need of such treatment an effective amount of a compound of the present invention, or a composition thereof.

In yet another aspect, provided is a method of inducing sleep and maintaining substantially the level of REM sleep that is found in normal sleep, wherein substantial rebound insomnia is not induced, comprising administering an effective amount of a compound of the present invention.

In yet another aspect, provided is a method of alleviating or preventing premenstrual syndrome (PMS) or postnatal depression (PND) in a subject, comprising administering to the subject in need of such treatment an effective amount of a compound of the present invention.

In yet another aspect, provided is a method of treating or preventing mood disorders in a subject, comprising administering to the subject in need of such treatment an effective amount of a compound of the present invention. In certain embodiments the mood disorder is depression.

In yet another aspect, provided is a method of cognition enhancement or treating memory disorder by administering to the subject a therapeutically effective amount of a compound of the present invention. In certain embodiments, the disorder is Alzheimer's disease. In certain embodiments, the disorder is Rett syndrome.

In yet another aspect, provided is a method of treating attention disorders by administering to the subject a therapeutically effective amount of a compound of the present invention. In certain embodiments, the attention disorder is ADHD.

In certain embodiments, the compound is administered to the subject chronically. In certain embodiments, the compound is administered to the subject orally, subcutaneously, intramuscularly, or intravenously.

### Neuroendocrine Disorders and Dysfunction

Provided herein are methods that can be used for treating neuroendocrine disorders and dysfunction. As used herein, "neuroendocrine disorder" or "neuroendocrine dysfunction" refers to a variety of conditions caused by imbalances in the body's hormone production directly related to the brain. Neuroendocrine disorders involve interactions between the nervous system and the endocrine system. Because the hypothalamus and the pituitary gland are two areas of the brain that regulate the production of hormones, damage to the hypothalamus or pituitary gland, e.g., by traumatic brain injury, may impact the production of hormones and other neuroendocrine functions of the brain. In some embodiments, the neuroendocrine disorder or dysfunction is associated with a women's health disorder or condition (e.g., a women's health disorder or condition described herein). In some embodiments, the neuroendocrine disorder or dysfunction is associated with a women's health disorder or condition is polycystic ovary syndrome.

Symptoms of neuroendocrine disorder include, but are not limited to, behavioral, emotional, and sleep-related symptoms, symptoms related to reproductive function, and somatic symptoms; including but not limited to fatigue, poor memory, anxiety, depression, weight gain or loss, emotional lability, lack of concentration, attention difficulties, loss of lipido, infertility, amenorrhea, loss of muscle mass, increased belly body fat, low blood pressure, reduced heart rate, hair loss, anemia, constipation, cold intolerance, and dry skin.

### Neurodegenerative Diseases and Disorders

The methods described herein can be used for treating neurodegenerative diseases and disorders. The term "neurodegenerative disease" includes diseases and disorders that are associated with the progressive loss of structure or function of neurons, or death of neurons. Neurodegenerative diseases and disorders include, but are not limited to, Alzheimer's disease (including the associated symptoms of mild, moderate, or severe cognitive impairment); amyotrophic lateral sclerosis (ALS); anoxic and ischemic injuries; ataxia and convulsion (including for the treatment and prevention and prevention of seizures that are caused by schizoaffective disorder or by drugs used to treat schizophrenia); benign forgetfulness; brain edema; cerebellar ataxia including McLeod neuroacanthocytosis syndrome (MLS); closed head injury; coma; contusive injuries (*e.g*., spinal cord injury and head injury); dementias including multi-infarct dementia and senile dementia; disturbances of consciousness; Down syndrome; drug-induced or medication-induced Parkinsonism (such as neuroleptic-induced acute akathisia, acute dystonia, Parkinsonism, or tardive dyskinesia, neuroleptic malignant syndrome, or medication-induced postural tremor); epilepsy; fragile X syndrome; Gilles de la Tourette's syndrome; head trauma; hearing impairment and loss; Huntington's disease; Lennox syndrome; levodopa-induced dyskinesia; mental retardation; movement disorders including akinesias and akinetic (rigid) syndromes (including basal ganglia calcification, corticobasal degeneration, multiple system atrophy, Parkinsonism-ALS dementia complex, Parkinson's disease, postencephalitic parkinsonism, and progressively supranuclear palsy); muscular spasms and disorders associated with muscular spasticity or weakness including chorea (such as benign hereditary chorea, drug-induced chorea, hemiballism, Huntington's disease, neuroacanthocytosis, Sydenham's chorea, and symptomatic chorea), dyskinesia (including tics such as complex tics, simple tics, and symptomatic tics), myoclonus (including generalized myoclonus and focal cyloclonus), tremor (such as rest tremor, postural tremor, and intention tremor) and dystonia (including axial dystonia, dystonic writer's cramp, hemiplegic dystonia, paroxysmal dystonia, and focal dystonia such as blepharospasm, oromandibular dystonia, and spasmodic dysphonia and torticollis); neuronal damage including ocular damage, retinopathy or macular degeneration of the eye; neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest; Parkinson's disease; seizure; status epilecticus; stroke; tinnitus; tubular sclerosis, and viral infection induced neurodegeneration (*e.g*., caused by acquired immunodeficiency syndrome (AIDS) and encephalopathies). Neurodegenerative diseases also include, but are not limited to, neurotoxic injury which follows cerebral stroke, thromboembolic stroke, hemorrhagic stroke, cerebral ischemia, cerebral vasospasm, hypoglycemia, amnesia, hypoxia, anoxia, perinatal asphyxia and cardiac arrest. Methods of treating or preventing a neurodegenerative disease also include treating or preventing loss of neuronal function characteristic of neurodegenerative disorder.

### Mood disorders

Also provided herein are methods for treating a mood disorder, for example clinical depression, postnatal depression or postpartum depression, perinatal depression, atypical depression, melancholic depression, psychotic major depression, cataonic depression, seasonal affective disorder, dysthymia, double depression, depressive personality disorder, recurrent brief depression, minor depressive disorder, bipolar disorder or manic depressive disorder, depression caused by chronic medical conditions, treatment-resistant depression, refractory depression, suicidality, suicidal ideation, or suicidal behavior. In some embodiments, the method described herein provides therapeutic effect to a subject suffering from depression (e.g., moderate or severe depression). In some embodiments, the mood disorder is associated with a disease or disorder described herein (e.g., neuroendocrine diseases and disorders, neurodegenerative diseases and disorders (e.g., epilepsy), movement disorders, tremor (e.g., Parkinson's Disease), women's health disorders or conditions).

**Clinical depression** is also known as major depression, major depressive disorder (MDD), severe depression, unipolar depression, unipolar disorder, and recurrent depression, and refers to a mental disorder characterized by pervasive and persistent low mood that is accompanied by low self-esteem and loss of interest or pleasure in normally enjoyable activities. Some people with clinical depression have trouble sleeping, lose weight, and generally feel agitated and irritable. Clinical depression affects how an individual feels, thinks, and behaves and may lead to a variety of emotional and physical problems. Individuals with clinical depression may have trouble doing day-to-day activities and make an individual feel as if life is not worth living.

**Peripartum depression** refers to depression in pregnancy. Symptoms include irritability, crying, feeling restless, trouble sleeping, extreme exhaustion (emotional and/or physical), changes in appetite, difficulty focusing, increased anxiety and/or worry, disconnected feeling from baby and/or fetus, and losing interest in formerly pleasurable activities.

**Postnatal** depression **(PND)** is also referred to as **postpartum depression** (**PPD**), and refers to a type of clinical depression that affects women after childbirth. Symptoms can include sadness, fatigue, changes in sleeping and eating habits, reduced sexual desire, crying episodes, anxiety, and irritability. In some embodiments, the PND is a treatment-resistant depression (e.g., a treatment-resistant depression as described herein). In some embodiments, the PND is refractory depression (e.g., a refractory depression as described herein).

In some embodiments, a subject having PND also experienced depression, or a symptom of depression during pregnancy. This depression is referred to herein as) **perinatal depression.** In an embodiment, a subject experiencing perinatal depression is at increased risk of experiencing PND.

**Atypical depression (AD)** is characterized by mood reactivity (*e.g*., paradoxical anhedonia) and positivity, significant weight gain or increased appetite. Patients suffering from AD also may have excessive sleep or somnolence (hypersomnia), a sensation of limb heaviness, and significant social impairment as a consequence of hypersensitivity to perceived interpersonal rejection.

**Melancholic depression** is characterized by loss of pleasure (anhedonia) in most or all activities, failures to react to pleasurable stimuli, depressed mood more pronounced than that of grief or loss, excessive weight loss, or excessive guilt.

**Psychotic major depression (PMD)** or psychotic depression refers to a major depressive episode, in particular of melancholic nature, where the individual experiences psychotic symptoms such as delusions and hallucinations.

**Catatonic depression** refers to major depression involving disturbances of motor behavior and other symptoms. An individual may become mute and stuporose, and either is immobile or exhibits purposeless or bizarre movements.

**Seasonal affective disorder (SAD)** refers to a type of seasonal depression wherein an individual has seasonal patterns of depressive episodes coming on in the fall or winter.

**Dysthymia** refers to a condition related to unipolar depression, where the same physical and cognitive problems are evident. They are not as severe and tend to last longer (*e.g.,* at least 2 years).

**Double depression** refers to fairly depressed mood (dysthymia) that lasts for at least 2 years and is punctuated by periods of major depression.

**Depressive Personality Disorder (DPD)** refers to a personality disorder with depressive features.

**Recurrent Brief Depression (RBD)** refers to a condition in which individuals have depressive episodes about once per month, each episode lasting 2 weeks or less and typically less than 2-3 days.

**Minor depressive disorder** or minor depression refers to a depression in which at least 2 symptoms are present for 2 weeks.

**Bipolar disorder or manic depressive disorder** causes extreme mood swings that include emotional highs (mania or hypomania) and lows (depression). During periods of mania the individual may feel or act abnormally happy, energetic, or irritable. They often make poorly thought out decisions with little regard to the consequences. The need for sleep is usually reduced. During periods of depression there may be crying, poor eye contact with others, and a negative outlook on life. The risk of suicide among those with the disorder is high at greater than 6% over 20 years, while self-harm occurs in 30-40%. Other mental health issues such as anxiety disorder and substance use disorder are commonly associated with bipolar disorder.

**Depression caused by chronic medical conditions** refers to depression caused by chronic medical conditions such as cancer or chronic pain, chemotherapy, chronic stress.

**Treatment-resistant depression** refers to a condition where the individuals have been treated for depression, but the symptoms do not improve. For example, antidepressants or physchological counseling (psychotherapy) do not ease depression symptoms for individuals with treatment-resistant depression. In some cases, individuals with treatment-resistant depression improve symptoms, but come back. **Refractory depression** occurs in patients suffering from depression who are resistant to standard pharmacological treatments, including tricyclic antidepressants, MAOIs, SSRIs, and double and triple uptake inhibitors and/or anxiolytic drugs, as well as non-pharmacological treatments (e.g., psychotherapy, electroconvulsive therapy, vagus nerve stimulation and/or transcranial magnetic stimulation).

**Post-surgical depression** refers to feelings of depression that follow a surgical procedure (e.g., as a result of having to confront one's mortality). For example, individuals may feel sadness or empty mood persistently, a loss of pleasure or interest in hobbies and activities normally enjoyed, or a persistent felling of worthlessness or hopelessness.

**Mood disorder associated with conditions or disorders of women's health** refers to mood disorders (e.g., depression) associated with (e.g., resulting from) a condition or disorder of women's health (e.g., as described herein).

**Suicidality, suicidal ideation, suicidal behavior** refers to the tendency of an individual to commit suicide. Suicidal ideation concerns thoughts about or an unusual preoccupation with suicide. The range of suicidal ideation varies greatly, from e.g., fleeting thoughts to extensive thoughts, detailed planning, role playing, incomplete attempts. Symptoms include talking about suicide, getting the means to commit suicide, withdrawing from social contact, being preoccupied with death, feeling trapped or hopeless about a situation, increasing use of alcohol or drugs, doing risky or self-destructive things, saying goodbye to people as if they won't be seen again.

**Symptoms** of depression include persistent anxious or sad feelings, feelings of helplessness, hopelessness, pessimism, worthlessness, low energy, restlessness, difficulty sleeping, sleeplessness, irritability, fatigue, motor challenges, loss of interest in pleasurable activities or hobbies, loss of concentration, loss of energy, poor self-esteem, absence of positive thoughts or plans, excessive sleeping, overeating, appetite loss, insomnia, self-harm, thoughts of suicide, and suicide attempts. The presence, severity, frequency, and duration of symptoms may vary on a case to case basis. Symptoms of depression, and relief of the same, may be ascertained by a physician or psychologist (e.g., by a mental state examination).

In some embodiments, the method comprises monitoring a subject with a known depression scale, *e.g.,* the Hamilton Depression (HAM-D) scale, the Clinical Global Impression-Improvement Scale (CGI), and the Montgomery-Åsberg Depression Rating Scale (MADRS). In some embodiments, a therapeutic effect can be determined by reduction in Hamilton Depression (HAM-D) total score exhibited by the subject. Reduction in the HAM-D total score can happen within 4, 3, 2, or 1 days; or 96, 84, 72, 60, 48, 24, 20, 16, 12, 10, 8 hours or less. The therapeutic effect can be assessed across a specified treatment period. For example, the therapeutic effect can be determined by a decrease from baseline in HAM-D total score after administering a compound described herein, *e.g.,* a compound of Formula (I) (e.g., 12, 24, or 48 hours after administration; or 24, 48, 72, or 96 hours or more; or 1 day, 2 days, 14 days, 21 days, or 28 days; or 1 week, 2 weeks, 3 weeks, or 4 weeks; or 1 month, 2 months, 6 months, or 10 months; or 1 year, 2 years, or for life).

In some embodiments, the subject has a mild depressive disorder, *e.g*., mild major depressive disorder. In some embodiments, the subject has a moderate depressive disorder, *e.g*., moderate major depressive disorder. In some embodiments, the subject has a severe depressive disorder, *e.g*., severe major depressive disorder. In some embodiments, the subject has a very severe depressive disorder, *e.g*., very severe major depressive disorder. In some embodiments, the baseline HAM-D total score of the subject (i.e., prior to treatment with a compound described herein, *e.g.,* a compound of Formula (I)) is at least 24. In some embodiments, the baseline HAM-D total score of the subject is at least 18. In some embodiments, the baseline HAM-D total score of the subject is between and including 14 and 18. In some embodiments, the baseline HAM-D total score of the subject is between and including 19 and 22. In some embodiments, the HAM-D total score of the subject before treatment with a compound described herein, *e.g.,* a compound of Formula (I), is greater than or equal to 23. In some embodiments, the baseline score is at least 10, 15, or 20. In some embodiments, the HAM-D total score of the subject after treatment with a compound described herein, *e.g.,* a compound of Formula (I), is about 0 to 10 (e.g., less than 10; 0 to 10, 0 to 6, 0 to 4, 0 to 3, 0 to 2, or 1.8). In some embodiments, the HAM-D total score after treatment with a compound described herein, *e.g.,* a compound of Formula (I), is less than 10, 7, 5, or 3. In some embodiments, the decrease in HAM-D total score is from a baseline score of about 20 to 30 (e.g., 22 to 28, 23 to 27, 24 to 27, 25 to 27, 26 to 27) to a HAM-D total score at about 0 to 10 (e.g., less than 10; 0 to 10, 0 to 6, 0 to 4, 0 to 3, 0 to 2, or 1.8) after treatment with a compound described herein, *e.g.,* a compound of Formula (I). In some embodiments, the decrease in the baseline HAM-D total score to HAM-D total score after treatment with a compound described herein, *e.g.,* a compound of Formula (I), is at least 1, 2, 3, 4, 5, 7, 10, 25, 40, 50, or 100 fold). In some embodiments, the percentage decrease in the baseline HAM-D total score to HAM-D total score after treatment with a compound described herein, *e.g.,* a compound of Formula (I), is at least 50% (e.g., 60%, 70%, 80%, or 90%). In some embodiments, the therapeutic effect is measured as a decrease in the HAM-D total score after treatment with a compound described herein, *e.g.,* a compound of Formula (I), relative to the baseline HAM-D total score (e.g., 12, 24, 48 hours after administration; or 24, 48, 72, 96 hours or more; or 1 day, 2 days, 14 days, or more) is at least 10, 15, or 20 points.

In some embodiments, the method of treating a depressive disorder, e.g., major depressive disorder provides a therapeutic effect (e.g., as measured by reduction in Hamilton Depression Score (HAM-D)) within 14, 10, 4, 3, 2, or 1 days, or 24, 20, 16, 12, 10, or 8 hours or less. In some embodiments, the method of treating the depressive disorder, e.g., major depressive disorder, provides a therapeutic effect (*e.g.,* as determined by a statistically significant reduction in HAM-D total score) within the first or second day of the treatment with a compound described herein, *e.g.,* a compound of Formula (I). In some embodiments, the method of treating the depressive disorder, e.g., major depressive disorder, provides a therapeutic effect (*e.g.,* as determined by a statistically significant reduction in HAM-D total score) within less than or equal to 14 days since the beginning of the treatment with a compound described herein, *e.g.,* a compound of Formula (I). In some embodiments, the method of treating the depressive disorder, e.g., major depressive disorder, provides a therapeutic effect (*e.g.,* as determined by a statistically significant reduction in HAM-D total score) within less than or equal to 21 days since the beginning of the treatment with a compound described herein, *e.g.,* a compound of Formula (I). In some embodiments, the method of treating the depressive disorder, e.g., major depressive disorder, provides a therapeutic effect (*e.g.,* as determined by a statistically significant reduction in HAM-D total score) within less than or equal to 28 days since the beginning of the treatment with a compound described herein, *e.g.,* a compound of Formula (I). In some embodiments, the therapeutic effect is a decrease from baseline in HAM-D total score after treatment with a compound described herein, *e.g.,* a compound of Formula (I) (e.g., treatment with a compound described herein, *e.g.,* a compound of Formula (I), once a day for 14 days). In some embodiments, the HAM-D total score of the subject before treatment with a compound described herein, *e.g.,* a compound of Formula (I), is at least 24. In some embodiments, the HAM-D total score of the subject before treatment with a compound described herein, *e.g.,* a compound of Formula (I), is at least 18. In some embodiments, the HAM-D total score of the subject before treatment with a compound described herein, *e.g.,* a compound of Formula (I), is between and including 14 and 18. In some embodiments, the decrease in HAM-D total score after treating the subject with a compound described herein, *e.g.,* a compound of Formula (I), relative to the baseline HAM-D total score is at least 10. In some embodiments, the decrease in HAM-D total score after treating the subject with a compound described herein, *e.g.,* a compound of Formula (I), relative to the baseline HAM-D total score is at least 15 (*e.g.,* at least 17). In some embodiments, the HAM-D total score associated with treating the subject with a compound described herein, *e.g.,* a compound of Formula (I), is no more than a number ranging from 6 to 8. In some embodiments, the HAM-D total score associated with treating the subject with a compound described herein, *e.g.,* a compound of Formula (I), is no more than 7.

In some embodiments, the method provides therapeutic effect (e.g., as measured by reduction in Clinical Global Impression-Improvement Scale (CGI)) within 14, 10, 4, 3, 2, or 1 days, or 24, 20, 16, 12, 10, or 8 hours or less. In some embodiments, the CNS-disorder is a depressive disorder, *e.g*., major depressive disorder. In some embodiments, the method of treating the depressive disorder, *e.g.,* major depressive disorder provides a therapeutic effect within the second day of the treatment period. In some embodiments, the therapeutic effect is a decrease from baseline in CGI score at the end of a treatment period (e.g., 14 days after administration).

In some embodiments, the method provides therapeutic effect (e.g., as measured by reduction in Montgomery-Åsberg Depression Rating Scale (MADRS)) within 14, 10, 4, 3, 2, or 1 days, or 24, 20, 16, 12, 10, or 8 hours or less. In some embodiments, the CNS-disorder is a depressive disorder, *e.g.,* major depressive disorder. In some embodiments, the method of treating the depressive disorder, *e.g.,* major depressive disorder provides a therapeutic effect within the second day of the treatment period. In some embodiments, the therapeutic effect is a decrease from baseline in MADRS score at the end of a treatment period (e.g., 14 days after administration).

A therapeutic effect for major depressive disorder can be determined by a reduction in Montgomery-Åsberg Depression Rating Scale (MADRS) score exhibited by the subject. For example, the MADRS score can be reduced within 4, 3, 2, or 1 days; or 96, 84, 72, 60, 48, 24, 20, 16, 12, 10, 8 hours or less. The Montgomery-Åsberg Depression Rating Scale (MADRS) is a ten-item diagnostic questionnaire (regarding apparent sadness, reported sadness, inner tension, reduced sleep, reduced appetite, concentration difficulties, lassitude, inability to feel, pessimistic thoughts, and suicidal thoughts) which psychiatrists use to measure the severity of depressive episodes in patients with mood disorders.

In some embodiments, the method provides therapeutic effect (e.g., as measured by reduction in Edinburgh Postnatal Depression Scale (EPDS)) within 4, 3, 2, 1 days; 24, 20, 16, 12, 10, 8 hours or less. In some embodiments, the therapeutic effect is an improvement measured by the EPDS.

In some embodiments, the method provides therapeutic effect (e.g., as measured by reduction in Generalized Anxiety Disorder 7-Item Scale (GAD-7)) within 4, 3, 2, 1 days; 24, 20, 16, 12, 10, 8 hours or less.

### Anxiety Disorders

Provided herein are methods for treating anxiety disorders (e.g., generalized anxiety disorder, panic disorder, obsessive compulsive disorder, phobia, post-traumatic stress disorder). **Anxiety disorder** is a blanket term covering several different forms of abnormal and pathological fear and anxiety. Current psychiatric diagnostic criteria recognize a wide variety of anxiety disorders.

**Generalized anxiety disorder** is a common chronic disorder characterized by long-lasting anxiety that is not focused on any one object or situation. Those suffering from generalized anxiety experience non-specific persistent fear and worry and become overly concerned with everyday matters. Generalized anxiety disorder is the most common anxiety disorder to affect older adults.

In **panic disorder,** a person suffers from brief attacks of intense terror and apprehension, often marked by trembling, shaking, confusion, dizziness, nausea, difficulty breathing. These panic attacks, defined by the APA as fear or discomfort that abruptly arises and peaks in less than ten minutes, can last for several hours and can be triggered by stress, fear, or even exercise; although the specific cause is not always apparent. In addition to recurrent unexpected panic attacks, a diagnosis of panic disorder also requires that said attacks have chronic consequences: either worry over the attacks' potential implications, persistent fear of future attacks, or significant changes in behavior related to the attacks. Accordingly, those suffering from panic disorder experience symptoms even outside of specific panic episodes. Often, normal changes in heartbeat are noticed by a panic sufferer, leading them to think something is wrong with their heart or they are about to have another panic attack. In some cases, a heightened awareness (hypervigilance) of body functioning occurs during panic attacks, wherein any perceived physiological change is interpreted as a possible life threatening illness (i.e. extreme hypochondriasis).

**Obsessive compulsive disorder** is a type of anxiety disorder primarily characterized by repetitive obsessions (distressing, persistent, and intrusive thoughts or images) and compulsions (urges to perform specific acts or rituals). The OCD thought pattern may be likened to superstitions insofar as it involves a belief in a causative relationship where, in reality, one does not exist. Often the process is entirely illogical; for example, the compulsion of walking in a certain pattern may be employed to alleviate the obsession of impending harm. And in many cases, the compulsion is entirely inexplicable, simply an urge to complete a ritual triggered by nervousness. In a minority of cases, sufferers of OCD may only experience obsessions, with no overt compulsions; a much smaller number of sufferers experience only compulsions.

The single largest category of anxiety disorders is that of **phobia,** which includes all cases in which fear and anxiety is triggered by a specific stimulus or situation. Sufferers typically anticipate terrifying consequences from encountering the object of their fear, which can be anything from an animal to a location to a bodily fluid.

**Post-traumatic stress disorder** or **PTSD** is an anxiety disorder which results from a traumatic experience. Post-traumatic stress can result from an extreme situation, such as combat, rape, hostage situations, or even serious accident. It can also result from long term (chronic) exposure to a severe stressor, for example soldiers who endure individual battles but cannot cope with continuous combat. Common symptoms include flashbacks, avoidant behaviors, and depression.

### Women's Health Disorders

Provided herein are methods for treating conditions or disorders related to women's health. Conditions or disorders related to women's health include, but are not limited to, gynecological health and disorders (e.g., premenstrual syndrome (PMS), premenstrual dysphoric disorder (PMDD)), **pregnancy issues** (e.g., miscarriage, abortion), infertility and related disorders (e.g., polycystic ovary syndrome (PCOS)), other disorders and conditions, and issues related to women's overall health and wellness (e.g., menopause).

**Gynecological health and disorders** affecting women include menstruation and menstrual irregularities; urinary tract health, including urinary incontinence and pelvic floor disorders; and such disorders as bacterial vaginosis, vaginitis, uterine fibroids, and vulvodynia.

**Premenstrual syndrome (PMS)** refers to physical and emotional symptoms that occur in the one to two weeks before a women's period. Symptoms vary but can include bleeding, mood swings, tender breasts, food cravings, fatigue, irritability, acne, and depression.

**Premenstrual dysphoric disorder (PMDD)** is a severe form of PMS. The symptoms of PMDD are similar to PMS but more severe and may interfere with work, social activity, and relationships. PMDD symptoms include mood swings, depressed mood or feelings of hopelessness, marked anger, increased interpersonal conflicts, tension and anxiety, irritability, decreased interest in usual activities, difficulty concentrating, fatigue, change in appetite, feeling out of control or overwhelmed, sleep problems, physical problems (e.g., bloating, breast tenderness, swelling, headaches, joint or muscle pain).

**Pregnancy issues** include preconception care and prenatal care, pregnancy loss (miscarriage and stillbirth), preterm labor and premature birth, sudden infant death syndrome (SIDS), breastfeeding, and birth defects.

**Miscarriage** refers to a pregnancy that ends on its own, within the first 20 weeks of gestation.

**Abortion** refers to the deliberate termination of a pregnancy, which can be performed during the first 28 weeks of pregnancy.

**Infertility and related disorders** include uterine fibroids, polycystic ovary syndrome, endometriosis, and primary ovarian insufficiency.

**Polycystic ovary syndrome (PCOS)** refers to an endocrine system disorder among women of reproductive age. PCOS is a set of symptoms resulting from an elevated male hormone in women. Most women with PCOS grow many small cysts on their ovaries. Symptoms of PCOS include irregular or no menstrual periods, heavy periods, excess body and facial hair, acne, pelvic pain, difficulty getting pregnant, and patches of thick, darker, velvety skin. PCOS may be associated with conditions including type 2 diabetes, obesity, obstructive sleep apnea, heart disease, mood disorders, and endometrial cancer.

**Other disorders and conditions** that affect only women include Turner syndrome, Rett syndrome, and ovarian and cervical cancers.

**Issues related to women's overall health and wellness** include violence against women, women with disabilities and their unique challenges, osteoporosis and bone health, and menopause.

**Menopause** refers to the 12 months after a woman's last menstrual period and marks the end of menstrual cycles. Menopause typically occurs in a woman's 40s or 50s. Physical symptoms such as hot flashes and emotional symptoms of menopause may disrupt sleep, lower energy, or trigger anxiety or feelings of sadness or loss. Menopause includes natural menopause and surgical menopause, which is a type of induced menopause due to an event such as surgery (e.g., hysterectomy, oophorectomy; cancer). It is induced when the ovaries are gravely damaged by, e.g., radiation, chemotherapy, or other medications.

### Epilepsy

The compound of Formula (I), or pharmaceutically acceptable salt, or a pharmaceutically acceptable composition thereof, can be used in a method described herein, for example in the treatment of a disorder described herein such as epilepsy, status epilepticus, or seizure.

Epilepsy is a brain disorder characterized by repeated seizures over time. Types of epilepsy can include, but are not limited to generalized epilepsy, e.g., childhood absence epilepsy, juvenile nyoclonic epilepsy, epilepsy with grand-mal seizures on awakening, West syndrome, Lennox-Gastaut syndrome, partial epilepsy, e.g., temporal lobe epilepsy, frontal lobe epilepsy, benign focal epilepsy of childhood.

### Epileptogenesis

The compounds and methods described herein can be used to treat or prevent epileptogenesis. Epileptogenesis is a gradual process by which a normal brain develops epilepsy (a chronic condition in which seizures occur). Epileptogenesis results from neuronal damage precipitated by the initial insult (e.g., status epilepticus).

### Status epilepticus (SE)

Status epilepticus (SE) can include, *e.g.,* convulsive status epilepticus, *e.g.,* early status epilepticus, established status epilepticus, refractory status epilepticus, super-refractory status epilepticus; non-convulsive status epilepticus, *e.g*., generalized status epilepticus, complex partial status epilepticus; generalized periodic epileptiform discharges; and periodic lateralized epileptiform discharges. Convulsive status epilepticus is characterized by the presence of convulsive status epileptic seizures, and can include early status epilepticus, established status epilepticus, refractory status epilepticus, super-refractory status epilepticus. Early status epilepticus is treated with a first line therapy. Established status epilepticus is characterized by status epileptic seizures which persist despite treatment with a first line therapy, and a second line therapy is administered. Refractory status epilepticus is characterized by status epileptic seizures which persist despite treatment with a first line and a second line therapy, and a general anesthetic is generally administered. Super refractory status epilepticus is characterized by status epileptic seizures which persist despite treatment with a first line therapy, a second line therapy, and a general anesthetic for 24 hours or more.

Non-convulsive status epilepticus can include, *e.g.,* focal non-convulsive status epilepticus, *e.g*., complex partial non-convulsive status epilepticus, simple partial non-convulsive status epilepticus, subtle non-convulsive status epilepticus; generalized non-convulsive status epilepticus, *e.g*., late onset absence non-convulsive status epilepticus, atypical absence non-convulsive status epilepticus, or typical absence non-convulsive status epilepticus.

The compound of Formula (I) or pharmaceutically acceptable salt, or a pharmaceutically acceptable composition thereof, can also be administered as a prophylactic to a subject having a CNS disorder *e.g.,* a traumatic brain injury, status epilepticus, *e.g.,* convulsive status epilepticus, *e.g*., early status epilepticus, established status epilepticus, refractory status epilepticus, super-refractory status epilepticus; non-convulsive status epilepticus, *e.g*., generalized status epilepticus, complex partial status epilepticus; generalized periodic epileptiform discharges; and periodic lateralized epileptiform discharges; prior to the onset of a seizure.

### Seizure

A seizure is the physical findings or changes in behavior that occur after an episode of abnormal electrical activity in the brain. The term "seizure" is often used interchangeably with "convulsion." Convulsions are when a person's body shakes rapidly and uncontrollably. During convulsions, the person's muscles contract and relax repeatedly.

Based on the type of behavior and brain activity, seizures are divided into two broad categories: generalized and partial (also called local or focal). Classifying the type of seizure helps doctors diagnose whether or not a patient has epilepsy.

Generalized seizures are produced by electrical impulses from throughout the entire brain, whereas partial seizures are produced (at least initially) by electrical impulses in a relatively small part of the brain. The part of the brain generating the seizures is sometimes called the focus.

There are six types of generalized seizures. The most common and dramatic, and therefore the most well-known, is the generalized convulsion, also called the grand-mal seizure. In this type of seizure, the patient loses consciousness and usually collapses. The loss of consciousness is followed by generalized body stiffening (called the "tonic" phase of the seizure) for 30 to 60 seconds, then by violent jerking (the "clonic" phase) for 30 to 60 seconds, after which the patient goes into a deep sleep (the "postictal" or after-seizure phase). During grand-mal seizures, injuries and accidents may occur, such as tongue biting and urinary incontinence.

Absence seizures cause a short loss of consciousness (just a few seconds) with few or no symptoms. The patient, most often a child, typically interrupts an activity and stares blankly. These seizures begin and end abruptly and may occur several times a day. Patients are usually not aware that they are having a seizure, except that they may be aware of "losing time."

Myoclonic seizures consist of sporadic jerks, usually on both sides of the body. Patients sometimes describe the jerks as brief electrical shocks. When violent, these seizures may result in dropping or involuntarily throwing objects.

Clonic seizures are repetitive, rhythmic jerks that involve both sides of the body at the same time.

Tonic seizures are characterized by stiffening of the muscles.

Atonic seizures consist of a sudden and general loss of muscle tone, particularly in the arms and legs, which often results in a fall.

Seizures described herein can include epileptic seizures; acute repetitive seizures; cluster seizures; continuous seizures; unremitting seizures; prolonged seizures; recurrent seizures; status epilepticus seizures, e.g., refractory convulsive status epilepticus, non-convulsive status epilepticus seizures; refractory seizures; myoclonic seizures; tonic seizures; tonic-clonic seizures; simple partial seizures; complex partial seizures; secondarily generalized seizures; atypical absence seizures; absence seizures; atonic seizures; benign Rolandic seizures; febrile seizures; emotional seizures; focal seizures; gelastic seizures; generalized onset seizures; infantile spasms; Jacksonian seizures; massive bilateral myoclonus seizures; multifocal seizures; neonatal onset seizures; nocturnal seizures; occipital lobe seizures; post traumatic seizures; subtle seizures; Sylvan seizures; visual reflex seizures; or withdrawal seizures. In some embodiments, the seizure is a generalized seizure associated with Dravet Syndrome, Lennox-Gastaut Syndrome, Tuberous Sclerosis Complex, Rett Syndrome or PCDH19 Female Pediatric Epilepsy.

### Movement Disorders

Also described herein are methods for treating a movement disorder. As used herein, "movement disorders" refers to a variety of diseases and disorders that are associated with hyperkinetic movement disorders and related abnormalities in muscle control. Exemplary movement disorders include, but are not limited to, Parkinson's disease and parkinsonism (defined particularly by bradykinesia), dystonia, chorea and Huntington's disease, ataxia, tremor (e.g., essential tremor), myoclonus and startle, tics and Tourette syndrome, Restless legs syndrome, stiff person syndrome, and gait disorders.

### Tremor

The methods described herein can be used to treat tremor, for example the compound of Formula (I) can be used to treat cerebellar tremor or intention tremor, dystonic tremor, essential tremor, orthostatic tremor, parkinsonian tremor, physiological tremor, psychogenic tremor, or rubral tremor. Tremor includes hereditary, degenerative, and idiopathic disorders such as Wilson's disease, Parkinson's disease, and essential tremor, respectively; metabolic diseases (e.g., thyroid-parathyroid-, liver disease and hypoglycemia); peripheral neuropathies (associated with Charcot-Marie-Tooth, Roussy-Levy, diabetes mellitus, complex regional pain syndrome); toxins (nicotine, mercury, lead, CO, Manganese, arsenic, toluene); drug-induced (narcoleptics, tricyclics, lithium, cocaine, alcohol, adrenaline, bronchodilators, theophylline, caffeine, steroids, valproate, amiodarone, thyroid hormones, vincristine); and psychogenic disorders. Clinical tremor can be classified into physiologic tremor, enhanced physiologic tremor, essential tremor syndromes (including classical essential tremor, primary orthostatic tremor, and task- and position-specific tremor), dystonic tremor, parkinsonian tremor, cerebellar tremor, Holmes' tremor (i.e., rubral tremor), palatal tremor, neuropathic tremor, toxic or drug-induced tremor, and psychogenic tremor.

**Tremor** is an involuntary, at times rhythmic, muscle contraction and relaxation that can involve oscillations or twitching of one or more body parts (*e.g*., hands, arms, eyes, face, head, vocal folds, trunk, legs).

**Cerebellar tremor** or **intention tremor** is a slow, broad tremor of the extremities that occurs after a purposeful movement. Cerebellar tremor is caused by lesions in or damage to the cerebellum resulting from, *e.g.,* tumor, stroke, disease (*e.g.,* multiple sclerosis, an inherited degenerative disorder).

**Dystonic tremor** occurs in individuals affected by dystonia, a movement disorder in which sustained involuntary muscle contractions cause twisting and repetitive motions and/or painful and abnormal postures or positions. Dystonic tremor may affect any muscle in the body. Dystonic tremors occurs irregularly and often can be relieved by complete rest.

**Essential tremor** or benign essential tremor is the most common type of tremor. Essential tremor may be mild and nonprogressive in some, and may be slowly progressive, starting on one side of the body but affect both sides within 3 years. The hands are most often affected, but the head, voice, tongue, legs, and trunk may also be involved. Tremor frequency may decrease as the person ages, but severity may increase. Heightened emotion, stress, fever, physical exhaustion, or low blood sugar may trigger tremors and/or increase their severity. Symptoms generally evolve over time and can be both visible and persistent following onset.

**Orthostatic tremor** is characterized by fast (*e.g.,* greater than 12 Hz) rhythmic muscle contractions that occurs in the legs and trunk immediately after standing. Cramps are felt in the thighs and legs and the patient may shake uncontrollably when asked to stand in one spot. Orthostatic tremor may occurs in patients with essential tremor.

**Parkinsonian tremor** is caused by damage to structures within the brain that control movement. Parkinsonian tremor is often a precursor to Parkinson's disease and is typically seen as a "pill-rolling" action of the hands that may also affect the chin, lips, legs, and trunk. Onset of parkinsonian tremor typically begins after age 60. Movement starts in one limb or on one side of the body and can progress to include the other side.

**Physiological tremor** can occur in normal individuals and have no clinical significance. It can be seen in all voluntary muscle groups. Physiological tremor can be caused by certain drugs, alcohol withdrawal, or medical conditions including an overactive thyroid and hypoglycemia. The tremor classically has a frequency of about 10 Hz.

**Psychogenic tremor** or hysterical tremor can occur at rest or during postural or kinetic movement. Patient with psychogenic tremor may have a conversion disorder or another psychiatric disease.

**Rubral tremor** is characterized by coarse slow tremor which can be present at rest, at posture, and with intention. The tremor is associated with conditions that affect the red nucleus in the midbrain, classical unusual strokes.

**Parkinson's Disease** affects nerve cells in the brain that produce dopamine. Symptoms include muscle rigidity, tremors, and changes in speech and gait. **Parkinsonism** is characterized by tremor, bradykinesia, rigidity, and postural instability. Parkinsonism shares symptoms found in Parkinson's Disease, but is a symptom complex rather than a progressive neurodegenerative disease.

**Dystonia** is a movement disorder characterized by sustained or intermittent muscle contractions causing abnormal, often repetitive movements or postures. Dystonic movements can be patterned, twisting, and may be tremulous. Dystonia is often initiated or worsened by voluntary action and associated with overflow muscle activation.

**Chorea** is a neurological disorder characterized by jerky involuntary movements typically affecting the shoulders, hips, and face. **Huntington's Disease** is an inherited disease that causes nerve cells in the brain to waste away. Symptoms include uncontrolled movements, clumsiness, and balance problems. Huntington's disease can hinder walk, talk, and swallowing.

**Ataxia** refers to the loss of full control of bodily movements, and may affect the fingers, hands, arms, legs, body, speech, and eye movements.

**Myloclonus and Startle** is a response to a sudden and unexpected stimulus, which can be acoustic, tactile, visual, or vestibular.

Tics are an involuntary movement usually onset suddenly, brief, repetitive, but non-rhythmical, typically imitating normal behavior and often occurring out of a background of normal activity. Tics can be classified as motor or vocal, motor tics associated with movements while vocal tics associated with sound. Tics can be characterized as simple or complex. For example simple motor tics involve only a few muscles restricted to a specific body part. **Tourette Syndrome** is an inherited neuropsychiatric disorder with onset in childhood, characterized by multiple motor tics and at least one vocal tic.

**Restless Legs Syndrome** is a neurologic sensorimotor disorder characterized by an overwhelming urge to move the legs when at rest.

**Stiff Person Syndrome** is a progressive movement disorder characterized by involuntary painful spasms and rigidity of muscles, usually involving the lower back and legs. Stiff-legged gait with exaggerated lumbar hyperlordosis typically results. Characteristic abnormality on EMG recordings with continuous motor unit activity of the paraspinal axial muscles is typically observed. Variants include "stiff-limb syndrome" producing focal stiffness typically affecting distal legs and feet.

**Gait disorders** refer to an abnormality in the manner or style of walking, which results from neuromuscular, arthritic, or other body changes. Gait is classified according to the system responsible for abnormal locomotion, and include hemiplegic gait, diplegic gait, neuropathic gait, myopathic gait, parkinsonian gait, choreiform gait, ataxic gait, and sensory gait.

### Anesthesia / Sedation

Anesthesia is a pharmacologically induced and reversible state of amnesia, analgesia, loss of responsiveness, loss of skeletal muscle reflexes, decreased stress response, or all of these simultaneously. These effects can be obtained from a single drug which alone provides the correct combination of effects, or occasionally with a combination of drugs (*e.g.,* hypnotics, sedatives, paralytics, analgesics) to achieve very specific combinations of results. Anesthesia allows patients to undergo surgery and other procedures without the distress and pain they would otherwise experience.

Sedation is the reduction of irritability or agitation by administration of a pharmacological agent, generally to facilitate a medical procedure or diagnostic procedure.

Sedation and analgesia include a continuum of states of consciousness ranging from minimal sedation (anxiolysis) to general anesthesia.

**Minimal sedation is** also known as anxiolysis. Minimal sedation is a drug-induced state during which the patient responds normally to verbal commands. Cognitive function and coordination may be impaired. Ventilatory and cardiovascular functions are typically unaffected.

**Moderate sedation/analgesia (conscious sedation) is** a drug-induced depression of consciousness during which the patient responds purposefully to verbal command, either alone or accompanied by light tactile stimulation. No interventions are usually necessary to maintain a patent airway. Spontaneous ventilation is typically adequate. Cardiovascular function is usually maintained.

**Deep sedation/analgesia is** a drug-induced depression of consciousness during which the patient cannot be easily aroused, but responds purposefully (not a reflex withdrawal from a painful stimulus) following repeated or painful stimulation. Independent ventilatory function may be impaired and the patient may require assistance to maintain a patent airway. Spontaneous ventilation may be inadequate. Cardiovascular function is usually maintained.

**General anesthesia is** a drug-induced loss of consciousness during which the patient is not arousable, even to painful stimuli. The ability to maintain independent ventilatory function is often impaired and assistance is often required to maintain a patent airway. Positive pressure ventilation may be required due to
depressed spontaneous ventilation or drug-induced depression of neuromuscular function. Cardiovascular function may be impaired.

Sedation in the intensive care unit (ICU) allows the depression of patients' awareness of the environment and reduction of their response to external stimulation. It can play a role in the care of the critically ill patient, and encompasses a wide spectrum of symptom control that will vary between patients, and among individuals throughout the course of their illnesses. Heavy sedation in critical care has been used to facilitate endotracheal tube tolerance and ventilator synchronization, often with neuromuscular blocking agents.

In some embodiments, sedation (*e.g*., long-term sedation, continuous sedation) is induced and maintained in the ICU for a prolonged period of time (*e.g.,* 1 day, 2 days, 3 days, 5 days, 1 week, 2 week, 3 weeks, 1 month, 2 months). Long-term sedation agents may have long duration of action. Sedation agents in the ICU may have short elimination half-life.

Procedural sedation and analgesia, also referred to as conscious sedation, is a technique of administering sedatives or dissociative agents with or without analgesics to induce a state that allows a subject to tolerate unpleasant procedures while maintaining cardiorespiratory function.

### Examples

In order that the invention described herein may be more fully understood, the following examples are set forth. The synthetic and biological examples described in this application are offered to illustrate the compounds, pharmaceutical compositions, and methods provided herein and are not to be construed in any way as limiting the scope of the invention as defined in the claims.

### Materials and Methods

The compounds provided herein can be prepared from readily available starting materials using the following general methods and procedures. It will be appreciated that where typical or preferred process conditions (*i.e.,* reaction temperatures, times, mole ratios of reactants, solvents, pressures, *etc*.) are given, other process conditions can also be used unless otherwise stated. Optimum reaction conditions may vary with the particular reactants or solvent used, but such conditions can be determined by one skilled in the art by routine optimization.

Additionally, as will be apparent to those skilled in the art, conventional protecting groups may be necessary to prevent certain functional groups from undergoing undesired reactions. The choice of a suitable protecting group for a particular functional group as well as suitable conditions for protection and deprotection are well known in the art. For example, numerous protecting groups, and their introduction and removal, are described in T. W. Greene and P. G. M. Wuts, Protecting Groups in Organic Synthesis, Second Edition, Wiley, New York, 1991, and references cited therein.

The compounds provided herein may be isolated and purified by known standard procedures. Such procedures include (but are not limited to) recrystallization, column chromatography, HPLC, or supercritical fluid chromatography (SFC). The following schemes are presented with details as to the preparation of representative oxysterols that have been listed herein. The compounds provided herein may be prepared from known or commercially available starting materials and reagents by one skilled in the art of organic synthesis. Exemplary chiral columns available for use in the separation/purification of the enantiomers/diastereomers provided herein include, but are not limited to, CHIRALPAK^{®} AD-10, CHIRALCEL^{®} OB, CHIRALCEL^{®} OB-H, CHIRALCEL^{®} OD, CHIRALCEL^{®} OD-H, CHIRALCEL^{®} OF, CHIRALCEL^{®} OG, CHIRALCEL^{®} OJ and CHIRALCEL^{®} OK.

**¹H-NMR** reported herein (e.g., for the region between δ (ppm) of about 0.5 to about 4 ppm) will be understood to be an exemplary interpretation of the NMR spectrum (e.g., exemplary peak integratations) of a compound.

**LC-ELSD/MS:** (Mobile Phase: 1.5ML/4L TFA in water (solvent A) and 0.75ML/4L TFA in acetonitrile (solvent B), using the elution gradient 30%-90% (solvent B) over 0.9 minutes and holding at 90% for 0.6 minutes at a flow rate of 1.2 ml/min; Column: Xtimate C18 2.1*30mm, 3um; Wavelength: UV 220 nm; Column temperature: 50°C; MS ionization: ESI; Detector: PDA & ELSD.

**Abbreviations:** CAN: acetonitrile; PE: petroleum ether; DCM: dichloromethane; EtOAc: ethylacetate; EDCI: N-Ethyl-N'-(3-dimethylaminopropyl)carbodiimide hydrochloride.

### INTERMEDIATES

### Procedure for synthesis of INT3

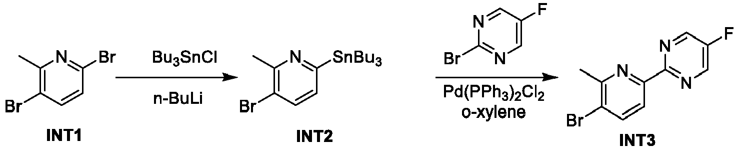

### Synthesis of INT2

To a solution of 3,6-dibromo-2-methylpyridine (1.0 g, 3.98 mmol) in toluene (50 mL) at -70 °C was slowly added n-BuLi (2.5 M in hexanes, 1.90 mL, 4.77 mmol). After stirring for 2 h at -70 °C, tributyl(chloro)stannane (1.68 g, 5.17 mmol) was added. After stirring for 1 h at - 70°C, the reaction mixture was warmed to -10°C and NH₄Cl saturated aqueous solution (10 mL) was added. The mixture was extracted with ethyl acetate (2 x 50 mL) and the combined organic phase was dried over Na₂SO₄, filtered and concentrated under vacuum to give a residue, which was purified by flash column chromatography (PE) to give **INT2** (1.61 g, 88%) as an oil.

**¹H NMR** (400 MHz, CDCl₃) δ 7.63-7.56 (m, 1H), 7.11-7.03 (m, 1H), 2.66 (s, 3H), 1.63-1.43 (m, 5H), 1.36-1.29 (m, 6H), 1.12-1.05 (m, 4H), 0.93-0.85 (m, 12H).

### Synthesis of INT3

To a suspension of **INT2** (1.3 g, 2.81 mmol) in xylene (15 mL) was added 2-bromo-5-fluoropyrimidine (470 mg, 2.66 mmol), Pd(PPh₃)₂Cl₂ (100 mg, 0.1424 mmol), PPh₃ (75 mg, 0.2859 mmol) under N₂. After stirring at 135°C for 16 h, the reaction mixture was quenched with water (50 mL) and extracted with DCM (2 x 50 mL). The combined organic phase was washed with brine (50 mL), dried over Na₂SO₄, filtered and concentrated under vacuum to give a residue, which was purified by flash column chromatography (ethyl acetate in PE, 15%) to give **INT3** (400 mg, 53%) as a solid.

**¹H NMR**(400 MHz, CDCl₃) δ 8.76 (s, 2H), 8.17 (d, *J=* 8.0 Hz, 1H), 7.98 (d, *J=* 8.0 Hz, 1H), 2.83 (s, 3H); **¹⁹F NMR** (376 MHz, CDCl₃) δ -137.524.

### Synthesis of INT4 and INT5

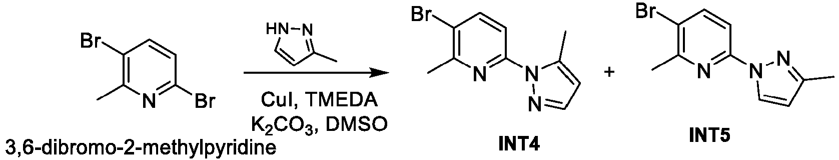

To a suspension of 3,6-dibromo-2-methylpyridine (1.5 g, 5.97 mmol), 3-methyl-1H-pyrazole (977 mg, 11.9 mmol) and TMEDA (1.38 g, 11.9 mmol) in DMSO (30 mL) was added K₂CO₃ (2.47 g, 17.9 mmol) under N₂. The suspension was degassed under vacuum and purged with N₂ for three times. CuI (1.13 g, 5.97 mmol) was then added to the suspension and degassed under vacuum and purged with N₂ for three times. After stirring initially at 20°C for 30 min and then heating to 90°C for 12 h, the reaction mixture was diluted water (50 mL) and ethyl acetate (50 mL). The aqueous layer was extracted with ethyl acetate (50 mL) and the combined organic solution was washed with water (50 mL) and brine (50 mL), dried over sodium sulfate, and concentrated in vacuum. The residue was purified by flash column (PE) to give a solid (1.5 g) that was further purified by SFC (Column: Chiralpak AD-3 150×4.6mm I.D., 3um; Mobile phase: A: CO₂ B:ethanol (0.05% DEA); Gradient: from 5% to 40% of B in 5 min and hold 40% for 2.5 min, then 5% of B for 2.5 min; Flow rate: 2.5mL/min) to afford **INT5** (384 mg, 26%, Peak 1) and **INT4** (106 mg, 7%, Peak 2) both as solids. The structures were confirmed by 2D NMR.

**INT5: ¹H NMR** (400 MHz, CDCl₃) δ 7.88 (d, *J =* 8.4 Hz, 1H), 7.60 (d, *J =* 8.4 Hz, 1H), 7.56 (d, *J=* 1.2 Hz, 1H), 6.18 (s, 1H), 2.68 (s, 3H), 2.65 (s, 3H).

### EXAMPLE 1: Synthesis of (3R,5R,8R,9R,10S,13S,14S,17S)-3-(ethoxymethyl)-3-hydroxy-13-methyl-N-(pyridin-4-yl)hexadecahydro-1H-cyclopenta[a]phenanthrene-17-carboxamide (A2)

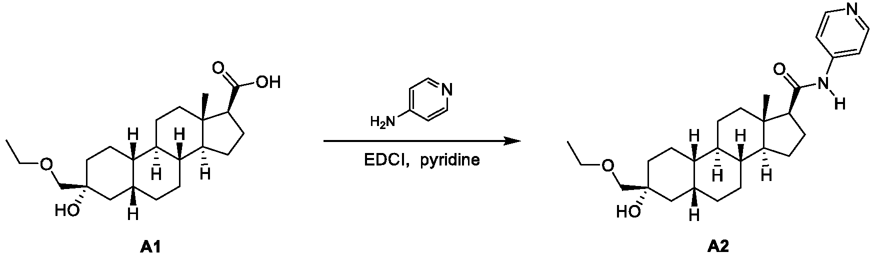

**General Method** 1: To a mixture of **A1** (100 mg, 0.274 mmol, reported in WO 2018013613) in pyridine (5 mL) was added EDCI (57.4 mg, 0.301 mmol). After stirring at rt for 15 mins, pyridin-4-amine (28.3 mg, 0.301 mmol) was added to the mixture. After stirring at 60°C for 16 h, the reaction mixture was quenched with HCl (1M, 10 mL) and extracted with EtOAc (2x 10 mL). The combined organic solution was washed with brine (5 mL), dried over Na₂SO₄, filtered and concentrated. The residue was purified by prep-HPLC (column: Xbridge 150*30mm*10um, Condition: water (0.05% ammonia hydroxide v/v)-ACN, Begin B: 60%, End B: 90%, Gradient Time: 7 min, Flow Rate: 25 ml/min) to give **A2** (29 mg, 24%) as a solid. **¹H NMR** (400 MHz, CDCl₃) δ 8.55-8.45 (m, 2H), 7.55-7.45 (m, 2H), 7.20-7.15 (s, 1H), 3.60-3.40 (m, 4H), 2.80-2.75 (m, 1H), 2.40-2.25 (m, 2H), 2.00-1.95 (m, 1H), 1.85-1.55 (m, 9H), 1.50-1.00 (s, 15H), 0.74 (s, 3H); **LC-ELSD/MS** purity 99%, MS ESI calcd. for C₂₇H₄₁N₂O₃ [M+H]⁺ 441, found 441.

### EXAMPLE 2: Synthesis of (3R,5R,8R,9R,10S,13S,14S,17S)-3-(ethoxymethyl)-3-hydroxy-13-methyl-N-(3-methylpyridin-2-yl)hexadecahydro-1H-cyclopenta[a]phenanthrene-17-carboxamide (A4)

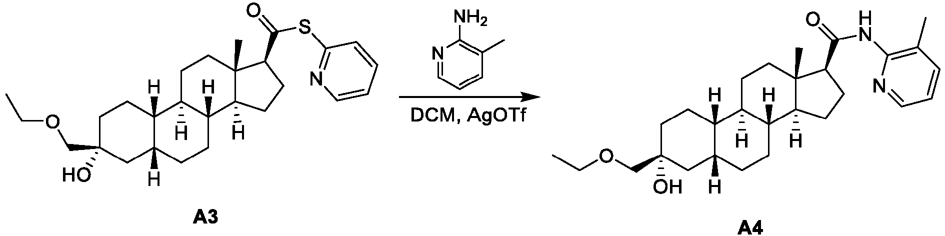

**General Method 2:** To a solution of **A3** (200 mg, 0.436 mmol, reported in WO 2018013613) in DCM (3 mL) was added AgOTf (56.0 mg, 0.218 mmol) and 3-methylpyridin-2-amine (70.7 mg, 0.654 mmol). After stirring at rt for 16 h, the reaction mixture was filtered, and the filtered cake was washed with DCM (3 x 3 mL). The combined organic solution was washed with 1 M HCl (100 mL) and brine (100 mL), dried over anhydrous sodium sulfate, filtered and concentrated. The residue was purified by HPLC (Column Waters Xbridge 150*25 5u Condition water (10mM NH4HCO₃)-ACN Begin B 57 End B 77 Gradient Time(min) 6 100%B Hold Time(min) 2 FlowRate (ml/min) 25) to give **A4** (9 mg, 5%) as a solid.

**¹H NMR** (400 MHz, CDCl₃) δ 8.26-8.22 (m, 1H), 7.58-7.52 (m, 1H), 7.35-7.31 (m, 1H), 7.12-7.06 (m, 1H), 3.60-3.50 (m, 2H), 3.48-3.38 (m, 2H), 2.71 (s, 1H), 2.46-2.38 (m, 1H), 2.31-2.10 (m, 5H), 1.90-1.56 (m, 8H), 1.56-1.05 (m, 16H), 0.81 (s, 3H); **LC-ELSD/MS** purity 100 %, MS ESI calcd. for C₂₈H₄₃N₂O₃ [M+H]⁺ 455, found 455.

### EXAMPLE 3: Synthesis of (3R,5R,8R,9R,10S,13S,14S,17S)-3-(ethoxymethyl)-3-hydroxy-13-methyl-N-(3-(trifluoromethyl)pyridin-2-yl)hexadecahydro-1H-cyclopenta[a]phenanthrene-17-carboxamide (A5)

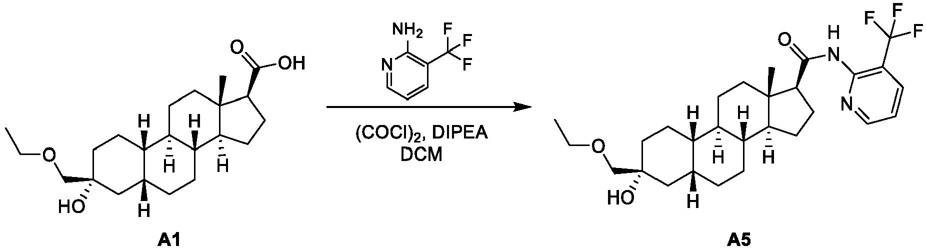

**General Method 3:** To a solution of **A1** (200 mg, 0.548 mmol) in anhydrous DCM (2 mL) at rt was added oxalyl dichloride (76.4 mg, 0.602 mmol) and DMF (2 mg, 0.0274 mmol) successively. After stirring for 1 h, 3-(trifluoromethyl)pyridin-2-amine (133 mg, 0.822 mmol) and DIPEA (352 mg, 2.73 mmol) were added. After 16 h at rt. the reaction mixture was quenched with water (2 mL) and extracted with DCM (2 x 2 mL). The combined organic solution was concentrated, purified by prep. HPLC (Column: YMC-Actus Triart C18 100*30mm*5um; Condition: water (0.05%HC1)-ACN; Gradient: 60%~90%B; FlowRate: 25 mL/min) and lyophilized to give product (80 mg). The solid was dissolved in DCM (10 mL) and washed with saturated NaHCO₃ (10 mL) & brine (10 mL), dried over anhydrous sodium sulfate, filtered, concentrated and dried in vacuum drier at 70°C for 24 hrs to give A5 (41 mg, 15%) as a solid.

**¹H NMR** (400 MHz, CDCl₃) δ 8.68 (d, *J=* 4.0 Hz, 1H), 7.98-7.92 (m, 1H), 7.53 (s, 1H), 7.24-7.20 (m, 1H), 3.57-3.50 (m, 2H), 3.47-3.38 (m, 2H), 2.72 (s, 1H), 2.52-2.43 (m, 1H), 2.36-2.25 (m, 1H), 2.11-2.03 (m, 1H), 1.90-1.70 (m, 6H), 1.68-1.59 (m, 2H), 1.51-1.34 (m, 7H), 1.29-1.09 (m, 9H), 0.80 (s, 3H); **¹⁹F NMR** (400 MHz, CDCl₃) δ -61.82; **LC-ELSD/MS** purity 99% MS ESI calcd. for C₂₈H₄₀F₃N₂O₃ [M+H]⁺ 509, found 509.

### EXAMPLE 4: Synthesis of (3R,5R,8R,9R,108,13S,14S,17S)-3-(ethoxymethyl)-3-hydroxy-13-methyl-N-(6-methylpyridin-2-yl)hexadecahydro-1H-cyclopenta[a]phenanthrene-17-carboxamide (A8)

### Synthesis of A7

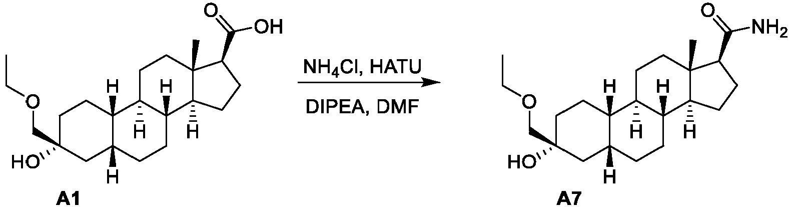

To a solution of **A1** (2.00 g, 5.48 mmol, reported in WO 2018013613) in DMF (20 mL) was added HATU (1.98 g, 8.22 mmol) and DIPEA (5.66 g, 43.8 mmol). After stirring for 15 mins at rt, NH₄Cl (1.17 g, 21.9 mmol) was added. After stirring for 16 h, the reaction mixture was diluted with EtOAc (50 mL), washed with water (50 mL), 3% of LiCl aqueous (50 mL), water (50 mL) and brine (50 mL), dried over sodium sulfate, filtered and concentrated. The residue was purified by flash column (0~10% of DCM in CH₃OH) to give **A7** (2.0 g, 100%) as a solid.

**¹H NMR** (400 MHz, CDCl₃) δ 5.47-5.03 (m, 2H), 3.53 (q, *J*=7.0 Hz, 2H), 3.42 (q, *J*=9.2 Hz, 2H), 2.25-2.05 (m, 2H), 1.99-1.92 (m, 1H), 1.85-1.34 (m, 14H), 1.34-1.00 (m, 11H), 0.71 (s, 3H).

### Synthesis of A8

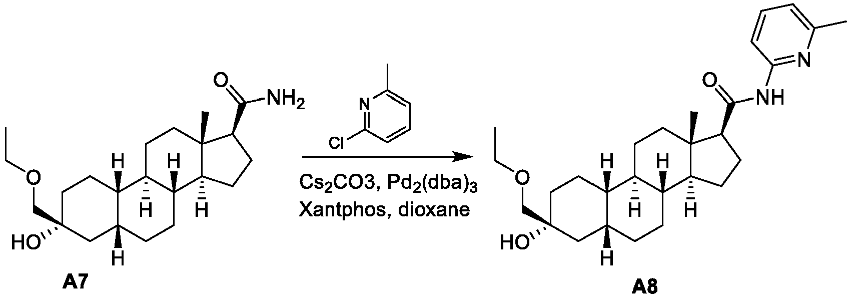

**General Method 4:** To a mixture of 2-chloro-6-methylpyridine (105 mg, 0.824 mmol), **A7** (200 mg, 0.550 mmol), Xantphos (31.8 mg, 0.055 mmol) and cesium carbonate (355 mg, 1.09 mmol) in dioxane (5 mL) sparged with nitrogen for 2 min, was added Pd₂(dba)₃ (50.3 mg, 0.055 mmol). After heating in microwave at 105°C for 1 h, the reaction was filtered, concentrated and purified by prep-HPLC (Column Xtimate C18 150*25mm*5um; Condition water(0.225%FA)-ACN Begin B 60; End B 90 Gradient Time(min) 8; 100%B Hold Time(min) 2; FlowRate (ml/min) 30) to give **A8** (43 mg, 17%) as a solid.

**1H NMR** (400 MHz, CDCl₃) δ 8.03 (d, *J*=7.8 Hz, 1H), 7.67 (br s, 1H), 7.57 (t, *J*=7.8 Hz, 1H), 6.87 (d, *J*=7.4 Hz, 1H), 3.53 (q, *J*=6.9 Hz, 2H), 3.47-3.39 (m, 2H), 2.73 (s, 1H), 2.44 (s, 3H), 2.36-2.22 (m, 2H), 2.05 (br d, *J*=11.8 Hz, 1H), 1.88-1.56 (m, 6H), 1.56-1.30 (m, 9H), 1.30-1.00 (m, 9H), 0.74 (s, 3H); **LC-ELSD/MS** purity 99%, MS ESI calcd. for C₂₈H₄₃N₂O₃ [M+H]⁺ 455, found 455.

### EXAMPLE 5: Synthesis of (3R,5S,8R,9S,10S,13S,14S,17S)-3-hydroxy-3-(methoxymethyl)-10,13-dimethyl-N-(6-methylpyridin-2-yl)hexadecahydro-1H-cyclopenta[a]phenanthrene-17-carboxamide (A13)

### Synthesis of A11

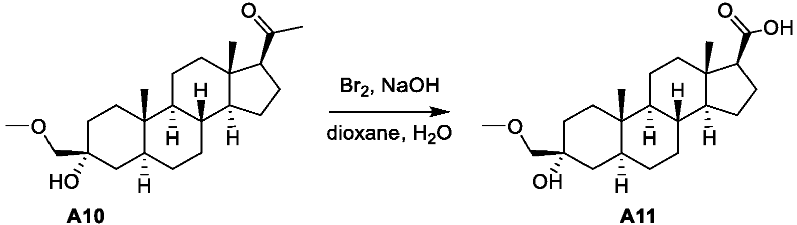

Liquid bromine (2.63 g, 16.5 mmol) was added slowly to a vigorously stirred sodium hydroxide aqueous (18.3 mL, 3 M, 55.1 mmol) at 0°C. When the bromine was dissolved, the mixture was diluted with cold dioxane (10 mL) and was added slowly to a stirred solution of **A10** (2.0 g, 5.51 mmol, reported in WO 2016061527) in dioxane (25 mL) and water (10 mL). The homogeneous solution became colorless slowly and a precipitate was formed. After stirring for 16 h at rt, the reaction was quenched by Na₂SO₃ aqueous (30 mL) and the pH adjusted with hydrochloride acid (3 N) yielding a precipitate. The suspension was dissolved in EtOAc (50 mL) and the organic phase was collected. The aqueous phase was extracted EtOAc (2 X 50 mL). The combined organic was washed with saturated brine (50 mL), dried over anhydrous Na₂SO₄, filtered and concentrated to give **A11** (2.0 g) as a solid.

### Synthesis of A12

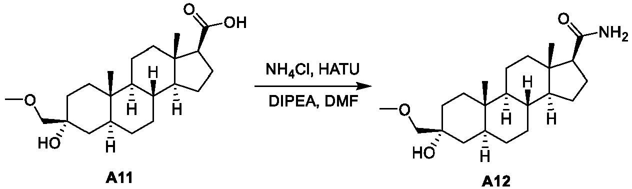

To a solution of **A11** (750 mg, 2.05 mmol) in DMF (10 mL) was added HATU (990 mg, 4.11 mmol) and DIPEA (2.11 g, 16.4 mmol). After stirring for 15 mins at rt, NH₄Cl (438 mg, 8.20 mmol) was added. After stirring for 16 h, the reaction mixture was diluted with EtOAc (50 mL), washed with water (50 mL), 3% of LiCl aqueous (50 mL), water (50 mL) and brine (50 mL), dried over sodium sulfate, filtered, concentrated and purified by flash column (0~50% of DCM in CH₃OH) to give **A12** (530 mg, 71%) as a solid. **¹H NMR** (400 MHz, CDCl₃) δ 5.31-5.20 (m, 2H), 3.38 (s, 3H), 3.18 (s, 2H), 2.22-1.95 (m, 4H), 1.78-1.56 (m, 4H), 1.56-0.78 (m, 16H), 0.76 (s, 3H), 0.71 (s, 3H). **LC-ELSD/MS** purity 99%, MS ESI calcd. for C₂₂H₃₈NO₃ [M +H]⁺ 364, found 364.

### Synthesis of A13

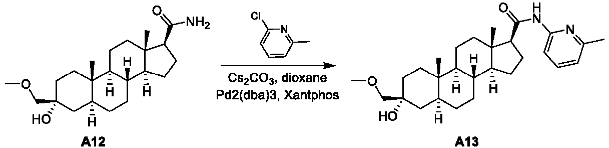

**General Method 5:** A mixture of 2-chloro-6-methylpyridine (105 mg, 0.824 mmol), **A12** (200 mg, 0.550 mmol), Xantphos (31.8 mg, 0.055 mmol) and cesium carbonate (357 mg, 1.09 mmol) in dioxane (3 mL) was sparged with nitrogen for 2 min, then Pd₂(dba)₃ (50.3 mg, 0.055 mmol) was added. After heating in microwave at 130°C for 1 h, the reaction mixture was filtered and purified by prep-HPLC (Column Xtimate C18 150*25mm*5um; Condition water (0.225%FA)-ACN Begin B 80 End B 100 Gradient Time (min) 7; 100%B Hold Time(min) 2 FlowRate (ml/min) 25) to give **A13** (137 mg, 55%) as a solid.

**¹H NMR** (400 MHz, CDCl₃) δ 8.03 (d, J=8.3 Hz, 1H), 7.70 (s, 1H), 7.56 (t, J=7.8 Hz, 1H), 6.86 (d, J=7.5 Hz, 1H), 3.38 (s, 3H), 3.18 (s, 2H), 2.43 (s, 3H), 2.35- 2.23 (m, 2H), 2.04 (br d, J=11.3 Hz, 2H), 1.85-1.56 (m, 3H), 1.56-1.10 (m, 15H), 1.05-0.80 (m, 2H), 0.75 (s, 3H), 0.73 (s, 3H). **LC-ELSD/MS** purity 99%, MS ESI calcd. for C₂₈H₄₃N₂O₃ [M+H]⁺ 455, found 455.

### EXAMPLE 6: Synthesis of (3R,5R,8R,9R,10S,13S,14S,17S)-3-hydroxy-3,13-dimethyl-N-(2-methylimidazo[1,2-a]pyridin-3-yl)hexadecahydro-1H-cyclopenta[a]phenanthrene-17-carboxamide

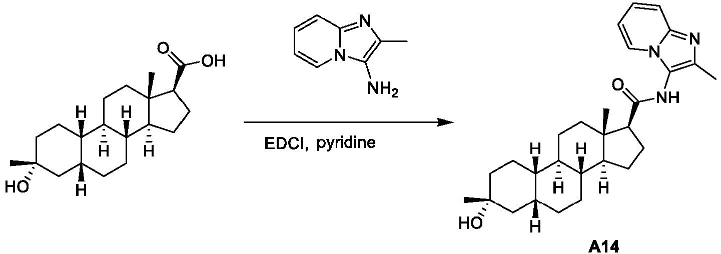

To a solution of (3R,5R,8R,9R,10S,13S,14S,17S)-3-hydroxy-3,13-dimethylhexadecahydro-1H-cyclopenta[a]phenanthrene-17-carboxylic acid (100 mg, 0.312 mmol) in pyridine (2 mL) was added EDCI (71.7 mg, 0.374 mmol) and 2-methylimidazo[1,2-a]pyridin-3-amine (45.9 mg, 0.312 mmol) at 25°C. After stirring at 25°C for 16 h, the reaction was quenched with water (10 mL) and extracted with EtOAc (2 x 20 mL). The combined organic solution was dried over Na₂SO₄, filtered and concentrated in vacuum to give product which was purified by prep. HPLC (column: YMC-Actus Triart C18 100*30mm*5um, gradient: 10-80% B (A= 0.05%HCl-ACN, B= acetonitrile), flow rate: 25 mL/min) to give **A14** (75 mg, 63%) as a solid.

**¹H NMR** (400 MHz, MeOD) δ 7.90 (d, J=6.8 Hz, 1H), 7.46 (d, J=8.8 Hz, 1H), 7.35-7.27 (m, 1H), 6.97-6.93 (m, 1H), 2.62 (t, J=9.4 Hz, 1H), 2.33 (s, 3H), 2.30-2.08 (m, 2H), 2.00-1.65 (m, 8H), 1.64-1.31 (m, 10H), 1.28-1.10 (m, 8H), 0.83 (s, 3H); **LC-ELSD/MS** purity 99%, MS ESI calcd. for C₂₈H₄₀N₃O₂ [M+H]⁺ 450, found 450.

### EXAMPLE 7: Synthesis of (3R,5S,8R,9R,10S,13S,14S,17S)-3-hydroxy-3-(methoxymethyl)-13-methyl-N-(6-methylpyridin-2-yl)hexadecahydro-1H-cyclopenta[a]phenanthrene-17-carboxamide (A18)

### Synthesis of A16

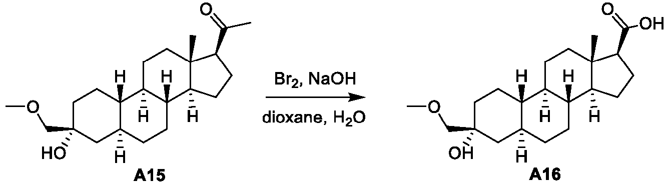

Liquid bromine (1.5 g, 9.43 mmol) was added slowly to a vigorously stirred sodium hydroxide aqueous (1.48 g NaOH, 37.1 mmol, 2.5 M) at 0°C. After stirring for 30 mins at rt, the mixture was added slowly to a stirred solution of **A15** (1.0 g, 2.86 mmol, reported in WO 2013056181) in dioxane (10 mL). The homogeneous solution became colorless slowly and a precipitate was formed. After stirring for 16 h, the reaction was quenched with Na₂SO₃ aqueous (50 mL), the pH adjusted with aq. HCl until pH (~6) resulting in a precipitate. The solid was filtered and washed with water (3 x 20 mL) to give a solid, which was dried under vacuum to afford **A16** (1.0 g) as a solid.

**¹H NMR** (400 MHz, CDCl₃) δ 11.89 (s, 1H), 4.01 (s, 1H), 3.23 (s, 3 H), 3.04 (s, 2H), 2.26 (m, 1H), 1.90 (m, 2H), 1.71 (m, 5H), 1.45 (m, 2H), 1.13 (m, 12H), 0.61 (s, 5H).

### Synthesis of A17

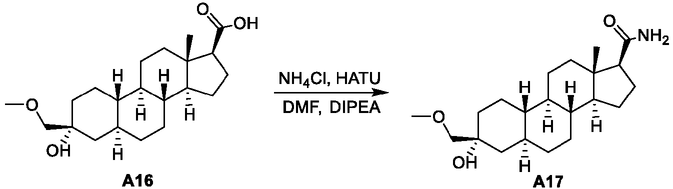

To a solution of **A16** (500 mg, 1.42 mmol), HATU (809 mg, 2.13 mmol) and NH₄Cl (151 mg, 2.84 mmol) in DMF (5 mL) at rt was added DIPEA (549 mg, 4.26 mmol). After stirring for 18 h, the mixture was poured into water (20 mL) and extracted with EtOAc (3 x 20 mL). The combined organic solution was washed with brine (2 x 20 mL), dried over anhydrous Na₂SO₄, filtered and concentrated. The product was purified by prep-HPLC separation (column: YMC-Actus Triart C18 100*30mm*5um), gradient: 65-95% B (A= water (0.05% HCl), B= ACN), flow rate: 25 mL/min) to give **A17** (600 mg) as a solid which was triturated from MeOH 10 mL and water 10 mL to give **A17** (330 mg, 55 %) as a solid.

**¹H NMR** (400 MHz, CDCl₃) δ 6.86 (s, 1H), 6.70 (s, 1H), 4.01 (s, 1H), 3.23 (s, 3H), 3.04 (s, 2H), 2.11 (m, 1H), 1.97 (m, 1H), 1.74 (m, 2H), 1.57 (m, 6H), 1.32 (m, 4H), 1.03 (m, 8H), 0.57 (m, 5H).

### Synthesis of A18

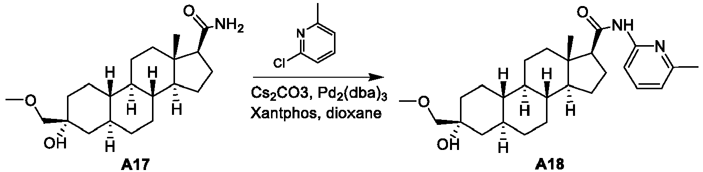

General Method 7: To a suspension of **A17** (300 mg, 0.858 mmol) and 2-chloro-6-methylpyridine (218 mg, 1.71 mmol) in anhydrous dioxane (4 mL) was added Pd₂(dba)₃ (78.5 mg, 0.0858 mmol), XantPhos (49.6 mg, 0.0858 mmol) and Cs₂CO₃ (557 mg, 1.71 mmol). After heating in microwave at 130°C for 1 h, the mixture was poured into water (10 mL) and extracted with EtOAc (3 x 10 mL). The combined organic solution was washed with brine (2 x 20 mL), dried over anhydrous Na₂SO₄, filtered and concentrated. The residue was purified by flash column (30% EtOAc in PE) to give **A18** (200 mg) as a solid. The solid was triturated with n-hexane (10 mL) at rt to give **A18** (167 mg) as a solid.

**¹H NMR** (400 MHz, CDCl₃) δ 8.01 (d, *J =* 8.4 Hz, 1H), 7.64 (s, 1H), 7.56 (t, *J =* 7.6 Hz, 1H), 6.85 (d, *J=* 7.6 Hz, 1H), 3.38 (s, 3H), 3.18 (s, 2H), 2.43 (s, 3H), 2.30 (m, 2H), 2.02 (m, 2H), 1.76 (m, 6H), 1.57 (m, 8 H), 1.21 (m, 5H), 0.74 (m, 5H). **LC-ELSD/MS** purity 99%, MS ESI calcd. for C₃₄H₄₈N₂O₃ [M+H]⁺ 441, found 441.

### EXAMPLE 8: Synthesis of (3R,5R,8S,9S,10S,13S,14S,17S)-N-(1,3-dimethyl-1H-pyrazol-5-yl)-3-(ethoxymethyl)-10-ethyl-3-hydroxy-13-methylhexadecahydro-1H-cyclopenta[a]phenanthrene-17-carboxamide (29)

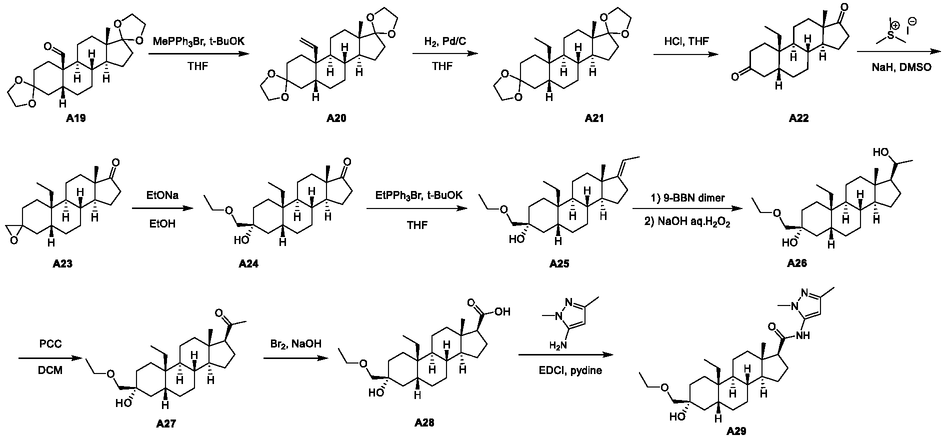

### Synthesis of A20

To a suspension of PPh₃MeBr (548 g, 1536 mmol) in THF (1L) was added t-BuOK (172 g, 1536 mmol) at rt. After stirring at 45°C for 1 h, a solution of **A19** (200 g, 512 mmol, reported in WO2016/134301) in THF (500 mL) was added. After stirring at 45°C for 10 min, saturated NH₄Cl solution (500 mL) and EtOAc (500 mL) were added to the mixture. The organic solution was separated, and the aqueous layer was extracted with EtOAc (2 x 300 mL). The combined organic solution was washed with brine (2 x 300 mL), dried over anhydrous Na₂SO₄, filtered, concentrated and triturated from MeOH/H₂O (1 L/1 L) at 15°C and purified by silica gel chromatography (PE/EtOAc = 50/1 to 20/1) to give **A20** (137 g, 69%) as an oil.

**¹H NMR** (400 MHz, CDCl₃) δ 6.40-6.25 (m, 1H), 5.15-5.00 (m, 2H), 3.95-3.75 (m, 9H),2.10-1.65 (m, 9H), 1.65-1.25 (m, 5H), 1.25-1.05 (m, 6H),0.85-0.75 (m, 4H).

### Synthesis of A21

To a solution of **A20** (70 g, 180 mmol) in THF (1L) was added Pd/C (dry, 10% on carbon, 10 g) under N₂. The suspension was degassed under vacuum and purged with H₂ for three times. After hydrogenatiing at 206,843 Pa (30 psi) of hydrogen at rt for 16 h, the reaction mixture was filtered through a pad of Celite and washed with THF (3 x 500 mL). The filtrate was concentrated to give **A21** (141 g, from two parallel reactions combined) as an oil.

**¹H NMR** (400 MHz, CDCl₃) δ_{H}3.95-3.75 (m, 9H),2.10-1.90 (m, 2H), 1.90-1.65 (m, 8H), 1.65-1.25 (m, 4H), 1.25-1.05 (m, 8H), 0.80-0.45 (s, 7H).

### Synthesis of A22

To a solution of **A21** (141 g, 361 mmol) in THF (1000 mL) was added aq. HCl (361 mL, 4M, 1444 mmol). After stirring at rt for 16 h, the mixture was poured into water (500 mL). The organic phase was collected, and the aqueous layer was extracted with EtOAc (2 x 300 mL). The combined organic solution was washed with aqueous saturated NaHCO₃solution (500 mL), H₂O (2 x 300 mL), brine (200 mL), dried over anhydrous Na₂SO₄, filtered, concentrated in vacuum and triturated from PE (300 mL) to give **A22** (82 g, 73 %) as a solid.

**¹H NMR** (400 MHz, CDCl₃) δ_{H}2.80-2.70 (m, 1H),2.55-2.45 (m, 3H),2.45-2.15 (m, 1H), 2.15-2.00 (m, 3H), 2.00-1.90 (m, 1H), 1.90-1.65 (m, 6H), 1.65-1.30 (m, 2H), 1.30-1.10 (m, 7H),0.88 (s, 3H),1.85-0.75 (m, 3H).

### Synthesis of A23

To a stirred solution of trimethylsulfonium iodide (22.2 g, 109 mmol) in DMSO/THF (100 mL/500mL) was added NaH (4.35 g, 109 mmol, 60 % in oil) at 0°C. After for 1 h, the mixture was added to a solution of **A23** (30 g, 99.1 mmol) in DMSO (100 mL). After stirring rt for 4 h, the reaction was diluted with water (200 mL) and extracted with EtOAc (2x100 mL). The combined organic solution was washed with water (2x100 mL), brine (100 mL), dried over anhydrous Na₂SO₄, filtered and concentrated in vacuum to give **A23** (35 g) as an oil.

### Synthesis of A24 & A24a

To anhydrous EtOH (200 mL) was added Na (25.4 g, 1107 mmol) at 15°C in ten potions. After stirring at 75°C for1 h, a solution of **A23** (22 g, 69.5 mmol) in anhydrous ethanol (50 mL) was added. After stirring at 75°C for 16 h, the reaction was diluted with water (500ml) and concentrated to remove most of the solvent. The mixture was extracted with EtOAc (2 x 200 mL) and the combined organic solution was washed with saturated brine (2 x 100 mL), dried over anhydrous Na₂SO₄, filtered, concentrated. The residue was purified by flash column (0~20% of EtOAc in PE) to give **A24** (12 g) as an oil and **A24a** (7 g) as a solid.
**A24: ¹H NMR** (400 MHz, CDCl₃) δ_{H} 3.60-3.45 (m, 2H),3.45-3.30 (m, 2H),2.45-2.30 (m, 1H),2.10-1.90 (m, 4H), 1.90-1.55 (m, 5H), 1.55-1.30 (m, 7H), 1.30-1.10 (m, 11H), 0.90-0.80 (m, 3H), 0.80-0.60 (m, 3H).
**A24a: ¹H NMR** (400 MHz, CDCl₃) δ_{H} 3.55-3.46 (m, 2H), 3.25-3.15 (m, 2H),2.47-2.35 (m, 1H),2.10-1.95 (m, 4H), 1.95-1.85 (m, 1H), 1.85-1.60 (m, 5H), 1.55-1.35 (m, 6H), 1.35-1.15 (m, 11H), 0.90-0.80 (m, 6H)

### Synthesis of A24

To a suspension of bromo(ethyl)triphenylphosphorane (48.9 g, 132 mmol) in THF (200 mL) was added *t*-BuOK (14.7 g, 132 mmol) at rt under N₂. After stirring at 45°C for 1 h, a solution of **A24** (12 g, 33.0 mmol) in THF (20 mL) was added. After stirring at 45°C for 16 h, the mixture was treated with NH₄Cl (100 mL) and the organic solution collected. The aqueous phase was extracted with EtOAc (2 x 50 mL) and the combined organic solution was washed with brine (2 x 40 mL), dried over anhydrous Na₂SO₄, filtered, concentrated in vacuum. The residue triturated from MeOH/H₂O (50mL/50 mL) at rt to give **A25** (17 g) as an oil.

### Synthesis of A26

To a solution of **A25** (17 g, 45.3 mmol) in THF (100 mL) was added 9-BBN dimer (22.1 g, 90.6 mmol) at rt. After stirring for 16 h, the reaction was cooled to 0°C and ethanol (20.8 g, 453 mmol) and NaOH (90.6 mL, 5 M, 453 mmol) were added very slowly. After the addition was completed, H₂O₂ (45.3 mL, 453 mmol, 30%) was added slowly and the inner temperature was maintained below 15°C. The resulting solution was stirred at 75°C for 1 hrs. Saturated aqueous Na₂S₂O₃ (100 mL) was added and the mixture was stirred at 0 °C for 1h before adding the mixture to water (2 L). The mixture was extracted with EtOAc (2 x 200 mL) and the combined organic solution was washed with brine (50 mL), dried over anhydrous Na₂SO₄, filtered and concentrated under vacuum to give **A26** (18 g) as an oil.

### Synthesis of A27

To a solution of **A26** (18 g, 45.8 mmol) in DCM (200 mL) was added PCC (19.6 g, 91.6 mmol) and silica gel (15 g) at rt. After stirring for 3h, the reaction mixture was filtered, and the residue was washed with anhydrous DCM (2 x 100 mL). The combined organic solution was concentrated and purified by flash column (0~20% of EtOAc in PE) to give **A27** (8 g, 45%) as a solid.

**¹H NMR** (400 MHz, CDCl₃) δ_{H}3.60-3.50 (m, 2H), 3.50-3.30 (m, 2H),2.74 (s, 1H), 2.60-2.45 (m, 1H),2.20-2.05 (m, 4H),2.05-1.75 (m, 2H), 1.75-1.50 (m, 7H), 1.50-1.30 (m, 7H), 1.30-1.05 (m, 10H), 0.80-0.65 (m, 3H), 0.59 (s, 3H); **LC-ELSD/MS** purity 99%, MS ESI calcd. for C₂₅H₄₁O₂ [M+H-H₂O]⁺373, found 373.

### Synthesis of A28

Liquid bromine (7.15 g, 44.8 mmol) was added slowly to a vigorously stirred sodium hydroxide aqueous (35.6 mL, 3 M, 107 mmol) at 0°C. When the bromine was dissolved, the mixture was diluted with cold dioxane (10 mL) and was added slowly to a stirred solution of A27 (3.5 g, 8.9 mmol) in dioxane (30 mL) and water (15 mL). After stirring at rt for 16 h, the reaction was quenched with Na₂SO₃ aqueous (30 mL) and the mixture was then heated at 80°C until the solid material was dissolved. Acidification of the solution with hydrochloride acid (3 N) to pH~6 furnished a precipitate. The precipitated solid was filtered out, washed with water (2 x 50 mL) and dried to give **A28** (3.8 g) as a solid.

**¹H NMR** (400 MHz, CDCl₃)δ_{H} 3.65-3.50 (m, 2H), 3.50-3.30 (m, 2H), 2.45-2.30 (m, 1H), 2.15-2.00 (m, 2H), 2.00-1.80 (m, 1H), 1.80-1.50 (m, 8H), 1.50-1.25 (m, 7H), 1.25-1.05 (m, 11H), 0.90-0.75 (m, 3H), 0.71 (s, 3H); **LC-ELSD/MS** purity 99%, MS ESI calcd. for C₂₄H₄₀O₄ Na [M+Na]⁺415, found 415.

### Synthesis of A29

General Method 8: To a solution of **A28** (200 mg, 0.5094 mmol) in pyridine (5 mL) was added EDCI (389 mg, 2.03 mmol). After stirring at rt 10 mins. 1,3-dimethyl-1H-pyrazol-5-amine (67.9 mg, 0.6112 mmol) was added. After stirring for 16 h, the reaction mixture was added water (20 mL) and EtOAc (20 mL). The organic solution was separated. The aqueous phase was extracted with EtOAc (2 x 10 mL). The combined organic solution was washed with brine (2 x 10 mL), dried over anhydrous Na₂SO₄, filtered, concentrated and purified by HPLC ((column: YMC-Actus Triart C18 100*30mm*5um), gradient: 75-92% B (A= water(0.05%HCl), B= MeCN), flow rate: 25 mL/min) to give **A29** (150mg) as a solid. The solid (150mg) was dissolved in EtOAc (20 mL). treated with saturated NaHCO₃ solution (10 mL). After stirring for 30 mins, the organic phase was separated. The aqueous phase was extracted with EtOAc (3 x 10 mL). The combined organic solution was washed with saturated brine (10 mL), dried over anhydrous Na₂SO₄, filtered and concentrated under vacuum to give **A29** (43 mg, 29 %) as a solid.

**¹H NMR** (400 MHz, CDCl₃) δ 6.71 (s, 1H), 6.00 (s, 1H), 3.67 (s, 3H),3.65-3.50 (m, 2H), 3.50-3.30 (m, 2H), 2.76 (s, 1H), 2.35-2.15 (m, 5H), 2.00-1.50 (m, 8H),1.50-1.25 (m, 9H), 1.25-1.05 (m, 9H), 0.90-0.75 (m, 3H), 0.73 (s, 3H); **LC-ELSD/MS** purity 99%, MS ESI calcd. for C₂₉H₄₈N₃O₃ [M+H]⁺486, found 486.

### EXAMPLE 9: Synthesis of (3R,5R,8S,9S,10R,13S,14S,17S)-N-(5-cyanopyridin-2-yl)-3-(ethoxymethyl)-3-hydroxy-10-(isopropoxymethyl)-13-methylhexadecahydro-1H-cyclopenta[a]phenanthrene-17-carboxamide (A38)

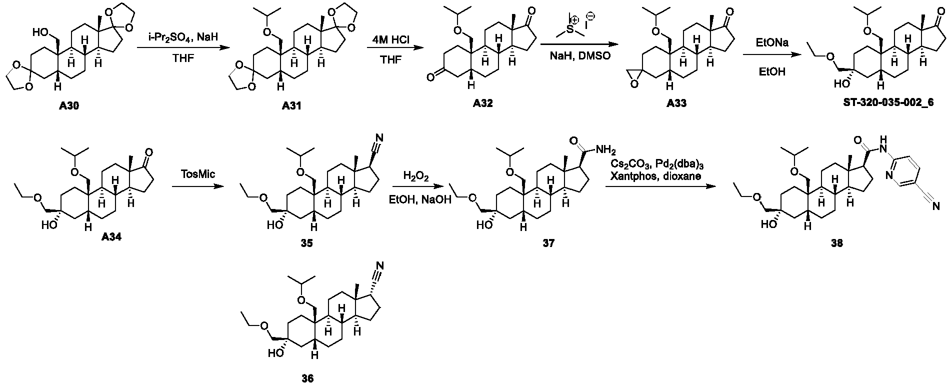

### Synthesis of A31 & A32

To a solution of A30 (10 g, 25.4 mmol, reported in WO2016/134301) in THF (100 mL) at 0°C under nitrogen were added sodium hydride (3.03 g, 76.1 mmol, w/w, 60%) and then diisopropyl sulfate (32.6 g, 177 mmol) in THF (50 mL) portion wise. The mixture was slowly warmed to 20°C and then heated to 60°C. After stirring at 60°C for 72 h, the mixture was diluted with water (200 mL) and extracted with ethyl acetate (3 x 200 mL). The combined organic solution was washed with brine (100 mL), dried with anhydrous sodium sulfate, filtered, concentrated and purified by flash column chromatography (ethyl acetate in PE, 20%) to afford a mixture of **A31** and **A32** (5.2 g) as solid.

### Synthesis of A32

To a solution of **A31** (5.2 g, mixture with **A32**) in THF (80 mL) was added HCl (4 M, 9 mL, 36.0 mmol) at rt. After stirring for 16 h, the reaction was extracted with ethyl acetate (3 x 80 mL). The combined organic solution was washed with saturated NaHCO₃ aqueous (100 mL), brine (100 mL), dried over Na₂SO₄, filtered and concentrated under vacuum to give **A32** (3 g, 73%) as a solid.

**¹H NMR** (400 MHz, CDCl₃) δ 3.55 (d, *J=* 9.2 Hz, 1H), 3.41 (td, *J =* 6.0, 12.0 Hz, 1H), 3.29 (d, *J*= 9.2 Hz, 1H), 2.60-2.50 (m, 1H), 2.40 (dd, *J* = 8.4, 19.2 Hz, 1H), 2.35-2.28 (m, 1H), 2.25-2.17 (m, 2H), 2.09-2.02 (m, 1H), 2.02-1.74 (m, 7H), 1.70-1.08 (m, 9H), 1.06 (dd, *J* = 1.6, 6.0 Hz, 6H), 0.83 (s, 3H).

### Synthesis of A33

To a stirred solution of trimethylsulfonium iodide (1.53 g, 7.50 mmol) in DMSO (10 mL) and THF (10 mL) was added NaH (283 mg, 7.12 mmol, 60 % in oil) at 0°C for 1 h under N₂. To the mixture was added a solution of **A32** (2 g, 5.77 mmol) in DMSO (10 mL) at 0°C. After stirring at rt for 16 h, the reaction was diluted with water (150 mL) and extracted with EtOAc (3 x 100 mL). The combined organic solution was washed with water (2 x 100 mL), brine (100 mL), dried over anhydrous Na₂SO₄, filtered, and concentrated in vacuum to give a residue, which was combined with another batch prepared from 1.9 of **A32.** The combined residue was purified by flash column chromatography (12% of ethyl acetate in PE) to give **A33** (3.9 g) as an oil.

### Synthesis of A34

To a solution of **A33** (3.9 g, 10.8 mmol) in ethanol (150 mL) was added EtONa (11.0 g, 162 mmol) at rt. After stirring at 78°C for 16 h, the reaction mixture was quenched with saturated NH₄Cl aqueous (100 mL) and extracted with EtOAc (3 x 100 mL). The combined organic solution was washed with brine (100 mL), dried over Na₂SO₄, filtered and concentrated under vacuum to give **A34** (1 g, 23%) and **A34a** (910 mg, 21%), both as oils.

**A34: ¹H NMR** (400 MHz, CDCl₃) δ 3.60-3.39 (m, 5H), 3.24-3.18 (m, 1H), 2.71 (s, 1H), 2.43 (dd, *J*= 8.4, 19.2 Hz, 1H), 2.16-2.07 (m, 1H), 1.99-1.62 (m, 9H), 1.20 (t, J = 6.8 Hz, 14H), 1.13 (d, *J=* 6.0 Hz, 7H), 0.85 (s, 3H).

### Synthesis of A35 & A36

To a solution of t-BuOK (3.29 g, 29.4 mmol) and in t-BuOH (8 mL) under N₂ was added **A34** (600 mg, 1.47 mmol) in DME (5 mL) at rt. After stirring for 30 min, TosMic (1.14 g, 5.88 mmol) in DME (3 mL) was added. After stirring at rt for 16 h, the residue was poured into ice-water (100 mL) and extracted with EtOAc (3 x 80 mL). The combined organic solution was washed with brine (50 mL), dried over anhydrous Na₂SO₄, filtered and concentrated. The residue was purified by flash column chromatography (ethyl acetate in PE, 12%) to afford **A35** (250 mg, 41%) and **A36** (80 mg, 13%) as solids.

**A35: ¹H NMR** (400 MHz, CDCl₃) δ 3.61-3.37 (m, 6H), 3.20 (d, *J* = 9.2 Hz, 1H), 2.77-2.64 (m, 1H), 2.32-2.23 (m, 1H), 2.19-2.05 (m, 1H), 1.99-1.70 (m, 6H), 1.67-1.24 (m, 12H), 1.20 (t, *J* = 7.2 Hz, 4H), 1.13 (d, *J* = 6.0 Hz, 6H), 1.10-0.97 (m, 2H), 0.89 (s, 3H); **LC-ELSD/MS** purity 99%, MS ESI calcd. for C₂₆H₄₃NO₃Na [M+Na]⁺ 440, found 440.

**A36: ¹H NMR** (400 MHz, CDCl₃) δ 3.57-3.38 (m, 6H), 3.20 (d, *J=* 9.2 Hz, 1H), 2.64 (s, 1H), 2.55 (dd, *J* = 2.0, 9.2 Hz, 1H), 2.23-2.11 (m, 1H), 2.06-1.93 (m, 1H), 1.91-1.23 (m, 19H), 1.20 (t, *J* = 6.8 Hz, 4H), 1.12 (d, *J =* 6.0 Hz, 6H), 0.79 (s, 3H); **LC-ELSD/MS** purity 97%, MS ESI calcd. for C₂₁H₃₁N [M-iPrOH-EtOH-H2O+H]⁺296, found 296.

### Synthesis of A37

To a solution of **A35** (230 mg, 0.5507 mmol) in THF (5 mL) was added NaOH solution (2.3 g in glycol/H₂O, 8 mL/2 mL). The mixture was evaporated under vacuum to remove THF. After stirring at 160°C for 3 h, additional KOH (1.54 g, 27.5 mmol) and H₂O₂ (0.33 mL, 10 M, 3.30 mmol) were added. After stirring at 160°C for 3 h additionally, the reaction mixture was cooled and poured into HCl (1.0 M, 150 mL) to give a precipitate, which was filtered and washed with water (3 x 50 mL) to give a solid, which was purified by flash column chromatography (ethyl acetate in PE/DCM(1:1), 60%) yielding **A37** (70 mg, 29%) as a solid.

**¹H NMR** (400 MHz, CDCl₃) δ 5.37 (s, 1H), 5.24 (s, 1H), 3.59-3.35 (m, 5H), 3.20 (d, *J*= 9.2 Hz, 1H), 2.22-2.09 (m, 1H), 1.98-1.66 (m, 6H), 1.64-1.37 (m, 13H), 1.21 (t, *J* = 7.2 Hz, 8H), 1.12 (d, *J=* 6.0 Hz, 6H), 0.70 (s, 3H).

### Synthesis of A38

General Method 9: A mixture of **A37** (70 mg, 0.1606 mmol), 6-chloropyridine-3-carbonitrile (75 mg, 0.4851 mmol), Cs₂CO₃ (105 mg, 0.3212 mmol), Pd₂(dba)₃ (30 mg, 0.03278 mmol) and xantphos (20 mg, 0.03460 mmol) in dioxane (3 mL) was stirred at 130°C in microwave for 1.5 h. The reaction mixture was filtered and concentrated under vacuum to give a residue. The solid was purified by flash column chromatography (ethyl acetate in PE, 15%) and then by prep-HPLC (YMC-Actus Triart C18 100*30mm*5um; water (0.05%HCl)-ACN, 70%-100% in 11 min; FlowRate: 25mL/min) to give **A38** (15 mg, 17%) as a solid.

**¹H NMR** (400 MHz, CDCl₃) δ8.53 (d, *J* = 1.6 Hz, 1H), 8.37 (d, *J* = 9.2 Hz, 1H), 7.95-7.88 (m, 2H), 3.58-3.50 (m, 3H), 3.49-3.38 (m, 3H), 3.21 (d, *J* = 9.2 Hz, 1H), 2.39-2.32 (m, 1H), 2.31-2.18 (m, 1H), 2.00 (d, *J* = 7.2 Hz, 1H), 1.92-1.70 (m, 6H), 1.55-1.15 (m, 18H), 1.13-1.09 (m, 6H), 0.71 (s, 3H); **LC-ELSD/MS** purity 99%, MS ESI calcd. for C₃₂H₄₈N₃O₄ [M+H]⁺538, found 538.

### EXAMPLE 10: Synthesis of (3R,5R,8R,9R,10S,13S,14S,17S)-N-(5-fluoropyridin-2-yl)-3-hydroxy-3-(methoxymethyl)-13-methylhexadecahydro-1H-cyclopenta[a]phenanthrene-17-carboxamide (A47)

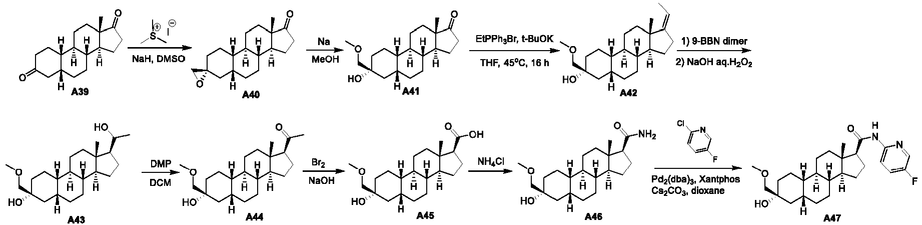

### Synthesis of A40

To a solution of NaH (8.00 g, 200 mmol) in DMSO (100 mL) was added a solution of trimethylsulfonium iodide (40.7 g, 200 mmol) in THF (100 mL) dropwise at 0°C over a period of 30 mins under N₂. The reaction mixture was then added into a solution of **A39** (50 g, 182 mmol) in DMSO (100 mL). After stirring at rt for 12 h, the residue was poured into ice-water (w/w = 1/1) (400 mL) and stirred for 20 min. The aqueous phase was extracted with EtOAc (3 x 400 mL). The combined organic solution was washed with brine (2 x 200 mL), dried over anhydrous Na₂SO₄, filtered and concentrated. The residue was triturated from MeOH (300 mL) to give **A40** (45 g) as a solid.

**¹H NMR** (400MHz, CDCl₃ ) δ 2.67-2.37 (m, 2H), 2.28-2.13 (m, 2H), 2.12-2.04 (m, 1H), 1.99-1.89 (m, 1H), 1.88-1.72 (m, 4H), 1.70-1.60 (m, 2H), 1.58-1.43 (m, 5H), 1.41-1.04 (m, 8H), 0.92-0.82 (m, 3H).

### Synthesis of A41

Na (21.5 g, 935 mmol) was added into MeOH (250 mL) at rt in portions. After stirring for 2 h under N₂, **A40** (45 g, 156 mmol) in MeOH (150 mL) was added. After warming to 75°C for 12 h, the residue was poured into water (400 mL) and extracted with EtOAc (3 x 400 mL). The combined organic solution was washed with brine (2 x 200 mL), dried over anhydrous Na₂SO₄, filtered and concentrated. The residue was purified by flash column (0~15% of EtOAc in PE) to give **A41** (30 g) as an oil and **A41a** (12 g, 24.0 %) as soild.

**A41: ¹H NMR** (400MHz, CDCl₃ ) δ 3.47-3.31 (m, 5H), 2.42 (dd, J=8.4, 19.2 Hz, 1H), 2.12-2.02 (m, 1H), 1.96-1.89 (m, 1H), 1.86-1.67 (m, 6H), 1.60-1.41 (m, 7H), 1.38-1.17 (m, 7H), 1.10-1.00 (m, 1H), 0.85 (s, 3H).

### Synthesis of A42

To a solution of EtPPh₃Br (30.0 g, 84.0 mmol) and t-BuOK (9.40 g, 84.0 mmol) in THF (100 mL) under N₂ at 25°C. After stirring for 1 h, a solution of **A41** (9 g, 28.0 mmol) in THF (50 mL) was added. After stirring at 40°C for 3 h, the solution was combined with other two batches prepared from 9 g and 18 g respectively and was poured into aq. NH₄Cl (200 mL). The aqueous phase was extracted with EtOAc (3 x 200 mL). The combined organic solution was washed with brine (2 x 100 mL), dried over anhydrous Na₂SO₄, filtered and concentrated. The residue was triturated from MeOH (300 mL) and water (300 mL) to give **A42** (30 g) as an oil.

**¹H NMR** δ 5.17-5.05 (m, 1H), 3.46-3.32 (m, 5H), 2.59 (br s, 1H), 2.43-2.12 (m, 4H), 1.90-1.79 (m, 2H), 1.77-1.69 (m, 7H), 1.67-1.61 (m, 4H), 1.43-1.32 (m, 3H), 1.23-1.03 (m, 6H), 0.87 (s, 3H).

### Synthesis of A43

To a solution of **A42** (30 g, 90.2 mmol) in THF (150 mL) at 25°C was added 9-BBN dimer (43.9 g, 180 mmol). After stirring for 1h, NaOH (108 mL, 5M in water, 541 mmol) was added at 0°C followed by dropwise addition of hydrogen peroxide (54.1 mL, 541 mmol). After stirring at 78°C for 3 h, the reaction was quenched with saturated aqueous Na₂S₂O₃ (100 mL) and and ice-water (300 mL). After stirring for 20 min, the aqueous phase was extracted with EtOAc (3 x 250 mL). The combined organic solution was washed with brine (2 x 250 mL), dried over anhydrous Na₂SO₄, filtered and concentrated. The residue was triturated from CH₃OH (100 mL) and water (500 mL) to give **A43** (35 g) as an oil, which was purified by flash column (0~15% of EtOAc in PE) to give **A43** (12 g, 34%) as a solid.

**¹H NMR** (400MHz, CDCl₃) δ 3.74-3.66 (m, 1H), 3.43-3.36 (m, 5H), 2.60 (s, 1H), 1.95-1.71 (m, 6H), 1.64 (s, 4H), 1.60-1.44 (m, 6H), 1.35-1.19 (m, 6H), 1.16-1.08 (m, 4H), 0.92-0.80 (m, 1H), 0.75 (s, 1H), 0.65 (s, 3H).

### Synthesis of A44

To a solution of **A43** in DCM (100 mL) was added DMP (33.1 g, 78.2 mmol) in one portion at 25°C. After stirring at 35°C for 30 min, the residue was washed with NaHCO₃ (100 mL) and filtered, then the mixture was washed with Na₂SO₃ and NaHCO₃ (3:1) in water (100 mL). The aqueous phase was extracted with DCM (100 mL). The combined organic solution was washed with brine (2 x 100 mL), dried over anhydrous Na₂SO₄, filtered and concentrated. The residue was purified by flash column (0~15% of EtOAc in PE) to give **A44** (5 g, 42%) as a solid and inpure product (4 g). The inpure product was repurified by silica gel chromatography (PE/EtOAc = 30/1 to 5/1) to afford additional **A44** (2 g, 50%) as a solid.

**¹H NMR** (400MHz, CDCl₃) δ 3.45-3.34 (m, 5H), 2.62 (s, 1H), 2.59-2.46 (m, 1H), 2.26-2.13 (m, 1H), 2.13-2.09 (m, 1H), 2.11 (s, 3H), 2.03-1.97 (m, 1H), 2.03-1.97 (m, 1H), 1.83 (br d, J=15.2 Hz, 1H), 1.78-1.63 (m, 5H), 1.52-1.33 (m, 5H), 1.31-1.00 (m, 8H), 0.61 (s, 3H).

### Synthesis of 45

Br₂ (2.73 g, 17.1 mmol) was added to aqueous NaOH (2.74 g, 10% in water, 17.1 mmol) at 0°C. After stirring at 0°C for 30 min, the solution was added to a solution of A44 (2 g, 5.73 mmol) in dioxane (20 mL) and water (20 mL). After stirring at rt for 3 h, the reaction mixture was washed with EtOAc (60 mL). The aqueous layer was acidified with HCl (12 M in water, 22 mL) to adjust the pH to 1 and stirred for 30 min. The precipitated solid was filtered out, washed with water (2 x 60 mL) and dried to give **A45** (1.6 g, 59%) as a soild.

**¹H NMR** (400MHz, CDCl₃) δ 3.44-3.34 (m, 5H), 2.44-2.35 (m, 1H), 2.13-2.01 (m, 2H), 1.88-1.72 (m, 5H), 1.71-1.53 (m, 5H), 1.51-1.31 (m, 7H), 1.30-1.18 (m, 4H), 1.09-0.99 (m, 2H), 0.72 (s, 3H).

### Synthesis of A46

To a solution of **A45** (1.6 g, 4.56 mmol) in DMF (20 mL) were added HATU (2.59 g, 6.84 mmol) and DIPEA (2.35 g, 18.2 mmol). After stirring at rt for 10 min, NH₄Cl (487 mg, 9.12 mmol) was added. After stirring for 16 h at rt, the residue was poured into water (50 mL) and extracted with EtOAc (3 x 40 mL). The combined organic solution was washed with brine (2 x 20 mL), dried over anhydrous Na₂SO₄,filtered and concentrated to give **A46** (1.6 g) as a soild. The solid was dissolved in EtOAc (50 mL) and washed with NH4Cl (2 x 50 mL) and LiCl (2 x 100 mL, 10 g in 200 mL of water) and concentrate in a vacuum to give **A46** (1.5 g, 94.3 %) as a soild.

**¹H NMR** (400MHz, CDCl₃) δ 5.43-5.18 (m, 2H), 3.46-3.34 (m, 5H), 2.63 (s, 1H), 2.23-2.07 (m, 2H), 2.23-2.07 (m, 1H), 1.96 (td, J=3.2, 12 Hz, 1H), 1.87-1.69 (m, 5H), 1.64-1.54 (m, 3H), 1.45-1.32 (m, 5H), 1.28-1.06 (m, 7H), 0.71 (s, 3H).

### Synthesis of A47

General Method 10: A mixture of 2-chloro-5-fluoropyridine (75.2 mg, 0.5722 mmol), **A46** (100 mg, 0.2861 mmol), Xantphos (16.5 mg, 0.02861 mmol) and cesium carbonate (187 mg, 0.5722 mmol) in dioxane (5 mL) was sparged with nitrogen for 2 min, then Pd₂(dba)₃ (26.1 mg,0.02861 mmol) was added. After stirring at 110°C for 16 h, the residue was poured into water (50 mL) and extracted with EtOAc (3 x 400 mL). The combined organic solution was washed with saturated brine (2 x 200 mL), dried over anhydrous Na₂SO₄,filtered and concentrated. The residue was purified by HPLC separation (column: YMC-Actus Triart C18 100*30mm*5um, gradient: 65-92% condition: water (0.05%HCl)-ACN, flow rate: 25 mL/min) to give **A47** (24 mg, 19%) as a soild.

**¹H NMR** (400MHz, CDCl₃) δ 8.30-8.19 (m, 1H), 8.10 (br s, 1H), 7.71 (br s, 1H), 7.47-7.38 (m, 1H), 3.40 (s, 5H), 2.63 (s, 1H), 2.71-2.59 (m, 1H), 2.42-2.20 (m, 2H), 2.03 (br d, J=16.5 Hz, 1H), 2.01-1.97 (m, 1H), 1.86-1.71 (m, 6H), 1.55-1.34 (m, 8H), 1.32-1.03 (m, 5H), 0.74 (s, 3H). **¹⁹F NMR** (376MHz, CDCl₃) -132.876. **LC-ELSD/MS** purity 99%, MS ESI calcd. for C₂₆H₃₈FN₂O₃ [M+H]⁺ 445, found 445.

### EXAMPLE 11: Synthesis of (3R,5R,8S,9S,10S,13S,14S,17S)-N-(5-cyanopyrazin-2-yl)-3-(ethoxymethyl)-10-ethyl-3-hydroxy-13-methylhexadecahydro-1H-cyclopenta[a]phenanthrene-17-carboxamide (A49)

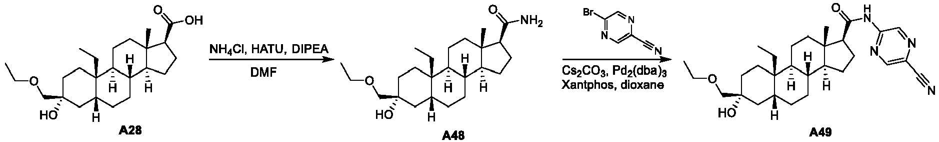

### Synthesis of A48

To a solution of **A28** (200 mg, 0.509 mmol) in DMF (5 mL) were added HATU (183 mg, 0.763 mmol) and DIPEA (526 mg, 4.07 mmol). After stirring for 15 mins at rt, NH₄Cl (108 mg, 2.03 mmol) was added. After stirring for 16 h, the reaction mixture was diluted with EtOAc (50 mL), washed with water (10 mL), 3% of LiCl aqueous (10 mL), water (10 mL) and brine (10 mL), dried over sodium sulfate, filtered and concentrated. The residue was purified by flash column (0~100% of EtOAc in PE) to afford A48 (180 mg, 90%) as a solid.

**¹H NMR** (400 MHz, CDCl₃) δ 5.27 (d, J=26.8 Hz, 2H), 3.58-3.50 (m, 2H), 3.46-3.35 (m, 2H), 2.74 (s, 1H), 2.20-2.10 (m, 2H), 1.98-1.90 (m, 2H), 1.80-1.56 (m, 7H), 1.56-1.15 (m, 17H), 0.80-0.74 (m, 3H), 0.70 (s, 3H); **LC-ELSD/MS** purity 99%, MS ESI calcd. for C₂₄H₃₂NO₃ [M +H]⁺ 392, found 392.

### Synthesis of A49

General Method 11: A mixture of 5-chloropyrazine-2-carbonitrile (85.3 mg, 0.612 mmol), **A48** (160 mg, 0.408 mmol), Xantphos (23.6 mg, 0.041 mmol) and cesium carbonate (265 mg, 0.816 mmol) in dioxane (3 mL) was sparged with nitrogen for 2 min, then Pd₂(dba)₃ (37.3 mg, 0.041 mmol) was added. After heating in microwave at 105°C for 1 h, the reaction mixture was filtered, concentrated and purified prep-HPLC (Column Xtimate C18 150*25mm*5um;Condition water(0.225%FA)-ACN Begin B 90;End B 100 Gradient Time(min) 7;100%B Hold Time(min) 1 FlowRate (ml/min) 25) to give **A49** (38 mg, 19%) as a solid.

**¹H NMR** (400 MHz, CDCl₃) δ 9.60 (d, J=1.2 Hz, 1H), 8.54 (d, J=1.2 Hz, 1H), 7.83 (m, 1H), 3.57-3.50 (m, 2H), 3.46-3.35 (m, 2H), 2.77 (s, 1H), 2.43-2.25 (m, 2H), 2.05-1.56 (m, 9H), 1.56-1.15 (m, 17H), 0.80-0.74 (m, 3H), 0.72 (s, 3H); **LC-ELSD/MS** purity 99%, MS ESI calcd. for C₂₉H₄₃N₄O₃ [M+H]⁺ 495, found 495.

### EXAMPLE 12: Synthesis of (3R,5S,8S,9S,10S,13S,14S,17S)-N-(5-cyanopyridin-2-yl)-3-(ethoxymethyl)-10-ethyl-3-hydroxy-13-methylhexadecahydro-1H-cyclopenta[a]phenanthrene-17-carboxamide (A68)

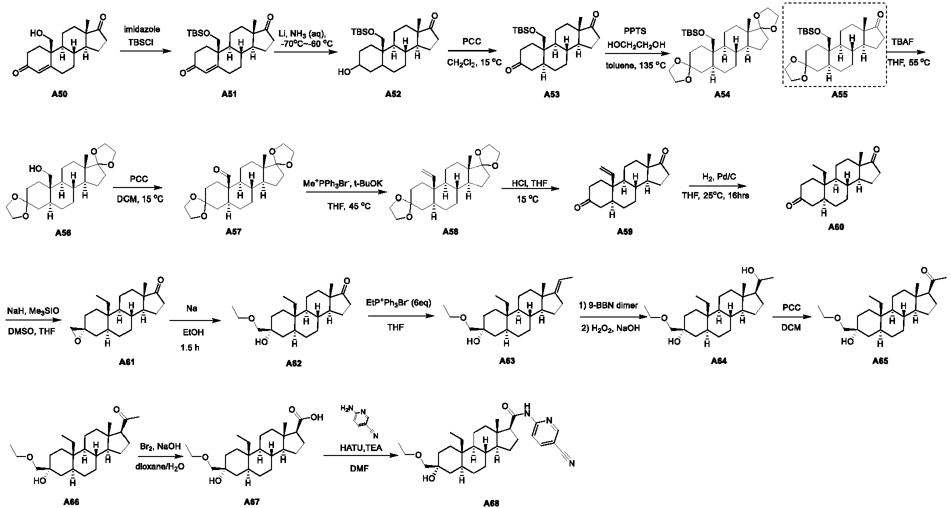

### Synthesis of A51

To a solution of **A50** (50.0 g, 165 mmol) in DCM (1000 mL) were added imidazole (16.7 g, 247 mmol) and TBSCl (37.2 g, 247 mmol) at rt. After 12 h, the reaction was purified together with another batch **A51.** The mixture was diluted water (1000 mL) and extracted with DCM (3 x 600 mL). The combined organic solution was washed with 1N HCl (500 mL), saturated NaHCO₃ (500 mL), saturated brine (500 mL), dried over anhydrous Na₂SO₄, filtered and concentrated under vacuum to give **A51** (110.0 g, 80%) as an oil.

**¹H NMR** (400 MHz, CDCl₃) δ_{H} 5.87 (s, 1H), 4.00-3.80 (m, 2H), 2.68-2.25 (m, 6H), 2.15-1.45 (m, 9H), 1.30-1.00 (m, 4H), 0.91 (s, 3H), 0.85 (s, 9H), 0.04 (s, 3H), 0.03 (s, 3H).

### Synthesis of A52

Lithium (9.2 g, 1314 mmol) was added to fresh prepared liquid ammonia (1.2 L) in portions at -70°C. After stirring at -70 °C for 1 h, a solution of **A51** (110.0 g, 263 mmol) in dry THF (800 mL) and t-butanol (38.9 g, 526 mmol) was added. After stirring at -70°C for 1 h, ammonium chloride (500 g) was added and the mixture was warmed to rt. After stirring for 16 h, the reaction was diluted with water (1L) and extracted with EtOAc (3 x 500 mL). The combined organic solution were washed with aqueous HCl solution (1 M, 2 x 500 mL), saturated aqueous NaHCO₃ solution (500 mL) and brine (1 L), dried over anhydrous Na₂SO₄, filtered and concentrated under reduced pressure to afford **A52** (110 g) as an oil, which was used directly for the next step without further purification.

**¹H NMR** (400 MHz, CDCl₃) δ_{H} 3.95 (d, *J=* 10.8 Hz, 1H), 3.89 (d, *J=* 10.8 Hz, 1H), 3.70-3.62 (m, 1H), 2.55-2.42 (m, 2H), 2.35-2.25 (m, 1H), 2.18-2.05 (m, 1H), 1.90-1.52 (m, 9H), 1.50-1.17 (m, 7H), 1.10-0.84 (m, 15H), 0.10 (s, 3H), 0.08 (s, 3H).

### Synthesis of A53

To a solution of **A52** (110 g, 261 mmol) in DCM (1500 mL) was added PCC (84.0 g, 391 mmol) and silica gel (100 g). After stirring at rt for 1 h, the suspension was filtered, and the filter cake was washed with DCM (2 x 500 mL). The combined filtrate was concentrated, and the material was purified by column chromatography on silica gel (PE/EtOAc=10/1~5/1) to afford **A53** (56.0 g, 51.3%) as a solid.

**¹H NMR** (400 MHz, CDCl₃) δ_{H} 3.96 (d, *J=* 10.8 Hz, 1H), 3.89 (d, *J=* 10.8 Hz, 1H), 2.51-2.40 (m, 4H), 2.36-2.28 (m, 1H), 2.20-2.02 (m, 2H), 2.00-1.35 (m, 10H), 1.30-1.15 (m, 4H), 1.10-0.95 (m, 1H), 0.93-0.87 (m, 12H), 0.09 (s, 3H), 0.08 (s, 3H).

### Synthesis of A54 & A55

To a solution of **A54** (24.0 g, 57.3 mmol) in toluene (250 mL) was added PPTS (2.86 g, 11.4 mmol) and ethane-1,2-diol (28.4 g, 458 mmol). After stirring 135°C for 16 h with Dean-Stark trap, the reaction mixture was concentrated, dissolved in EtOAc (500 mL) and washed with water (500 mL), brine (300 mL), dried over anhydrous Na₂SO₄, filtered and concentrated. The residue was triturated from EtOAc:PE (1:1, 50 mL) to give mixture of **A55 & A56** as a solid that was purified by silica gel chromatography (PE/EtOAc = 4/1) to afford **A55** and **A56** as solids.

**A55: ¹H NMR** (400 MHz, CDCl₃) δ_{H} 3.95-3.80 (m, 9H), 3.65 (d, *J* = 10.4 Hz, 1H), 2.27-2.20 (m, 1H), 2.03-1.87 (m, 1H), 1.83-1.32 (m, 14H), 1.30-1.15 (m, 3H), 1.05-0.75 (m, 15H), 0.05 (s, 3H), 0.04 (s, 3H).

A56: ¹H NMR (400 MHz, CDCl₃) δ_{H} 3.94 (s, 4H), 3.85 (d, *J* = 10.4 Hz, 1H), 3.65 (d, *J* = 10.0 Hz, 1H), 2.43 (dd, *J* = 6.6, 18.8 Hz, 1H), 2.27-2.17 (m, 1H), 2.00-1.87 (m, 2H), 1.82-1.35 (m, 11H), 1.28-1.12 (m, 5H), 1.07-0.95 (m, 2H), 0.92-0.88 (m, 12H), 0.06 (s, 3H), 0.05 (s, 3H).

### Synthesis of A57

To a solution of **A55** (33.0 g, 65.1 mmol) in THF (300 mL) was added TBAF.3H₂O (101.0 g, 325 mmol). After stirring at 55°C for 12 h, the mixture was poured into water (500 mL) and extracted with EtOAc (2 x 300 mL). The organic solution was washed with saturated brine (2 x 200 mL), dried over anhydrous Na₂SO₄, filtered and concentrated in vacuum to give product (25.5 g) which was purified by flash column (0~50% of EtOAc in PE) to give **A57** (18.7 g, 73.6%) as a solid.

**¹H NMR** (400 MHz, CDCl₃) δ_{H} 3.95-3.75 (m, 10H), 2.25-2.18 (m, 1H), 2.03-1.93 (m, 1H), 1.85-1.60 (m, 7H), 1.58-1.37 (m, 7H), 1.30-1.03 (m, 5H), 1.02-0.80 (m, 5H).

### Synthesis of A58

To a solution of **A57** (18.7 g, 47.6 mmol) in DCM (200 mL) was added PCC (15.3 g, 71.4 mmol) and silica gel (20.0 g) at rt. After stirring for 1 h, the suspension was filtered and the filter cake was washed with DCM (2 x 100 mL). The combined filtrate was concentrated to afford **A58** (18.5 g) as a solid, which was used directly for the next step without further purification.

**¹H NMR** (400 MHz, CDCl₃) δ_{H} 10.03 (s, 1H), 4.10-3.70 (m, 8H), 2.33 (d, *J* = 12.4 Hz, 1H), 2.10-1.15 (m, 18H), 1.13-1.00 (m, 3H), 0.76 (s, 3H).

### Synthesis of A59

To a suspension of MePPh₃Br (50.3 g, 141 mmol) in THF (200 mL) was added t-BuOK (15.8 g, 141 mmol). After stirring at 45°C for 0.5 h, a solution of **A58** (18.5 g, 47.3 mmol) in THF (100 mL) was added. After stirring at 45°C for 1 h, saturated NH₄Cl solution (200 mL) was added to the mixture and the organic solution was separated. The aqueous layer was collected and extracted with EtOAc (2 x 100 mL). The combined organic solution was washed with saturated brine (200 mL), filtered and concentrated under reduced pressure. The residue was purified by column chromatography (PE/EtOAc=10/1~4/1) to afford **A59** (13.6 g, 74.3%) as a solid.

**¹H NMR** (400 MHz, CDCl₃) δ_{H} 5.88 (dd, *J=* 10.8, 17.6 Hz, 1H), 5.35 (dd, *J=* 11.2 Hz, 1H), 5.02 (dd, *J*= 1.2, 18.0 Hz, 1H), 4.00-3.82 (m, 8H), 2.20-2.12 (m, 1H), 2.00-1.90 (m, 1H), 1.85-1.12 (m, 18H), 1.07-0.92 (m, 1H), 0.90-0.80 (m, 1H), 0.74 (s, 3H).

### Synthesis of A60

To a solution of **A59** (13.6 g, 35.0 mmol) in THF (80 mL) was added 12M HCl (29.1 mL, 350 mmol). After stirring at 15°C for 16 h, the reaction mixture was diluted with H₂O (50 mL) and adjust to pH = 9 with solid Na₂CO₃ (20 g). The aqueous layer was extracted with EtOAc (3 x 200 mL). The combined organic solution was washed with saturated brine (200 mL), dried over anhydrous Na₂SO₄, filtered and concentrated (10.5 g) and purified by flash column (15~20% of EtOAc in PE) to give **A60** (8.0 g, 76.9%) as a solid.

**¹H NMR** (400 MHz, CDCl₃) δ_{H} 6.03 (dd, *J=* 11.2, 18.0 Hz, 1H), 5.52 (d, *J =* 11.2 Hz, 1H), 5.20 (d, *J*= 18.0 Hz, 1H), 2.55-2.40 (m, 3H), 2.32-1.55 (m, 13H), 1.55-1.15 (m, 4H), 1.10-0.95 (m, 1H), 0.93-0.83 (m, 1H), 0.80 (s, 3H).

### Synthesis of A61

To a solution of **A60** (8.0 g, 26.6 mmol) in THF (200 mL) was added Pd-C (wet, 50%, 2.0 g) under Ar. The suspension was degassed under vacuum and purged with H₂ for three times. After hydrogenating at 206,843 Pa (30 psi) of hydrogen at rt for 16 h, the reaction mixture was filtered through a pad of Celite and washed with THF (2 x 200 mL). The filtrate was concentrated under reduced pressure and purified by flash column (15~20% of EtOAc in PE) to give **A61** (8.0 g, 99.5%) as a solid.

**¹H NMR** (400 MHz, CDCl₃) δ_{H} 2.55-2.25 (m, 5H), 2.15-1.15 (m, 17H), 1.10 (t, *J =* 8.0 Hz, 3H), 1.05-0.95 (m, 1H), 0.91 (s, 3H), 0.85-0.65 (m, 1H); **LC-ELSD/MS** purity 99%, MS ESI calcd. for C₂₀H₃₁O₂ [M+H]⁺ 303, found 303.

### Synthesis of A62

To a stirred solution of Me₃SIO (4.35 g, 19.8 mmol) in DMSO (30 mL) and THF (30 mL) was added NaH (791 mg, 19.8 mmol, 60 % in oil) at 0°C in portions. After stirring for 1 h under N₂, the mixture was added to a solution of **A61** (5.0 g, 16.5 mmol) in DMSO (30 mL). After stirring at rt 16 h, the reaction mixture was poured into ice-water (300 mL) and extracted with EtOAc (2 x 100 mL). The combined organic solution was washed with water (2 x 100 mL) and brine (100 mL), dried over anhydrous Na₂SO₄, filtered, and concentrated to give **A62** (5.0 g) as an oil, which was used as is.

**¹H NMR** (400 MHz, CDCl₃) δ_{H} 2.61 (s, 2H), 2.42 (dd, *J* = 8.8, 19.6 Hz, 1H), 2.15-1.40 (m, 11H), 1.35-1.05 (m, 8H), 1.02-0.70 (m, 10H).

### Synthesis of A63

To a fresh prepared solution of ethoxysodium (31.5 mL, 31.5 mmol, 1M in EtOH) was added **A62** (1.0 g, 3.15 mmol) in anhydrous ethanol (20 mL). After stirring at 75°C for 16 h, the reaction was diluted with water (50 mL) and concentrated to remove most of the solvent. The mixture was extracted with EtOAc (2 x 30 mL). The combined organic solution was washed with saturated brine (50 mL), dried over anhydrous Na₂SO₄, filtered and concentrated. The residue was purified by silica gel chromatography (0-40% of EtOAc in PE) to give **A63** (512 mg, 44.9%) as an oil.

**¹H NMR** (400 MHz, CDCl₃) δ_{H} 3.52 (q, *J =* 6.8 Hz, 2H), 3.21 (s, 2H), 2.42 (dd, *J =* 8.8, 19.6 Hz, 1H), 2.15-1.50 (m, 9H), 1.50-1.10 (m, 15H), 1.05-0.99 (m, 1H), 0.95 (t, *J =* 7.6 Hz, 3H), 0.90-0.65 (m, 4H); **LC-ELSD/MS** purity 99%, MS ESI calcd. for C₂₃H₃₈O₃Na [M+Na]⁺ 385, found 385.

### Synthesis of A64

To a suspension of bromo(ethyl)triphenylphosphorane (22.4 g, 60.4 mmol) in THF (100 mL) was added t-BuOK (6.76 g, 60.4 mmol) at 15°C under N₂. After stirring at 45°C for 1 h, a solution of **A63** (5.5 g, 15.1 mmol) in THF (100 mL) was added. After stirring at 45°C for 16 h, the mixture was treated with NH₄Cl (300 mL). The organic solution was separated and the collected aqueous phase was extracted with EtOAc (2 x 150 mL). The combined organic solution was washed with brine (2 x 100 mL), dried over anhydrous Na₂SO₄, filtered, concentrated in vacuum. The residue was purified by flash column (0~15% of EtOAc in PE) to give **A64** (5.0 g, 88%) as an oil.

**¹H NMR** (400 MHz, CDCl₃) δ_{H} 5.15-5.05 (m, 1H), 3.52 (q, *J =* 6.8 Hz, 2H), 3.21 (s, 2H), 2.40-2.10 (m, 4H), 1.85-1.75(m, 1H), 1.70-1.40 (m, 7H), 1.35-1.10 (m, 14H), 1.10-0.80 (m, 11H).

### Synthesis of A65

To a solution of **A64** (3.0 g, 8.0 mmol) in THF (50 mL) was added 9-BBN dimer (3.90 g, 16.0 mmol). After stirring at rt for 16 hr, the mixture was cooled to 0°C, ethanol (3.68 g, 80.0 mmol) and then NaOH (16 mL, 5 M, 80.0 mmol) were added slowly. After the addition was complete, H₂O₂ (8.01 mL, 80.0 mmol, 30%) was added slowly and the inner temperature was maintained below 15°C. After stirring at 75°C for 1 h, the reaction mixture was workup and purified together with another batch (done on same scale). Saturated aqueous Na₂S₂O₃ (300 mL) was added and the mixture was stirred at 0°C for another 1 h. The mixture was diluted with water (200 mL) and extracted with EtOAc (3x100 mL). The combined organic solution was washed with saturated brine (200 mL), dried over anhydrous Na₂SO₄, filtered and concentrated under vacuum to give **A65** (5.0 g) as an oil, which was used as is.

**¹H NMR** (400 MHz, CDCl₃) δ_{H} 3.75-3.65 (m, 1H), 3.51 (q, *J=* 6.8 Hz, 2H), 3.20 (s, 2H), 1.95-1.75 (m, 12H), 1.74-1.40 (m, 13H), 1.39-0.95 (m, 8H), 0.92 (t, *J =* 7.6 Hz, 3H), 0.66 (s, 3H).

### Synthesis of A66

To a solution of **A65** (0.8 g, 2.0 mmol) in DCM (20 mL) was added PCC (872 mg, 4.1 mmol) and silica gel (1 g). After stirring at rt for 3h, the reaction mixture was filtered, and the residue was washed with anhydrous DCM (2 x 50 mL). The combined organic solution was concentrated in vacuum and purified by flash column (0~20% of EtOAc in PE) to give **A66** (0.75 g, 94.6%) as a solid.

**¹H NMR** (400 MHz, CDCl₃) δ_{H} 3.52 (q, *J =* 6.8 Hz, 2H), 3.21 (s, 2H), 2.52 (t, *J =* 8.8 Hz, 1H), 2.25-2.10 (m, 5H), 2.05-1.95 (m, 1H), 1.90-1.80 (m, 1H), 1.75-1.65 (m, 4H), 1.55-1.05 (m, 18H), 1.00-0.80 (m, 5H), 0.62 (s, 3H); **LC-ELSD/MS** purity 99%, MS ESI calcd. for C₂₅H₄₁O₂ [M-H₂O+H]⁺ 373, found 373.

### Synthesis of A67

Liquid bromine (1.22 g, 7.7 mmol) was added slowly to a vigorously stirred aqueous sodium hydroxide (10.2 mL, 3 M, 30.7 mmol) at 0°C. When all the bromine was dissolved, the mixture was added slowly to a stirred solution of **A66** (1.0 g, 2.6 mmol) in dioxane (18 mL) and water (6 mL). After stirring at rt for 16 h, the reaction was quenched with aqueous Na₂SO₃ solution (30 mL) and pH adjusted with hydrochloride acid (3 N) to yield a precipitate. The suspension was diluted with EtOAc (50 mL) and the organic phase was collected. The aqueous phase was extracted EtOAc (2 x 50 mL). The combined organic solution was washed with saturated brine (50 mL), dried over anhydrous Na₂SO₄, filtered and concentrated in vacuum to give **A67** (0.8 g, 80%) as a solid.

**¹H NMR** (400 MHz, CDCl₃) δ_{H} 3.71 (s, 1H), 3.52 (q, *J =* 6.8 Hz, 2H), 3.21 (s, 2H), 2.37 (t, *J* = 9.2 Hz, 1H), 2.15-2.00 (m, 3H), 1.90-1.70 (m, 2H), 1.60-1.10 (m, 20H), 1.00-0.65 (m, 5H), 0.74 (s, 3H).

### Synthesis of A68

To a solution of **A67** (200 mg, 0.5 mmol) in pyridine (5 mL) was added EDCI (389 mg, 2.0 mmol). After stirring rt for 10 mins, 6-aminopyridine-3-carbonitrile (120 mg, 1.0 mmol) was added. After stirring at 45°C for 16 h, the reaction was poured into water (50 mL) and extracted with EtOAc (2 x 20 mL). The combined organic solution was washed with saturated brine (2 x 20 mL), dried over anhydrous Na₂SO₄, filtered and concentrated in vacuum. The residue was purified by flash column (10~20% of EtOAc in PE) to afford **A68** (26 mg, 10.3%) as a solid. **¹H NMR** (400 MHz, CDCl₃) δ_{H} 8.52 (d, *J=* 1.6 Hz, 1H), 8.36 (d, *J*= 8.8 Hz, 1H), 7.92 (dd, J = 2.4, 6.4 Hz, 1H), 7.86 (s, 1H), 3.52 (q, *J*= 6.8 Hz, 2H), 3.21 (s, 2H), 2.40-2.20 (m, 2H), 2.15-1.95 (m, 2H), 1.90-1.65 (m, 6H), 1.50-1.05 (m, 16H), 1.00-0.80 (m, 6H), 0.75 (s, 3H); **LC-ELSD/MS** purity 99%, MS ESI calcd. for C₃₀H₄₄N₃O₃ [M+H]⁺ 494, found 494.

### EXAMPLE 13: Synthesis of (3R,5R,8R,9R,10S,13S,14S,17S)-3-hydroxy-3,13-dimethyl-N-(2-methyl-5,6,7,8-tetrahydroimidazo[1,2-a]pyridin-3-yl)hexadecahydro-1H-cyclopenta[a]phenanthrene-17-carboxamide (A69)

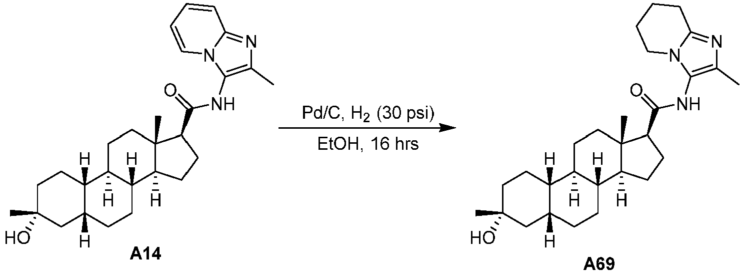

To a solution of **A14** (50 mg, 0.111 mmol) in EtOH (5 mL) was added Pd/C (50 mg, >50% water). After hydrogenating under 206,843 Pa (30 psi) of hydrogen at 25°C for 16 h, the mixture was filtered through a pad of celite and the filtrate was concentrated in vacuum to give product which was purified by SFC (column: DAICEL CHIRALCEL OD-H (250mm*30mm, 5um)), gradient: 40-40% B (A= 0.1%NH₃/H₂O, B= MeOH), flow rate: 60 mL/min) to give **ST-320-018-024** (Rt: 4.870, 39 mg, 65%) as a solid.

**¹H NMR** (400 MHz, MeOD) δ 3.67 (t, J=5.8 Hz, 2H), 2.74 (t, *J*=6.4 Hz, 2H), 2.47 (t, *J*=9.2 Hz, 1H), 2.25-2.11 (m, 1H), 2.02 (s, 3H), 2.00-1.93 (m, 3H), 1.92-1.83 (m, 5H), 1.82-1.65 (m, 4H), 1.56-1.28 (m, 11H), 1.24 (s, 3H), 1.21-1.08 (m, 4H), 0.77 (s, 3H); **LC-ELSD/MS** purity 98%, MS ESI calcd. for C₂₈H₄₄N₃O₂ [M+H]⁺ 454, found 454.

### EXAMPLE 14: Synthesis of (3R,5R,8R,9R,10S,13S,14S,17S)-N-(5-cyanopyrazin-2-yl)-3-hydroxy-13-methylhexadecahydro-1H-cyclopenta[a]phenanthrene-17-carboxamide (A75)

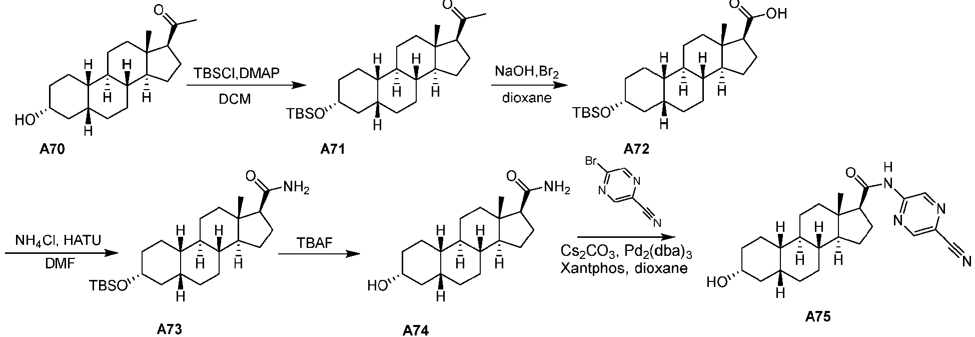

### Synthesis of A71

To a solution of 19-Norpregnan-20-one, 3-hydroxy-,(3α,5β)- (CAS# 16051-97-1) (500 mg, 1.64 mmol), imidazole (334 mg, 4.92 mmol) and DMAP (386 mg, 1.64 mmol) in DCM (10 mL) was added TBSCl (988 mg, 6.56 mmol) at 0°C under N₂. After stirring at 30°C for 16 h, the mixture was diluted with water (15 mL) and extracted with DCM (35 mL). The combined organic phases were washed with brine (20 mL), dried with anhydrous Na₂SO₄, filtered and concentrated to give a solid (610 mg) which was purified by flash column (0~5% of EtOAc in PE) to afford **A71** (590 mg, 86%) a solid.

**¹H NMR** (400 MHz, CDCl₃) δ_{H} 3.64-3.52 (m, 1H), 2.53 (t, J=8.9 Hz, 1H), 2.23-2.07 (m, 4H), 2.02-1.81 (m, 2H), 1.78-1.59 (m, 6H), 1.52-1.38 (m, 4H), 1.34-0.96 (m, 10H), 0.92-0.87 (m, 9H), 0.60 (s, 3H), 0.09-0.03 (m, 6H).

### Synthesis of A72

To aqueous NaOH (10% in water 11.7 g, 29.7 mmol) was added Br₂ (1.18 g, 7.44 mmol) at 0°C. After stirring at 0°C for 30 min, the resulting mixture was added to a solution of A71 (590 mg, 1.40 mmol) in dioxane (6 mL). After stirring at 25°C for 6 h, the reaction mixture was added another batch of HBrO prepared from aqueous NaOH (10% in water 11.7 g, 29.7 mmol) and Br₂ (1.18 g, 7.44 mmol) at 0°C. After stirring at rt for 16 h additionally, the reaction mixture was diluted with Na₂SO₃ aqueous (15 mL) and stirred at 75°C for 1 h. After cooling to rt, the pH was adjusted to 3~4 with HCl (4 M) and stirred for 30 min. The precipitated solid was filtered and dried to give **A72** (514 mg, 87%) as a gum.

**¹H NMR** (400 MHz, CDCl₃) δ_{H} 3.58 (m, 2H), 2.34 (m, 1H), 2.08-2.01 (m, 2H), 1.94-1.60 (m, 8H), 1.59-1.36 (m, 2H), 1.33-0.98 (m, 11H), 0.89 (s, 9H), 0.70 (s, 3H), 0.12--0.04 (m, 6H).

### Synthesis of A73

To a solution of **A72** (300 mg, 0.713 mmol) in DMF (8 mL) was added HATU (809 mg, 2.13 mmol) and DIPEA (275 mg, 2.13 mmol). After stirring at 25°C for 30 min, NH₄Cl (190 mg, 3.56 mmol) was added. After stirring at 25°C for 16 h, the reaction mixture was diluted with water (50 mL) and extracted with EtOAc (50 mL). The organic solution was washed with brine (3 x 50 mL), dried over sodium sulfate, filtered and concentrated. The residue was triturated with PE (10 mL) to give **A73** (160 mg) as an oil, which was purified by flash column (0~60% of EtOAc in PE) to give **A73** (60 mg, 38%) as oil.

**¹H NMR** (400 MHz, CDCl₃) δ_{H} 5.51-5.15 (m, 2H), 3.69-3.48 (m, 1H), 2.24-2.06 (m, 2H), 2.00-1.83 (m, 3H), 1.70-1.61 (m, 6H), 1.53-1.37 (m, 4H), 1.35-1.03 (m, 9H), 0.89 (s, 9H), 0.76-0.68 (m, 3H), 0.06 (s, 6H).

### Synthesis of A74

To a solution of **A73** (60 mg, 0.142 mmol) in THF (2 mL) was added TBAF (295 mg, 1.13 mmol). After stirring at 60°C for 16 h, the mixture was poured into water (15 mL) and extracted with EtOAc (2 x 30 mL). The combined organic solution was washed with brine (50 mL), dried over anhydrous Na₂SO₄, filtered and concentrated under vacuum to give a product, which was purified by prep-HPLC (Column Xtimate C18 150*25mm*5um Condition water(0.225%FA)-ACN Begin B: 28% End B: 58% Gradient Time(min) 7.5 100%B Hold Time(min) 2; FlowRate (ml/min) 30) to give **A74** (9.2 mg, 21%) as a solid.

**¹H NMR** (400 MHz, CDCl₃) δ_{H} 5.46-5.11 (m, 2H), 3.64 (m, 1H), 2.26-2.06 (m, 2H), 2.01-1.86 (m, 2H), 1.85-1.56 (m, 9H), 1.50-1.34 (m, 3H), 1.32-1.04 (m, 9H), 0.72 (s, 3H); **LC-ELSD/MS** purity 99%, MS ESI calcd. for C₁₉H₃₂NO₂ [M+H]⁺306, found 306.

### Synthesis of A75

A mixture of **A74** (200 mg, 0.654 mmol), 5-bromopyrazine-2-carbonitrile (239 mg, 1.30 mmol), Xantphos (37.8 mg, 0.065 mmol) and Cs₂CO₃ (423 mg, 1.30 mmol) in dioxane (10 mL) was sparged with nitrogen for 2 min, then Pd₂(dba)₃ (59.8 mg, 0.065 mmol) was added. After stirring at 115°C for 16 h, the reaction mixture was filtered through a pad of Celite and extracted with DCM (3 x 20 mL). The combined organic solution was concentrated and purified by flash column (0~25% of EtOAc in PE) to give **A75** (35 mg, 13%) as a solid.

**¹H NMR** (400MHz, CDCl₃) δ_{H} 9.67 (d, J=1.3 Hz, 1H), 8.55 (d, J=1.5 Hz, 1H), 7.84 (s, 1H), 3.70-3.60 (m, 1H), 2.49-2.38 (m, 1H), 2.31-2.23 (m, 1H), 2.01-1.95 (m, 1H), 1.88-1.60 (m, 9H), 1.56-1.05 (m, 13H), 0.75 (s, 3H). **LC-ELSD/MS** purity 99%, MS ESI calcd. for C₂₄H₃₃N₄O₂[M+H]⁺409, found 409.

### EXAMPLE 15: Synthesis of (3R,5R,8R,9S,10S,13S,14S,17S)-N-(3-chloro-6-methylpyridin-2-yl)-3-ethyl-3-hydroxy-10,13-dimethylhexadecahydro-1H-cyclopenta[a]phenanthrene-17-carboxamide (A80)

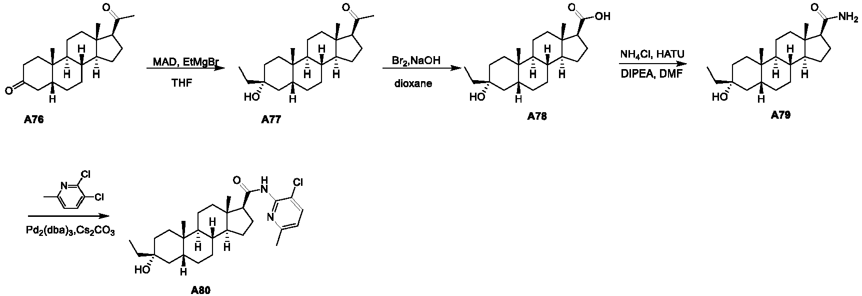

### Synthesis of A77

To a solution of 2, 6-di-tert-butyl-4-methylphenol (22.5 g, 102 mmol) in toluene (50 mL) was added drop-wise AlMe₃ (25.5 mL, 51.0 mmol, 2 M in toluene) at 0°C. The mixture was stirred at 25°C for 1 h and used directly as MAD solution. To the MAD (51.0 mmol) was added a solution of Pregnane-3,20-dione, (5β)- (5 g, 15.7 mmol) in DCM (25 mL) dropwise at -70°C. After stirring at -70°C for 1 h under N₂, bromo(ethyl)magnesium (15.6 mL, 47.0 mmol, 3 M in ethyl ether) was added drop wise at -70°C. After stirring at -70°C for another 1 h, the reaction mixture was poured into saturated aqueous citric acid (50 mL) at below 10°C, followed by adding ice-water (60 mL) and stirred for another 10 min. The aqueous phase was extracted with EtOAc (2 x 40 mL). The combined organic solution was washed with brine (20 mL), dried over anhydrous Na₂SO₄, filtered and concentrated. The residue was purified by flash column (10% of EtOAc in PE) to give **A77** (3.7 g) as solid.

**¹H NMR** (400 MHz, CDCl₃) δ_{H} 2.59-2.46 (m, 1H), 2.22-2.09 (m, 4H), 2.04 (s, 1H), 2.03-1.97 (m, 1H), 1.94-1.78 (m, 2H), 1.73-1.63 (m, 3H), 1.62-1.35 (m, 8H), 1.31-1.20 (m, 6H), 1.19-0.95 (m, 3H), 0.93 (s, 3H), 0.88 (t, *J=* 7.6 Hz, 3H), 0.59 (s, 3H).

### Synthesis of A78

Br₂ (2.52 g, 15.8 mmol) was added to aqueous NaOH (1.90 g, 10% in water, 47.5 mmol). The mixture was stirred at 0°C for 30 min and added to a solution of **A77** (1.1 g, 3.17 mmol) in dioxane (6 mL) and water (2 mL). After stirring at 25°C for 2 h, the reaction mixture was washed with EtOAc (20 mL), and the aqueous layer was acidified with HCl (12 M in water, 12 mL) to adjust the pH to 1 and stirred for 30 min. The precipitated solid was filtered, washed with water (2 x 12 mL), dried and purified by flash column (0~30% of EtOAc in PE) to give **A78** (1.06 g) as a solid.

**¹H NMR** (400 MHz, CDCl₃) δ_{H} 2.39 (t, *J=* 9.6 Hz, 1H), 2.15-2.01 (m, 2H), 1.94-1.80 (m, 3H), 1.70 (br d, *J=* 14.4 Hz, 3H), 1.59 (td, *J=* 7.6, 14.9 Hz, 5H), 1.45 (br dd, *J=* 10.4, 19.6 Hz, 7H), 1.31-1.18 (m, 6H), 0.94 (s, 3H), 0.88 (t, *J* = 7.6 Hz, 3H), 0.71 (s, 3H). **LC-ELSD/MS** purity 99%, MS ESI calcd. for C₂₂H₃₅O₂[M-H₂O+H]⁺331, found 331

### Synthesis of A79

To a solution of **A78** (530 mg, 1.52 mmol) in DMF (5 mL) at rt was added HATU (866 mg, 2.28 mmol) and DIPEA (785 mg, 6.08 mmol) followed by NH₄Cl (161 mg, 3.04 mmol). After stirring for 16 h, reaction was poured into water (50 mL) and extracted with EtOAc (3 x 40 mL). The combined organic phase was washed with brine (2 x 50 mL), then the organic phase was wished with LiCl (3 x 50 mL, 5% in water) and dried over anhydrous Na₂SO₄, filtered and concentrated to give **A79** (266 mg) as a solid.
**¹H NMR** (400 MHz, CDCl₃) δ_{H} 5.25 (br d, *J=* 11.6 Hz, 2H), 2.23-2.08 (m, 2H), 2.02-1.95 (m, 1H), 1.93-1.61 (m, 6H), 1.54-1.35 (m, 8H), 1.34-1.06 (m, 9H), 0.94 (s, 3H), 0.88 (t, *J* = 7.6 Hz, 3H), 0.70 (s, 3H). **LC-ELSD/MS** purity 99%, MS ESI calcd. for C₂₂H₃₈NO₂[M+H]⁺348, found 348

### Synthesis of A80

A mixture of 2,3-dichloro-6-methylpyridine (79.8 mg, 0.493 mmol), **A79** (150 mg, 0.449 mmol), Xantphos (25.9 mg, 0.0449 mmol) and Cs₂CO₃(172 mg, 0.898 mmol) in dioxane (3 mL) was sparged with nitrogen for 2 min, then Pd₂(dba)₃ (41.1 mg, 0.0449 mmol) was added. After stirring at 130°C for 16 h, the mixture was poured into water (8 mL) and extracted with EtOAc (2 x 8 mL). The combined organic solution was washed with brine (8 mL), dried over anhydrous Na₂SO₄, filtered and concentrated. The residue was purified by flash column (0~75% of EtOAc in PE) to give **A80** (120.2 mg, 57%) as a solid.

**¹H NMR** (400 MHz, CDCl₃) δ_{H} 7.58 (d, *J=* 8.0 Hz, 1H), 7.48 (s, 1H), 6.92 (d, *J* = 8.4 Hz, 1H), 2.52 (s, 3H), 2.50-2.44 (m, 1H), 2.37-2.28 (m, 1H), 2.14 (br d, *J=* 10.3 Hz, 1H), 1.94-1.79 (m, 3H), 1.77-1.61 (m, 3H), 1.56-1.36 (m, 8H), 1.35-1.15 (m, 8H), 1.05-0.97 (m, 1H), 0.94 (s, 3H), 0.88 (t, *J =* 7.6 Hz, 3H), 0.79 (s, 3H); **LC-ELSD/MS** purity 99%, MS ESI calcd. for C₂₈H₄₂ClN₂O₂[M+H]⁺473, found 473.

### EXAMPLE 16: Synthesis of (3R,5R,8R,9R,10S,13S,14S,17S)-N-(3-chloro-6-methylpyridin-2-yl)-3-ethyl-3-hydroxy-13-methylhexadecahydro-1H-cyclopenta[a]phenanthrene-17-carboxamide (A85)

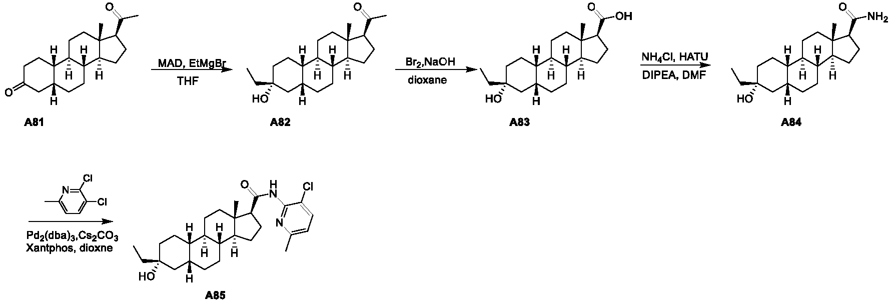

### Synthesis of A82

To a solution of BHT (12 g, 54.4 mmol) in toluene (120 mL) under nitrogen at 0°C was added trimethylaluminum (2 M in toluene, 14 mL, 28 mmol) dropwise. The mixture was stirred at 25°C for 1 h and used directly as a solution of MAD without further purification. To the above freshly prepared MAD solution was added a solution of **A81** (6 g, 19.8 mmol, CAS# 10594-58-8) in DCM (60 mL) dropwise at -70°C. After stirring at -70°C for 1 h under N₂, EtMgBr (20 mL, 60 mmol, 3 M in ethyl ether) was added dropwise at -70°C. After stirring at -70°C for 1 h, the reaction mixture was poured into saturated aqueous citric acid (600 mL) at below 10°C and extracted with DCM (2 x 800 mL). The combined organic solution was dried over Na₂SO₄, filtered and concentrated in vacuum to give product, which was triturated by PE (10 mL) to give **A82** (3.83 g, 58%) as a solid.

**¹H NMR** (400 MHz, CDCl₃) δ_{H} 2.56-2.50 (m, 1H), 2.24-2.10 (m, 4H), 2.07-1.99 (m, 1H), 1.89-1.51 (m, 9H), 1.50-1.20 (m, 12H), 1.19-1.00 (m, 3H), 0.98-0.80 (m, 3H), 0.61 (s, 3H).

### Synthesis of A83

Br₂ (2.39 g, 15 mmol) was added to aqueous NaOH (10% in water 1.9 g, 47.5 mmol). The mixture was stirred at 0°C for 30 min and added to a solution of **A82** (1 g, 3 mmol) in dioxane (9 mL) and water (3 mL). After stirring at 25°C for 2 h, the reaction mixture was washed with EtOAc (10 mL). The aqueous layer was acidified with HCl (12 M in water, 12 mL) to pH ~ 1 and stirred for 30 min. The precipitated solid was filtered out, washed with water (2 x 12 mL), dried and purified by flash column (0~30% of EtOAc in PE) to give **A83** (500 mg, 50%) as a solid.

**¹H NMR** (400 MHz, CDCl₃) δ_{H} 2.43-2.38 (m, 1H), 2.20-2.00 (m, 2H), 1.87-1.57 (m, 8H), 1.56-149 (m, 3H), 1.48-1.25 (m, 10H), 1.24-1.00 (m, 4H), 0.89-0.72 (m, 3H), 0.73 (s, 3H).

### Synthesis of A84

To a solution of **A83** (500 mg, 1.49 mmol) in DMF (3 mL) was added HATU (847 mg, 2.23 mmol) and DIPEA (770 mg, 5.96 mmol). After stirring at rt for 10 min, ammonia chloride (157 mg, 2.98 mmol) was added. After stirring overnight, the reaction mixture was poured into water (8 mL) and extracted with EtOAc (3 x 8 mL). The combined organic solution was washed with brine (2 x 8 mL). The organic phase was wished with LiCl (3 x 8 mL, 5% in water) and dried over anhydrous Na₂SO₄, filtered and concentrated to give **A84** (300 mg, 60%) as a solid.

**¹H NMR** (400 MHz, CDCl₃) δ_{H} 6.00-5.85 (m, 1H), 5.70-5.55 (m, 1H), 2.26-2.24 (m, 1H), 2.23-2.10 (m, 1H), 2.0-1.85 (m, 1H), 1.80-154 (m, 9H), 1.50-1.20 (m, 12H), 1.20-1.05 (m, 3H), 0.95-0.82 (m, 3H), 0.90 (s, 3H).

### Synthesis of A85

A mixture of 2,3-dichloro-6-methylpyridine (73 mg, 0.45 mmol), **A84** (150 mg, 0.449 mmol), Xantphos (26 mg, 0.044 mmol) and Cs₂CO₃(300 mg, 0.923 mmol) in dioxane (3 mL) was sparged with nitrogen for 2 min, then Pd₂(dba)₃ (42 mg, 0.045 mmol) was added. After stirring at 130°C for 16 h, the reaction mixture was poured into water (8 mL) and extracted with EtOAc (2 x 8 mL). The combined organic solution was washed with brine (8 mL), dried over anhydrous Na₂SO₄, filtered and concentrated. The residue was purified by flash column (0~75% of EtOAc in PE) to give **A85** (76.6 mg) as a solid, which was combined with another batch of 63.6 mg prepared from 150 mg of **A84.** The combined material (140 mg) was purified by SFC (column: DAICEL CHIRALPAK AS-H (250mm*30mm, 5um), condition: 0.1%NH₃H₂O EtOH, Begin B: 30%, End B: 30%, FlowRate (ml/min):65) to give **A85** (101 mg) as a solid.

**¹H NMR** (400 MHz, CDCl₃) δ_{H} 7.58 (d, J=8.0, 1H), 7.26 (s, 1H), 6.91 (d, *J*=8.0, 1H), 2.52-2.48 (m, 4H), 2.30-2.25 (m, 1H), 2.20-2.10 (m, 1H), 1.90-1.70 (m, 6H), 1.69-1.51 (m, 6H), 1.50-1.25 (m, 12H), 0.90-0.86 (m, 3H), 0.81 (m, 3H). **LC-ELSD/MS** purity 99%, MS ESI calcd. for C₂₇H₄₀ClN₂O₂ [M+H]+ 459, found 459.

The following examples were made using the general procedures in Examples 1 to 16 with the listed reactant and SM.

| **Example** | **SM** | **method** | **Reactant** | **STRUCTURE** | **¹H NMR** | **LC-ELSD/MS** |
|---|---|---|---|---|---|---|
| **20** | **A1** | 1 | pyridin-2-amine | 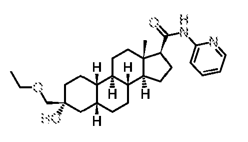 | (400 MHz, CDCl₃) δ = 11.75 (s, 1H), 8.84 (d, *J*=9.0 Hz, 1H), 8.29 - 8.07 (m, 2H), 7.35 (t, *J*=6.0 Hz, 1H), 3.52 (q, *J*=6.9 Hz, 2H), 3.48 - 3.43 (m, 1H), 3.41 - 3.34 (m, 1H), 2.69 (t, *J*=9.3 Hz, 1H), 2.30 - 2.18 (m, 1H), 2.05 - 1.91 (m, 2H), 1.78 - 1.73 (m, 4H), 1.72 - 1.63 (m, 5H), 1.48 - 1.31 (m, 8H), 1.20 (t, *J*=7.0 Hz, 4H), 1.16 - 0.93 (m, 3H), 0.75 (s, 3H). | purity 99%, MS ESI calcd. for C₂₇H₄₁N₂ O₃[M+H] ⁺ 441, found 441 |
| **21** | **A1** | 1 | pyridin-3-amine | 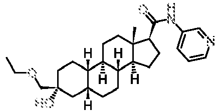 | (400 MHz, CDCl₃) δ = 8.53 (d, J*=*2.5 Hz, 1H), 8.33 (m, 1H), 8.18 (d, 1H), 7.24 (s, 1H), 7.06 (s, 1H), 3.58-3.37 (m, 4H), 2.76 (s, 1H), 2.38-2.21 (m, 2H), 2.03-1.95 (m, 1H), 1.89-1.69 (m, 6H), 1.65-1.35 (m, 10H), 1.32-1.24 (m, 2H), 1.21 (m, 4H), 1.16-1.07 (m, 2H), 0.75 (s, 3H). | purity 99%, MS ESI calcd. for C27H41 N2O3 [M +H]+ 441, found 441. |
| **22** | **A1** | 3 | 6-aminopyr idine-3-carbonitri le | 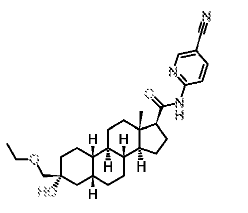 | (400 MHz, CDCl₃) δ 8.60-8.47 (m, 1H), 8.41-8.33 (m, 1H), 7.96-7.90 (m, 1H), 7.87 (s, 1H), 3.57-3.50 (m, 2H), 3.47-3.39 (m, 2H), 2.75 (m, 1H), 2.41-2.34 (m, 1H), 2.31-2.19 (m, 1H), 2.05-1.98 (m, 1H), 1.86-1.72 (m, 6H), 1.52-1.36 (m, 8H), 1.30-1.06 (m, 10H), 0.73 (s, 3H) | purity 99%, MS ESI calcd. for C28H40 N3O3 [M+H]+ 466, found 466. |
| **23** | **A1** | 3 | 6-amino-5-methylpy ridine-3-carbonitri le | 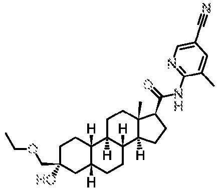 | (400 MHz, CDCl₃) δ = 8.59-8.46 (m, 1H), 7.80 (s, 1H), 7.44 (s, 1H), 3.57-3.50 (m, 2H), 3.47-3.39 (m, 2H), 2.74 (s, 1H), 2.48-2.40 (m, 1H), 2.31 (s, 3H), 2.12-2.05 (m, 1H), 1.88-1.72 (m, 6H), 1.66-1.58 (m, 3H), 1.52-1.38 (m, 7H), 1.30-1.10 (m, 9H), 0.78 (s, 3H) | purity 99%, MS ESI calcd. for C29H42 N3O3 [M+H]+ 480, found 480. |
| **24** | **A7** | 4 | 2-chloro-6-methylpy ridine | 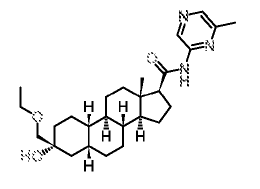 | (400 MHz, CDCl₃) δ 9.35 (s, 1H), 8.19 (s, 1H), 7.55 (s, 1H), 3.53 (q, J=7.0 Hz, 2H), 3.49-3.37 (m, 2H), 2.76 (s, 1H), 2.45 (s, 3H), 2.26-2.10 (m, 2H), 2.02 (br s, 1H), 1.95-1.56 (m, 8H), 1.53-0.99 (m, 16H), 0.75 (s, 3H) | purity 99%, MS ESI calcd. for C27H42 N3O3 [M+H]+ 456, found 456 |
| **25** | **A7** | 4 | 2-chloro-5-methylpy razine | 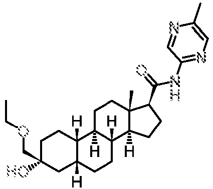 | (400 MHz, CDCl₃) δ 9.43 (s, 1H), 8.08 (s, 1H), 7.56 (s, 1H), 3.53 (q, J=7.0 Hz, 2H), 3.49-3.37 (m, 2H), 2.75 (s, 1H), 2.52 (s, 3H), 2.36-2.20 (m, 2H), 2.08-1.98 (m, 1H), 1.92-1.56 (m, 8H), 1.53-0.99 (m, 16H), 0.75 (s, 3H) | purity 99%, MS ESI calcd. for C27H42 N3O3 [M+H]+ 456, found 456. |
| **26** | **A7** | 4 | 5,6-dichlorop yridine-3-carbonitri le | 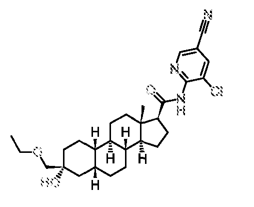 | (400 MHz, CDCl₃) δ 8.64 (d, J=2.0 Hz, 1H), 7.94 (d, J=2.0 Hz, 1H), 7.89 (s, 1H), 3.53 (q, J=6.9 Hz, 2H), 3.48-3.37 (m, 2H), 2.74 (br s, 1H), 2.57-2.48 (m, 1H), 2.38-2.25 (m, 1H), 2.06 (br d, J=11.5 Hz, 1H), 1.92-1.58 (m, 8H), 1.52-1.33 (m, 6H), 1.33-1.05 (m, 10H), 0.78 (s, 3H) | purity 99%, MS ESI calcd. for C28H39 ClN3O3 [M+H]+ 500, found 500 |
| **27** | **A7** | 4 | 5-bromo-6-methylpy ridine-2-carbonitri le | 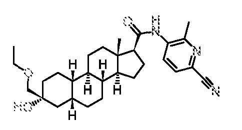 | (400 MHz, CDCl3)δ = 8.72 (d, J=8.5 Hz, 1H), 7.56 (d, J=8.5 Hz, 1H), 7.01 (s, 1H), 3.54 (q, J=7.0 Hz, 2H), 3.48-3.34 (m, 2H), 2.85-2.64 (m, 1H), 2.55 (s, 3H), 2.44-2.33 (m, 1H), 2.32-2.20 (m, 1H), 2.02 (br d, J=11.5 Hz, 1H), 1.93-1.56 (m, 7H), 1.50-1.05 (m, 17H), 0.74 (s, 3H) | purity 99%, MS ESI calcd. for C29H42 N3O3 [M+H]+ 480, found 480 |
| **28** | **A1** | 3 | 5-aminopyr azine-2-carbonitri le | 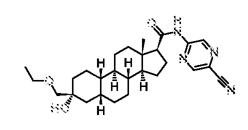 | (400 MHz, CDCl₃) δ = 9.66 (d, J=1.3 Hz, 1H), 8.55 (d, J=1.3 Hz, 1H), 7.83 (s, 1H), 3.57-3.50 (m, 2H), 3.47-3.39 (m, 2H), 2.76 (s, 1H), 2.45-2.39 (m, 1H), 2.32-2.23 (m, 1H), 2.03-1.97 (m, 1H), 1.86-1.70 (m, 6H), 1.65-1.59 (m, 2H), 1.48-1.39 (m, 6H), 1.31-1.08 (m, 10H), 0.75 (s, 3H) | purity 99%, MS ESI calcd. for C27H39 N4O3 [M+H]+ 467, found 467 |
| **29** | **A1** | 3 | 6-aminopyr idazine-3-carbonitri le | 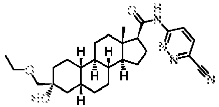 | (400 MHz, CDCl₃) δ = 8.64 (d, J = 9.3 Hz, 1H), 8.40 (s, 1H), 7.78 (d, J = 9.3 Hz, 1H), 3.57-3.51 (m, 2H), 3.46-3.39 (m, 2H), 2.75 (s, 1H), 2.51-2.44 (m, 1H), 2.32-2.22 (m, 1H), 2.07-2.01 (m, 1H), 1.85-1.75 (m, 5H), 1.65-1.58 (m, 2H), 1.47-1.38 (m, 6H), 1.33-1.08 (m, 11H), 0.74 (s, 3H) | purity 99%, MS ESI calcd. for C27H39 N4O3 [M+H]+ 467, found 467 |
| **30** | **A1** | 1 | 1,3-dimethyl-1H-pyrazol-5-amine | 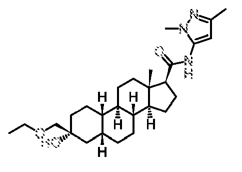 | (400 MHz, CDCl₃) δ 6.78 (s, 1H), 5.99 (s, 1H), 3.66 (s, 3H), 3.53 (q, J = 6.8 Hz, 2H), 3.42 (q, J = 9.2 Hz, 2H), 2.76 (s, 1H), 2.21 (s, 5H), 2.03-1.95 (m, 1H), 1.76 (br s, 6H), 1.68-1.60 (m, 2H), 1.51-1.08 (m, 16H), 0.75 (s, 3H) | purity 99%, MS ESI calcd. for C27H44 N3O3 [M+H]+ 458, found 458 |
| **31** | **A9 (3R,5R, 8R,9S,1 0S,13S,1 4S,17S)-3-(ethoxy methyl)-3-hydroxy -10,13-dimethy lhexade cahydro -1H-cyclope nta[a]p henanth rene-17-carboxy lic acid (reporte d in** WO 2018013 613**)** | 3 | 6-aminopyr idine-3-carbonitri le | 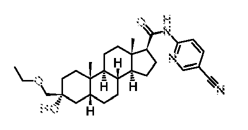 | (400 MHz, CDCl₃) δ = 8.55-8.52 (m, 1H), 8.37 (d, J=8.5 Hz, 1H), 7.95-7.90 (m, 1H), 7.87 (s, 1H), 3.54 (q, J=7.0 Hz, 2H), 3.47-3.37 (m, 2H), 2.39-2.33 (m, 1H), 2.31-2.20 (m, 1H), 2.04-1.98 (m, 1H), 1.97-1.65 (m, 6H), 1.48-1.09 (m, 17H), 1.05-0.98(m, 1H), 0.94 (s, 3H), 0.71 (s, 3H) | purity 99%, MS ESI calcd. for C29H42 N3O3 [M+H]+ 480, found 480. |
| **32** | **A13** | 6 | 3-bromo-2-methylpy ridine | 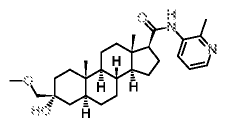 | (400 MHz, CDCl₃) δ 8.34 (d, J=8.0 Hz, 1H), 8.24 (s, 1H), 7.18-7.13 (m, 1H), 6.83 (s, 1H), 3.38 (s, 3H), 3.18 (s, 2H), 2.51 (s, 3H), 2.35-2.23 (m, 2H), 2.08-1.98 (m, 2H), 1.90-1.56 (m, 4H), 1.56-1.15 (m, 14H), 1.10-0.80 (m, 2H), 0.76 (s, 3H), 0.75 (s, 3H) | LC-ELSD/M S purity 99%, MS ESI calcd. for C28H43 N2O3 [M+H]+ 455, found 455 |
| **33** | **A28** | 3 | 6-aminopyr idine-3-carbonitri le | 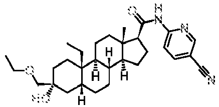 | (400 MHz, CDCl₃) δH 8.53 (d, J=2.0 Hz, 1H), 8.37 (d, J=8.8 Hz, 1H), 7.95-7.87 (m, 2H), 3.58-3.50 (m, 2H), 3.46-3.37 (m, 2H), 2.76 (s, 1H), 2.42-2.19 (m, 2H), 2.04-1.81 (m, 3H), 1.80-1.60 (m, 6H), 1.53-1.34 (m, 7H), 1.34-1.24 (m, 4H), 1.24-1.16 (m, 6H), 0.78 (t, J=7.4 Hz, 3H), 0.71 (s, 3H). | purity 99%, MS ESI calcd. for C30H44 N3O3[M +H]+494 , found 494 |
| **35** | **A28** | 11 | 5-amino-1-methyl-1H-pyrazole-3-carbonitri le | 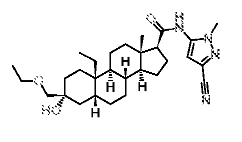 | 1H NMR (400 MHz, CDCl₃) δH 7.13 (s, 1H), 6.62 (s, 1H), 3.81 (s, 3H), 3.55 (q, J = 6.9 Hz, 2H), 3.46-3.35 (m, 2H), 2.81 (s, 1H), 2.39-2.30 (m, 1H), 2.28-2.16 (m, 1H), 1.98-1.71 (m, 9H), 1.68-1.26 (m, 10H), 1.22 (t, J=7.0 Hz, 5H), 1.19-1.10 (m, 2H), 0.78 (t, J=7.4 Hz, 3H), 0.73 (s, 3H). | purity 99%, MS ESI calcd. for.C29H 43N4O2 [M-H2O+H] + 479, found 479. |
| **36** | **A28** | 11 | 3-amino-1-methyl-1H-pyrazole-5-carbonitri le | 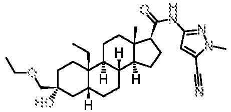 | (400 MHz, CDCl₃) δ 7.54 (s, 1H), 7.21 (s, 1H), 3.93 (s, 3H), 3.55-3.50 (q, J = 9.2 Hz, 2H), 3.50-3.40 (q, J = 7.2 Hz, 2H), 3.75 (s, 1H), 2.26-2.20 (m, 2H), 2.00-1.75 (m, 7H), 1.70-1.20 (m, 19H), 0.77 (t, J = 7.2, 3H), 0.68 (s, 3H) | purity 99%, MS ESI calcd. for C29H45 N4O3 [M+H]+ 497, found 497 |
| **37** | **A1** | 1 | 5-amino-1-methyl-1H-pyrazole-3-carbonitri le | 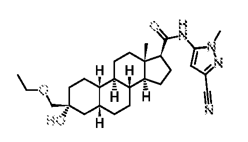 | (400 MHz, CDCl3) δH 7.18 (s, 1H), 6.63 (s, 1H), 3.81 (s, 3H), 3.54 (q, J = 6.8 Hz, 2H), 3.47 - 3.36 (m, 2H), 2.82 (br s, 1H), 2.42 - 2.31 (m, 1H), 2.22 (br d, J = 10.4 Hz, 1H), 1.76 (br d, J = 6.8 Hz, 10H), 1.50 - 1.11 (m, 15H), 0.75 (s, 3H) | purity 99%, MS ESI calcd. for C27H40 N4O3 [M+H-H2O]+ 451, found 451 |
| **38** | **A46** | 10 | 2-chloro-6-methylpy ridine | 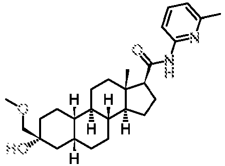 | (400 MHz, CDCl3) δH 7.96 (d, J=8.3 Hz, 1H), 7.71-7.41 (m, 2H), 6.80 (d, J=7.5 Hz, 1H), 3.40-3.23 (m, 5H), 2.59 (s, 1H), 2.37 (s, 3H), 2.29-2.16 (m, 2H), 2.04-1.96 (m, 1H), 1.83-1.58 (m, 8H), 1.45-1.00 (m, 13H), 0.67 (s, 3H) | purity 99%, MS ESI calcd. for C20H31 [M+H-H2O]+ 441, found 441 |
| **39** | **A46** | 10 | 2,3-dichlorop yridine | 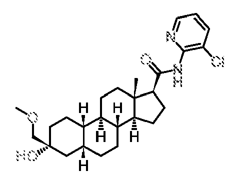 | (400 MHz, CDCl3) δH 8.38 (dd, J=4.6, 1.1 Hz, 1H), 7.49-7.77 (m, 2H), 7.05 (dd, J=7.9, 4.8 Hz, 1H), 3.29-3.65 (m, 5H), 2.63 (s, 1H), 2.49 (t, J=8.9 Hz, 1H), 2.27-2.39 (m, 1H), 2.12 (d, J=12.0 Hz, 1H), 1.67-1.92 (m, 7H), 1.46-1.66 (m, 7H), 1.26-1.44 (m, 7H), 0.80 (s, 3H) | LC-ELSD/M S purity 99%, MS ESI calcd. for C20H31 [M+H-H2O]+ 461, found 461 |
| **40** | **A46** | 10 | 5-bromo-2-methylpy ridine | 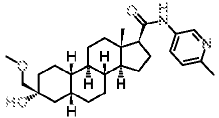 | (400 MHz, CDCl3) δH 8.39 (s, 1H), 8.06 (dd, J=2.5, 8.3 Hz, 1H), 7.12 (d, J=8.5 Hz, 1H), 6.93 (s, 1H), 3.43-3.33 (m, 5H), 2.64 (s, 1H), 2.51 (s, 3H), 2.35-2.25 (m, 2H), 1.99 (br d, J=11.5 Hz, 1H), 1.90-1.56 (m, 7H), 1.50-1.05 (m, 14H), 0.75 (s, 3H) | purity 99%., MS ESI calcd. for C₂₇H₄₁N₂ O₃ [M+H]⁺ 441, found |
| **41** | **A46** | 10 | 5-bromo-6-methylpy ridine-2-carbonitri le | 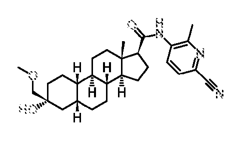 | (400 MHz, CDCl3) δH 8.72 (d, J=8.5 Hz, 1H), 7.56 (d, J=8.5 Hz, 1H), 7.01 (s, 1H), 3.45-3.35 (m, 5H), 2.73-2.58 (m, 1H), 2.55 (s, 3H), 2.44-2.34 (m, 1H), 2.33-2.20 (m, 1H), 2.05-1.96 (m, 1H), 1.92-1.63 (m, 7H), 1.56-1.05 (m, 14H), 0.74 (s, 3H) | purity 99%., MS ESI calcd. for C28H40 N3O3 [M+H]+ 466, found 466 |
| **42** | **A46** | 10 | 5-bromo-6-chloropyr idine-2-carbonitri le | 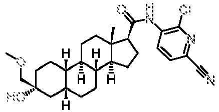 | (400 MHz, CDCl3) δH 8.98 (d, J=8.5 Hz, 1H), 7.75 (s, 1H), 7.64 (d, J=8.5 Hz, 1H), 3.56-3.29 (m, 5H), 2.64 (s, 1H), 2.49-2.37 (m, 1H), 2.36-2.19 (m, 1H), 2.08 (br d, J=11.8 Hz, 1H), 1.95-1.56 (m, 7H), 1.50-1.02 (m, 14H), 0.72 (s, 3H) | purity 99%., MS ESI calcd. for C27H37 ClN3O3 [M+H]+ 486, found 486 |
| **43** | **A46** | 10 | 3-bromo-2-chloro-6-methylpy ridine | 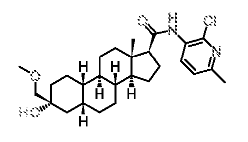 | (400 MHz, CDCl3) δH 8.63 (d, J = 8.4 Hz, 1H), 7.46 (s, 1H), 7.09 (d, J = 8.4 Hz, 1H), 3.40 (s, 5H), 2.62 (br s, 1H), 2.48 (s, 3H), 2.42-2.35 (m, 1H), 2.32-2.24 (m, 1H), 2.12 (d, J = 12.0 Hz, 1H), 1.87-1.73 (m, 5H), 1.63-1.59 (m, 4H), 1.48-1.37 (m, 7H), 1.31-1.07 (m, 5H), 0.73 (s, 3H) | purity 98%, MS ESI calcd. for C27H40 ClN2O3 [M+H]+ 475, found 475 |
| **44** | **A46** | 10 | 2,3-dichloro-6-methylpy ridine | 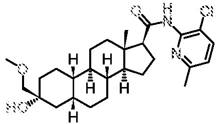 | (400 MHz, CDCl3) δ = 7.58 (d, J=8.0 Hz, 1H), 7.49 (s, 1H), 6.92 (d, J=8.0 Hz, 1H), 3.36-3.44 (m, 5H), 2.77-2.55 (m, 1H), 2.51 (s, 4H), 2.25-2.37 (m, 1H), 2.19-2.07 (m, 1H), 1.88-1.80 (m, 2H), 1.79-1.64 (m, 5H), 1.23-1.51 (m, 11H), 1.06-1.18 (m, 3H), 0.81 ppm (s, 3H) | MS ESI calcd. for C27H40 ClN2O3 [M+H]+ 475, found 475 |
| **45** | **A46** | 10 | 2-bromo-6-(3-methyl-1H-pyrazol-1-yl)pyridine | 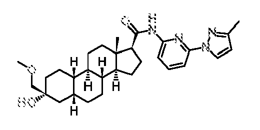 | (400 MHz, CDCl3) δH 8.31 (d, J = 2.4 Hz, 1H), 8.05 (d, J = 7.6 Hz, 1H), 7.77 (t, J = 8.0 Hz, 1H), 7.67-7.53 (m, 2H), 6.23 (d, J = 2.4 Hz, 1H), 3.44-3.37 (m, 5H), 2.64 (s, 1H), 2.41-2.24 (m, 5H), 2.13-2.03 (m, 1H), 1.89-1.70 (m, 6H), 1.53-1.32 (m, 8H), 1.32-1.03 (m, 7H), 0.76 (s, 3H) | purity 99%, MS ESI calcd. for C30H43 N4O3 [M+H]+ 507.3, found 507.3 |
| **46** | **A46** | 10 | 3-bromo-2-methyl-6-(trifluoro methyl)p yridine | 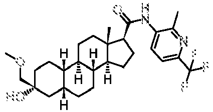 | (400 MHz, CDCl3) δH 8.61 (d, J = 8.4 Hz, 1H), 7.47 (d, J = 8.4 Hz, 1H), 6.90 (s, 1H), 3.43-3.27 (m, 5H), 2.51 (s, 4H), 2.36-2.15 (m, 2H), 2.02-1.92 (m, 1H), 1.88-1.65 (m, 6H), 1.56 (br d, J = 10.0 Hz, 2H), 1.43-1.27 (m, 7H), 1.26-1.14 (m, 3H), 1.13-0.97 (m, 3H), 0.68 (s, 3H); 19F NMR (377 MHz, CDCl3) δF -67.43 (s, 1F) | purity 99%, MS ESI calcd. for C28H40 F3N2O3 [M+H]+ 509.3, found 509.3 |
| **47** | **A46** | 10 | 3-bromo-2-methyl-6-(5-methyl-1H-pyrazol-1-yl)pyridine (INT4) | 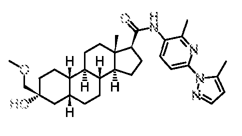 | 1H NMR (400 MHz, CDCl3) δ 8.41 (d, J = 8.8 Hz, 1H), 7.68 (d, J = 8.8 Hz, 1H), 7.57 (d, J = 1.6 Hz, 1H), 6.87 (s, 1H), 6.19 (s, 1H), 3.51-3.36 (m, 5H), 2.71-2.61 (m, 4H), 2.53 (s, 3H), 2.42-2.26 (m, 2H), 2.10 (br d, J = 11.2 Hz, 1H), 1.96-1.72 (m, 6H), 1.66 (br d, J = 11.2 Hz, 2H), 1.56-1.40 (m, 7H), 1.36-1.25 (m, 3H), 1.24-1.07 (m, 3H), 0.81 (s, 3H) | LC-ELSD/M S purity 99%, MS ESI calcd. for C31H45 N4O3 [M+H]+ 521, found 521 |
| **48** | **A46** | 10 | 2-(5-bromo-6-methylpy ridin-2-yl)-5-fluoropyri midine (INT3) | 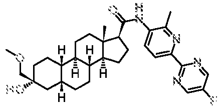 | (400 MHz, CDCl3) δ 8.74 (s, 2H), 8.68 (d, J = 8.4 Hz, 1H), 8.34 (d, J = 8.4 Hz, 1H), 7.02 (s, 1H), 3.46-3.36 (m, 5H), 2.69 (s, 3H), 2.44-2.37 (m, 1H), 2.36-2.26 (m, 1H), 2.08 (d, J = 11.6 Hz, 1H), 1.94-1.68 (m, 6H), 1.55-1.03 (m, 16H), 0.78 (s, 3H); 19F NMR (376 MHz, CDCl3) δ - 138.712 | LC-ELSD/M S purity 99%, MS ESI calcd. for C31H42 FN4O3 [M+H]+ 537, found 537. |
| **49** | **A46** | 10 | 1-(5-bromo-6-methylpy ridin-2-yl)pyrroli din-2-one | 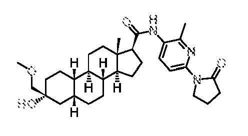 | (400 MHz, CDCl3) δ = 8.20-8.12 (m, 2H), 6.78 (s, 1H), 4.09 (t, J=7.2 Hz, 2H), 3.42-3.39 (m, 5H), 2.64 (t, J=8.0 Hz, 2H), 2.38-2.26 (m, 2H), 2.05-2.16 (m, 2H), 2.05-1.98 (m, 1H), 1.88-1.80 (m, 2H), 1.80-1.70 (m, 5H), 1.52-1.24 (m, 14H), 1.19-1.00 (m, 4H), 0.77 (s, 3H) | MS ESI calcd. for C31H46 N3O4+ [M+H]+ 524, found 524 |
| **50** | **A46** | 10 | 1-(6-bromopyr idin-2-yl)pyrroli din-2-one | 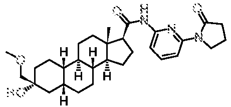 | (400 MHz, CDCl3) δ = 8.08 (d, J=8.0 Hz, 1H), 7.91 (d, J=8.0 Hz, 1H), 7.68 (t, J=8.2 Hz, 1H), 7.46 (s, 1H), 3.94-4.08 (m, 2H), 3.34-3.45 (m, 5H), 2.58-2.69 (m, 3H), 2.41-2.22 (m, 2H), 2.16-2.00 (m, 4H), 1.88-1.70 (m, 6H), 1.52-1.35 (m, 7H), 1.34-0.84 (m, 7H), 0.74 (s, 3H). | MS ESI calcd. for C30H44 N3O4+ [M+H]+ 510, found 510. |
| **51** | **A46** | 10 | 3-bromo-2-methylpy ridine | 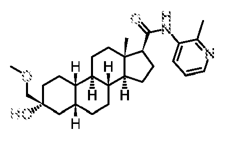 | (400MHz, CDCl3) δ 8.34 (br d, J=8.2 Hz, 1H), 8.25 (br d, J=4.0 Hz, 1H), 7.15 (br dd, J=4.4, 8.0 Hz, 1H), 6.83 (br s, 1H), 3.46 - 3.32 (m, 5H), 2.64 (s, 1H), 2.52 (s, 3H), 2.41 - 2.22 (m, 2H), 2.06 (br d, J=11.6 Hz, 1H), 1.90 - 1.71 (m, 6H), 1.54 - 1.35 (m, 8H), 1.33 - 1.22 (m, 4H), 1.19 - 1.08 (m, 3H), 0.76 (s, 3H) | LC-ELSD/M S 99% purity, MS ESI calcd. For C27H40 N2O3 [M+H]+ 441, found 441 |
| **52** | **A46** | 10 | 3-bromo-2-methyl-6-(3-methyl-1H-pyrazol-1-yl)pyridine (INT5) | 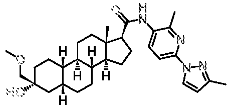 | (400 MHz, CDCl3) δ 8.38 (d, J=2.4 Hz, 1H), 8.31 (d, J=8.8 Hz, 1H), 7.72 (d, J=8.8 Hz, 1H), 6.79 (s, 1H), 6.21 (d, J=2.4 Hz, 1H), 3.45-3.35 (m, 5H), 2.63 (s, 1H), 2.49 (s, 3H), 2.40-2.23 (m, 5H), 2.08 (br d, J=13.2 Hz, 1H), 1.93-1.74 (m, 6H), 1.49-1.21 (m, 12H), 1.17-0.97 (m, 3H), 0.78 (s, 3H) | LC-ELSD/M S purity 99%, MS ESI calcd. for C31H45 N4O3[M +H]+521 .3, found 521.3 |
| **53** | **A46** | 10 | 2,6-dimethylp yridin-3-amine | 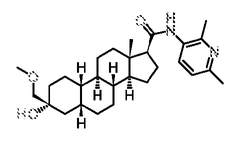 | (400MHz, DMSO-d6) δ 9.69 (s, 1H), 8.42 (d, J=8.6 Hz, 1H), 7.68 (d, J=8.6 Hz, 1H), 3.34-3.19 (m, 5H), 2.65 (s, 3H), 2.57 (s, 3H), 2.16-2.00 (m, 1H), 1.86 (br d, J=12.4 Hz, 1H), 1.78-1.45 (m, 9H), 1.44-0.92 (m, 14H), 0.65 (s, 3H) | LC-ELSD/M S purity 96%, MS ESI calcd. for C28H42 N2O3 [M+H-H2O]+ 455, found 455 |
| **54** | **A1** | 1 | 2-methyl-4,5,6,7-tetrahydr o-2H-indazol-3-amine | 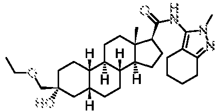 | (400 MHz, CDCl3) δ 6.49 (s, 1 H), 3.67 (s, 3 H), 3.58-3.49 (m, 2 H) 3.47-3.36 (m, 2 H), 2.76 (s, 1 H), 2.64-2.56 (m, 2 H), 2.41-2.17 (m, 4 H), 2.07-1.97 (m, 1 H), 1.89-1.62 (m, 11 H), 1.53-1.53 (m, 8 H), 1.28-1.05 (m, 9 H), 0.77 (s, 3 H) | purity 99%, C30H47 N3O3 MS ESI calcd. For C30H47 N3O3 [M+H] + 498, found 498 |
| **55** | **A1** | 1 | 1-cyclopent yl-1H-pyrazol-5-amine | 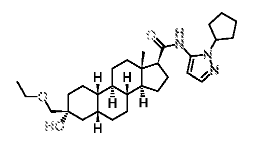 | (400 MHz, CDCl3) δH 7.45 (s, 1 H), 6.74 (s, 1 H), 6.21 (s, 1 H), 4.51-4.40 (m, 1 H) 3.59-3.50 (m, 2 H) 3.48-3.38 (m, 2 H) 2.76 (s, 1 H), 2.38-2.19 (m, 2 H), 2.15-1.98(m, 5 H), 1.96-1.72 (m, 8 H), 1.71-1.6 (m, 4 H), 1.56-1.34 (m, 8 H), 1.33-1.03 (m, 8 H), 0.76 (s, 3 H) | 100%, MS ESI calcd. For C30H47 N3O3 [M+H]+ 498, found 498 |
| **56** | **A1** | 1 | 3-cycloprop yl-1-methyl-1H-pyrazol-5-amine | 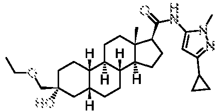 | (400 MHz, CDCl3) δ 6.74 (s, 1 H), 5.88 (s, 1 H), 3.65 (s, 3 H), 3.5-3.49 (m, 2 H), 3.47-3.37 (m, 2 H), 2.76 (s, 1 H), 2.36-2.17 (m, 2 H), 2.03-1.93 (m, 1 H) 1.90-1.64 (m, 8 H), 1.53-1.35 (m, 7 H), 1.31-1.0 (m, 10 H), 0.92-0.83 (m, 2 H), 0.75 (s, 3 H), 0.70-0.62 (m, 2 H) | purity 99%, C29H45 N3O3 MS ESI calcd. For C29H45 N3O3 [M+H] + 484, found 484 |
| **57** | **A3** | 2 | 5-amino-1-methyl-1H-pyrazole-4-carbonitrile | 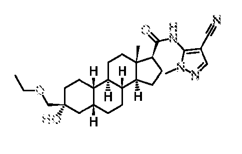 | (400 MHz, CDCl3) δ = 7.72 (s, 1H), 7.24 (br s, 1H), 3.77 (s, 3H), 3.57-3.38 (m, 4H), 2.78 (s, 1H), 2.47-2.38 (m, 1H), 2.30-2.05 (m, 2H), 1.95-1.70 (m, 6H), 1.67-1.63 (m, 1H), 1.56-1.34 (m, 8H), 1.33-1.09 (m, 9H), 0.79 (s, 3H) | ESI calcd. For C27H41 N4O3 [M+H]+ 470, found 470 |
| **58** | **A7** | 4 | 6-chloropyr azine-2-carbonitrile | 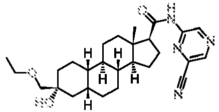 | δH 9.78 (s, 1H), 8.63 (s, 1H), 7.75 (s, 1H), 3.54 (q, J=7.0 Hz, 2H), 3.43 (q, J=9.3 Hz, 2H), 2.76 (s, 1H), 2.48-2.36 (m, 1H), 2.20-2.30 (m, 1H), 2.01 (d, J=11.5 Hz, 1H), 1.89-1.70 (m, 5H), 1.69-1.59 (m, 4H), 1.49-1.38 (m, 6H), 1.35-1.03 (m, 9H), 0.75 (s, 3H) | LC-ELSD/M S purity 99%, MS ESI calcd. For C27H39 N4O3 [M+H] + 467, found 467. |
| **59** | **A7** | 4 | 2-chloro-3,6-dimethylp yridine | 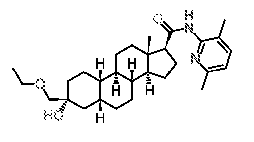 | δH 7.43 (d, J=7.8 Hz, 1H), 7.33 (br s, 1H), 6.94 (d, J=7.8 Hz, 1H), 3.53 (q, J=7.0 Hz, 2H), 3.43 (d, J=8.0 Hz, 2H), 2.73 (s, 1H), 2.45 (s, 3H), 2.41-2.30 (m, 1H), 2.22 (s, 4H), 2.15-2.03 (m, 1H), 1.89-1.66 (m, 7H), 1.52-1.33 (m, 7H), 1.31-1.01 (m, 10H), 0.81 (s, 3H) | LC-ELSD/M S purity 99%, MS ESI calcd. For C29H45 N2O3 [M+H] + 469, found 469 |
| **60** | **A7** | 4 | 6-chloropyr idine-2-carbonitrile | 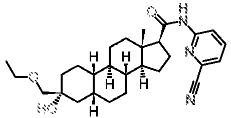 | (400 MHz, CDCl3) δH 8.50 (d, J = 8.4 Hz, 1H), 7.80 (t, J = 8.8 Hz, 2H), 7.41 (d, J = 6.8 Hz, 1H), 3.54 (q, J = 6.8 Hz, 2H), 3.43 (q, J = 9.2 Hz, 2H), 2.74 (br s, 1H), 2.39-2.32 (m, 1H), 2.31-2.20 (m, 1H), 2.06-2.00 (m, 1H), 1.89-1.73 (m, 6H), 1.65-1.58 (m, 2H), 1.47-1.38 (m, 6H), 1.31-1.18 (m, 7H), 1.17-1.01 (m, 3H), 0.73 (s, 3H) | LC-ELDS/M S purity 99%, MS ESI calcd. for C28H40 N3O3 [M+H]+ 466, found 466. |
| **61** | **A7** | 4 | 6-chloro-5-methylpy ridine-2-carbonitrile | 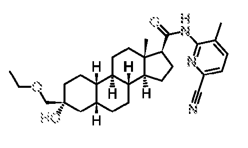 | (400 MHz, CDCl3) δ 7.69-7.67 (m, 1H), 7.52-7.48 (m, 1H), 7.43 (s, 1H), 3.54-3.51 (m, 2H), 3.48-3.39 (m, 2H), 2.73 (s, 1H), 2.46-2.33 (m, 4H), 2.29-2.06 (m, 2H), 1.94-1.71 (m, 6 H), 1.69-1.58 (m, 2H), 1.52-1.35 (m, 7H), 1.33-1.03 (m, 9H), 0.79 (s, 3H) | purity 99%, MS ESI calcd. For C29H41 N3O3 [M+H] + 480, found 484 |
| **62** | **A7** | 4 | 2-chloro-3,5-dimethylp yridine | 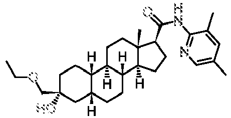 | (400 MHz, CDCl3) δ 8.04 (s, 1H), 7.39-7.28 (m, 2H), 3.57-3.50 (m, 2H), 3.47-3.38 (m, 2H), 2.72 (s, 1H), 2.44-2.33 (m, 1H), 2.28 (s, 3H), 2.24 (s, 3H), 2.11 (m, 1H), 1.90-1.72 (m, 5H), 1.67-1.58 (m, 6H), 1.48-1.33 (m, 6H), 1.27-1.05 (m, 8H), 0.80 (s, 3H) | purity 99%, MS ESI calcd. For C29H44 N2O3 [M+H] + 469, found 469 |
| **63** | **A7** | 4 | 4-chloro-2-methylpy rimidine | 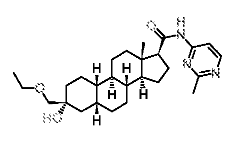 | (400 MHz, CDCl3) δ 8.53-8.50 (m, 1H), 8.12-8.08 (m, 1H), 7.92 (s, 1H), 3.59-3.50 (m, 2H), 3.47-3.37 (m, 2H), 2.70 (s, 4H), 2.44-2.34 (m, 1H), 2.32-2.10 (m, 1H), 2.09-1.94 (m, 1H), 1.86-1.70 (m, 6H), 1.61-1.58 (m, 2H), 1.53-1.34 (m, 8H),1.27- 1.06 (m, 8H), 0.73 (s, 3H) | LC-ELSD/M S purity 99%, MS ESI calcd. For C27H42 N3O3 [M+H] + 456, found 456 |
| **64** | **A7** | 4 | 1-(6-bromopyr idin-2-yl)pyrroli din-2-one | 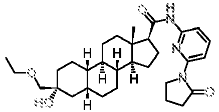 | (400MHz, CDCl3) δ 8.07 (d, J=7.6 Hz, 1H), 7.92 (d, J=7.6 Hz, 1H), 7.68 (t, J=8.0 Hz, 1H), 7.47 (s, 1H), 4.12-3.94 (m, 2H), 3.56-3.39 (m, 4H), 2.76 (s, 1H), 2.69-2.61 (m, 2H), 2.39-2.23 (m, 2H), 2.17-1.99 (m, 3H), 1.86-1.64 (m, 7H), 1.50-1.32 (m, 8H), 1.26-1.07 (m, 9H), 0.74 (s, 3H) | LC-ELSD/M S purity 99%, MS ESI calcd. for C28H47 O [M+H-H2O]+ 524, found 524 |
| **65** | **A7** | 4 | 1-(5-bromo-6-methylpy ridin-2-yl)pyrroli din-2-one | 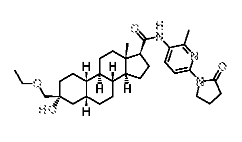 | (400 MHz, CDCl3) δ 8.19 (d, J=8.8 Hz, 1H), 8.11 (d, J=8.8 Hz, 1H), 6.75 (s, 1H), 4.08 (t, J=7.3 Hz, 2H), 3.54 (q, J=7.0 Hz, 2H), 3.43 (q, J=9.3 Hz, 2H), 2.74 (br s, 1H), 2.64 (t, J=8.2 Hz, 2H), 2.42 (s, 3H), 2.37 - 2.21 (m, 2H), 2.16 - 2.01 (m, 3H), 1.90 - 1.70 (m, 5H), 1.68 - 1.53 (m, 4H), 1.52 - 1.35 (m, 7H), 1.34 - 1.05 (m, 8H), 0.77 (s, 3H) | LC-ELSD/M S purity 99%, MS ESI calcd. for C32H48 N3O4 [M+H]+ 538, found 538 |

### EXAMPLE 66: Synthesis of (3R,5R,8R,9R,10S,13S,14S,17S)-3-(ethoxymethyl)-N-(2-fluoropyridin-3-yl)-3-hydroxy-13-methylhexadecahydro-1H-cyclopenta[a]phenanthrene-17-carboxamide (A89)

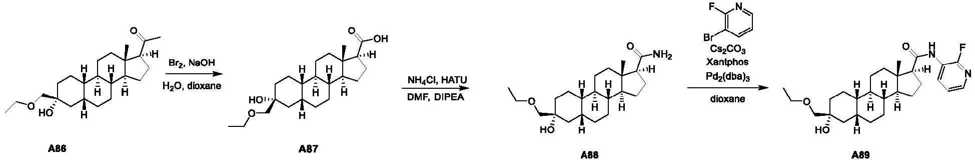

### Synthesis of A87

Liquid bromine (6.55 g, 41.0 mmol) was added slowly to a vigorously stirred sodium hydroxide aqueous (54.6 mL, 3 M, 164 mmol) at 0°C. When all the bromine was dissolved, the mixture was diluted with cold dioxane (15 mL) and added slowly to a stirred solution of **A86** (5 g, 13.7 mmol) in dioxane (20 mL) and water (15 mL). The homogeneous yellow solution became colorless slowly and a white precipitate was formed. After stirring at 25°C for 5 h, the remaining oxidizing reagent was quenched by Na₂S₂O₃ aqueous (30 mL) and the mixture was then heated to 80°C until the solid material dissolved. The solution was acidified with HCl (3 M, 40 mL) and a white solid was precipitated. The solid was filtered and washed with water (3 x 100 mL) to give a solid, which was dried under vacuum to afford **A87** (5 g) as a solid.

**¹H NMR** (400 MHz, CDCl3) δ 11.89 (br s, 1H), 4.13 (br s, 1H), 3.46 (q, J=7.0 Hz, 2H), 3.32-3.26 (m, 2H), 2.29 (t, *J* = 9.2 Hz, 1H), 1.99-1.89 (m, 2H), 1.78-1.46 (m, 7H), 1.41-1.14 (m, 11H), 1.11 (t, J= 7.0 Hz, 3H), 1.07-0.91 (m, 3H), 0.62 (s, 3H).

### Synthesis of A88

To a solution of **A87** (150 mg, 0.411 mmol) in DMF (3 mL) were added HATU (234 mg, 0.616 mmol) and DIPEA (211 mg, 1.64 mmol). After stirring at 25°C for 15 min, NH₄Cl (43.9 mg, 0.822 mmol) was added. After stirring at 25°C for 16 h, the mixture was poured into water (15 mL) and extracted with EtOAc (2 x 30 mL). The combined organic phase was washed with brine (20 mL), dried over anhydrous Na₂SO₄, filtered and concentrated. The residue was purified by HPLC (Column: Xtimate C18 150 x 25mm, 5um; Condition: water (0.04%NH₃H₂O+10mM NH₄HCO₃)-ACN; Gradient: from 34% to 59% of B in 9min and hold 100% for 2 min; Flow rate: 30mL/min) to afford **A88** (64 mg, 42.9 %) as a solid. **¹H NMR** (400 MHz, CDCl3) δ 5.25 (d, *J =* 15.6 Hz, 2H), 3.53 (q, *J =* 7.2 Hz, 2H), 3.43 (q, *J =* 9.2 Hz, 2H), 2.73 (s, 1H), 2.23-2.08 (m, 2H), 1.99-1.92 (m, 1H), 1.86-1.58 (m, 8H), 1.50-1.34 (m, 6H), 1.21 (s, 10H), 0.72 (s, 3H). **LCMS:** purity 100%, MS ESI calcd. for C₂₂H₃₈NO₃ [M+H]⁺ 364, found 364.

### Synthesis of A89

A mixture of **A88** (300 mg, 0.825 mmol), Pd₂(dba) ₃ (75.5 mg, 0.0825 mmol), Xantphos (47.7 mg, 0.0825 mmol), Cs₂CO₃ (537 mg, 1.65 mmol) and 3-bromo-2-fluoropyridine (290 mg, 1.65 mmol) in dioxane (4 mL) under N₂ was stirred at 100°C for 16 h. The reaction mixture combined with another batch from 100 mg of **A88.** The combined reactions were quenched with water (10 mL) and extracted with EtOAc (3 x 10 mL). The combined organic layer was dried over anhydrous Na₂SO₄, filtered and concentrated. The residue was purified by flash column (10%~50% of EtOAc in PE) to give **A89** (260 mg) as a solid. **A89** (60 mg, 0.130 mmol) was further purified by prep-HPLC (Column: Phenomenex Gemini-NX 150*30mm*5um; Condition: water (0.04%NH₃H₂O+10mM NH₄HCO₃)-ACN; Begin B: 58%, End B: 88%; Gradient Time (min): 8; 100%B Hold Time (min): 2; FlowRate (ml/min): 30) to give **A89** (16.8 mg, 28.1%) as a solid.

**¹H NMR** (400 MHz, CDCl₃) δ_{H} 8.81-8.72 (m, 1H), 7.89-7.83 (m, 1H), 7.22-7.14 (m, 2H), 3.58-3.49 (m, 2H), 3.48-3.38 (m, 2H), 2.74 (s, 1H), 2.41-2.33 (m, 1H), 2.32-2.20 (m, 1H), 2.06-1.98 (m, 1H), 1.91-1.70 (m, 6H), 1.68-1.58 (m, 2H), 1.54-1.36 (m, 7H), 1.34-1.06 (m, 9H), 0.73 (s, 3H). **¹⁹F NMR** (376.5 MHz, CDCl₃) δ_{F} -83.93. **LC-ELSD/MS** purity >99% MS ESI calcd. for C₂₇H₄₀FN₂O₃ [M +H]⁺ 459, found 459.

### EXASMLE 67: Synthesis of (3R, 5R, 8R, 9R, 10S, 13S, 14S, 17S)-N-(5-cyanopyridin-2-yl)-3-hydroxy-13-methyl-3-propylhexadecahydro-1H-cyclopenta[a]phenanthrene-17-carboxamide (A94)

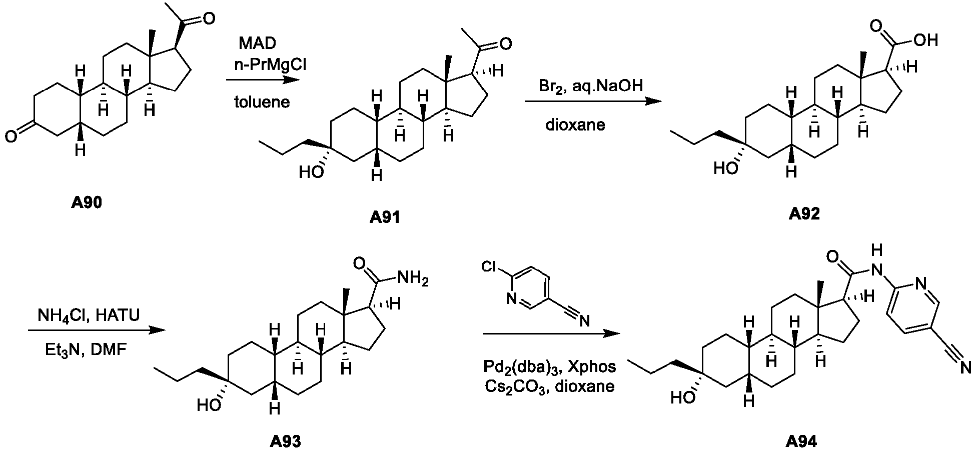

### Synthesis of A91

To a solution of 2,6-di-tert-butyl-4-methylphenol (13.1 g, 59.6 mmol) in toluene (20 mL) was added AlMe₃(14.9 mL, 29.8 mmol, 2 M in toluene) dropwise at 0°C. After stirring at 25°C for 30 min, to the MAD solution was added a solution of **A90** (3 g, 9.91 mmol) in anhydrous toluene (40 mL) dropwise at -70°C. After stirring at -70°C for 1 h under N₂, n-PrMgCl (14.8 mL, 29.7 mmol, 2 M in diethyl ether) was added dropwise at -70°C. After stirring at -70°C for 2 h, the reaction mixture was poured into saturated aqueous citric acid (100 mL) at 10°C and extracted with EtOAc (2 x 100 mL). The combined organic layer was dried over Na₂SO₄, filtered and concentrated in vacuum. The residue was purified by flash column (0~10% of EtOAc in PE) to give **A91** (1.7 g, 49%) as a solid.

**¹H NMR** (400 MHz, CDCl₃) δ_{H} 2.53 (t, *J =* 8 Hz, 1H), 2.16-2.11 (m, 4H), 2.04-1.98 (m, 1H), 1.83-1.52 (m, 3H), 1.50-1.30 (m, 5H), 1.27-1.02 (m, 10H), 0.97-0.77 (m, 11H), 0.61 (s, 3H).

### Synthesis of A92

Liquid bromine (1.92 g, 12.0 mmol) was added slowly to a vigorously stirred sodium hydroxide aqueous (15.0 mL, 4 M, 60.2 mmol) at 0°C. When all the bromine was dissolved, the mixture was added slowly to a stirred solution of **A91** (700 mg, 2.0 mmol) in dioxane (12 mL) and water (3 mL). The homogeneous yellow solution became colorless slowly and a white precipitate was formed. After stirring at 25°C for 16 h, the remaining oxidizing reagent was quenched by Na₂SO₃ aqueous (30 mL, sat.) and the mixture was then heated at 80°C until the solid material dissolved. Acidification of the solution with hydrochloride acid (3 M, 40 mL) furnished a white precipitate. The solid was filtered and washed with water (3 x 50 mL) to give a solid, which was dried under vacuum to afford **A92** (600 mg, 86%) as a solid.

**¹H NMR** (400 MHz, CDCl₃) δ_{H} 2.41 (t, *J* = 9.2 Hz, 1H), 2.18-1.98 (m, 2H), 1.95-1.59 (m, 9H), 1.52-1.28 (m, 9H), 1.25-1.00 (m, 9H), 0.93 (t, *J =* 7.6 Hz, 3H), 0.73 (s, 3H).

### Synthesis of A93

To a solution of **A92** (600 mg, 1.72 mmol) in DMF (10 mL) were added HATU (977 mg, 2.57 mmol), Et₃N (868 mg, 8.60 mmol) and NH₄Cl (184 mg, 3.44 mmol). After stirring at 40°C for 16 h, the mixture was added into water (100 mL) and filtered. The filter cake was washed with water (2 x 50 mL) and concentrated to give **A93** (600 mg) as a solid. **A93** (400 mg, 1.15 mmol) was triturated from acetonitrile (5 mL) at 20°C to give **A93** (155.7 mg, 39%) as a solid.

**¹H NMR** (400 MHz, CDCl₃) δ_{H} 5.35-5.15 (m, 2H), 2.25-2.05 (m, 2H), 2.02-1.90 (m, 1H), 1.90-1.60 (m, 7H), 1.52-1.30 (m, 9H), 1.28-1.05 (m, 10H), 0.93 (t, *J =* 7.6 Hz, 3H), 0.72 (s, 3H).**LC-ELSD/MS** purity 99%, MS ESI calcd. for C₂₂H₃₈NO₂ [M +H]⁺ 348.3, found 348.3.

### Synthesis of A94

To a solution of **A93** (200 mg, 0.58 mmol), 6-chloropyridine-3-carbonitrile (159 mg, 1.15 mmol), X-Phos (27.3 mg, 0.058 mmol) and Cs₂CO₃ (374 mg, 1.15 mmol) in dioxane (10 mL) was added Pd₂ (dba) ₃ (52.6 mg, 0.058 mmol) in one portion under N₂. After stirring at 101°C for 20 h, the residue was poured into water (50 mL) and extracted with EtOAc (3 x 20 mL). The combined organic phase was washed with brine (2 x 50 mL), dried over anhydrous Na₂SO₄, filtered and concentrated. The residue was purified by flash column (0~30% of EtOAc in PE) to give **A94** (140 mg) as a solid. **A94** (140 mg, 0.311 mmol) was triturated by acetonitrile (2 mL) at 20°C to give **A94** (71.3 mg, 51%) as a solid.

**¹H NMR** (400 MHz, CDCl₃) δ_{H} 8.53 (d, *J =* 1.6 Hz, 1H), 8.37 (d, *J =* 8.8 Hz, 1H),7.92 (dd, *J*=2.0, 8.8 Hz, 1H), 7.86 (s, 1H), 2.37 (t, *J =* 8.8 Hz, 1H), 2.35-2.20 (m, 1H), 2.03-1.95 (m, 1H), 1.95-1.59 (m, 8H), 1.52-1.30 (m, 13H), 1.28-1.05 (m, 5H), 0.94 (t, *J =* 7.2 Hz, 3H), 0.73 (s, **3H).LC-ELSD/MS** purity 99%, MS ESI calcd. for C₁₈H₄₀N₃O₁ [M +H]⁺ 450.3, found 450.3.

### EXAMPLE 68: Synthesis of (3R, 5R, 8R, 9S, 10S, 13S, 14S, 17S)-N-(5-cyanopyridin-2-yl)-3-hydroxy-10, 13-dimethyl-3-propylhexadecahydro-1H-cyclopenta[a]phenanthrene-17-carboxamide (A99)

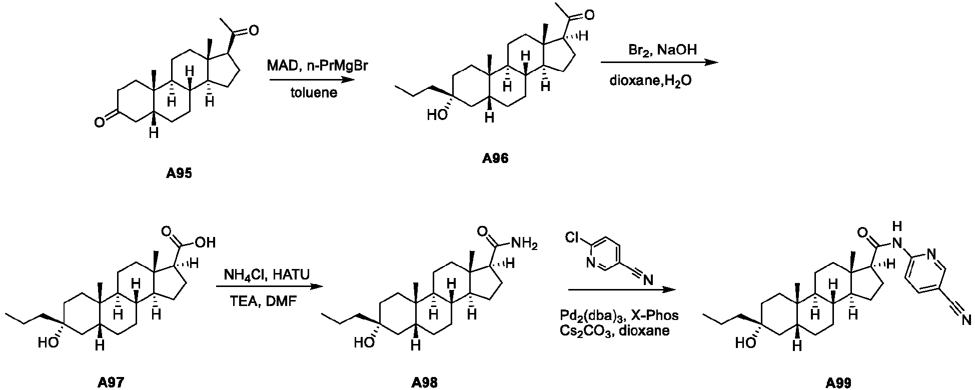

### Synthesis of A96

To a solution of 2,6-*di-t*-butyl-*p*-cresol (butylated hydroxytoluene) (33.5 g, 152 mmol) in toluene (100 mL) under nitrogen at 0°C was added trimethylaluminum (2 M in toluene, 38 mL, 76 mmol) dropwise. After stirring at 20°C for 1 h, a solution of **A95** (8.0 g, 25.2 mmol) in toluene (50 mL) was added to the above solution dropwise under N₂ at -70°C. After stirring at -70°C for 1 h, n-PrMgCl (37.8 mL, 75.6 mmol, 2M in THF) was added dropwise. After stirring at -70°C for 0.5 h, the reaction mixture was poured to ice-cooled aqueous citric acid (500 mL) and extracted with EtOAc (2 x 500 mL). The combined organic layer was washed with brine (2 x 300 mL), dried over anhydrous Na₂SO₄, filtered and concentrated. The residue was combined with another solution of crude product to be purified by flash column (0 ~ 25% of EtOAc in PE) to give **A96** (5.9 g) as a solid.

**¹H NMR** (400 MHz, CDCl₃) δ_{H} 2.53 (t, *J =* 8.8 Hz, 1H), 2.19-2.13 (m, 1H), 2.11 (s, 3H), 2.06-1.64 (m, 6H), 1.54-1.36 (m, 11H), 1.32-1.00 (m, 9H), 0.96-0.91 (m, 6H), 0.59 (s, 3H).

### Synthesis of A97

Liquid bromine (1.32 g, 8.28 mmol) was added slowly to a vigorously stirred sodium hydroxide aqueous (10.3 mL, 4 M, 41.4 mmol) at 0°C. When all the bromine was dissolved, the mixture was added slowly to a stirred solution of **A96** (500 mg, 1.38 mmol) in dioxane (10 mL) and water (2.5 mL). The homogeneous yellow solution became colorless slowly and a white precipitate formed. After stirring at 25°C for 16 h, the reaction mixture was acidified with HCl (4 M in water, 13 mL) to adjust the pH to 1 and stirred for 30 min. The precipitated solid was filtered out, washed with water (2 x 30 mL) and dried to give **A97** (500 mg) as a solid.

**¹H NMR** (400 MHz, CDCl₃) δ_{H} 2.42-2.04 (m, 3H), 1.88-1.46 (m, 13H), 1.39-1.01 (m, 13H), 0.96-0.91 (m, 6H), 0.71 (s, 3H)

### Synthesis of A98

To a solution of **A97** (500 mg, 1.37 mmol) in DMF (10 mL) were added HATU (779 mg, 2.05 mmol), Et₃N (693 mg, 6.85 mmol) and NH₄Cl (146 mg, 2.74 mmol). After stirring at 40°C for 16 h, the mixture was added into water (100 mL) and stirred at 15°C for 10 min. The mixture was filtered; the filter cake was washed with water (2 x 30 ml) to give **A98** (1.2 g) as a solid, which was triturated from acetonitrile (20 ml) at 15°C to give **A98** (250 mg) as a solid.

**¹H NMR** (400 MHz, CDCl₃) δ_{H} 5.34-5.16 (m, 2H), 2.22-2.02 (m, 3H), 1.99-1.67 (m, 7H), 1.50-1.33 (m, 9H), 1.30-1.05 (m, 9H), 0.97-0.89 (m, 6H), 0.70 (s, 3H).**LC-ELSD/MS:** purity 99%; MS ESI calcd. for C₂₃H₄₀NO₂ [M +H]⁺ 362.3, found 362.3.

### Synthesis of A99

To a solution of **A98** (150 mg, 0.41 mmol), 6-chloropyridine-3-carbonitrile (114 mg, 0.83 mmol), X-Phos (19.7 mg, 0.041 mmol) and Cs₂CO₃ (270 mg, 0.83 mmol) in dioxane (10 mL) was added Pd₂ (dba) ₃ (37.9 mg, 0.041 mmol) in one portion under N₂. After stirring at 110°C for 16 h, the residue was poured into water (50 mL) and extracted with EtOAc (3 x 40 mL). The combined organic phase was washed with brine (2 x 50 mL). The organic phase was dried over anhydrous Na₂SO₄, filtered and concentrated. The residue was purified by flash column (0~40% of EtOAc in PE) to give **A99** (100 mg), which was triturated from acetonitrile (5 ml) at 15°C to give **A99** (31.0 mg, 31%) as a solid.

**¹H NMR** (400 MHz, CDCl₃) δ_{H} 8.53 (d, *J*=1.2 Hz, 1H), 8.37 (d, *J*=8.8 Hz, 1H), 7.92 (dd, *J*=2.2, 8.8 Hz, 1H), 7.85 (s, 1H), 2.39-2.21 (m, 2H), 2.04-1.67 (m, 6H), 1.54-1.36 (m, 10H), 1.36-0.98 (m, 10H), 0.97-0.91 (m, 6H), 0.71 (s, 3H).**LC-ELSD/MS:** purity 99%, MS ESI calcd. for C₂₉H₄₂N₃O₂[M +H]⁺ 464.3, found 464.3.

### Steroid Inhibition of TBPS Binding

[³⁵S]-t-Butylbicyclophosphorothionate (TBPS) binding assays using rat brain cortical membranes in the presence of 5 mM GABA has been described (Gee et al, J. Pharmacol. Exp. Ther. 1987, 241, 346-353; Hawkinson et al, Mol. Pharmacol. 1994, 46, 977-985; Lewin, A.H et al., Mol. Pharmacol. 1989, 35, 189-194*).*

Briefly, cortices are rapidly removed following decapitation of carbon dioxide-anesthetized Sprague-Dawley rats (200-250 g). The cortices are homogenized in 10 volumes of ice-cold 0.32 M sucrose using a glass/teflon homogenizer and centrifuged at 1500 x g for 10 min at 4 °C. The resultant supernatants are centrifuged at 10,000 x g for 20 min at 4 °C to obtain the P2 pellets. The P2 pellets are resuspended in 200 mM NaCl/50 mM Na-K phosphate pH 7.4 buffer and centrifuged at 10,000 x g for 10 min at 4 °C. This washing procedure is repeated twice and the pellets are resuspended in 10 volumes of buffer. Aliquots (100 mL) of the membrane suspensions are incubated with 3 nM [³⁵S]-TBPS and 5 mL aliquots of test drug dissolved in dimethyl sulfoxide (DMSO) (final 0.5%) in the presence of 5 mM GABA. The incubation is brought to a final volume of 1.0 mL with buffer. Nonspecific binding is determined in the presence of 2 mM unlabeled TBPS and ranged from 15 to 25 %. Following a 90 min incubation at room temp, the assays are terminated by filtration through glass fiber filters (Schleicher and Schuell No. 32) using a cell harvester (Brandel) and rinsed three times with ice-cold buffer. Filter bound radioactivity is measured by liquid scintillation spectrometry. Non-linear curve fitting of the overall data for each drug averaged for each concentration is done using Prism (GraphPad). The data are fit to a partial instead of a full inhibition model if the sum of squares is significantly lower by F-test. Similarly, the data are fit to a two component instead of a one component inhibition model if the sum of squares is significantly lower by F-test. The concentration of test compound producing 50% inhibition (IC₅₀) of specific binding and the maximal extent of inhibition (Iₘₐₓ) are determined for the individual experiments with the same model used for the overall data and then the means ± SEM.s of the individual experiments are calculated. Picrotoxin serves as the positive control for these studies as it has been demonstrated to robustly inhibit TBPS binding.

Various compounds are or can be screened to determine their potential as modulators of [³⁵S]-TBPS binding *in vitro.* These assays are or can be performed in accordance with the above

In **Table 2** below, A indicates a TBPS IC₅₀ (µM) < 0.1 µM, B indicates a TBPS IC₅₀ (µM) of 0.1 µM to < 1 µM, C indicates a TBPS IC₅₀ (µM) of ≥ 1.0 µM.

**Table 2**

| **Example** | **Intermediate** | **STRUCTURE** | **IC₅₀** |
|---|---|---|---|
| 1 | A2 | 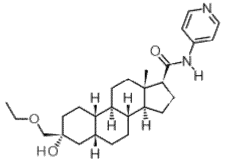 | A |
| 2 | A4 | 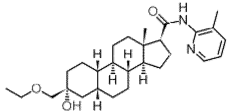 | C |
| 3 | A5 | 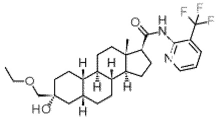 | B |
| 4 | A8 | 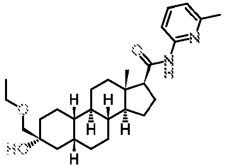 | A |
| 5 | A13 | 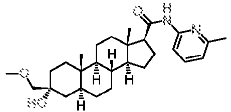 | A |
| 6 | A14 | 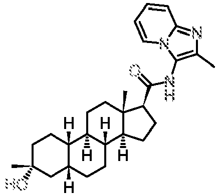 | B |
| 7 | A18 | 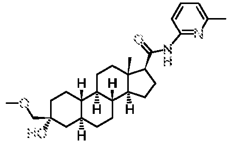 | A |
| 8 | A29 | 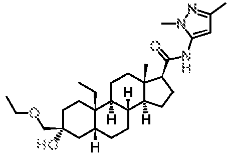 | A |
| 9 | A38 | 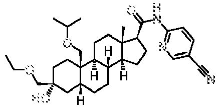 | A |
| 10 | A47 | 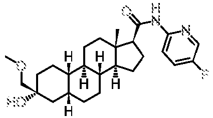 | A |
| 11 | A49 | 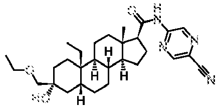 | A |
| 12 | A68 | 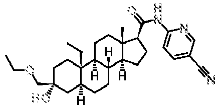 | A |
| 13 | A69 | 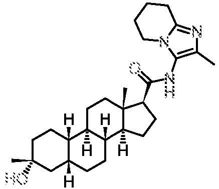 | B |
| 14 | A75 | 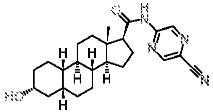 | A |
| 15 | A80 | 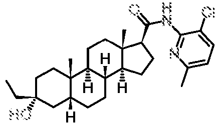 | B |
| 16 | A85 | 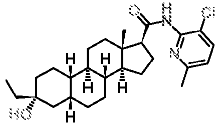 | B |
| 20 | | 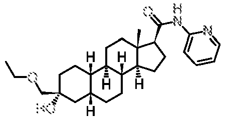 | A |
| 21 | | 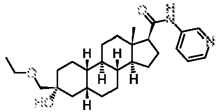 | B |
| 22 | | 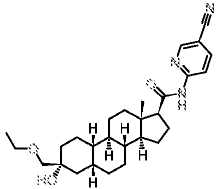 | A |
| 23 | | 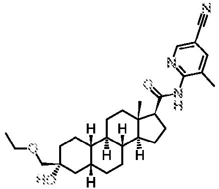 | B |
| 24 | | 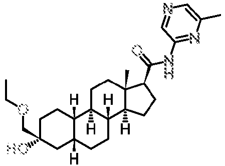 | A |
| 25 | | 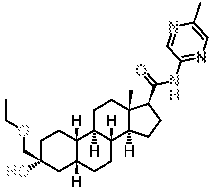 | A |
| 26 | | 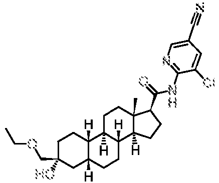 | A |
| 27 | | 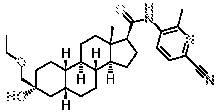 | A |
| 28 | | 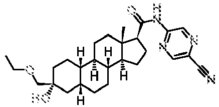 | A |
| 29 | | 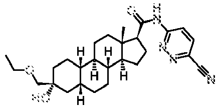 | A |
| 30 | | 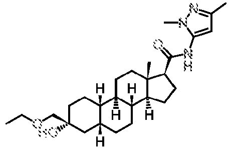 | A |
| 31 | | 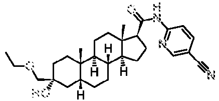 | A |
| 32 | | 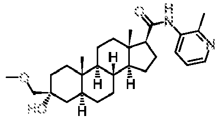 | A |
| 33 | | 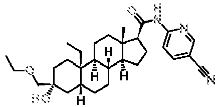 | A |
| 35 | | 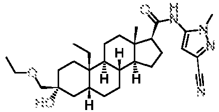 | A |
| 36 | | 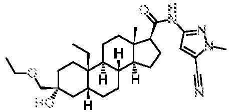 | A |
| 37 | | 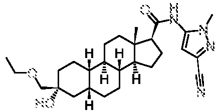 | A |
| 38 | | 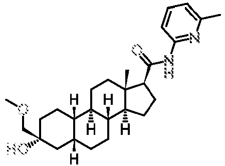 | A |
| 39 | | 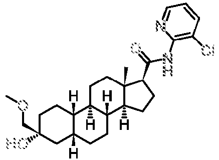 | B |
| 40 | | 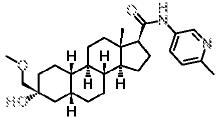 | A |
| 41 | | 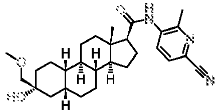 | A |
| 42 | | 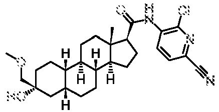 | A |
| 43 | | 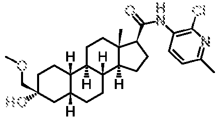 | A |
| 44 | | 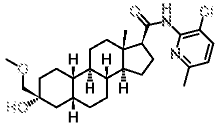 | B |
| 45 | | 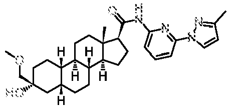 | A |
| 46 | | 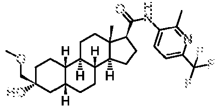 | A |
| 47 | | 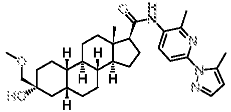 | A |
| 48 | | 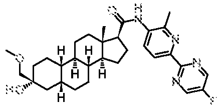 | A |
| 49 | | 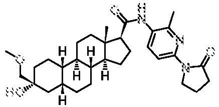 | A |
| 50 | | 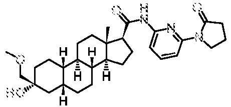 | A |
| 51 | | 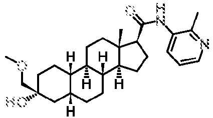 | A |
| 52 | | 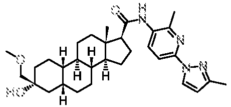 | A |
| 53 | | 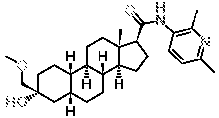 | |
| 54 | | 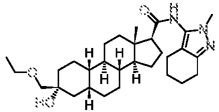 | B |
| 55 | | 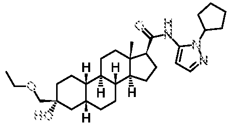 | A |
| 56 | | 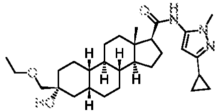 | A |
| 57 | | 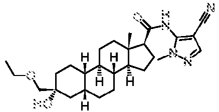 | C |
| 58 | | 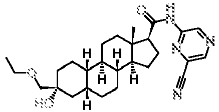 | A |
| 59 | | 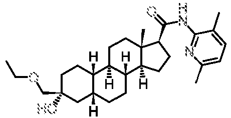 | B |
| 60 | | 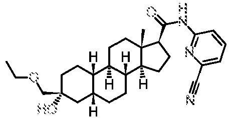 | A |
| 61 | | 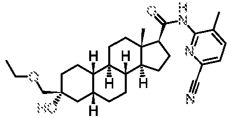 | B |
| 62 | | 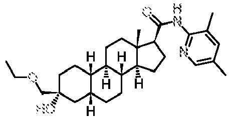 | B |
| 63 | | 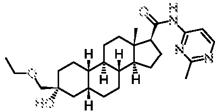 | A |
| 64 | | 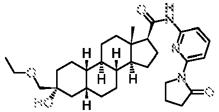 | A |
| 65 | | 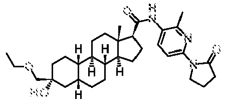 | A |
| 66 | | 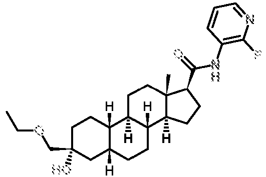 | A |
| 67 | | 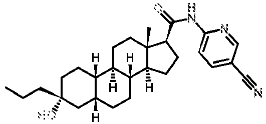 | A |
| 68 | | 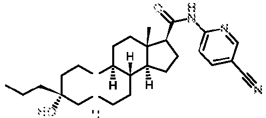 | A |

## Claims

1. A compound of Formula (I): 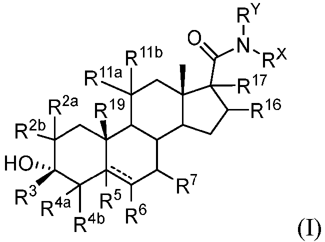 or a pharmaceutically acceptable salt thereof;
wherein:
- - - - - - represents a single or a double bond as valency permits;
each of R^{2a}, R^{2b}, R^{4a}, R^{4b}, R⁶, R⁷, R^{11a}, and R^{11b}, is independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, an oxygen protecting group when attached to an oxygen atom, a nitrogen protecting group when attached to a nitrogen atom, or two R^{D1} groups are joined to form an substituted or unsubstituted heterocyclic ring; or any one of R^{2a} and R^{1b}, R^{4a} and R^{4b}, R^{7a} and R^{7b}, R^{11a} and R^{11b} are joined to form an oxo (=O) group;
each of R¹⁶ and R¹⁷ is each independently hydrogen, halogen, cyano, hydroxyl, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}),-CN(R^{A1})₂, -C(O)R^{A1}, -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, - SC(=O)N(R^{A1})₂,-NHC(=O)R^{A1}, -NHC(=O)OR^{A1}, -NHC(=O)SR^{A1}, -NHC(=O)N(R^{A1})₂, - OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or - S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, -SO₂R^{A2}, -C(O)R^{A2}, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R³ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R⁵ is hydrogen or methyl when - - - - - - is a single bond; and when - - - - - - is a double bond, R⁵ is absent;
R¹⁹ is hydrogen or substituted or unsubstituted alkyl;
R^{X} is independently hydrogen, or substituted or unsubstituted alkyl
R^{Y} is independently substituted or unsubstituted heteroaryl.

2. The compound of claim 1, wherein the compound of Formula (I) is a compound of Formula (I-X) 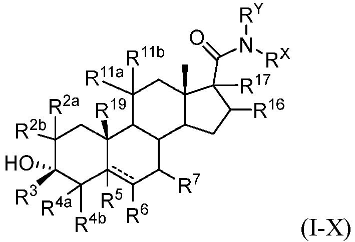 or
a compound of Formula (I-1) 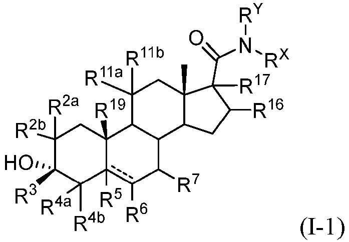 wherein:
- - - - - - represents a single or a double bond as valency permits;
each of R^{2a}, R^{2b}, R^{4a}, R^{4b}, R⁶, R⁷, R^{11a}, and R^{11b}, is independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, an oxygen protecting group when attached to an oxygen atom, a nitrogen protecting group when attached to a nitrogen atom, or two R^{D1} groups are joined to form an substituted or unsubstituted heterocyclic ring; or any one of R^{2a} and R^{2b}, R^{4a} and R^{4b}, R^{7a} and R^{7b}, R^{11a} and R^{11b} are joined to form an oxo (=O) group;
each of R¹⁶ and R¹⁷ is each independently hydrogen, halogen, cyano, hydroxyl, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}),-CN(R^{A1})₂, -C(O)R^{A1}, -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, - SC(=O)N(R^{A1})₂,-NHC(=O)R^{A1}, -NHC(=O)OR^{A1}, -NHC(=O)SR^{A1}, -NHC(=O)N(R^{A1})₂, - OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or - S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, -SO₂R^{A2}, -C(O)R^{A2}, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R³ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R⁵ is hydrogen or methyl when - - - - - - is a single bond; and when - - - - - - is a double bond, R⁵ is absent;
R¹⁹ is hydrogen or methyl;
R^{X} is independently hydrogen, or substituted or unsubstituted alkyl
R^{Y} is independently substituted or unsubstituted heteroaryl,
or a pharmaceutically acceptable salt thereof.

3. The compound of claim 1, wherein the compound of Formula (I) is: 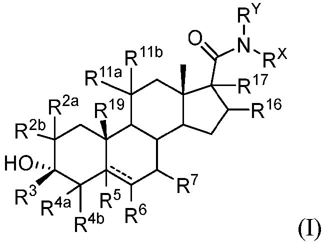 wherein:
- - - - - - represents a single or a double bond as valency permits;
each of R^{2a}, R^{2b}, R^{4a}, R^{4b}, R⁶, R⁷, R^{11a}, and R^{11b}, is independently hydrogen, halogen, cyano, hydroxyl, substituted or unsubstituted alkyl, substituted or unsubstituted alkenyl, substituted or unsubstituted heterocyclyl, or substituted or unsubstituted alkynyl, -OR^{D1},-OC(=O)R^{D1}, -NH₂, -N(R^{D1})₂, or -NR^{D1}C(=O)R^{D1}, wherein each instance of R^{D1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl, an oxygen protecting group when attached to an oxygen atom, a nitrogen protecting group when attached to a nitrogen atom, or two R^{D1} groups are joined to form an substituted or unsubstituted heterocyclic ring; or any one of R^{2a} and R^{2b}, R^{4a} and R^{4b}, R^{7a} and R^{7b}, R^{11a} and R^{11b} are joined to form an oxo (=O) group;
each of R¹⁶ and R¹⁷ is each independently hydrogen, halogen, cyano, hydroxyl, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, -OR^{A1}, -SR^{A1}, -N(R^{A1})₂, -N(R^{A1}),-CN(R^{A1})₂, -C(O)R^{A1}, -OC(=O)R^{A1}, -OC(=O)OR^{A1}, - OC(=O)SR^{A1}, -OC(=O)N(R^{A1})₂, -SC(=O)R^{A2}, -SC(=O)OR^{A1}, -SC(=O)SR^{A1}, - SC(=O)N(R^{A1})₂,-NHC(=O)R^{A1}, -NHC(=O)OR^{A1}, -NHC(=O)SR^{A1}, -NHC(=O)N(R^{A1})₂, - OS(=O)₂R^{A2}, -OS(=O)₂OR^{A1}, -S-S(=O)₂R^{A2}, -S-S(=O)₂OR^{A1}, -S(=O)R^{A2}, -SO₂R^{A2}, or - S(=O)₂OR^{A1}, wherein each instance of R^{A1} is independently hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, substituted or unsubstituted heteroaryl, an oxygen protecting group when attached to an oxygen atom, a sulfur protecting group when attached to a sulfur atom, a nitrogen protecting group when attached to a nitrogen atom, -SO₂R^{A2}, -C(O)R^{A2}, or two R^{A1} groups are joined to form an substituted or unsubstituted heterocyclic or heteroaryl ring; and R^{A2} is substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R³ is hydrogen, substituted or unsubstituted alkyl, substituted or unsubstituted alkenyl, substituted or unsubstituted alkynyl, substituted or unsubstituted carbocyclyl, substituted or unsubstituted heterocyclyl, substituted or unsubstituted aryl, or substituted or unsubstituted heteroaryl;
R⁵ is hydrogen or methyl when - - - - - - is a single bond; and when - - - - - - is a double bond, R⁵ is absent;
R¹⁹ is hydrogen or substituted or unsubstituted alkyl;
R^{X} is independently hydrogen, or substituted or unsubstituted alkyl
R^{Y} is independently substituted or unsubstituted heteroaryl.

4. The compound of any one of claims 1-3, wherein R^{2a} and R^{2b}
(a) is each independently hydrogen, halogen, cyano, hydroxyl, substituted or unsubstituted alkyl, substituted or unsubstituted alkenyl, -OR^{D1}, -OC(=O)R^{D1}, -NH₂, or - N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl;
(b) is each independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH;
(c) is each independently hydrogen, -CH₃, -CH₂CH₃, -OH, -OCH₃, or -CH(CH₃)₂;
(d) is each independently hydrogen; or
(e) are both hydrogen.

5. The compound of any one of claims 1-4, wherein R^{4a} and R^{4b}
(a) is each independently hydrogen, halogen, cyano, hydroxyl, substituted or unsubstituted alkyl, substituted or unsubstituted alkenyl, -OR^{D1}, -OC(=O)R^{D1}, -NH₂, or - N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl;
(b) is each independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH;
(c) is each independently hydrogen, -CH₃, -CH₂CH₃, -OH, -OCH₃, or -CH(CH₃)₂;
(d) is each independently hydrogen; or
(e) are both hydrogen.

6. The compound of any one of claims 1-5, wherein R^{11a} and R^{11b}
(a) is each independently hydrogen, halogen, cyano, substituted or unsubstituted alkyl, substituted or unsubstituted alkenyl, -OR^{D1}, -OC(=O)R^{D1}, -NH₂, or -N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl;
(b) is each independently hydrogen, substituted or unsubstituted C₁-C₆ alkyl, substituted or unsubstituted C₁-C₆ haloalkyl, substituted or unsubstituted C₁-C₆ alkoxy, or substituted or unsubstituted C₁-C₆ alkoxyhalo;
(c) is each independently hydrogen, -CH₃, -CH₂CH₃, -OH, -OCH₃, or -CH(CH₃)₂;
(d) is each independently hydrogen; or
(e) are both hydrogen.

7. The compound of any one of claims 1-6, wherein R⁷ is
(a) independently hydrogen, halogen, cyano, hydroxyl, substituted or unsubstituted alkyl, substituted or unsubstituted alkenyl, -OR^{D1}, -OC(=O)R^{D1}, -NH₂, or - N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl;
(b) independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH;
(c) independently hydrogen, -CH₃, -CH₂CH₃, -OH, -OCH₃, or -CH(CH₃)₂; or
(d) hydrogen.

8. The compound of any one of claims 1-7, wherein R⁶ is
(a) independently hydrogen, halogen, cyano, hydroxyl, substituted or unsubstituted alkyl, substituted or unsubstituted alkenyl, -OR^{D1}, -OC(=O)R^{D1}, -NH₂, or - N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl;
(b) independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH;
(c) independently hydrogen, -CH₃, -CH₂CH₃, -OH, -OCH₃, or -CH(CH₃)₂; or
(d) hydrogen.

9. The compound of any one of claims 1-8, wherein R¹⁶ is
(a) independently hydrogen, halogen, cyano, hydroxyl, substituted or unsubstituted alkyl, substituted or unsubstituted alkenyl, -OR^{D1}, -OC(=O)R^{D1}, -NH₂, or - N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl;
(b) independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH;
(c) independently hydrogen, -CH₃, -CH₂CH₃, -OH, -OCH₃, or -CH(CH₃)₂; or
(d) hydrogen.

10. The compound of any one of claims 1-9, wherein R¹⁷ is
(a) independently hydrogen, halogen, cyano, hydroxyl, substituted or unsubstituted alkyl, substituted or unsubstituted alkenyl, -OR^{D1}, -OC(=O)R^{D1}, -NH₂, or - N(R^{D1})₂, wherein each instance of R^{D1} is independently hydrogen, substituted or unsubstituted alkyl, substituted or unsubstituted carbocyclyl, substituted or unsubstituted heterocyclyl, substituted or unsubstituted aryl, or substituted or unsubstituted heteroaryl;
(b) independently hydrogen, substituted or unsubstituted C₁-C₆ alkyl, substituted or unsubstituted C₁-C₆ haloalkyl, substituted or unsubstituted C₁-C₆ alkoxy, substituted or unsubstituted C₁-C₆ alkoxyhalo, or -OH;
(c) independently hydrogen, -CH₃, -CH₂CH₃, -OH, -OCH₃, or -CH(CH₃)₂; or
(d) hydrogen.

11. The compound of any one of claims 1-10, wherein R¹⁹ is
(a) independently hydrogen, or substituted or unsubstituted C₁-C₆ alkyl;
(b) independently hydrogen, or substituted or unsubstituted C₁-C₄ alkyl;
(c) independently hydrogen, -CH₃, -CH₂CH₃, or -CH₂OCH(CH₃)₂;
(d) hydrogen; or
(e) -CH₂OCH(CH₃)₂.

12. The compound of any one of claims 1-11, wherein R³ is
(a) substituted or unsubstituted alkyl, substituted or unsubstituted alkenyl, or substituted or unsubstituted alkynyl, substituted or unsubstituted carbocyclyl, or substituted or unsubstituted heterocyclyl;
(b) substituted or unsubstituted alkyl; or
(c) substituted or unsubstituted C₁-C₆ alkyl.

13. The compound of any one of claims 1-12,
wherein the compound of Formula (I) is a compound of Formula (I-a): 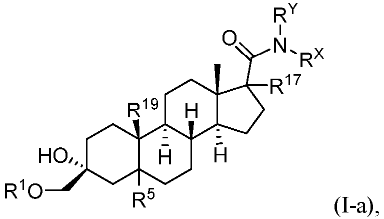
wherein R¹ is substituted or unsubstituted alkyl.

14. The compound of claim 13, wherein R¹
(a) is -CH₂CH₃ or-CH₃;
(b) is -CH₂CH₃; or
(c) is -CH_{3.}

15. The compound of any one of claims 1-14, wherein the compound of Formula (I) is a compound of Formula (I-b1) or Formula (I-b2): 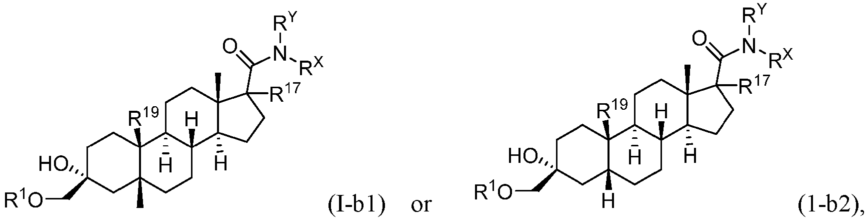 or
wherein the compound of Formula (I) is a compound of Formula (I-c1) or Formula (I-c2): 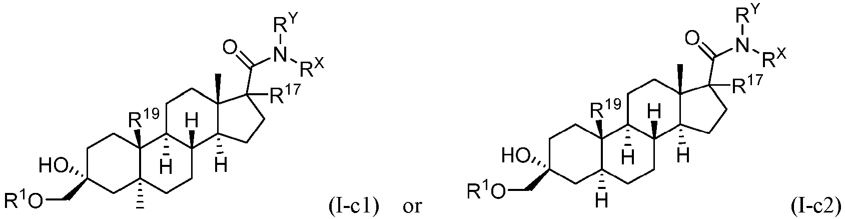 or
wherein the compound of Formula (I) is a compound of Formula (I-d1) or Formula (I-d2): 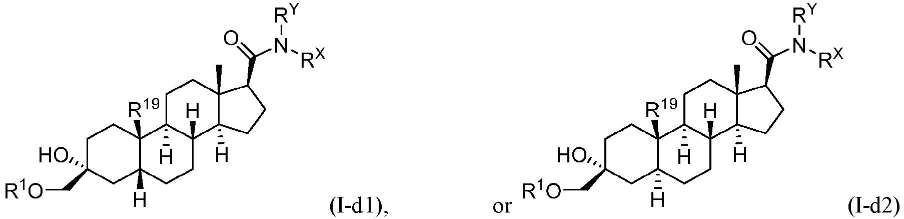 or
wherein the compound of Formula (I) is a compound of Formula (I-d1): 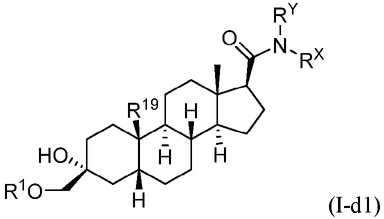 or
wherein the compound of Formula (I) is a compound of Formula (I-d2): 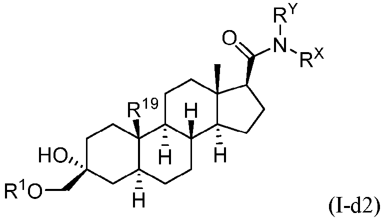 or
wherein the compound of Formula (I), is a compound of Formula (I-e): 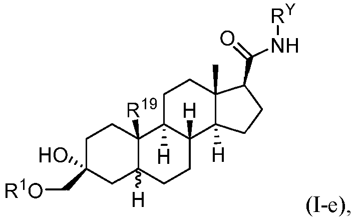
wherein R^{Y} is heteroaryl, and R¹ is -CH₂CH₃ or -CH₃,
or
wherein the compound of Formula (I) is a compound of Formula (I-f): 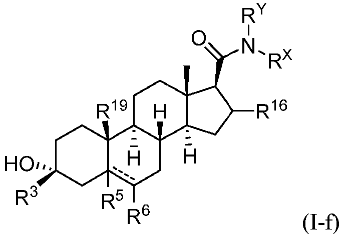 or
wherein the compound of Formula (I) is a compound of Formula (I-g): 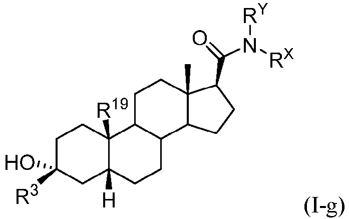
or a pharmaceutically acceptable salt thereof.

16. The compound of any one of claims 1-15, wherein R^{X} is
(a) independently hydrogen, or substituted or unsubstituted C₁₋₆ alkyl;
(b) independently hydrogen, or substituted or unsubstituted C₁₋₄ alkyl;
(c) independently hydrogen,-CH₃, or -CH₂CH₃; or
(d) hydrogen.

17. The compound of any one of claims 1-16, wherein R^{Y}
(a) is selected from: 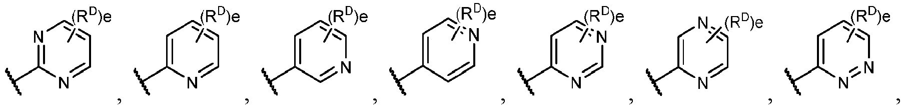 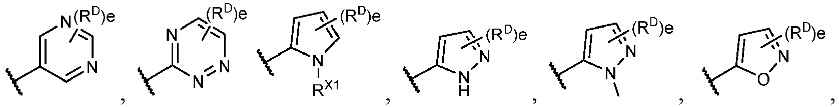 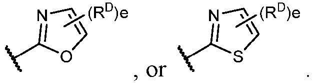 wherein each instance of R^{D} is, independently hydrogen, halogen, -NO₂, -CN, -OR^{GA}, - N(R^{GA})₂, -C(=O)R^{GA}, -C(=O)OR^{GA}, -C(=O)N(R^{GA})₂, -OC(=O)R^{GA}, -OC(=O)OR^{GA}, - N(R^{GA})C(=O)R^{GA}, -OC(=O)N(R^{GA})₂, -N(R^{GA})C(=O)OR^{GA}, -S(=O)₂R^{GA}, -S(=O)₂OR^{GA}, - OS(=O)₂R^{GA}, -S(=O)₂N(R^{GA})₂, or -N(R^{GA})S(=O)₂R^{GA}, substituted or unsubstituted C₁₋₆ alkyl, substituted or unsubstituted C₂₋₆ alkenyl, substituted or unsubstituted C₂₋₆ alkynyl, substituted or unsubstituted C₃₋₄ carbocyclyl, substituted or unsubstituted 3- to 6-membered heterocyclyl, C₅₋₁₀ substituted or unsubstituted aryl, substituted or unsubstituted 5- to 10- membered heteroaryl, or optionally two R^{GA} are taken with the intervening atoms to form a substituted or unsubstituted 3- to 4-membered carbocyclic or heterocyclic ring;
wherein each instance of R^{GA} is independently hydrogen, substituted or unsubstituted C₁₋₆ alkyl, substituted or unsubstituted C₂₋₆ alkenyl, substituted or unsubstituted C₂₋₆ alkynyl, substituted or unsubstituted C₃₋₆ carbocyclyl, substituted or unsubstituted 3- to 6- membered heterocyclyl, substituted or unsubstituted aryl, substituted or unsubstituted heteroaryl, an oxygen protecting group when attached to oxygen, a nitrogen protecting group when attached to nitrogen, or two R^{GA} groups are taken with the intervening atoms to form a substituted or unsubstituted carbocyclic or heterocyclic ring;
wherein each instance R^{X1} is hydrogen or substituted or unsubstituted alkyl, substituted or unsubstituted cycloalkyl, substituted or unsubstituted aryl, or substituted or unsubstituted carbocyclyl and
e is 0, 1, 2, 3, 4, or 5;
(b) is selected from: 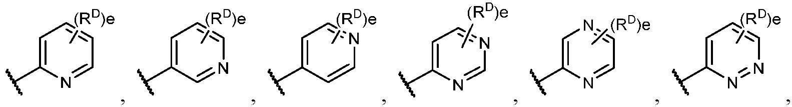 or 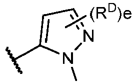
wherein each instance of R^{D} is, independently hydrogen, halogen, substituted or unsubstituted C₁₋₆ alkyl, -CN, substituted or unsubstituted 5-6 membered heteroaryl, substituted or unsubstituted 5-membered heterocyclyl,
e is 0, 1, 2, 3, or 4;
(c) is selected from: 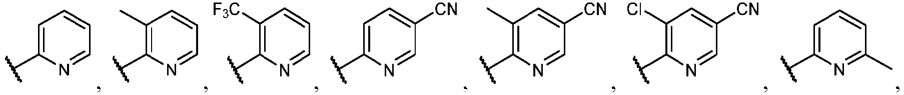 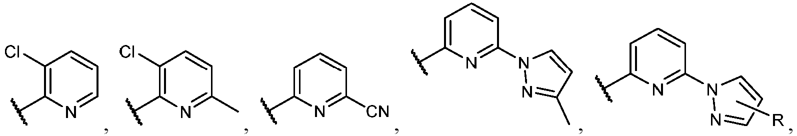 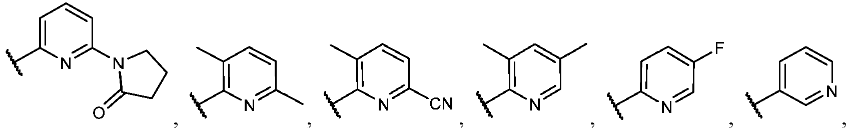 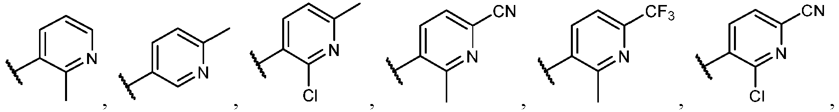 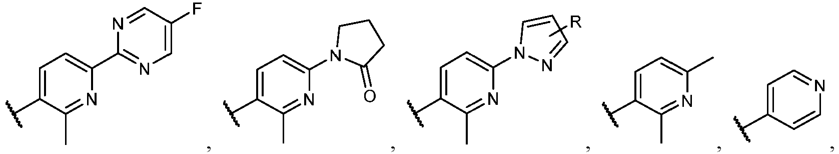 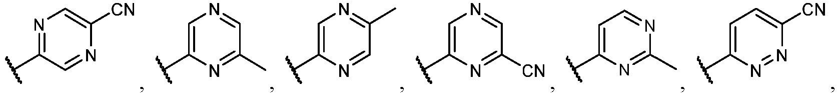 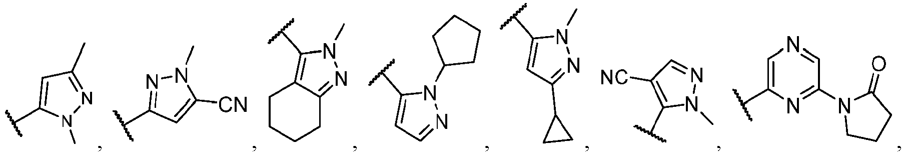 or 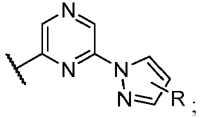 wherein R is -CH₃, -CH₂CH₃, -i-Pr, cyclopropyl or -CN;
(d) is selected from: 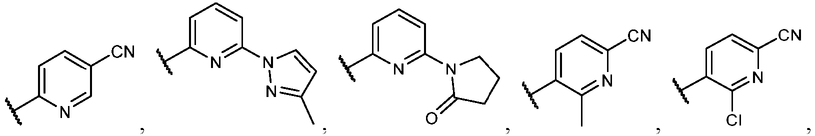 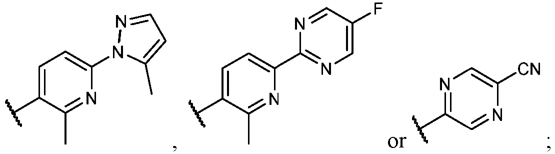
(e) is 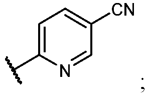 or
(f) is

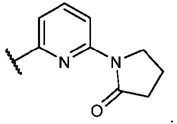

18. The compound of any one of claims 1-17, when the compound is selected from:
| **Example** | **STRUCTURE** |
|---|---|
| 1 | 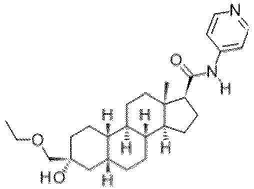 |
| 2 | 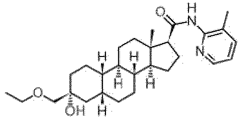 |
| 3 | 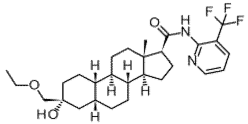 |
| 4 | 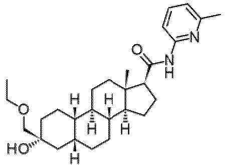 |
| 5 | 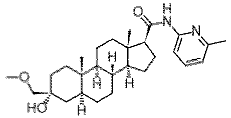 |
| 6 | 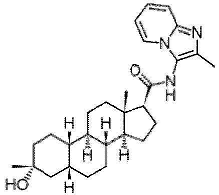 |
| 7 | 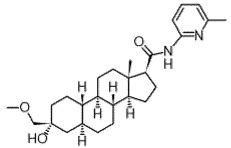 |
| 8 | 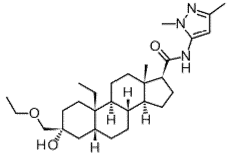 |
| 9 | 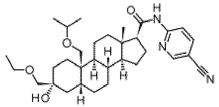 |
| 10 | 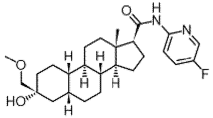 |
| 11 | 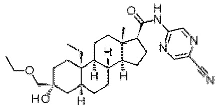 |
| 12 | 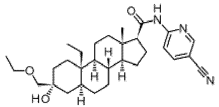 |
| 13 | 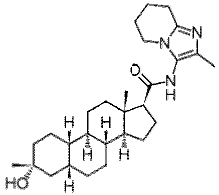 |
| 14 | 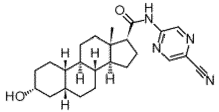 |
| 15 | 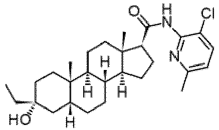 |
| 16 | 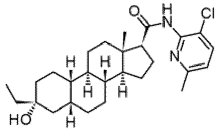 |
| 20 | 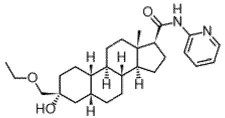 |
| 21 | 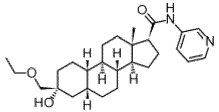 |
| 22 | 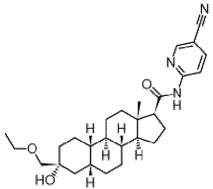 |
| 23 | 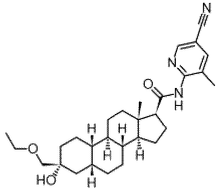 |
| 24 | 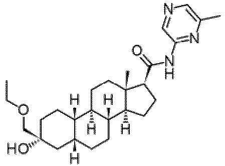 |
| 25 | 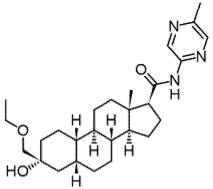 |
| 26 | 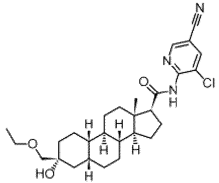 |
| 27 | 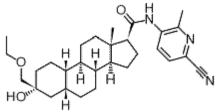 |
| 28 | 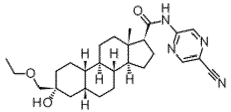 |
| 29 | 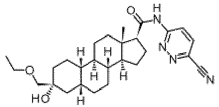 |
| 30 | 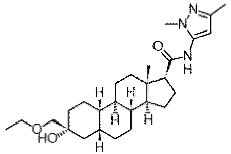 |
| 31 | 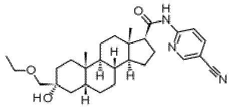 |
| 32 | 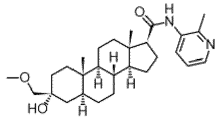 |
| 33 | 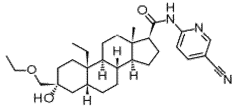 |
| 35 | 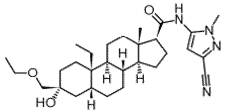 |
| 36 | 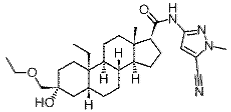 |
| 37 | 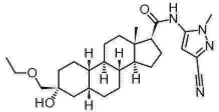 |
| 38 | 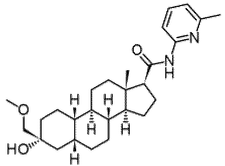 |
| 39 | 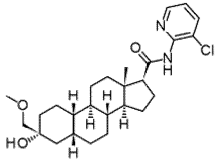 |
| 40 | 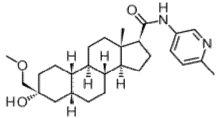 |
| 41 | 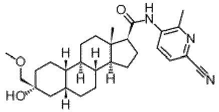 |
| 42 | 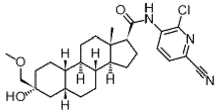 |
| 43 | 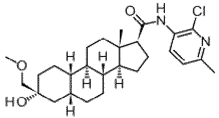 |
| 44 | 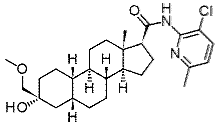 |
| 45 | 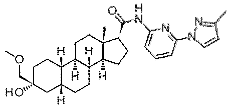 |
| 46 | 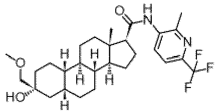 |
| 47 | 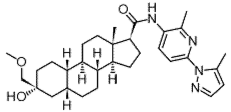 |
| 48 | 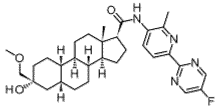 |
| 49 | 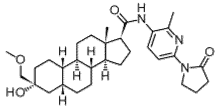 |
| 50 | 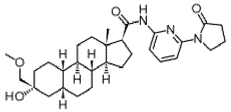 |
| 51 | 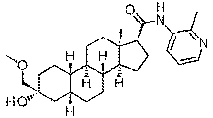 |
| 52 | 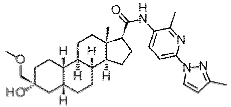 |
| 53 | 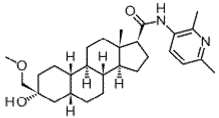 |
| 54 | 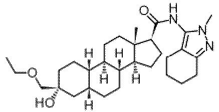 |
| 55 | 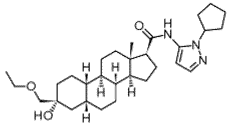 |
| 56 | 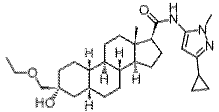 |
| 57 | 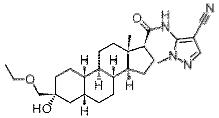 |
| 58 | 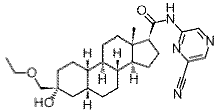 |
| 59 | 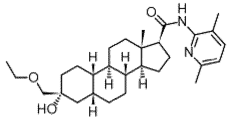 |
| 60 | 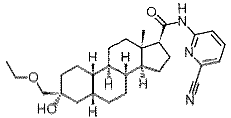 |
| 61 | 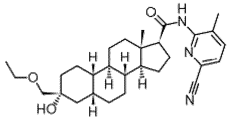 |
| 62 | 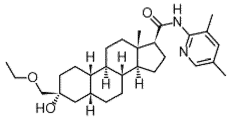 |
| 63 | 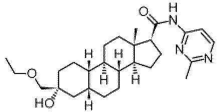 |
| 64 | 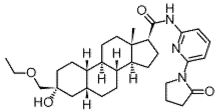 |
| 65 | 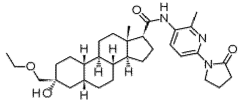 |
| 66 | 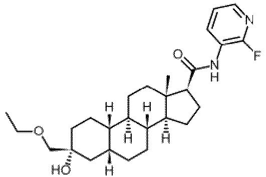 |
| 67 | 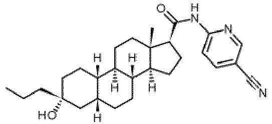 |
| 68 | 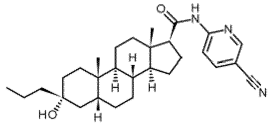 |

19. A compound of any one of claims 1-18, or a pharmaceutically acceptable salt thereof, for use in a method of treating a CNS-related disorder in a subject in need thereof.

20. The compound, or a pharmaceutically acceptable salt thereof, for use according to claim 19, wherein
(a) the CNS-related disorder is a sleep disorder, a mood disorder, a schizophrenia spectrum disorder, a convulsive disorder, a disorder of memory and/or cognition, a movement disorder, a personality disorder, autism spectrum disorder, pain, traumatic brain injury, a vascular disease, a substance abuse disorder and/or withdrawal syndrome, tinnitus, or status epilepticus;
(b) the CNS-related disorder is depression;
(c) the CNS-related disorder is postpartum depression;
(d) the CNS-related disorder is major depressive disorder;
(e) the CNS-related disorder is major depressive disorder and the major depressive disorder is moderate major depressive disorder; or
(f) the CNS-related disorder is major depressive disorder and the major depressive disorder is severe major depressive disorder.

## Patentansprüche

1. Verbindung der Formel (I): 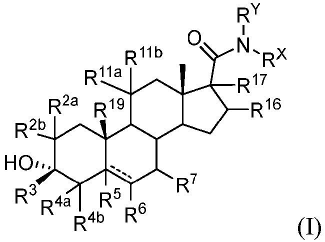 oder ein pharmazeutisch unbedenkliches Salz davon;
wobei:
**- - - - - -** für eine Einfach- oder eine Doppelbindung steht, wie es die Wertigkeit erlaubt;
jedes von R^{2a}, R^{2b}, R^{4a}, R^{4b}, R⁶, R⁷, R^{11a} und R^{11b} unabhängig Wasserstoff, Halogen, Cyano, Hydroxyl, substituiertes oder unsubstituiertes Alkyl, substituiertes oder unsubstituiertes Alkenyl, substituiertes oder unsubstituiertes Heterocyclyl oder substituiertes oder unsubstituiertes Alkinyl, -OR^{D1}, -OC(=O)R^{D1}, -NH₂, -N(R^{D1})₂ oder -NR^{D1}C (=O)R^{D1} ist, wobei jedes Vorkommen von R^{D1} unabhängig Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl, eine Sauerstoff-Schutzgruppe, wenn es an ein Sauerstoffatom gebunden ist, eine Stickstoff-Schutzgruppe ist, wenn es an ein Stickstoffatom gebunden ist, oder zwei R^{D1}-Gruppen zusammengenommen einen substituierten oder unsubstituierten heterocyclischen Ring bilden; oder beliebige von R^{2a} und R^{2b}, R^{4a} und R^{4b}, R^{7a} und R^{7b}, R^{11a} und R^{11b} zusammengenommen eine Oxogruppe (=O) bilden;
jedes von R¹⁶ und R¹⁷ unabhängig Wasserstoff, Halogen, Cyano, Hydroxyl, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl, -OR^{A1}, - SR^{A1}, -N(R^{A1})₂, -N(R^{A1}),-CN(R^{A1})₂, -C(O)R^{A1}, -OC(=O)R^{A1}, - OC (=O)OR^{A1}, -OC (=O) SR^{A1}, -OC (=O) N (R^{A1})₂, -SC(=O) R^{A2}, - SC (=O) OR^{A1}, -SC (=O) SR^{A1}, -SC (=O) N (R^{A1})₂, -NHC(=O)R^{A1}, - NHC (=O)OR^{A1}, -NHC (=O) SR^{A1}, -NHC (=O) N (R^{A1})₂, -OS (=O)₂R^{A2}, - OS (=O)₂OR^{A1}, -S-S (=O) ₂R^{A2}, -S-S (=O)₂OR^{A1}, -S(=O) R^{A2}, -SO₂R^{A2} oder -S (=O)₂OR^{A1} ist, wobei jedes Vorkommen von R^{A1} unabhängig Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl, eine Sauerstoff-Schutzgruppe, wenn es an ein Sauerstoffatom gebunden ist, eine Schwefel-Schutzgruppe, wenn es an ein Schwefelatom gebunden ist, eine Stickstoff-Schutzgruppe, wenn es an ein Stickstoffatom gebunden ist, -SO₂R^{A2}, -C(O)R^{A2} ist, oder zwei R^{A1}-Gruppen verbunden sind, um einen substituierten oder unsubstituierten heterocyclischen oder Heteroarylring zu bilden; und R^{A2} substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder
unsubstituiertes Heterocyclyl, substituiertes oder unsubstituiertes Aryl ist, oder substituiertes oder unsubstituiertes Heteroaryl;
R³ Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
R⁵ Wasserstoff oder Methyl ist, wenn - - - - - - eine Einfachbindung ist; und wenn - - - - - - eine Doppelbindung ist, dann R⁵ fehlt;
R¹⁹ Wasserstoff oder substituiertes oder unsubstituiertes Alkyl ist;
R^{X} unabhängig Wasserstoff oder substituiertes oder unsubstituiertes Alkyl ist;
R^{Y} unabhängig substituiertes oder unsubstituiertes Heteroaryl ist.

2. Verbindung nach Anspruch 1, wobei die Verbindung der Formel (I) eine Verbindung der Formel (I-X) ist: 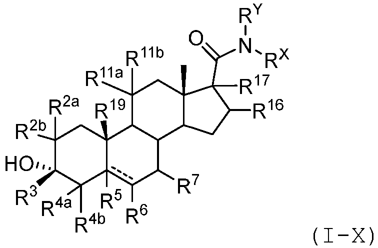 oder
eine Verbindung der Formel (I-1): 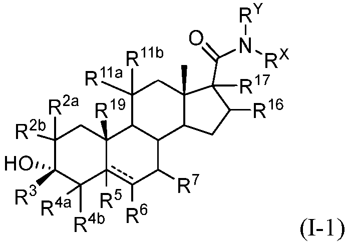
wobei:
- - - - - - für eine Einfach- oder eine Doppelbindung steht, wie es die Wertigkeit erlaubt;
jedes von R^{2a}, R^{2b}, R^{4a}, R^{4b}, R⁶, R⁷, R^{11a} und R^{11b} unabhängig Wasserstoff, Halogen, Cyano, Hydroxyl, substituiertes oder unsubstituiertes Alkyl, substituiertes oder unsubstituiertes Alkenyl, substituiertes oder unsubstituiertes Heterocyclyl oder substituiertes oder unsubstituiertes Alkinyl, -OR^{D1}, -OC(=O)R^{D1}, -NH₂, -N(R^{D1})₂ oder -NR^{D1}C(=O)R^{D1} ist, wobei jedes Vorkommen von R^{D1} unabhängig Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl, eine Sauerstoff-Schutzgruppe, wenn es an ein Sauerstoffatom gebunden ist, eine Stickstoff-Schutzgruppe ist, wenn es an ein Stickstoffatom gebunden ist, oder zwei R^{D1}-Gruppen zusammengenommen einen substituierten oder unsubstituierten heterocyclischen Ring bilden; oder beliebige von R^{2a} und R^{2b}, R^{4a} und R^{4b}, R^{7a} und R^{7b}, R^{11a} und R^{11b} zusammengenommen eine Oxogruppe (=O) bilden;
jedes von R¹⁶ und R¹⁷ jeweils unabhängig Wasserstoff, Halogen, Cyano, Hydroxyl, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl, -OR^{A1}, -SR^{A1}, -N(R^{A1})₂, - N(R^{A1}), -CN (R^{A1})₂, -C (O) R^{A1}, -OC(=O) R^{A1}, -OC(=O)OR^{A1}, - OC (=O) SR^{A1}, -OC (=O) N (R^{A1})₂, -SC (=O)R^{A2}, -SC (=O) OR^{A1}, - SC (=O) SR^{A1}, - SC (=O) N (R^{A1})₂, -NHC (=O) R^{A1}, -NHC (=O) OR^{A1}, - NHC (=O) SR^{A1}, -NHC (=O)N(R^{A1})₂, -OS(=O)₂R^{A2}, -OS(=O)₂OR^{A1}, -S-S (=O) ₂R^{A2}, -S-S (=O)₂OR^{A1}, -S(=O)R^{A2}, -SO₂R^{A2} oder -S(=O)₂OR^{A1} ist, wobei jedes Vorkommen von R^{A1} unabhängig Wasserstoff,
substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl, eine Sauerstoff-Schutzgruppe, wenn es an ein Sauerstoffatom gebunden ist, eine Schwefel-Schutzgruppe, wenn es an ein Schwefelatom gebunden ist, eine Stickstoff-Schutzgruppe, wenn es an ein Stickstoffatom gebunden ist, -SO₂R^{A2}, - C(O)R^{A2} ist, oder zwei R^{A1}-Gruppen verbunden sind, um einen substituierten oder unsubstituierten heterocyclischen oder Heteroarylring zu bilden; und R^{A2} substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl ist, oder substituiertes oder unsubstituiertes Heteroaryl;
R³ Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
R⁵ Wasserstoff oder Methyl ist, wenn - - - - - - eine Einfachbindung ist; und wenn - - - - - - eine Doppelbindung ist, dann R⁵ fehlt;
R¹⁹ Wasserstoff oder Methyl ist;
R^{X} unabhängig Wasserstoff oder substituiertes oder unsubstituiertes Alkyl ist;
R^{Y} unabhängig substituiertes oder unsubstituiertes Heteroaryl ist,
oder ein pharmazeutisch unbedenkliches Salz davon.

3. Verbindung nach Anspruch 1, wobei die Verbindung der Formel (I) wie folgt ist: 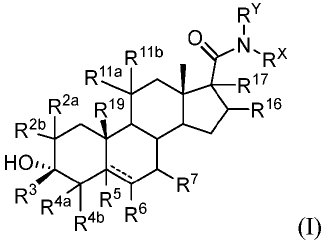 wobei:
- - - - - - für eine Einfach- oder eine Doppelbindung steht, wie es die Wertigkeit erlaubt;
jedes von R^{2a}, R^{2b}, R^{4a}, R^{4b}, R⁶, R⁷, R^{11a} und R^{11b} unabhängig Wasserstoff, Halogen, Cyano, Hydroxyl, substituiertes oder unsubstituiertes Alkyl, substituiertes oder unsubstituiertes Alkenyl, substituiertes oder unsubstituiertes Heterocyclyl oder substituiertes oder unsubstituiertes Alkinyl, -OR^{D1}, -OC(=O)R^{D1}, -NH₂, -N(R^{D1})₂ oder -NR^{D1}C(=O)R^{D1} ist, wobei jedes Vorkommen von R^{D1} unabhängig Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl, eine Sauerstoff-Schutzgruppe, wenn es an ein Sauerstoffatom gebunden ist, eine Stickstoff-Schutzgruppe ist, wenn es an ein Stickstoffatom gebunden ist, oder zwei R^{D1}-Gruppen zusammengenommen einen substituierten oder unsubstituierten heterocyclischen Ring bilden; oder beliebige von R^{2a} und R^{2b}, R^{4a} und R^{4b}, R^{7a} und R^{7b}, R^{11a} und R^{11b} zusammengenommen eine Oxogruppe (=O) bilden;
jedes von R¹⁶ und R¹⁷ unabhängig Wasserstoff, Halogen, Cyano, Hydroxyl, substituiertes oder unsubstituiertes Alkyl, substituiertes oder unsubstituiertes Alkenyl, substituiertes oder unsubstituiertes Alkinyl, substituiertes oder unsubstituiertes Carbocyclyl,
substituiertes oder unsubstituiertes Heterocyclyl, substituiertes oder unsubstituiertes Aryl, substituiertes oder unsubstituiertes Heteroaryl, -OR^{A1}, - SR^{A1}, -N(R^{A1})₂, -N(R^{A1}), -CN(R^{A1})₂, -C(O)R^{A1}, -OC(=O) R^{A1}, - OC (=O)OR^{A1}, -OC (=O)SR^{A1}, -OC (=O)N(R^{A1})₂, -SC(=O) R^{A2}, - SC(=O)OR^{A1}, -SC (=O)SR^{A1}, -SC (=O)N(R^{A1})₂, -NHC(=O) R^{A1}, - NHC (=O)OR^{A1}, -NHC (=O) SR^{A1}, -NHC (=O)N(R^{A1})₂, -OS (=O)₂R^{A2}, - OS (=O)₂OR^{A1}, -S-S (=O)₂R^{A2}, -S-S (=O)₂OR^{A1}, -S(=O) R^{A2}, -SO₂R^{A2} oder -S(=O)₂OR^{A1} ist, wobei jedes Vorkommen von R^{A1} unabhängig Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl, substituiertes oder unsubstituiertes Heteroaryl, eine Sauerstoff-Schutzgruppe, wenn es an ein Sauerstoffatom gebunden ist, eine Schwefel-Schutzgruppe, wenn es an ein Schwefelatom gebunden ist, eine Stickstoff-Schutzgruppe, wenn es an ein Stickstoffatom gebunden ist, -SO₂R^{A2}, -C(O)R^{A2} ist, oder zwei R^{A1}-Gruppen verbunden sind, um einen substituierten oder unsubstituierten heterocyclischen oder Heteroarylring zu bilden; und R^{A2} substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl ist, oder substituiertes oder unsubstituiertes Heteroaryl;
R³ Wasserstoff, substituiertes oder unsubstituiertes Alkyl, substituiertes oder unsubstituiertes Alkenyl, substituiertes oder unsubstituiertes Alkinyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
R⁵ Wasserstoff oder Methyl ist, wenn - - - - - - eine Einfachbindung ist; und wenn - - - - - - eine Doppelbindung ist, dann R⁵ fehlt;
R¹⁹ Wasserstoff oder substituiertes oder unsubstituiertes Alkyl ist;
R^{X} unabhängig Wasserstoff oder substituiertes oder unsubstituiertes Alkyl ist;
R^{Y} unabhängig substituiertes oder unsubstituiertes Heteroaryl ist.

4. Verbindung nach einem der Ansprüche 1-3, wobei R^{2a} und R^{2b}
(a) jeweils unabhängig Wasserstoff, Halogen, Cyano, Hydroxyl, substituiertes oder unsubstituiertes Alkyl, substituiertes oder unsubstituiertes Alkenyl, -OR^{D1}, - OC(=O)R^{D1}, -NH2 oder -N(R^{D1})₂ sind, wobei jedes Vorkommen von R^{D1} unabhängig Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
(b) jeweils unabhängig Wasserstoff, substituiertes oder unsubstituiertes C₁-C₆-Alkyl, substituiertes oder unsubstituiertes C₁-C₆-Halogenalkyl, substituiertes oder unsubstituiertes C₁-C₆-Alkoxy, substituiertes oder unsubstituiertes C₁-C₆-Alkoxyhalogen oder -OH sind;
(c) jeweils unabhängig Wasserstoff, -CH₃, -CH₂CH₃, -OH, -OCH₃ oder -CH(CH₃)₂ sind;
(d) jeweils unabhängig Wasserstoff sind; oder
(e) beide Wasserstoff sind.

5. Verbindung nach einem der Ansprüche 1-4, wobei R^{4a} und R^{4b}
(a) jeweils unabhängig Wasserstoff, Halogen, Cyano, Hydroxyl, substituiertes oder unsubstituiertes Alkyl, substituiertes oder unsubstituiertes Alkenyl, -OR^{D1}, - OC(=O)R^{D1}, -NH2 oder -N(R^{D1})₂ sind, wobei jedes Vorkommen von R^{D1} unabhängig Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
(b) jeweils unabhängig Wasserstoff, substituiertes oder unsubstituiertes C₁-C₆-Alkyl, substituiertes oder unsubstituiertes C₁-C₆-Halogenalkyl, substituiertes oder unsubstituiertes C₁-C₆-Alkoxy, substituiertes oder unsubstituiertes C₁-C₆-Alkoxyhalogen oder -OH sind;
(c) jeweils unabhängig Wasserstoff, -CH₃, -CH₂CH₃, -OH, -OCH₃ oder -CH(CH₃)₂ sind;
(d) jeweils unabhängig Wasserstoff sind; oder
(e) beide Wasserstoff sind.

6. Verbindung nach einem der Ansprüche 1-5, wobei R^{11a} und R^{11b}
(a) jeweils unabhängig Wasserstoff, Halogen, Cyano, substituiertes oder unsubstituiertes Alkyl, substituiertes oder unsubstituiertes Alkenyl, -OR^{D1}, - OC(=O)R^{D1}, -NH₂ oder -N(R^{D1})₂ sind, wobei jedes Vorkommen von R^{D1} unabhängig Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
(b) jeweils unabhängig Wasserstoff, substituiertes oder unsubstituiertes C₁-C₆-Alkyl, substituiertes oder unsubstituiertes C₁-C₆-Halogenalkyl, substituiertes oder unsubstituiertes C₁-C₆-Alkoxy oder substituiertes oder unsubstituiertes C₁-C₆-Alkoxyhalogen sind;
(c) jeweils unabhängig Wasserstoff, -CH₃, -CH₂CH₃, -OH, -OCH₃ oder -CH(CH₃)₂ sind;
(d) jeweils unabhängig Wasserstoff sind; oder
(e) beide Wasserstoff sind.

7. Verbindung nach einem der Ansprüche 1-6, wobei R⁷ wie folgt ist:
(a) unabhängig Wasserstoff, Halogen, Cyano, Hydroxyl, substituiertes oder unsubstituiertes Alkyl, substituiertes oder unsubstituiertes Alkenyl, -OR^{D1}, - OC(=O)R^{D1}, -NH2 oder -N(R^{D1})₂, wobei jedes Vorkommen von R^{D1} unabhängig Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
(b) unabhängig Wasserstoff, substituiertes oder unsubstituiertes C₁-C₆-Alkyl, substituiertes oder unsubstituiertes C₁-C₆-Halogenalkyl, substituiertes oder unsubstituiertes C₁-C₆-Alkoxy, substituiertes oder unsubstituiertes C₁-C₆-Alkoxyhalogen oder -OH;
(c) unabhängig Wasserstoff, -CH₃, -CH₂CH₃, -OH, -OCH₃ oder -CH(CH₃)₂; oder
(d) Wasserstoff.

8. Verbindung nach einem der Ansprüche 1-7, wobei R⁶ wie folgt ist:
(a) unabhängig Wasserstoff, Halogen, Cyano, Hydroxyl, substituiertes oder unsubstituiertes Alkyl, substituiertes oder unsubstituiertes Alkenyl, -OR^{D1}, - OC(=O)R^{D1}, -NH₂ oder -N(R^{D1})₂, wobei jedes Vorkommen von R^{D1} unabhängig Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
(b) unabhängig Wasserstoff, substituiertes oder unsubstituiertes C₁-C₆-Alkyl, substituiertes oder unsubstituiertes C₁-C₆-Halogenalkyl, substituiertes oder unsubstituiertes C₁-C₆-Alkoxy, substituiertes oder unsubstituiertes C₁-C₆-Alkoxyhalogen oder -OH;
(c) unabhängig Wasserstoff, -CH₃, -CH₂CH₃, -OH, -OCH₃ oder -CH(CH₃)₂; oder
(d) Wasserstoff.

9. Verbindung nach einem der Ansprüche 1-8, wobei R¹⁶ wie folgt ist:
(a) unabhängig Wasserstoff, Halogen, Cyano, Hydroxyl, substituiertes oder unsubstituiertes Alkyl, substituiertes oder unsubstituiertes Alkenyl, -OR^{D1}, - OC(=O)R^{D1}, -NH2 oder -N(R^{D1})₂, wobei jedes Vorkommen von R^{D1} unabhängig Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
(b) unabhängig Wasserstoff, substituiertes oder unsubstituiertes C₁-C₆-Alkyl, substituiertes oder unsubstituiertes C₁-C₆-Halogenalkyl, substituiertes oder unsubstituiertes C₁-C₆-Alkoxy, substituiertes oder unsubstituiertes C₁-C₆-Alkoxyhalogen oder -OH;
(c) unabhängig Wasserstoff, -CH₃, -CH₂CH₃, -OH, -OCH₃ oder -CH(CH₃)₂; oder
(d) Wasserstoff.

10. Verbindung nach einem der Ansprüche 1-9, wobei R¹⁷ wie folgt ist:
(a) unabhängig Wasserstoff, Halogen, Cyano, Hydroxyl, substituiertes oder unsubstituiertes Alkyl, substituiertes oder unsubstituiertes Alkenyl, -OR^{D1}, - OC(=O)R^{D1}, -NH₂ oder -N(R^{D1})₂, wobei jedes Vorkommen von R^{D1} unabhängig Wasserstoff, substituiertes oder unsubstituiertes Alkyl, substituiertes oder unsubstituiertes Carbocyclyl, substituiertes oder unsubstituiertes Heterocyclyl, substituiertes oder unsubstituiertes Aryl oder substituiertes oder unsubstituiertes Heteroaryl ist;
(b) unabhängig Wasserstoff, substituiertes oder unsubstituiertes C₁-C₆-Alkyl, substituiertes oder unsubstituiertes C₁-C₆-Halogenalkyl, substituiertes oder unsubstituiertes C₁-C₆-Alkoxy, substituiertes oder unsubstituiertes C₁-C₆-Alkoxyhalogen oder -OH;
(c) unabhängig Wasserstoff, -CH₃, -CH₂CH₃, -OH, -OCH₃ oder -CH(CH₃)₂; oder
(d) Wasserstoff.

11. Verbindung nach einem der Ansprüche 1-10, wobei R¹⁹ wie folgt ist:
(a) unabhängig Wasserstoff oder substituiertes oder unsubstituiertes C₁-C₆-Alkyl;
(b) unabhängig Wasserstoff oder substituiertes oder unsubstituiertes C₁-C₄-Alkyl;
(c) unabhängig Wasserstoff, -CH₃, -CH₂CH₃ oder - CH₂OCH(CH₃)₂;
(d) Wasserstoff; oder
(e) -CH₂OCH(CH₃)₂.

12. Verbindung nach einem der Ansprüche 1-11, wobei R³ wie folgt ist:
(a) substituiertes oder unsubstituiertes Alkyl, substituiertes oder unsubstituiertes Alkenyl oder substituiertes oder unsubstituiertes Alkinyl, substituiertes oder unsubstituiertes Carbocyclyl oder substituiertes oder unsubstituiertes Heterocyclyl;
(b) substituiertes oder unsubstituiertes Alkyl; oder
(c) substituiertes oder unsubstituiertes C₁-C₆-Alkyl.

13. Verbindung nach einem der Ansprüche 1-12,
wobei die Verbindung der Formel (I) eine Verbindung der Formel (I-a) ist: 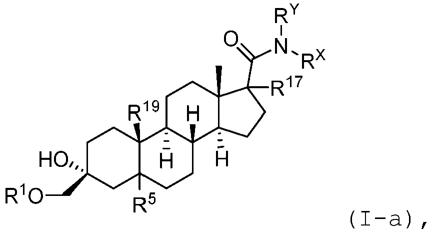
wobei R¹ substituiertes oder unsubstituiertes Alkyl ist.

14. Verbindung nach Anspruch 13, wobei R¹
(a) -CH₂CH₃ oder -CH₃ ist;
(b) -CH₂CH₃ ist; oder
(c) -CH₃ ist.

15. Verbindung nach einem der Ansprüche 1-14, wobei die Verbindung der Formel (I) eine Verbindung der Formel (I-b1) oder Formel (I-b2) ist: 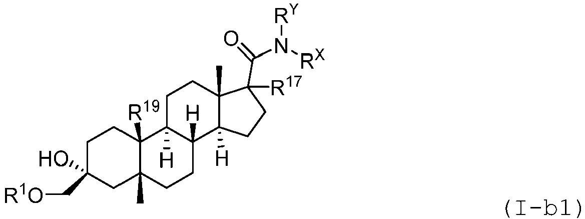 oder 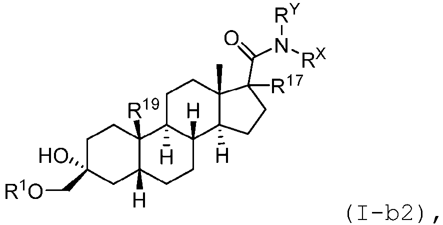 oder
wobei die Verbindung der Formel (I) eine Verbindung der Formel (I-c1) oder Formel (I-c2) ist: 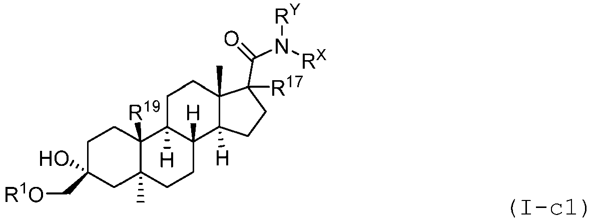 oder 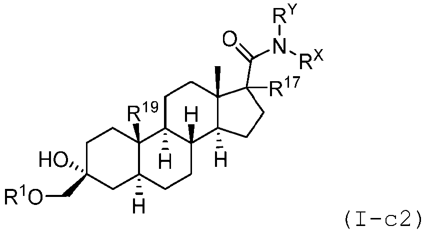 oder
wobei die Verbindung der Formel (I) eine Verbindung der Formel (I-d1) oder der Formel (I-d2) ist: 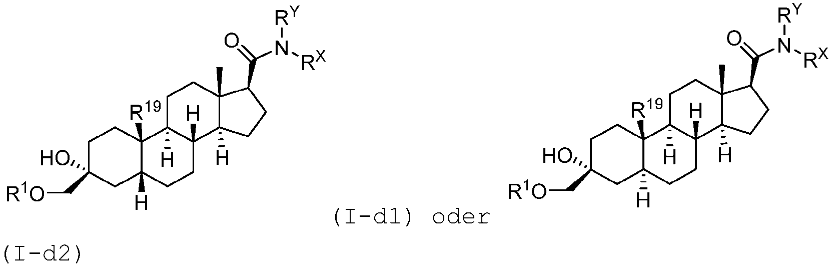 oder
wobei die Verbindung der Formel (I) eine Verbindung der Formel (I-d1) ist: 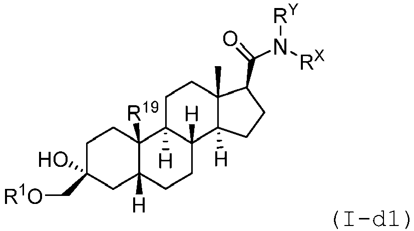 oder
wobei die Verbindung der Formel (I) eine Verbindung der Formel (I-d2) ist: 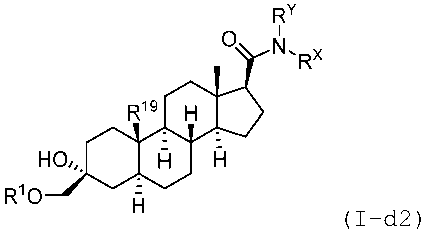 oder
wobei die Verbindung der Formel (I) eine Verbindung der Formel (I-e) ist: 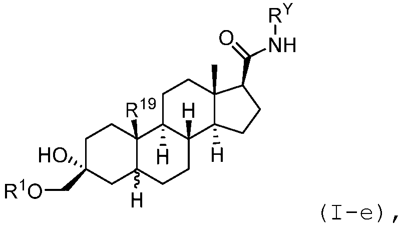
wobei R^{Y} Heteroaryl ist, und R¹ -CH₂CH₃ oder -CH₃ ist, oder
wobei die Verbindung der Formel (I) eine Verbindung der Formel (I-f) ist: 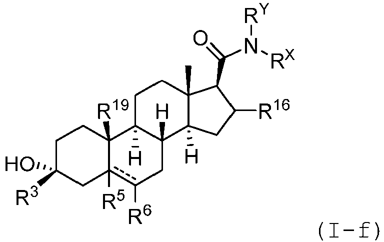 oder
wobei die Verbindung der Formel (I) eine Verbindung der Formel (I-g) ist: 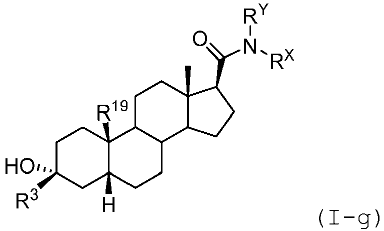
oder ein pharmazeutisch unbedenkliches Salz davon.

16. Verbindung nach einem der Ansprüche 1-15, wobei R^{X} wie folgt ist:
(a) unabhängig Wasserstoff oder substituiertes oder unsubstituiertes C₁₋₆-Alkyl;
(b) unabhängig Wasserstoff oder substituiertes oder unsubstituiertes C₁₋₄-Alkyl;
(c) unabhängig Wasserstoff, -CH₃ oder -CH₂CH₃; oder
(d) Wasserstoff.

17. Verbindung nach einem der Ansprüche 1-16, wobei R^{Y}
(a) ausgewählt ist aus: 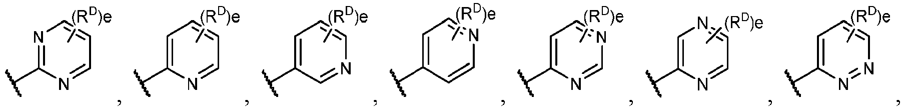 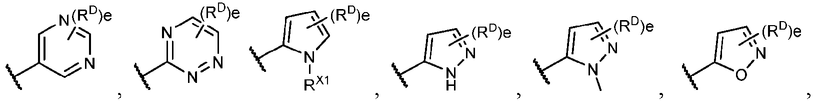 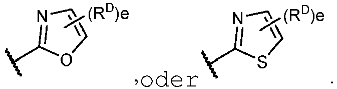
wobei jedes Vorkommen von R^{D} unabhängig Wasserstoff, Halogen, -NO₂, -CN, -OR^{GA}, -N(R^{GA})2, -C(=O)R^{GA}, -C(=O)OR^{GA}, -C(=O)N(R^{GA})₂, -OC(=O) R^{GA}, -OC(=O) OR^{GA}, -N(R^{GA})C (=O) R^{GA}, - OC (=O) N(R^{GA})₂, -N(R^{GA})C(=O)OR^{GA}, -S(=O)₂R^{GA}, -S (=O)₂OR^{GA}, - OS (=O)₂R^{GA}, -S (=O)₂N(R^{GA})₂ oder -N(R^{GA})S (=O)₂R^{GA}, substituiertes oder unsubstituiertes C₁₋₆-Alkyl, substituiertes oder unsubstituiertes C₂₋₆-Alkenyl, substituiertes oder unsubstituiertes C₂₋₆-Alkinyl, substituiertes oder unsubstituiertes C₃₋₄-Carbocyclyl, substituiertes oder unsubstituiertes 3- bis 6-gliedriges Heterocyclyl, substituiertes oder unsubstituiertes C₅₋₁₀-Aryl, substituiertes oder unsubstituiertes 5- bis 10-gliedriges Heteroaryl ist, oder gegebenenfalls zwei R^{GA} zusammen mit den dazwischenliegenden Atomen einen substituierten oder unsubstituierten 3- bis 4-gliedrigen carbocyclischen oder heterocyclischen Ring bilden;
wobei jedes Vorkommen von R^{GA} unabhängig Wasserstoff, substituiertes oder unsubstituiertes C₁₋₆-Alkyl, substituiertes oder unsubstituiertes C₂₋₆-Alkenyl, substituiertes oder unsubstituiertes C₂₋₆-Alkinyl, substituiertes oder unsubstituiertes C₃₋₆-Carbocyclyl, substituiertes oder unsubstituiertes 3- bis 6-gliedriges Heterocyclyl, substituiertes oder unsubstituiertes Aryl, substituiertes oder unsubstituiertes Heteroaryl, eine Sauerstoff-Schutzgruppe, wenn es an Sauerstoff gebunden ist, eine Stickstoff-Schutzgruppe ist, wenn es an Stickstoff gebunden ist, oder zwei R^{GA}-Gruppen zusammen mit den dazwischenliegenden Atomen einen substituierten oder unsubstituierten carbocyclischen oder heterocyclischen Ring bilden;
wobei jedes Vorkommen von R^{X1} Wasserstoff oder substituiertes oder unsubstituiertes Alkyl, substituiertes oder unsubstituiertes Cycloalkyl, substituiertes oder unsubstituiertes Aryl oder substituiertes oder unsubstituiertes Carbocyclyl ist, und
e 0, 1, 2, 3, 4 oder 5 ist;
(b) ausgewählt ist aus: 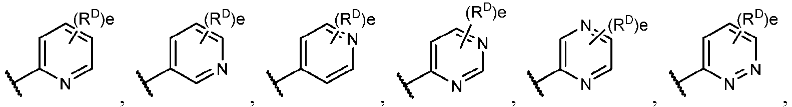 oder 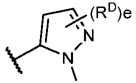
wobei jedes Vorkommen von R^{D} unabhängig Wasserstoff, Halogen, substituiertes oder unsubstituiertes C₁₋₆-Alkyl, -CN, substituiertes oder unsubstituiertes 5-6-gliedriges Heteroaryl, substituiertes oder unsubstituiertes 5-gliedriges Heterocyclyl ist,
e 0, 1, 2, 3 oder 4 ist;
(c) ausgewählt ist aus: 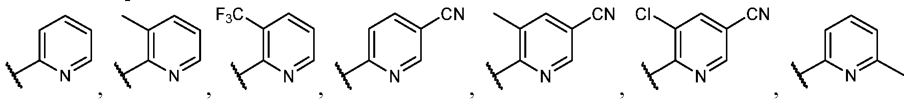 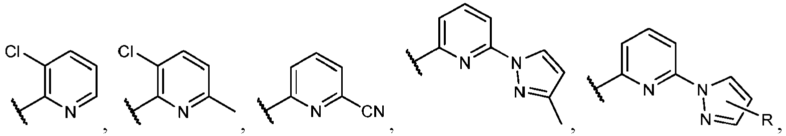 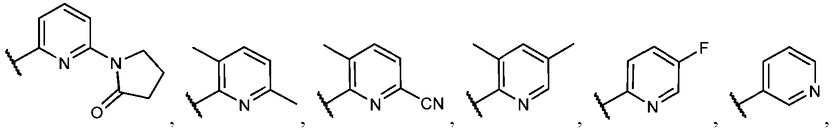 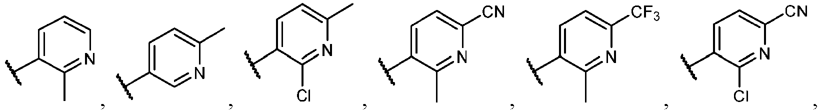 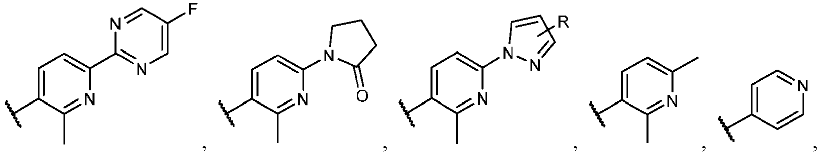 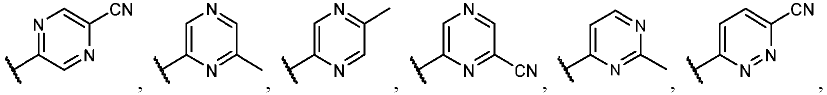 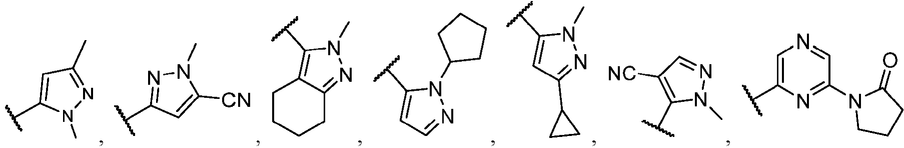 oder 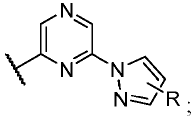 wobei R -CH₃, -CH₂CH₃, -i-Pr, Cyclopropyl oder -CN ist;
(d) ausgewählt ist aus: 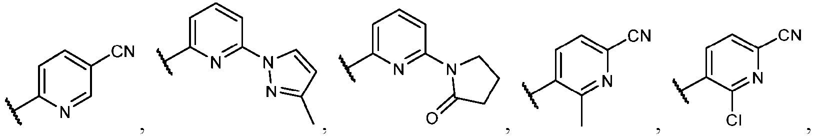 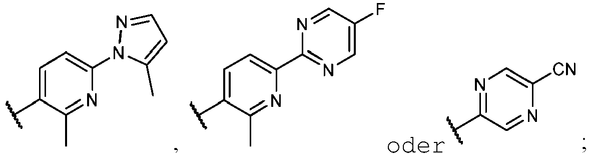
(e) 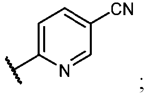 ist, oder
(f) 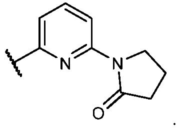 ist.

18. Verbindung nach einem der Ansprüche 1-17, wobei die Verbindung ausgewählt ist aus:
| Beispiel | STRUKTUR |
|---|---|
| 1 | 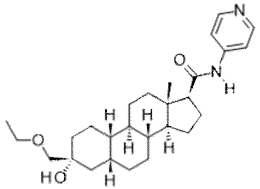 |
| 2 | 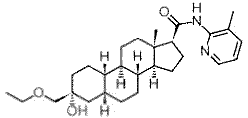 |
| 3 | 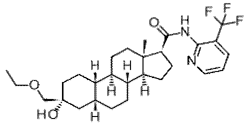 |
| 4 | 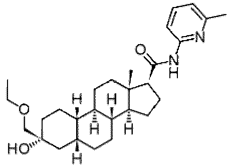 |
| 5 | 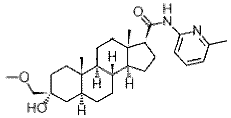 |
| 6 | 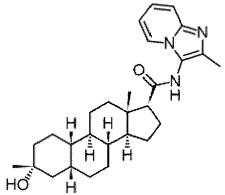 |
| 7 | 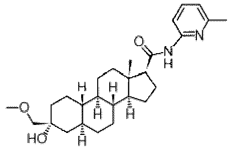 |
| 8 | 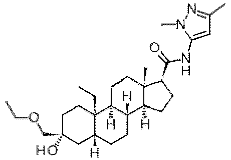 |
| 9 | 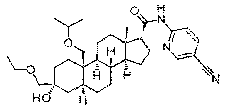 |
| 10 | 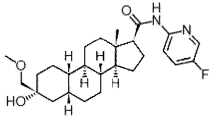 |
| 11 | 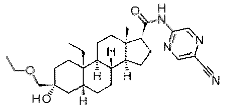 |
| 12 | 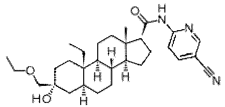 |
| 13 | 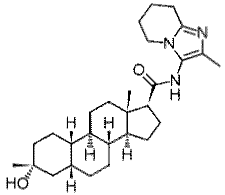 |
| 14 | 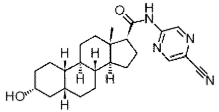 |
| 15 | 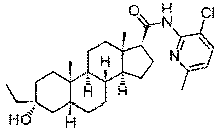 |
| 16 | 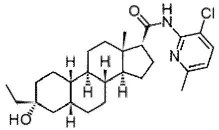 |
| 20 | 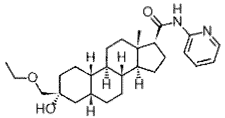 |
| 21 | 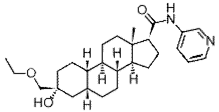 |
| 22 | 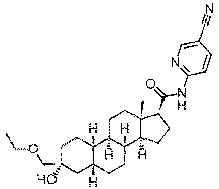 |
| 23 | 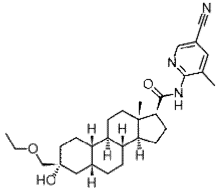 |
| 24 | 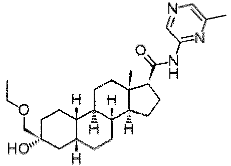 |
| 25 | 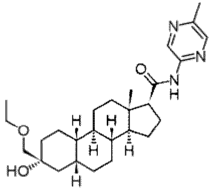 |
| 26 | 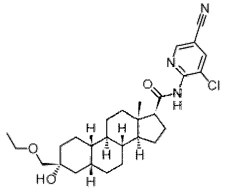 |
| 27 | 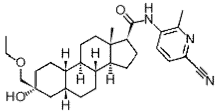 |
| 28 | 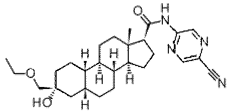 |
| 29 | 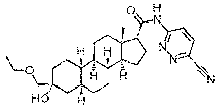 |
| 30 | 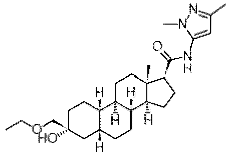 |
| 31 | 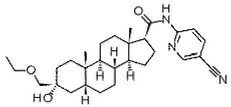 |
| 32 | 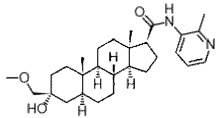 |
| 33 | 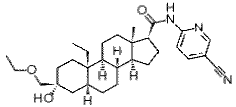 |
| 35 | 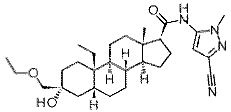 |
| 36 | 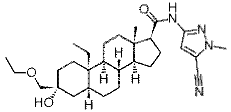 |
| 37 | 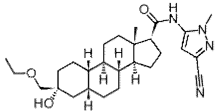 |
| 38 | 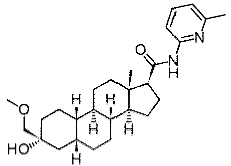 |
| 39 | 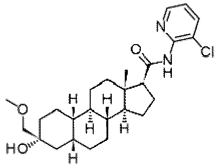 |
| 40 | 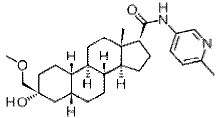 |
| 41 | 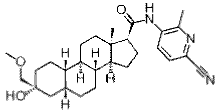 |
| 42 | 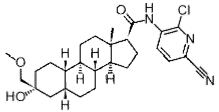 |
| 43 | 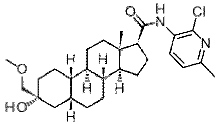 |
| 44 | 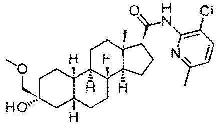 |
| 45 | 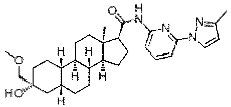 |
| 46 | 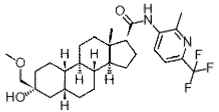 |
| 47 | 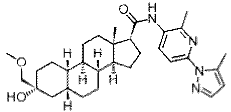 |
| 48 | 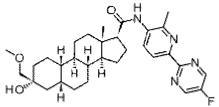 |
| 49 | 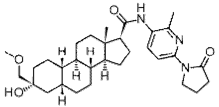 |
| 50 | 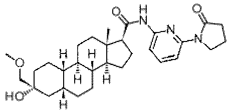 |
| 51 | 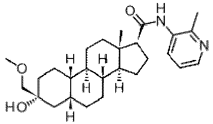 |
| 52 | 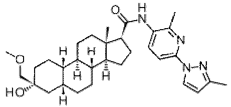 |
| 53 | 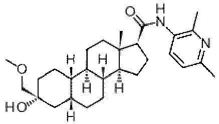 |
| 54 | 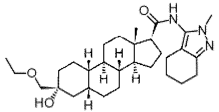 |
| 55 | 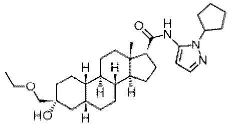 |
| 56 | 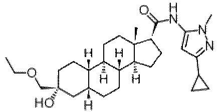 |
| 57 | 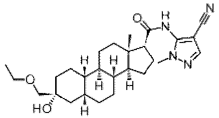 |
| 58 | 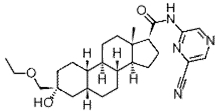 |
| 59 | 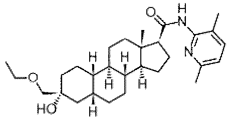 |
| 60 | 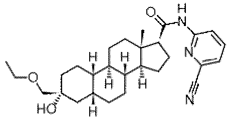 |
| 61 | 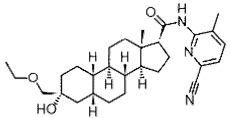 |
| 62 | 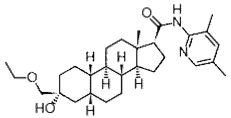 |
| 63 | 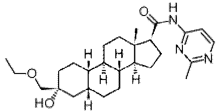 |
| 64 | 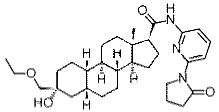 |
| 65 | 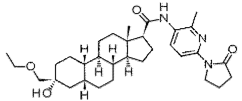 |
| 66 | 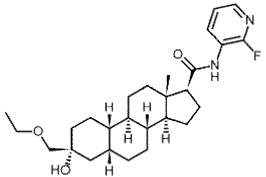 |
| 67 | 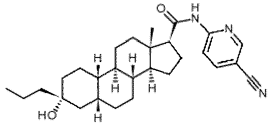 |
| 68 | 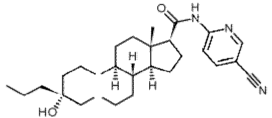 |

19. Verbindung nach einem der Ansprüche 1-18 oder ein pharmazeutisch unbedenkliches Salz davon zur Verwendung in einem Verfahren bei der Behandlung einer ZNS-bedingten Störung bei einem Individuum, bei dem diesbezüglicher Bedarf besteht.

20. Verbindung oder pharmazeutisch unbedenkliches Salz davon zur Verwendung nach Anspruch 19, wobei
(a) die ZNS-bedingte Störung eine Schlafstörung, eine affektive Störung, eine Störung des Schizophrenie-Spektrums, eine konvulsive Störung, eine Gedächtnis- und/oder Kognitionsstörung, eine Bewegungsstörung, eine Persönlichkeitsstörung, eine Störung des Autismus-Spektrums, Schmerzen, traumatische Hirnverletzung, eine Gefäßerkrankung, eine Substanzmissbrauchsstörung und/oder ein Entzugssyndrom, Tinnitus oder Status epilepticus ist;
(b) die ZNS-bedingte Störung Depression ist;
(c) die ZNS-bedingte Störung postpartale Depression;
(d) die ZNS-bedingte Störung majore depressive Störung ist;
(e) die ZNS-bedingte Störung majore depressive Störung ist, und die majore depressive Störung eine mittlere majore depressive Störung ist; oder
(f) die ZNS-bedingte Störung majore depressive Störung ist, und die majore depressive Störung eine schwere majore depressive Störung ist.

## Revendications

1. Composé de formule (I) : 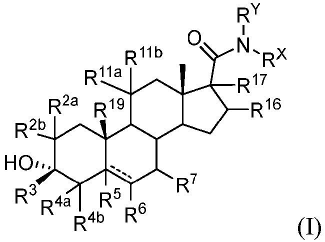 ou sel pharmaceutiquement acceptable de celui-ci ;
- - - - - - représentant une simple ou une double liaison comme la valence le permet ;
chacun parmi R^{2a}, R^{2b}, R^{4a}, R^{4b}, R⁶, R⁷, R^{11a}, et R^{11b}, étant indépendamment hydrogène, halogène, cyano, hydroxyle, alkyle substitué ou non substitué, alcényle substitué ou non substitué, hétérocyclyle substitué ou non substitué, alcynyle substitué ou non substitué, -OR^{D1}, -OC(=O)R^{D1}, - NH₂, -N(R^{D1})₂, ou -NR^{D1}C(=O)R^{D1}, chaque instance de R^{D1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué, un groupe protecteur d'oxygène lorsqu'il est fixé à un atome d'oxygène, un groupe protecteur d'azote lorsqu'il est fixé à un atome d'azote, ou deux groupes R^{D1} étant joints pour former un cycle hétérocyclique substitué ou non substitué ; ou l'un quelconque parmi R^{2a} et R^{2b}, R^{4a} et R^{4b}, R^{7a} et R^{7b}, R^{11a} et R^{11b} étant joints pour former un groupe oxo (=O) ;
chacun parmi R¹⁶ et R¹⁷ étant chacun indépendamment hydrogène, halogène, cyano, hydroxyle, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, hétéroaryle substitué ou non substitué, -OR^{A1}, -SR^{A1}, - N (R^{A1})₂, -N(R^{A1}),-CN(R^{A1})₂, -C (O)R^{A1}, -OC(=O)R^{A1}, - OC (=O) OR^{A1}, -OC (=O) SR^{A1}, -OC (=O) N (R^{A1})₂, -SC (=O) R^{A2}, - SC (=O) OR^{A1}, -SC (=O) SR^{A1}, -SC(=O)N(R^{A1})₂, -NHC(=O)R^{A1}, - NHC (=O) OR^{A1}, -NHC (=O) SR^{A1}, -NHC (=O) N (R^{A1})₂, -OS (=O)₂R^{A2}, - OS (=O)₂OR^{A1}, -S-S (=O)₂R^{A2}, -S-S(=O)₂OR^{A1}, -S (=O) R^{A2}, -SO₂R^{A2}, ou -S (=O)₂OR^{A1}, chaque instance de R^{A1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, hétéroaryle substitué ou non substitué, un groupe protecteur d'oxygène lorsqu'il est fixé à un atome d'oxygène, un groupe protecteur de soufre lorsqu'il est fixé à un atome de soufre, un groupe protecteur d'azote lorsqu'il est fixé à un atome d'azote, -SO₂R^{A2}, - C(O)R^{A2}, ou deux groupes R^{A1} étant joints pour former un cycle hétérocyclique ou hétéroaryle substitué ou non substitué ; et R^{A2} étant alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué ;
R³ étant hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué ;
R⁵ étant hydrogène ou méthyle lorsque - - - - - - est une simple liaison ; et lorsque - - - - - - est une double liaison, R⁵ est absent ;
R¹⁹ étant hydrogène ou alkyle substitué ou non substitué ; R^{X} étant indépendamment hydrogène, ou alkyle substitué ou non substitué
R^{Y} étant indépendamment hétéroaryle substitué ou non substitué.

2. Composé selon la revendication 1, le composé de formule (I) étant un composé de formule (I-X) 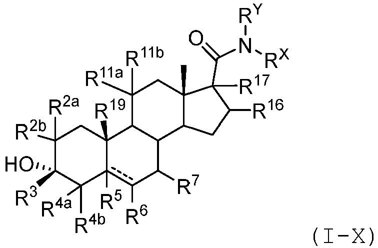 ou
un composé de formule (I-1) 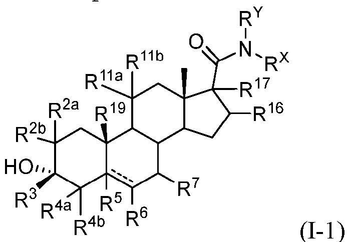
- - - - - - représentant une simple ou une double liaison comme la valence le permet ;
chacun parmi R^{2a}, R^{2b}, R^{4a}, R^{4b}, R⁶, R⁷, R^{11a}, et R^{11b}, étant indépendamment hydrogène, halogène, cyano, hydroxyle, alkyle substitué ou non substitué, alcényle substitué ou non substitué, hétérocyclyle substitué ou non substitué, alcynyle substitué ou non substitué, -OR^{D1}, -OC(=O)R^{D1}, - NH₂, -N(R^{D1})₂, ou -NR^{D1}C(=O)R^{D1}, chaque instance de R^{D1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué, un groupe protecteur d'oxygène lorsqu'il est fixé à un atome d'oxygène, un groupe protecteur d'azote lorsqu'il est fixé à un atome d'azote, ou deux groupes R^{D1} étant joints pour former un cycle hétérocyclique substitué ou non substitué ; ou l'un quelconque parmi R^{2a} et R^{2b}, R^{4a} et R^{4b}, R^{7a} et R^{7b}, R^{11a} et R^{11b} étant joints pour former un groupe oxo (=O) ;
chacun parmi R¹⁶ et R¹⁷ étant chacun indépendamment hydrogène, halogène, cyano, hydroxyle, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, hétéroaryle substitué ou non substitué, -OR^{A1}, -SR^{A1}, - N(R^{A1})₂, -N(R^{A1}), -CN(R^{A1})₂, -C(O)R^{A1}, -OC(=O)R^{A1}, - OC(=O)OR^{A1}, -OC(=O)SR^{A1}, -OC (=O)N(R^{A1})₂, -SC (=O) R^{A2}, - SC (=O)OR^{A1}, -SC (=O)SR^{A1}, - SC (=O) N (R^{A1})₂, -NHC(=O)R^{A1}, - NHC (=O) OR^{A1}, -NHC (=O) SR^{A1}, -NHC (=O)N(R^{A1})₂, -OS (=O)₂R^{A2}, - OS (=O)₂OR^{A1}, -S-S (=O) ₂R^{A2}, -S-S (=O)₂OR^{A1}, -S(=O)R^{A2}, -SO₂R^{A2}, ou -S(=O)₂OR^{A1}, chaque instance de R^{A1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, hétéroaryle substitué ou non substitué, un groupe protecteur d'oxygène lorsqu'il est fixé à un atome d'oxygène, un groupe protecteur de soufre lorsqu'il est fixé à un atome de soufre, un groupe protecteur d'azote lorsqu'il est fixé à un atome d'azote, -SO₂R^{A2}, - C(O)R^{A2}, ou deux groupes R^{A1} étant joints pour former un cycle hétérocyclique ou hétéroaryle substitué ou non substitué ; et R^{A2} étant alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué ;
R³ étant hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué ;
R⁵ étant hydrogène ou méthyle lorsque - est une simple liaison ; et lorsque - - - - - - est une double liaison, R⁵ est absent ;
R¹⁹ étant hydrogène ou méthyle ;
R^{X} étant indépendamment hydrogène, ou alkyle substitué ou non substitué
R^{Y} étant indépendamment hétéroaryle substitué ou non substitué,
ou sel pharmaceutiquement acceptable de celui-ci.

3. Composé selon la revendication 1, le composé de formule (I) étant : 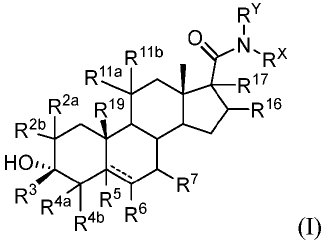
- - - - - - représentant une simple ou une double liaison comme la valence le permet ;
chacun parmi R^{2a}, R^{2b}, R^{4a}, R^{4b}, R⁶, R⁷, R^{11a}, et R^{11b}, étant indépendamment hydrogène, halogène, cyano, hydroxyle, alkyle substitué ou non substitué, alcényle substitué ou non substitué, hétérocyclyle substitué ou non substitué, alcynyle substitué ou non substitué, -OR^{D1}, -OC(=O)R^{D1}, - NH₂, -N(R^{D1})₂, ou -NR^{D1}C(=O)R^{D1}, chaque instance de R^{D1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué, un groupe protecteur d'oxygène lorsqu'il est fixé à un atome d'oxygène, un groupe protecteur d'azote lorsqu'il est fixé à un atome d'azote, ou deux groupes R^{D1} étant joints pour former un cycle hétérocyclique substitué ou non substitué ; ou l'un quelconque parmi R^{2a} et R^{2b}, R^{4a} et R^{4b}, R^{7a} et R^{7b}, R^{11a} et R^{11b} étant joints pour former un groupe oxo (=O) ;
chacun parmi R¹⁶ et R¹⁷ étant chacun indépendamment hydrogène, halogène, cyano, hydroxyle, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, hétéroaryle substitué ou non substitué, -OR^{A1}, -SR^{A1}, - N(R^{A1})₂, -N(R^{A1}) ,-CN(R^{A1})₂, -C(O)R^{A1}, -OC(=O)R^{A1}, - OC (=O) OR^{A1}, -OC(=O)SR^{A1}, -OC(=O)N(R^{A1})₂, -SC(=O)R^{A2}, - SC(=O)OR^{A1}, -SC(=O)SR^{A1}, -SC(=O)N(R^{A1})₂, -NHC(=O) R^{A1}, - NHC(=O)OR^{A1}, -NHC(=O)SR^{A1}, -NHC(=O)N(R^{A1})₂, -OS(=O)₂R^{A2}, - OS(=O)₂OR^{A1}, -S-S(=O)₂R^{A2}, -S-S(=O)₂OR^{A1}, -S(=O)R^{A2}, -SO₂R^{A2}, ou -S(=O)₂OR^{A1}, chaque instance de R^{A1} étant indépendamment hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, hétéroaryle substitué ou non substitué, un groupe protecteur d'oxygène lorsqu'il est fixé à un atome d'oxygène, un groupe protecteur de soufre lorsqu'il est fixé à un atome de soufre, un groupe protecteur d'azote lorsqu'il est fixé à un atome d'azote, -SO₂R^{A2}, - C(O)R^{A2}, ou deux groupes R^{A1} étant joints pour former un cycle hétérocyclique ou hétéroaryle substitué ou non substitué ; et R^{A2} étant alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué ;
R³ étant hydrogène, alkyle substitué ou non substitué, alcényle substitué ou non substitué, alcynyle substitué ou non substitué, carbocyclyle substitué ou non substitué, hétérocyclyle substitué ou non substitué, aryle substitué ou non substitué, ou hétéroaryle substitué ou non substitué ;
R⁵ étant hydrogène ou méthyle lorsque - - - - - - est une simple liaison ; et lorsque - - - - - - est une double liaison, R⁵ est absent ;
R¹⁹ étant hydrogène ou alkyle substitué ou non substitué ; R^{X} étant indépendamment hydrogène, ou alkyle substitué ou non substitué
R^{Y} étant indépendamment hétéroaryle substitué ou non substitué.

4. Composé selon l'une quelconque des revendications 1-3, R^{2a} et R^{2b}
(a) étant chacun indépendamment hydrogène, halogène, cyano, hydroxyle, alkyle substitué ou non substitué, alcényle substitué ou non substitué, -OR^{D1}, -OC(=O)R^{D1}, - NH₂, ou -N(R^{D1})₂, chaque instance de R^{D1} étant indépendamment hydrogène, alkyle substitué ou non substitué, carbocyclyle substitué ou non substitué, hétérocyclyle substitué ou non substitué, aryle substitué ou non substitué, hétéroaryle substitué ou non substitué ;
(b) étant chacun indépendamment hydrogène, C₁-C₆ alkyle substitué ou non substitué, C₁-C₆ halogénoalkyle substitué ou non substitué, C₁-C₆ alcoxy substitué ou non substitué, C₁-C₆ alcoxyhalogéno substitué ou non substitué, ou -OH ;
(c) étant chacun indépendamment hydrogène, -CH₃, - CH₂CH₃, -OH, -OCH₃, ou -CH(CH₃)₂ ;
(d) étant chacun indépendamment hydrogène ; ou
(e) étant tous deux hydrogène.

5. Composé selon l'une quelconque des revendications 1-4, R^{4a} et R^{4b}
(a) étant chacun indépendamment hydrogène, halogène, cyano, hydroxyle, alkyle substitué ou non substitué, alcényle substitué ou non substitué, -OR^{D1}, -OC(=O)R^{D1}, - NH₂, ou -N(R^{D1})₂, chaque instance de R^{D1} étant indépendamment hydrogène, alkyle substitué ou non substitué, carbocyclyle substitué ou non substitué, hétérocyclyle substitué ou non substitué, aryle substitué ou non substitué, hétéroaryle substitué ou non substitué ;
(b) étant chacun indépendamment hydrogène, C₁-C₆ alkyle substitué ou non substitué, C₁-C₆ halogénoalkyle substitué ou non substitué, C₁-C₆ alcoxy substitué ou non substitué, C₁-C₆ alcoxyhalogéno substitué ou non substitué, ou -OH ;
(c) étant chacun indépendamment hydrogène, -CH₃, - CH₂CH₃, -OH, -OCH₃, ou -CH(CH₃)₂ ;
(d) étant chacun indépendamment hydrogène ; ou
(e) étant tous deux hydrogène.

6. Composé selon l'une quelconque des revendications 1-5, R^{11a} et R^{11b}
(a) étant chacun indépendamment hydrogène, halogène, cyano, alkyle substitué ou non substitué, alcényle substitué ou non substitué, -OR^{D1}, -OC(=O)R^{D1}, -NH₂, ou - N(R^{D1})₂, chaque instance de R^{D1} étant indépendamment hydrogène, alkyle substitué ou non substitué, carbocyclyle substitué ou non substitué, hétérocyclyle substitué ou non substitué, aryle substitué ou non substitué, hétéroaryle substitué ou non substitué ;
(b) étant chacun indépendamment hydrogène, C₁-C₆ alkyle substitué ou non substitué, C₁-C₆ halogénoalkyle substitué ou non substitué, C₁-C₆ alcoxy substitué ou non substitué, C₁-C₆ alcoxyhalogéno substitué ou non substitué ;
(c) étant chacun indépendamment hydrogène, -CH₃, - CH₂CH₃, -OH, -OCH₃, ou -CH(CH₃)₂ ;
(d) étant chacun indépendamment hydrogène ; ou
(e) étant tous deux hydrogène.

7. Composé selon l'une quelconque des revendications 1-6, R⁷ étant
(a) indépendamment hydrogène, halogène, cyano, hydroxyle, alkyle substitué ou non substitué, alcényle substitué ou non substitué, -OR^{D1}, -OC(=O)R^{D1}, -NH₂, ou - N(R^{D1})₂, chaque instance de R^{D1} étant indépendamment hydrogène, alkyle substitué ou non substitué, carbocyclyle substitué ou non substitué, hétérocyclyle substitué ou non substitué, aryle substitué ou non substitué, hétéroaryle substitué ou non substitué ;
(b) indépendamment hydrogène, C₁-C₆ alkyle substitué ou non substitué, C₁-C₆ halogénoalkyle substitué ou non substitué, C₁-C₆ alcoxy substitué ou non substitué, C₁-C₆ alcoxyhalogéno substitué ou non substitué, ou -OH ;
(c) indépendamment hydrogène, -CH₃, -CH₂CH₃, -OH, -OCH₃, ou -CH(CH₃)₂ ; ou
(d) hydrogène.

8. Composé selon l'une quelconque des revendications 1-7, R⁶ étant
(a) indépendamment hydrogène, halogène, cyano, hydroxyle, alkyle substitué ou non substitué, alcényle substitué ou non substitué, -OR^{D1}, -OC(=O)R^{D1}, -NH₂, ou - N (R^{D1})₂, chaque instance de R^{D1} étant indépendamment hydrogène, alkyle substitué ou non substitué, carbocyclyle substitué ou non substitué, hétérocyclyle substitué ou non substitué, aryle substitué ou non substitué, hétéroaryle substitué ou non substitué ;
(b) indépendamment hydrogène, C₁-C₆ alkyle substitué ou non substitué, C₁-C₆ halogénoalkyle substitué ou non substitué, C₁-C₆ alcoxy substitué ou non substitué, C₁-C₆ alcoxyhalogéno substitué ou non substitué, ou -OH ;
(c) indépendamment hydrogène, -CH₃, -CH₂CH₃, -OH, -OCH₃, ou -CH(CH₃)₂ ; ou
(d) hydrogène.

9. Composé selon l'une quelconque des revendications 1-8, R¹⁶ étant
(a) indépendamment hydrogène, halogène, cyano, hydroxyle, alkyle substitué ou non substitué, alcényle substitué ou non substitué, -OR^{D1}, -OC(=O)R^{D1}, -NH₂, ou - N(R^{D1})₂, chaque instance de R^{D1} étant indépendamment hydrogène, alkyle substitué ou non substitué, carbocyclyle substitué ou non substitué, hétérocyclyle substitué ou non substitué, aryle substitué ou non substitué, hétéroaryle substitué ou non substitué ;
(b) indépendamment hydrogène, C₁-C₆ alkyle substitué ou non substitué, C₁-C₆ halogénoalkyle substitué ou non substitué, C₁-C₆ alcoxy substitué ou non substitué, C₁-C₆ alcoxyhalogéno substitué ou non substitué, ou -OH ;
(c) indépendamment hydrogène, -CH₃, -CH₂CH₃, -OH, -OCH₃, ou -CH(CH₃)₂ ; ou
(d) hydrogène.

10. Composé selon l'une quelconque des revendications 1-9, R¹⁷ étant
(a) indépendamment hydrogène, halogène, cyano, hydroxyle, alkyle substitué ou non substitué, alcényle substitué ou non substitué, -OR^{D1}, -OC(=O)R^{D1}, -NH₂, ou - N(R^{D1})₂, chaque instance de R^{D1} étant indépendamment hydrogène, alkyle substitué ou non substitué, carbocyclyle substitué ou non substitué, hétérocyclyle substitué ou non substitué, aryle substitué ou non substitué, hétéroaryle substitué ou non substitué ;
(b) indépendamment hydrogène, C₁-C₆ alkyle substitué ou non substitué, C₁-C₆ halogénoalkyle substitué ou non substitué, C₁-C₆ alcoxy substitué ou non substitué, C₁-C₆ alcoxyhalogéno substitué ou non substitué, ou -OH ;
(c) indépendamment hydrogène, -CH₃, -CH₂CH₃, -OH, -OCH₃, ou -CH(CH₃)₂ ; ou
(d) hydrogène.

11. Composé selon l'une quelconque des revendications 1-10, R¹⁹ étant
(a) indépendamment hydrogène, ou C₁-C₆ alkyle substitué ou non substitué ;
(b) indépendamment hydrogène, ou C₁-C₄ alkyle substitué ou non substitué ;
(c) indépendamment hydrogène, -CH₃, -CH₂CH₃, ou - CH₂OCH(CH₃)₂ ;
(d) hydrogène ; ou
(e) -CH₂OCH(CH₃)₂.

12. Composé selon l'une quelconque des revendications 1-11, R³ étant
(a) alkyle substitué ou non substitué, alcényle substitué ou non substitué, ou alcynyle substitué ou non substitué, carbocyclyle substitué ou non substitué, ou hétérocyclyle substitué ou non substitué ;
(b) alkyle substitué ou non substitué ; ou
(c) C₁-C₆ alkyle substitué ou non substitué.

13. Composé selon l'une quelconque des revendications 1-12,
le composé de formule (I) étant un composé de formule (I-a) : 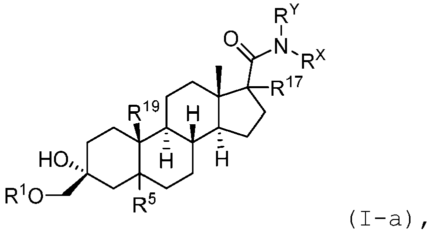
R¹ étant alkyle substitué ou non substitué.

14. Composé selon la revendication 13, R¹
(a) étant -CH₂CH₃ ou -CH₃ ;
(b) étant -CH₂CH₃ ; ou
(c) étant -CH₃.

15. Composé selon l'une quelconque des revendications 1-14, le composé de formule (I) étant un composé de formule (I-b1) ou formule (I-b2) : 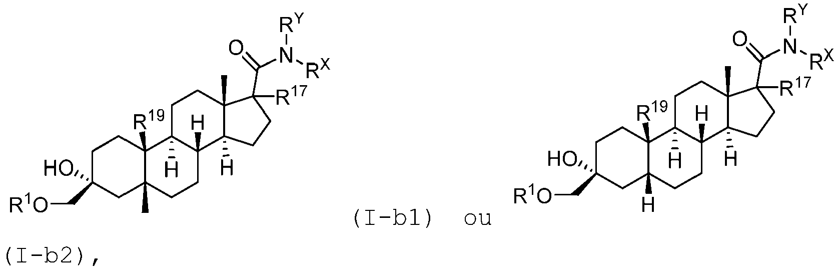 ou
le composé de formule (I) étant un composé de formule (I-c1) ou formule (I-c2) : 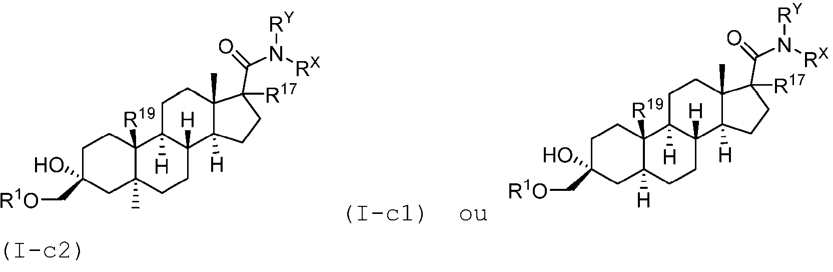 ou
le composé de formule (I) étant un composé de formule (I-d1) ou formule (I-d2) : 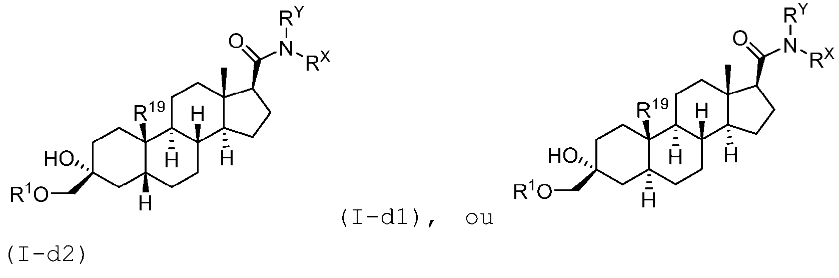 ou
le composé de formule (I) étant un composé de formule (I-d1) : 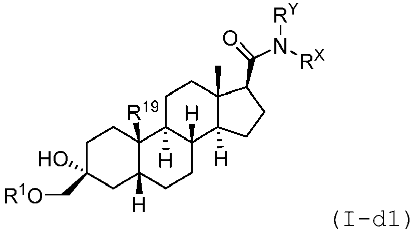 ou
le composé de formule (I) étant un composé de formule (I-d2) : 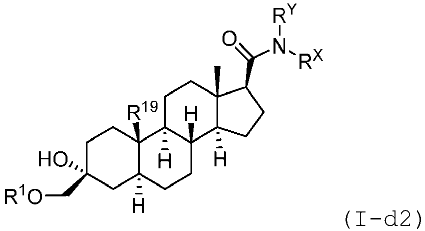 ou
le composé de formule (I), étant un composé de formule (I-e) : 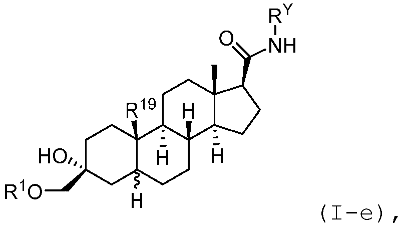
R^{Y} étant hétéroaryle, et R¹ étant -CH₂CH₃ ou -CH₃,
ou
le composé de formule (I) étant un composé de formule (If) : 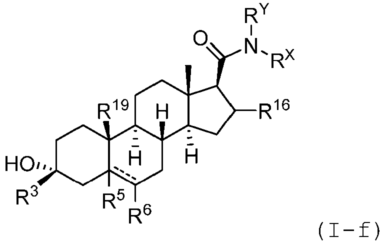 ou
le composé de formule (I) étant un composé de formule (I-g) : 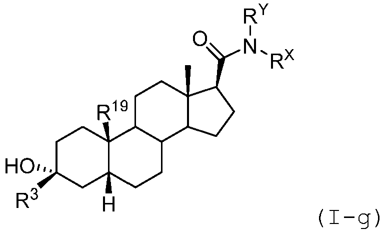
ou sel pharmaceutiquement acceptable de celui-ci.

16. Composé selon l'une quelconque des revendications 1-15, R^{X} étant
(a) indépendamment hydrogène, ou C₁₋₆ alkyle substitué ou non substitué ;
(b) indépendamment hydrogène, ou C₁₋₄ alkyle substitué ou non substitué ;
(c) indépendamment hydrogène,-CH₃, ou -CH₂CH₃ ; ou
(d) hydrogène.

17. Composé selon l'une quelconque des revendications 1-16, R^{Y}
(a) étant choisi parmi : 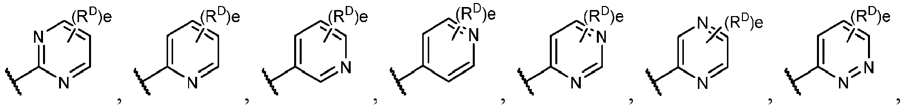 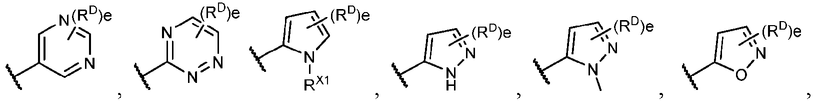 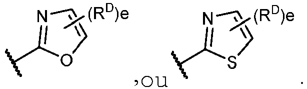
chaque instance de R^{D} étant, indépendamment hydrogène, halogène, -NO₂, -CN, -OR^{GA}, -N(R^{GA})₂, -C(=O)R^{GA}, -C (=O)OR^{GA}, -C(=O) N (R^{GA})₂, -OC(=O)R^{GA}, -OC(=O) OR^{GA}, -N(R^{GA}) C (=O)R^{GA}, - OC (=O) N (R^{GA}) ₂, -N(R^{GA}) C(=O)OR^{GA}, -S(=O) ₂R^{GA}, -S (=O) ₂OR^{GA}, - OS (=O) ₂R^{GA}, -S (=O) ₂N(R^{GA})₂, ou -N(R^{GA}) S (=O) ₂R^{GA}, C₁₋₆ alkyle substitué ou non substitué, C₂₋₆ alcényle substitué ou non substitué, C₂₋₆ alcynyle substitué ou non substitué, C₃₋₄ carbocyclyle substitué ou non substitué, hétérocyclyle à 3 à 6 chaînons substitué ou non substitué, C₅₋₁₀ aryle substitué ou non substitué, hétéroaryle à 5 à 10 chaînons substitué ou non substitué, ou éventuellement deux R^{GA} étant pris ensemble avec les atomes intervenant pour former un cycle carbocyclique ou hétérocyclique à 3 à 4 chaînons substitué ou non substitué ;
chaque instance de R^{GA} étant indépendamment hydrogène, C₁₋₆ alkyle substitué ou non substitué, C₂₋₆ alcényle substitué ou non substitué, C₂₋₆ alcynyle substitué ou non substitué, C₃₋₆ carbocyclyle substitué ou non substitué, hétérocyclyle à 3 à 6 chaînons substitué ou non substitué, aryle substitué ou non substitué, hétéroaryle substitué ou non substitué, un groupe protecteur d'oxygène lorsqu'il est fixé à un oxygène, un groupe protecteur d'azote lorsqu'il est fixé à un azote, ou deux groupes R^{GA} étant pris ensemble avec les atomes intervenant pour former un cycle carbocyclique ou hétérocyclique substitué ou non substitué ;
chaque instance de R^{X1} étant hydrogène ou alkyle substitué ou non substitué, cycloalkyle substitué ou non substitué, aryle substitué ou non substitué, ou carbocyclyle substitué ou non substitué et
e étant 0, 1, 2, 3, 4, ou 5 ;
(b) étant choisi parmi : 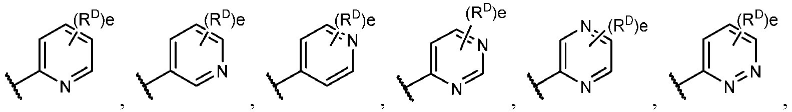 ou 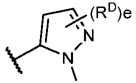 chaque instance de R^{D} étant, indépendamment hydrogène, halogène, C₁₋₆ alkyle substitué ou non substitué, -CN, hétéroaryle à 5 à 6 chaînons substitué ou non substitué, hétérocyclyle à 5 chaînons substitué ou non substitué, e étant 0, 1, 2, 3, ou 4 ;
(c) étant choisi parmi : 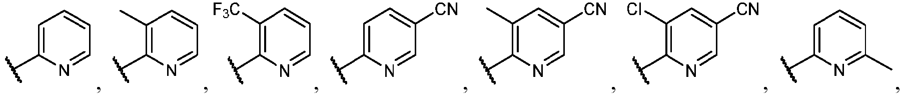 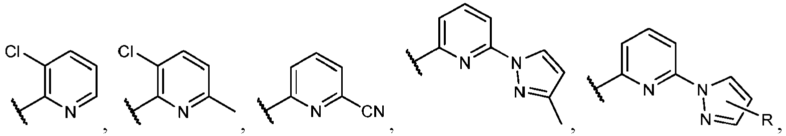 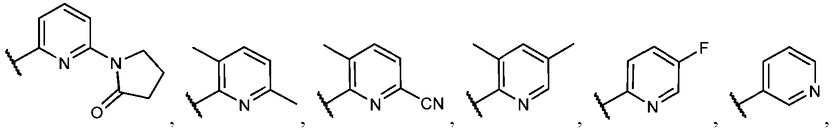 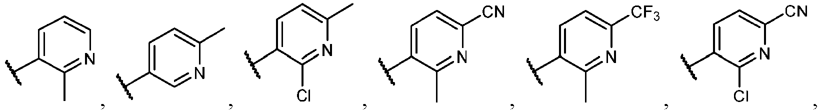 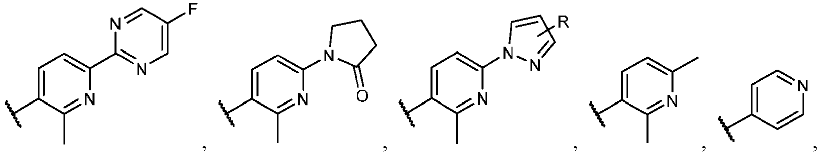 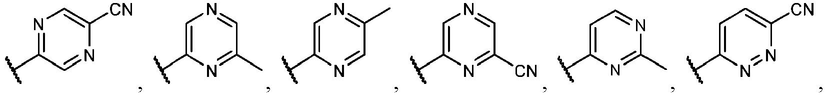 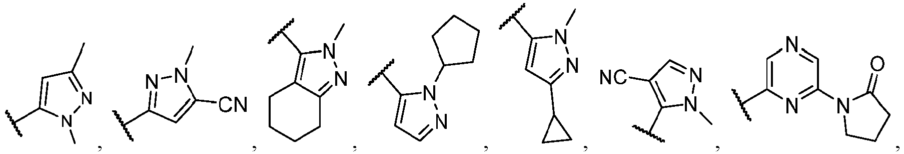 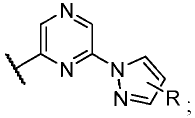 R étant -CH₃, -CH₂CH₃, -i-Pr, cyclopropyle ou -CN ;
(d) étant choisi parmi : 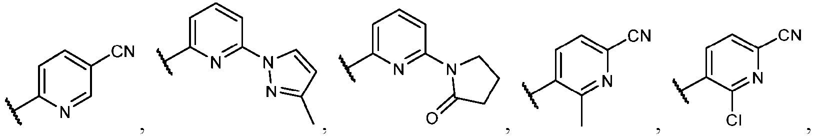 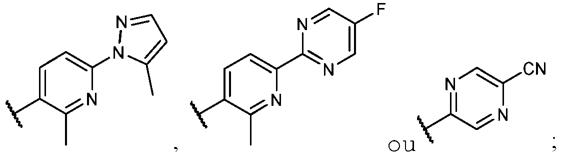
(e) étant 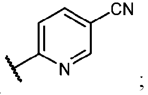 ou
(f) étant

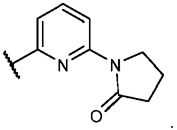

18. Composé selon l'une quelconque des revendications 1-17, le composé étant choisi parmi :
| Exemple | STRUCTURE |
|---|---|
| 1 | 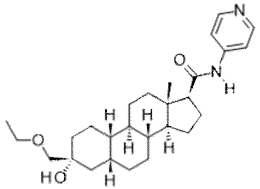 |
| 2 | 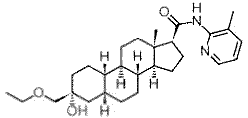 |
| 3 | 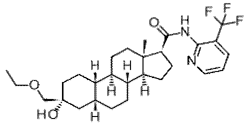 |
| 4 | 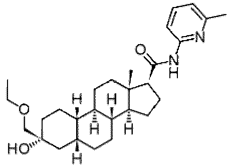 |
| 5 | 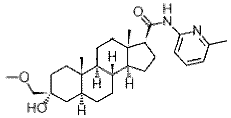 |
| 6 | 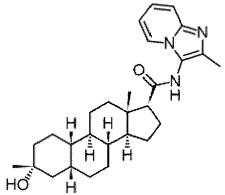 |
| 7 | 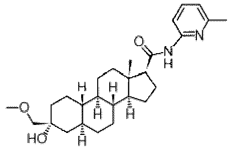 |
| 8 | 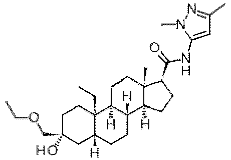 |
| 9 | 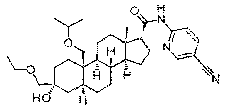 |
| 10 | 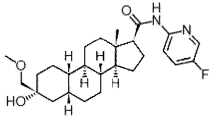 |
| 11 | 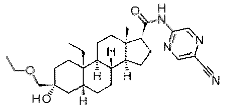 |
| 12 | 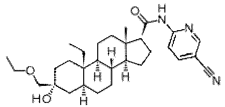 |
| 13 | 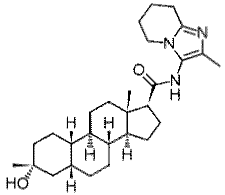 |
| 14 | 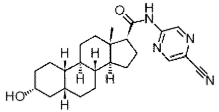 |
| 15 | 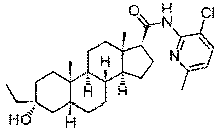 |
| 16 | 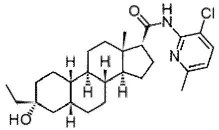 |
| 20 | 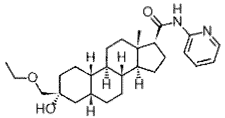 |
| 21 | 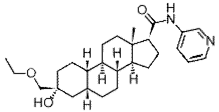 |
| 22 | 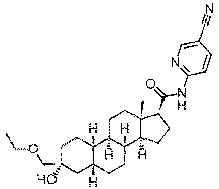 |
| 23 | 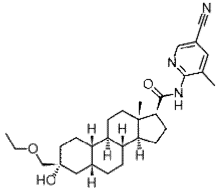 |
| 24 | 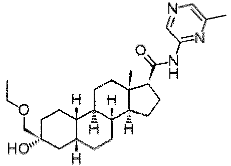 |
| 25 | 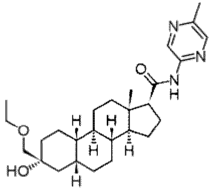 |
| 26 | 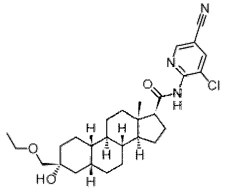 |
| 27 | 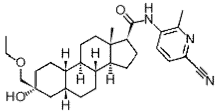 |
| 28 | 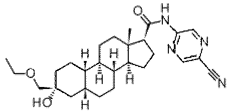 |
| 29 | 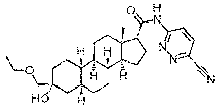 |
| 30 | 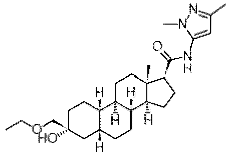 |
| 31 | 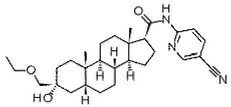 |
| 32 | 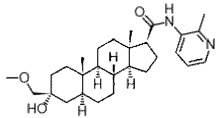 |
| 33 | 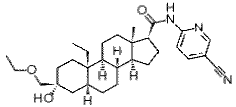 |
| 35 | 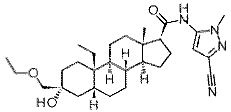 |
| 36 | 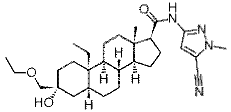 |
| 37 | 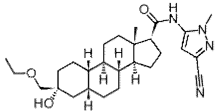 |
| 38 | 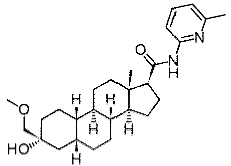 |
| 39 | 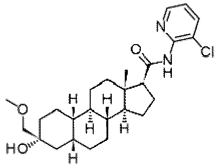 |
| 40 | 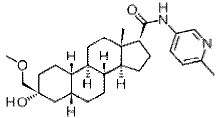 |
| 41 | 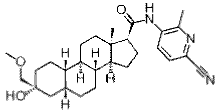 |
| 42 | 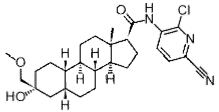 |
| 43 | 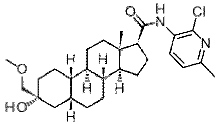 |
| 44 | 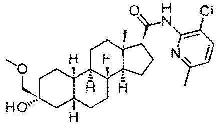 |
| 45 | 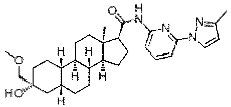 |
| 46 | 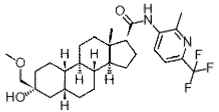 |
| 47 | 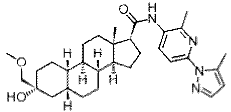 |
| 48 | 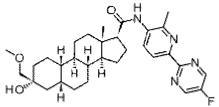 |
| 49 | 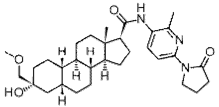 |
| 50 | 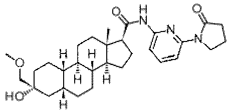 |
| 51 | 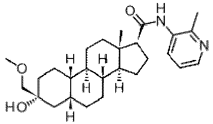 |
| 52 | 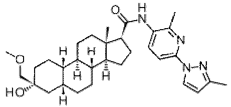 |
| 53 | 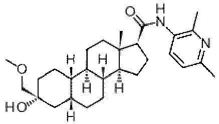 |
| 54 | 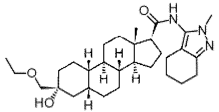 |
| 55 | 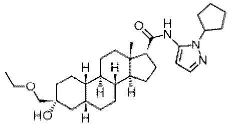 |
| 56 | 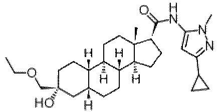 |
| 57 | 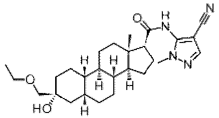 |
| 58 | 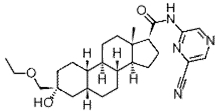 |
| 59 | 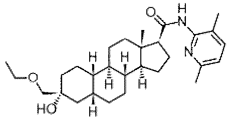 |
| 60 | 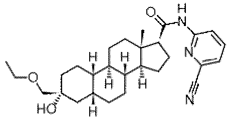 |
| 61 | 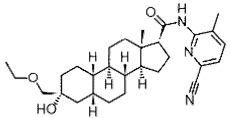 |
| 62 | 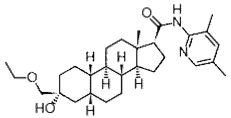 |
| 63 | 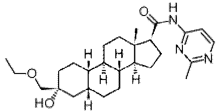 |
| 64 | 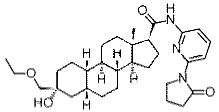 |
| 65 | 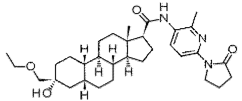 |
| 66 | 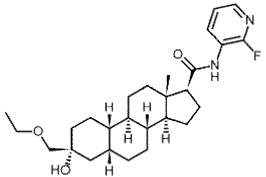 |
| 67 | 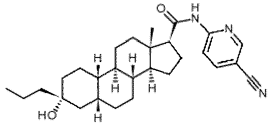 |
| 68 | 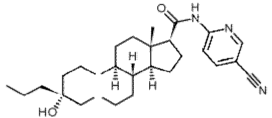 |

19. Composé selon l'une quelconque des revendications 1-18 ou sel pharmaceutiquement acceptable correspondant pour une utilisation dans un procédé de traitement d'un trouble lié au SNC chez un sujet qui en a besoin.

20. Composé, ou sel pharmaceutiquement acceptable de celui-ci, pour une utilisation selon la revendication 19,
(a) le trouble lié au SNC étant un trouble du sommeil, un trouble de l'humeur, un trouble du spectre de la schizophrénie, un trouble convulsif, un trouble de la mémoire et/ou cognitif, un trouble du mouvement, un trouble de la personnalité, un trouble du spectre de l'autisme, une douleur, une lésion cérébrale traumatique, une maladie vasculaire, un trouble de toxicomanie et/ou un syndrome de sevrage, un acouphène ou un état épileptique ;
(b) le trouble lié au SNC étant la dépression ;
(c) le trouble lié au SNC étant la dépression post-partum ;
(d) le trouble lié au SNC étant un trouble dépressif majeur ;
(e) le trouble lié au SNC étant un trouble dépressif majeur et le trouble dépressif majeur étant un trouble dépressif majeur modéré ; ou
(f) le trouble lié au SNC étant un trouble dépressif majeur et le trouble dépressif majeur étant un trouble dépressif majeur grave.
